# EUROPEAN PATENT APPLICATION

(11) **EP 4 702 991 A1**
(43) Date of publication of application: **04.03.2026**
(21) Application number: 24796215.2
(22) Date of filing: 26.04.2024
(51) Int. Cl.: A61K 47/68, A61K 47/60, A61K 31/454, A61K 31/4035, A61K 31/4439, C07K 16/28, A61P 35/00, A61P 35/02, A61P 35/04

(54) **ANTIBODY-DRUG CONJUGATE**

(30) Priority: 27.04.2023 CN 202310468007
(71) Applicant: CSPC Megalith Biopharmaceutical Co., Ltd., Shijiazhuang, Hebei 050025 (CN)
(72) Inventor: WU, Shuai, Shijiazhuang, Hebei 050025 (CN); BAO, Bin, Shijiazhuang, Hebei 050025 (CN); HONG, Dingjun, Shijiazhuang, Hebei 050025 (CN); ZHANG, Xiaodan, Shijiazhuang, Hebei 050025 (CN); XU, Hanqian, Shijiazhuang, Hebei 050025 (CN); DAN, Mo, Shijiazhuang, Hebei 050025 (CN); YAO, Bing, Shijiazhuang, Hebei 050025 (CN); CHENG, Qianyi, Shijiazhuang, Hebei 050025 (CN)
(74) Representative: Gille Hrabal Partnerschaftsgesellschaft mbB Patentanwälte
(86) International application number: PCT/CN2024/089969
(87) International publication number: WO 2024/222841

(57) **Abstract**

An antibody-drug conjugate of a domide compound, and the use thereof and a preparation method therefor. By means of introducing a hydrophilic linker to be linked with the above-mentioned domide compound, an antibody-drug conjugate having a good stability, hydrophilicity and good pharmacokinetic properties is obtained.

## Description

### CROSS-REFERENCE TO RELATED APPLICATIONS

The present application claims priority to the prior application with the patent application No. 2023104680075 and entitled "ANTIBODY-DRUG CONJUGATE" filed with the China National Intellectual Property Administration on April 27, 2023, which is incorporated herein by reference in its entirety.

### TECHNICAL FIELD

The present disclosure relates to the field of biotechnology and pharmaceuticals, and particularly to an antibody-drug conjugate, a preparation method therefor, and use thereof.

### BACKGROUND

Antibody-drug conjugates (ADCs) are currently one of the hottest spots in the research and development of anti-tumor drugs. To date, there are 14 types of antibody-drug conjugates on the market. They are: Mylotarg approved in 2000 for the treatment of CD33-positive acute myeloid leukemia (AML); Adcetris approved in 2011 for Hodgkin lymphoma; Kadcyla approved in 2013 for Her2-positive breast cancer; Besponsa approved in 2017 for B-cell lymphocytic leukemia; Lumoxiti approved in 2018 for recurrent or refractory hairy cell leukemia; Polivy approved in 2019 for recurrent or refractory diffuse large B-cell lymphoma; Padcev approved in 2019 for urothelial carcinoma; Enhertu approved in 2019 for Her2-positive breast cancer; Trodelvy approved in 2020 for triple negative breast cancer; Akalux approved in 2020 as a photo immunotherapy drug for head and neck cancer; Zynlonta approved in 2021 for large B-cell lymphoma; Disitamab Vedotin approved in 2021 for Her2-positive gastric cancer; Tivdak approved in 2022 for cervical cancer; and Elahere approved in 2022 for ovarian cancer. It is worth mentioning that Mylotarg was withdrawn from the market in 2010 due to its excessive toxicity, and then re-launched in 2017 after adjusting the clinical dosage. Additionally, Blenrep was granted accelerated approval in 2020 for multiple myeloma, but was later withdrawn from the market due to its unsatisfactory subsequent clinical results.

An antibody-drug conjugate comprises three parts: an antibody, a linker, and a bioactive molecule (drug), wherein the antibody is closely related to a disease target, while the linker and the drug determine the efficacy and safety of the ADC, thus being the most critical parts. Among the marketed ADC drugs, the majority employ the technology developed several decades ago by Seattle Genetics, in which a microtubule inhibitor is used as an active molecule, combined with a cathepsin-cleavable linker, and then conjugated to antibody cysteine via maleimide. ADCs obtained using this technology often exhibit relatively high toxicity, strong hydrophobicity, and poor pharmacokinetic properties. The ADCs obtained by Daiichi Sankyo using a hydrophilic tetrapeptide linker have achieved great improvements, but still show considerable differences in properties compared to naked antibodies. Trodelvy adopts a linker containing polyethylene glycol and salt-forming lysine; although the resulting ADC has relatively good hydrophilicity, it has relatively poor stability. It has been reported in the literature that a linker containing polysarcosine can greatly improve the hydrophilicity of an ADC, but the linker is only suitable for an active molecule containing a secondary amine structure, such as MMAE. For an active molecule with a primary amine structure like exatecan, the carbonamide bond formed by linking the linker has relatively poor stability.

Domide compounds have been used as clinical therapeutic drugs in the fields of immunomodulation and anti-tumor therapy for many years. For example, thalidomide has been used for a long time in leprosy and a variety of skin diseases, such as discoid lupus erythematosus, subacute cutaneous lupus erythematosus, and Behcet's syndrome. Pomalidomide and lenalidomide are commonly used in the treatment of multiple myeloma. In recent years, studies have shown that the mechanism of action of such domide molecules is similar to that of "molecular glue", which achieves therapeutic effects by degrading the corresponding target proteins. The marketed domide small molecules mainly have problems such as high toxicity, undesirable efficacy, etc. For example, thalidomide is the culprit behind the infamous "thalidomide tragedy". An increasing number of studies are focusing on structural modifications of such domide compounds. Orum Therapeutics has developed Smol006 through such modifications, and obtained the antibody-drug conjugates ORM-5029 and ORM-6151 by integrating antibody-conjugated delivery technology. The antibody-drug conjugates show excellent anti-tumor activity and good safety. However, during research, it was found that the activity of Smol006 is only at a moderate level. For tumors with relatively low receptor expression, Smol006 is far from sufficient as an active moiety of antibody-drug conjugates. The inventors have disclosed a domide molecular glue derivative with a more excellent anti-tumor effect and higher safety in the patent application PCT/CN2024/076883. Therefore, there is an urgent need to develop antibody-drug conjugates based on domide compounds with better activity.

### SUMMARY

In order to solve the problems in the prior art, the present disclosure provides an antibody-drug conjugate having a structure of formula (I), or a tautomer, a mesomer, a racemate, an enantiomer, a diastereoisomer, a pharmaceutically acceptable salt, a hydrate, a solvate or an isotopically labeled compound thereof:

A-(L-D)ₘ (I)

wherein A is a targeting ligand selected from an antibody (e.g., a monoclonal antibody) or an antigen-binding fragment, a small-molecule ligand, and a polypeptide;
L is a linker moiety, with one end linked to the ligand A and the other end linked to a bioactive molecule D;
D is an amino-containing bioactive molecule or a tautomer, a mesomer, a racemate, an enantiomer, a diastereoisomer or an isotopically labeled compound thereof, covalently linked to the linker moiety L via the amino group in its molecular structure;
m represents the molar ratio of the cytotoxic drug molecule to the antibody A (also called DAR, i.e., drug-to-antibody ratio).
m is an integer or a decimal from 1 to 12 (e.g., 1, 2, 3, 4, 5, 6, 7, 8, 9, 10, 11, or 12).

When m is a decimal, it refers to the average number of linker-drug molecules (L-D) conjugated per antibody unit (A).

In some embodiments, A is selected from an antibody or an antigen-binding fragment thereof targeting HER2 (ErbB2), HER3 (ErbB3), HER4 (ErbB4), EGFR, DLL3, TROP2, B7-H3, c-Met, CD20, CD22, CD30, CD33, CD123, CD44, CD47, CD56, CD70, CD73, CD79b, CD105, CEA, A33, Cripto, EphA2, G250, MUC1, Lewis Y, VEGFR, VEGF, PD-1, PD-L1, MET, RET, GPNMB, Integrin, PSMA, Tenascin-C, SLC44A4, or Mesothelin.

In some embodiments, A can be modified, for example by alteration, addition, or deletion of one or more amino acids.

In some embodiments, A is selected from an antibody or an antigen-binding fragment thereof targeting Her2, BCMA, CD33, or CD123.

In some embodiments, in the order of VHCDR1, VHCDR2, VHCDR3, VLCDR1, VLCDR2, and VLCDR3, a CDR combination of a heavy chain variable region VH and a light chain variable region of the antibody A is as follows:
CDR combination I:
   SEQ ID NO. 21: GFNIKDTYIH
   SEQ ID NO. 22: RIYPTNGYTRYADSVKG
   SEQ ID NO. 23: WGGDGFYAMDY
   SEQ ID NO. 24: RASQDVNTAVA
   SEQ ID NO. 25: SASFLYS
   SEQ ID NO. 26: QQHYTTPPT;
CDR combination II:
   SEQ ID NO. 27: GFTFTDYTMD
   SEQ ID NO. 28: DVNPNSGGSIYNORFKG
   SEQ ID NO. 29: NLGPSFYFDY
   SEQ ID NO. 30: KASQDVSIGVA
   SEQ ID NO. 31: SASYRYT
   SEQ ID NO. 32: OOYYIYPYT;
CDR combination III:
   SEQ ID NO. 33: DSNIH
   SEQ ID NO. 34: YIYPYNGGTDYNQKFKN
   SEQ ID NO. 35: GNPWLAY
   SEQ ID NO. 36: RASESLDNYGIRFLT
   SEQ ID NO. 37: AASNQGSG
   SEQ ID NO. 38: QQTKEVPWS;
CDR combination IV:
   SEQ ID NO. 39: DSNIH
   SEQ ID NO. 40: YIYPYNGGTDYNQKFKN
   SEQ ID NO. 41: GNPWLAY
   SEQ ID NO. 42: RASESLDNYGIRFLT
   SEQ ID NO. 43: AASNQGSG
   SEQ ID NO. 44: QQTKEVPWS;
CDR combination V:
   SEQ ID NO. 45: SYYIH
   SEQ ID NO. 46: VIYPGNDDISYNOKFOG
   SEQ ID NO. 47: EVRLRYFDV
   SEQ ID NO. 48: KSSQSVFFSSSQKNYLA
   SEQ ID NO. 49: WASTRES
   SEQ ID NO. 50: HQYLSSRT;
CDR combination VI:
   SEQ ID NO. 51: NYDIN
   SEQ ID NO. 52: WIYPGDGSTKYNEKFKA
   SEQ ID NO. 53: GYEDAMDY
   SEQ ID NO. 54: KASODINSYLS
   SEQ ID NO. 55: RANRLVD
   SEQ ID NO. 56: LQYDEFPLT;
CDR combination VII:
   SEQ ID NO. 57: DYNMH
   SEQ ID NO. 58: YIYPYNGGTGYNOKFK
   SEQ ID NO. 59: GRPAMDY
   SEQ ID NO. 60: RASESVDNYGISFMN
   SEQ ID NO. 61: AASNQGS
   SEQ ID NO. 62: QQSKEVPWT;
CDR combination VIII:
   SEQ ID NO. 63: SYYIH
   SEQ ID NO. 64: VIYPGNDDISYNOKFOG
   SEQ ID NO. 65: EVRLRYFDV
   SEQ ID NO. 66: KSSQSVFFSSSQKNYLA
   SEQ ID NO. 67: WASTRES
   SEQ ID NO. 68: HOYLSSRT;
CDR combination IX:
   SEQ ID NO. 69: SSIMH
   SEQ ID NO. 70: YIKPYNDGTKYNEKFKG
   SEQ ID NO. 71: EGGNDYYDTMDY
   SEQ ID NO. 72: RASODINSYLS
   SEQ ID NO. 73: RVNRLVD
   SEQ ID NO. 74: LQYDAFPYT; or
CDR combination X:
   SEQ ID NO. 75: NYWMH
   SEQ ID NO. 76: ATYRGHSDTYYNQKFKG
   SEQ ID NO. 77: GAIYDGYDVLDN
   SEQ ID NO. 78: SASQDISNYLN
   SEQ ID NO. 79: YTSNLHS
   SEQ ID NO. 80: QQYRKLPWT;
also preferably, A is selected from Trastuzumab (combination of heavy and light chains: SEQ ID NOs. 1-2), 007 (pertuzumab, combination of heavy and light chains: SEQ ID NOs. 3-4), 014 (Gemtuzumab, combination of heavy and light chains: SEQ ID NOs. 5-6), 018 (CD33AB, combination of heavy and light chains: SEQ ID NOs. 7-8), 019 (combination of heavy and light chains: SEQ ID NOs. 9-10), 020 (combination of heavy and light chains: SEQ ID NOs. 11-12), 021 (combination of heavy and light chains: SEQ ID NOs. 13-14), 022 (combination of heavy and light chains: SEQ ID NOs. 15-16), 023 (heavy and light chain combination: SEQ ID NOs. 17-18), 008 (combination of heavy and light chains: SEQ ID NOs. 19-20), and biosimilars thereof.

In some embodiments, the linker moiety -L- is represented by the following formula:

-L₁-L₂-L₃-L₄-,

wherein L₁ is selected from 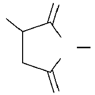 (or a ring-opened form thereof 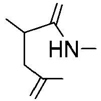 or 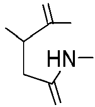 ), 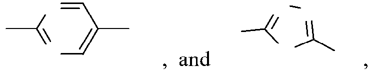 wherein * represents linkage to a sulfhydryl group of A (e.g., a monoclonal antibody), and ** represents linkage to L₂;
L₂ is a spacer, preferably selected from -L₂ₐ-C(O)-, -L₂ₐ-L_{2b}-C(O)-, -L₂ₐ-NH-C(O)-, -L₂ₐ-L_{2b}-NH-C(O)-, -L₂ₐ-L_{2b}-C(O)-NH-, -L₂ₐ-C(O)-NH-L_{2b}-C(O)-, -L₂ₐ-NH-C(O)-L_{2b}-C(O)-, -L₂ₐ-C(O)-NH-L_{2b}-C(O)-NH-, -L₂ₐ-NH-C(O)-L_{2b}-NH-C(O)-, -L₂ₐ-NR¹-SO₂-NH-C(O)-O-L_{2b}-C(O)-, and -L₂ₐ₋NR¹-SO₂-NH-C(O)-O-L_{2b}-NH-C(O)-, wherein L₂ₐ is selected from -C₁-C₈ alkylene-, -C₁-C₈ alkylene-C₃-C₈ cycloalkylene-, -C₆-C₁₄ arylene-, -C₆-C₁₄ arylene-C₁-C₈ alkylene-, -(5- to 6-membered heteroarylene)-, -(5- to 6-membered heteroarylene)-C₁-C₈ alkylene-, linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and (linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms)-(3- to 8-membered heterocyclylene), wherein the alkylene, cycloalkylene, arylene, heteroalkylene, heteroarylene, and heterocyclylene are each optionally substituted with one or more substituents independently selected from halogen, C₁-C₆ alkyl, heteroalkyl having 1-6 atoms, C₁-C₆ alkoxy, hydroxyl, amino, carboxyl, and C₃-C₈ cycloalkyl, the heteroalkylene, heterocyclylene, or heteroalkyl contains 1-12 (preferably 1-8, e.g., 1, 2, 3, 4, 5, 6, 7, 8, 9, 10, 11, or 12) heteroatoms, and the heteroatom in the heteroalkylene, heterocyclylene, heteroarylene, or heteroalkyl is selected from one or more of N, O, and S; L_{2b} is selected from -C₁-C₈ alkylene- and linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and R¹ is selected from hydrogen, C₁-C₆ alkyl, C₃-C₈ cycloalkyl, 3- to 8-membered heterocyclyl, C₁-C₆ haloalkyl, heteroalkyl having 2-8 atoms, C₆-C₁₄ aryl, and 5- to 6-membered heteroaryl, wherein the alkyl, heteroalkyl, aryl, heteroaryl, cycloalkyl, and heterocyclyl are each optionally substituted with one or more substituents independently selected from C₁-C₆ alkyl, heteroalkyl having 2-6 atoms, C₁-C₆ alkoxy, amino, and carboxyl, the heteroalkyl or heteroalkylene contains 1-12 (preferably 1-8, e.g., 1, 2, 3, 4, 5, 6, 7, 8, 9, 10, 11, or 12) heteroatoms, and the heteroatom in the heteroalkyl, heterocyclyl, heteroaryl, or heteroalkylene is selected from one or more of N, O, and S;
L₃ is a polypeptide sequence selected from a peptide residue consisting of 2-8 (e.g., 2, 3, 4, 5, 6, 7, or 8) natural or unnatural amino acids, wherein optionally, the amino acid is further substituted with one or more substituents selected from C₁-C₆ alkyl, heteroalkyl having 2-6 atoms, C₁-C₆ alkoxy, hydroxyl, amino, carboxyl, and C₃-C₈ cycloalkyl;
L₄ is a self-immolative fragment, and
further preferably, L₄ is a hydrophilic group-modified self-immolative fragment;
the self-immolative fragment is selected from: 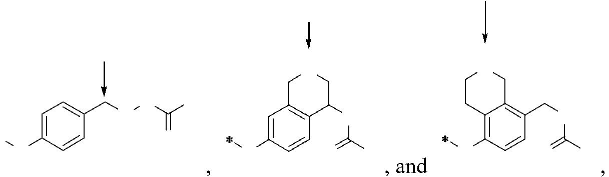
wherein * represents linkage to a carboxyl group of L₃ via an amide bond, ** represents linkage to the amino group of the bioactive molecule D, X is absent or 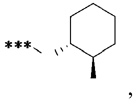 *** represents linkage to a carbon atom, **** represents linkage to an oxygen atom, and the arrow indicates a modification site of the hydrophilic group;
further preferably, the self-immolative fragment, prior to modification by the hydrophilic group, is selected from the following structures: 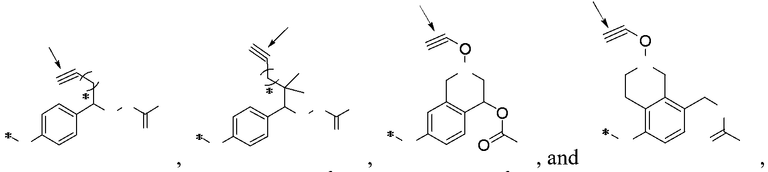
wherein Y is C₁-C₆ alkylene or -R³-C(O)-, wherein R³ is C₁-C₆ alkylene or heteroalkylene containing 1-8 - OCH₂CH₂- structural units; n is an integer from 0 to 6;
the hydrophilic group has at least one azido group and comprises a polyethylene glycol group, a poly-natural or unnatural amino acid group, a monosaccharide, an oligosaccharide, or a polysaccharide, or a combination thereof;
preferably, L₄ is selected from: 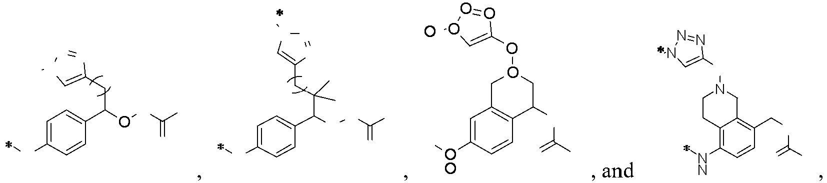
wherein * represents linkage to a carboxyl group of L₃ via an amide bond, and ** represents linkage to the amino group of the bioactive molecule D;
R² is a hydrophilic fragment, preferably selected from linear or branched heteroalkyl containing 4-50 (preferably 4-24, more preferably 6-24, and most preferably 8-24, e.g., 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) -OCH₂CH₂- structural units, a peptide chain containing 4-50 (preferably 4-24, more preferably 8-24, and most preferably 10-24, e.g., 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) proteinogenic amino acids (e.g., glycines) or non-proteinogenic amino acids (e.g., sarcosines), and linear or branched heteroalkyl containing a monosaccharide, an oligosaccharide, or a polysaccharide; X is absent or 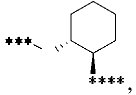 *** represents linkage to a carbon atom, and **** represents linkage to an oxygen atom; Y is C₁-C₆ alkylene or -R³-C(O)-, wherein R³ is C₁-C₆ alkylene or heteroalkylene containing 1-8 (e.g., 1, 2, 3, 4, 5, 6, 7, or 8) -OCH₂CH₂- structural units; n is an integer from 0 to 6 (e.g., 0, 1, 2, 3, 4, 5, or 6);
when A is selected from an antibody targeting CD33 or CD123, L₄ may also be: 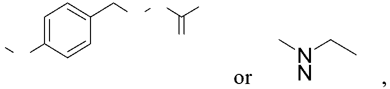
wherein * represents linkage to a carboxyl group of L₃ via an amide bond, ** represents linkage to the amino group or a hydroxyl group of the bioactive molecule D, X is absent or 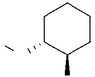 , *** represents linkage to a carbon atom, and **** represents linkage to an oxygen atom.

In some embodiments, R² is selected from the following structures: 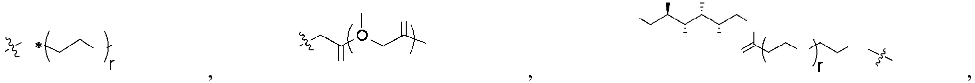 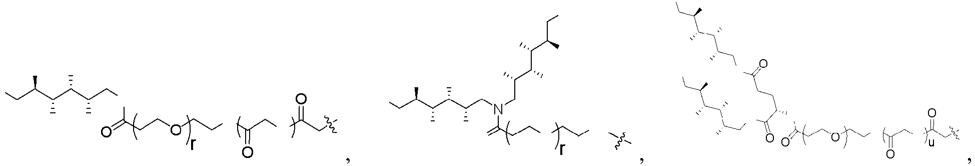 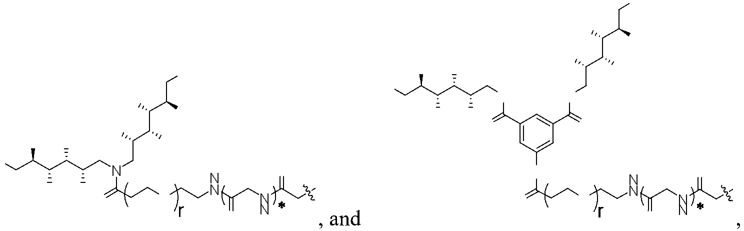 wherein r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50); Rₐ is selected from a bond and C₁-C₃ alkylene, such as methylene, ethylidene, *n-*propylidene, or isopropylidene; R_{b} is selected from C₁-C₃ alkyl, such as methyl, ethyl, n-propyl, or isopropyl. In some embodiments, R² is selected from the following structures:

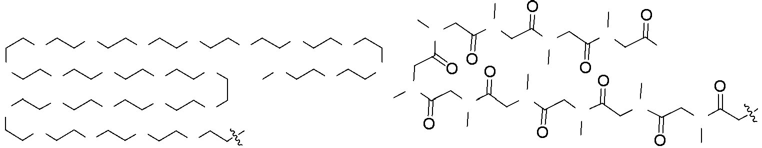

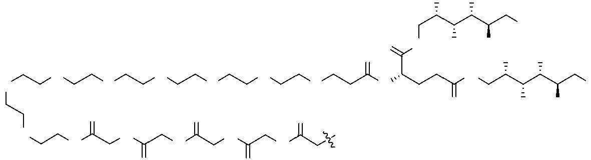

In some embodiments, Y is C₁-C₆ alkylene or -R³-C(O)-, wherein R³ is C₁-C₆ alkylene or -C₁-C₆ alkylene-(OCH₂CH₂)ₒ-, wherein o is an integer selected from 1-12 (e.g., 1, 2, 3, 4, 5, 6, 7, 8, 9, 10, 11, or 12).

In some embodiments, Y is C₁-C₃ alkylene or -R³-C(O)-, wherein R³ is C₁-C₃ alkylene or -C₁-C₃ alkylene-(OCH₂CH₂)ₒ-, wherein o is an integer selected from 1-12 (e.g., 1, 2, 3, 4, 5, 6, 7, 8, 9, 10, 11, or 12).

In some embodiments, Y is methylene, ethylidene, *n*-propylidene, isopropylidene, or -R³-C(O)-, wherein R³ is methylene-(OCH₂CH₂)ₒ-, ethylidene-(OCH₂CH₂)ₒ-, *n*-propylidene-(OCH₂CH₂)ₒ-, or isopropylidene-(OCH₂CH₂)ₒ-, wherein o is an integer selected from 1-12 (e.g., 1, 2, 3, 4, 5, 6, 7, 8, 9, 10, 11, or 12).

In some embodiments, Y is selected from -CH₂-(OCH₂CH₂)₄C(O)-.

In some embodiments, L₂ is selected from -L₂ₐ-C(O)-, -L₂ₐ-NH-C(O)-L_{2b}-C(O)-, and -L₂ₐ-NR¹-SO₂-NH-C(O)-O-L_{2b}-C(O)-, wherein L₂ₐ is selected from -C₁-C₆ alkylene-, -C₁-C₆ alkylene-C₃-C₆ cycloalkylene-, -C₆-C₁₀ arylene-, -C₆-C₁₀ arylene-C₁-C₆ alkylene-, -(5- to 6-membered heteroarylene)-, -(5- to 6-membered heteroarylene)-C₁-C₆ alkylene-, linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and (linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms)-(3- to 6-membered heterocyclylene), wherein the alkylene, cycloalkylene, arylene, heteroalkylene, heteroarylene, and heterocyclylene are each optionally substituted with one or more substituents independently selected from halogen, C₁-C₃ alkyl, heteroalkyl having 1-3 atoms, C₁-C₃ alkoxy, hydroxyl, amino, carboxyl, and C₃-C₆ cycloalkyl, the heteroalkylene, heterocyclylene, or heteroalkyl contains 1-12 (preferably 1-8, e.g., 1, 2, 3, 4, 5, 6, 7, 8, 9, 10, 11, or 12) heteroatoms, and the heteroatom in the heteroalkylene, heterocyclylene, heteroarylene, or heteroalkyl is selected from one or more of N, O, and S; L_{2b} is selected from - C₁-C₆ alkylene- and linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and R¹ is selected from hydrogen, C₁-C₃ alkyl, C₃-C₆ cycloalkyl, 3- to 6-membered heterocyclyl, C₁-C₃ haloalkyl, heteroalkyl having 2-6 atoms, C₆-C₁₀ aryl, and 5- to 6-membered heteroaryl, wherein the alkyl, heteroalkyl, aryl, heteroaryl, cycloalkyl, and heterocyclyl are each optionally substituted with one or more substituents independently selected from C₁-C₃ alkyl, heteroalkyl having 2-3 atoms, C₁-C₃ alkoxy, amino, and carboxyl, the heteroalkyl or heteroalkylene contains 1-12 (preferably 1-8, e.g., 1, 2, 3, 4, 5, 6, 7, 8, 9, 10, 11, or 12) heteroatoms, and the heteroatom in the heteroalkyl, heterocyclyl, heteroaryl, or heteroalkylene is selected from one or more of N, O, and S.

In some embodiments, L₂ is selected from -L₂ₐ-C(O)-, -L₂ₐ-NH-C(O)-L_{2b}-C(O)-, and -L₂ₐ-NR¹-SO₂-NH-C(O)-O-L_{2b}-C(O)-, wherein L₂ₐ is selected from methylene, ethylidene, *n*-propylidene, *n*-butylidene, *n*-pentylidene, *n-*hexylidene, -CH(CH₃)CH₂CH₂CH₂CH₂-, -C₁-C₃ alkylene-cyclohexylidene-, -phenylene-, -phenylene-C₁-C₃ alkylene-, -(5- to 6-membered heteroarylene)-C₁-C₃ alkylene-, linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and (linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms)-(3- to 6-membered heterocyclylene), wherein the alkylene, cycloalkylene, arylene, heteroalkylene, heteroarylene, and heterocyclylene are each optionally substituted with one or more substituents independently selected from halogen, methyl, ethyl, *n*-propyl, isopropyl, heteroalkyl having 1-3 atoms, methoxy, ethoxy, *n*-propoxy, isopropoxy, hydroxyl, amino, carboxyl or cyclopropyl, cyclobutyl, cyclopentyl, and cyclohexyl, the heteroalkylene, heterocyclylene, or heteroalkyl contains 1-12 (preferably 1-8, e.g., 1, 2, 3, 4, 5, 6, 7, 8, 9, 10, 11, or 12) heteroatoms, and the heteroatom in the heteroalkylene, heterocyclylene, heteroarylene, or heteroalkyl is selected from one or more of N, O, and S; L_{2b} is selected from methylene, ethylidene, *n*-propylidene, *n*-butylidene, *n*-pentylidene, *n-*hexylidene, -CH(CH₃)CH₂CH₂CH₂CH₂-, and linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and R¹ is selected from hydrogen, methyl, ethyl, *n*-propyl, isopropyl, cyclopropyl, cyclobutyl, cyclopentyl, cyclohexyl, 3- to 6-membered heterocyclyl, -CF₃, -CF₂CF₃, heteroalkyl having 2-3 atoms, phenyl, and 5- to 6-membered heteroaryl, wherein the alkyl, heteroalkyl, phenyl, heteroaryl, cycloalkyl, and heterocyclyl are each optionally substituted with one or more substituents independently selected from methyl, ethyl, *n*-propyl, isopropyl, heteroalkyl having 2-3 atoms, methoxy, ethoxy, *n*-propoxy, isopropoxy, amino, and carboxyl, the heteroalkyl or heteroalkylene contains 1-12 (preferably 1-8, e.g., 1, 2, 3, 4, 5, 6, 7, 8, 9, 10, 11, or 12) heteroatoms, and the heteroatom in the heteroalkyl, heterocyclyl, heteroaryl, or heteroalkylene is selected from one or more of N, O, and S.

In some embodiments, L₂ₐ is selected from -C₁-C₈ alkylene-, -C₁-C₈ alkylene-C₃-C₈ cycloalkylene, C₆-C₁₄ arylene-C₁-C₈ alkylene, and linear or branched heteroalkylene having 1-50 (preferably 1-20, more preferably 1-12, and most preferably 1-8) atoms, wherein the alkylene, cycloalkylene, arylene, and heteroalkylene are each optionally substituted with one or more substituents independently selected from C₁-C₆ alkyl, heteroalkyl having 2-6 atoms, C₁-C₆ alkoxy, hydroxyl, amino, carboxyl, and C₃-C₈ cycloalkyl, the heteroalkylene or heteroalkyl contains 1-12 (preferably 1-8, e.g., 1, 2, 3, 4, 5, 6, 7, 8, 9, 10, 11, or 12) heteroatoms, and the heteroatom in the heteroalkyl, heteroalkylene, or heteroarylene is selected from one or more of N, O, and S; L_{2b} is selected from -C₁-C₈ alkylene- and linear or branched heteroalkylene having 1-50 (preferably 1-20, more preferably 1-12, and most preferably 1-8) atoms, and R¹ is selected from hydrogen, C₁-C₆ alkyl, C₂-C₈ heteroalkyl, and C₆-C₁₄ aryl, wherein the alkyl, heteroalkyl, and aryl are each optionally substituted with one or more substituents independently selected from C₁-C₆ alkyl, heteroalkyl having 2-6 atoms, C₁-C₆ alkoxy, amino, and carboxyl, the heteroalkyl contains 1-12 (preferably 1-8, e.g., 1, 2, 3, 4, 5, 6, 7, 8, 9, 10, 11, or 12) heteroatoms, and the heteroatom in the heteroalkyl or heteroalkylene is selected from one or more of N, O, and S.

In some embodiments, L₂ₐ is selected from -CH₂CH₂CH₂CH₂CH₂-, -CH₂CH₂(OCH₂CH₂)ₚ-, -(OCH₂CH₂)ₚ-, - CH₂(OCH₂CH₂)ₚ-, -CH₂CH₂(OCH₂CH₂)ₚCH₂-, -CH₂CH₂(OCH₂CH₂)ₚCH₂CH₂-, -CH₂(OCH₂CH₂)ₚCH₂CH₂-, 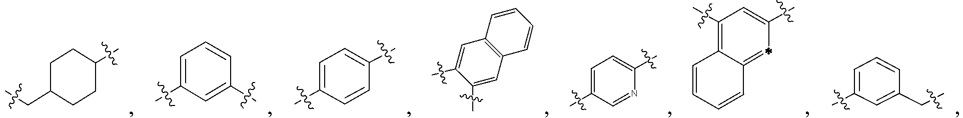 and 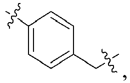 wherein p is an integer from 1 to 12 (preferably 2-8, e.g., 1, 2, 3, 4, 5, 6, 7, 8, 9, 10, 11, or 12); L_{2b} is selected from -CH₂OCH₂-, -CH₂CH₂OCH₂-, -CH₂CH₂OCH₂CH₂-, -CH₂O-, -CH₂CH₂O-, -CH₂-, -CH₂CH₂-, - CH₂CH₂CH₂-, -CH₂CH₂CH₂CH₂-, -CH₂CH₂CH₂CH₂CH₂-, -(OCH₂CH₂)_{q}-, -CH₂CH₂(OCH₂CH₂)_{q}-, - CH₂(OCH₂CH₂)_{q}-, -CH₂CH₂(OCH₂CH₂)_{q}CH₂-, -CH₂CH₂(OCH₂CH₂)_{q}CH₂CH₂-, and -CH₂(OCH₂CH₂)_{q}CH₂CH₂-, and R¹ is selected from hydrogen, methyl, ethyl, n-propyl, isopropyl, and cyclopropyl, wherein q is an integer from 1 to 12 (preferably 2-8, e.g., 1, 2, 3, 4, 5, 6, 7, 8, 9, 10, 11, or 12).

In some embodiments, L₂ is selected from the following structures:

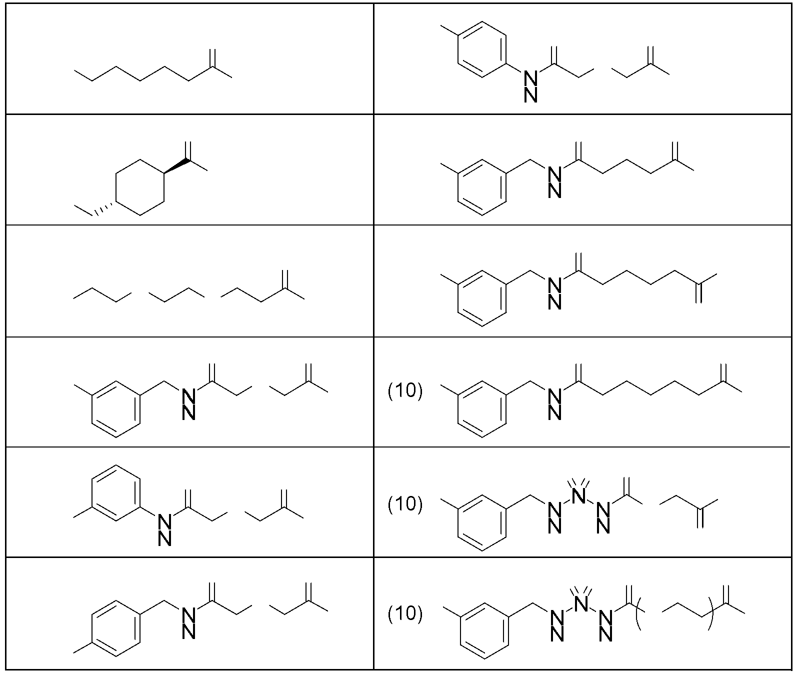

wherein w is an integer selected from 1-12 (e.g., 1, 2, 3, 4, 5, 6, 7, 8, 9, 10, 11, or 12), * represents linkage to L₁, and ** represents linkage to L₃.

In some embodiments, L₃ is selected from a peptide residue formed from 2-8 amino acids selected from phenylalanine (F), glycine (G), valine (V), citrulline (C), glutamic acid (E), alanine (A), lysine (K), C₁-C₆ alkyl-substituted lysine (e.g., C₁-C₃ alkyl-substituted lysine, such as methyl-substituted lysine, ethyl-substituted lysine, n-propyl-substituted lysine, or isopropyl-substituted lysine), etc. For example, L₃ is: -ValCit-; -CitVal-; -AlaAla-; - AlaCit-; -CitAla-; -AsnCit-; -CitAsn-; -CitCit-; -ValGlu-; -GluVal-; -SerCit-; -CitSer-; -LysCit-; -CitLys-; -AspCit-; -CitAsp-; -AlaVal-; -ValAla-; -PheAla-; -AlaPhe-; -PheLys-; -LysPhe-; -ValLys-; -LysVal-; -AlaLys-; -LysAla-; - PheCit-; -CitPhe-; -LeuCit-; -CitLeu-; -IleCit-; -CitIle-; -PheArg-; -ArgPhe-; -CitTrp-; -TrpCit-; -AlaAlaAla-; - PhePheLys-; -LysPhePhe-; -DPhePheLys-; -DLysPhePhe-; -GlyPheLys-; -LysPheGly-; -GlyPheLeuGly-; - GlyLeuPheGly-; -GluValCit-; -AlaLeuAlaLeu-; -GlyGlyGly-; -GlyGlyGlyGly-; -GlyPheValGly-; - GlyValPheGly-; -GlyGlyPheGly-; or -GlyGlyValGly-.

In some embodiments, L₃ is selected from the following structures: 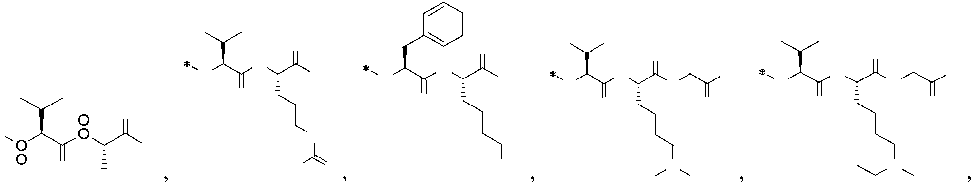 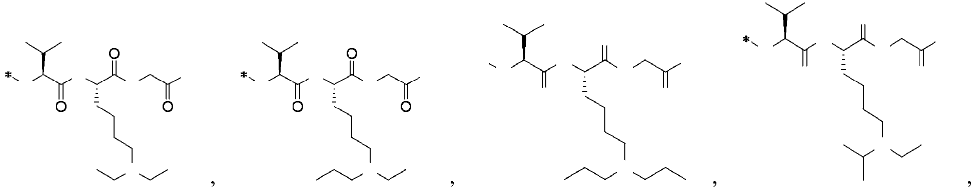 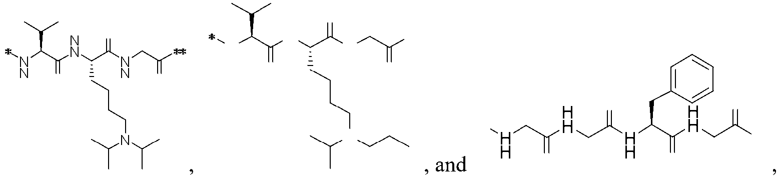 wherein * represents linkage to L₂, and ** represents linkage to L₄.

In some embodiments, L₄ is selected from: 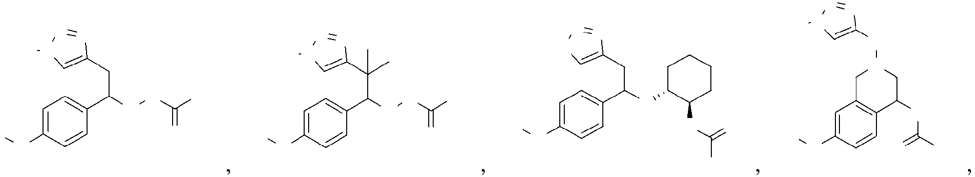 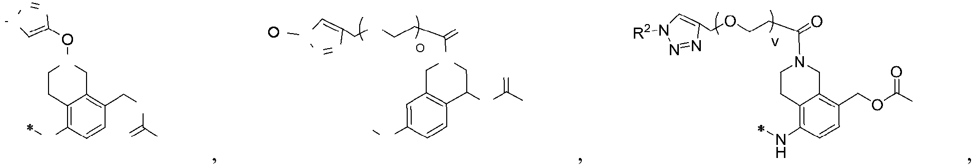 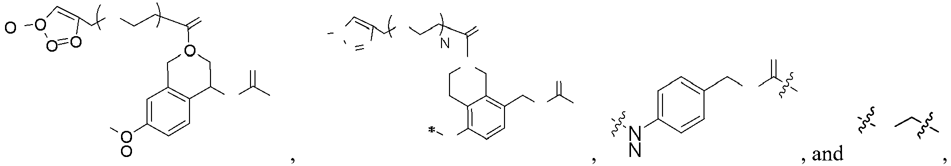 wherein v is an integer selected from 1-12 (e.g., 1, 2, 3, 4, 5, 6, 7, 8, 9, 10, 11, or 12), * represents linkage to a carboxyl group of L₃ via an amide bond, and ** represents linkage to the amino group of the bioactive molecule D. In some embodiments, L₄ is selected from: 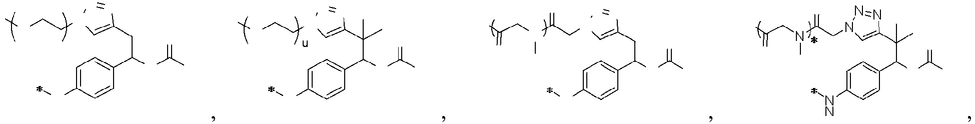 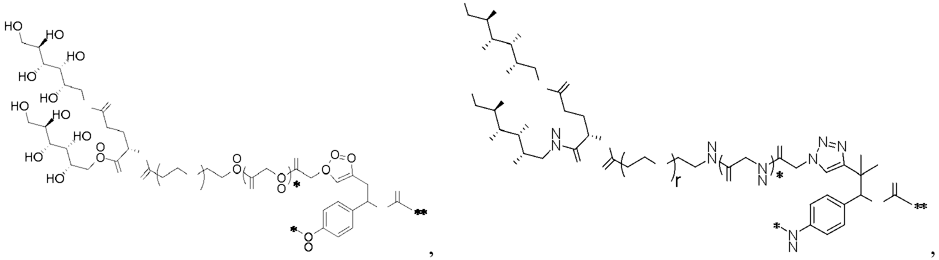 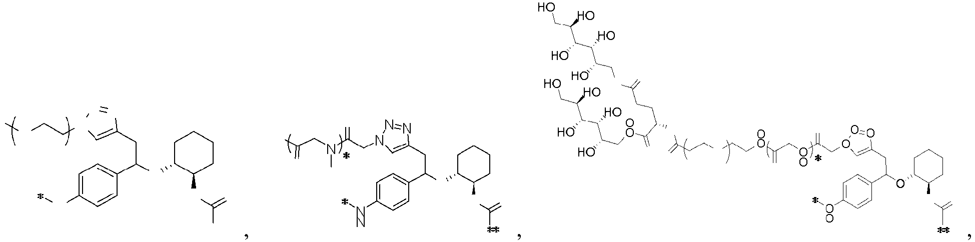 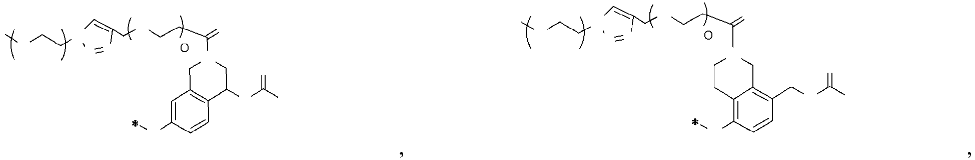 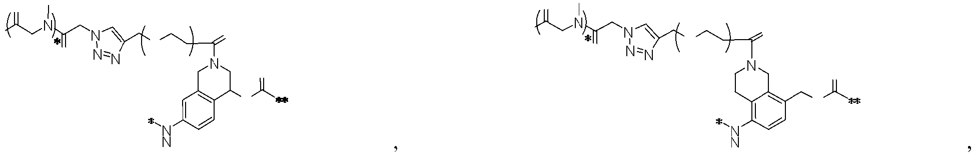 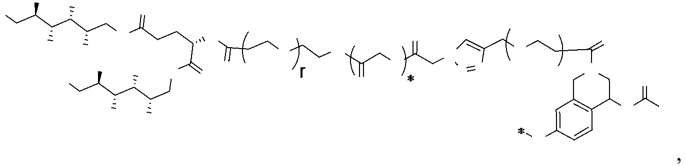 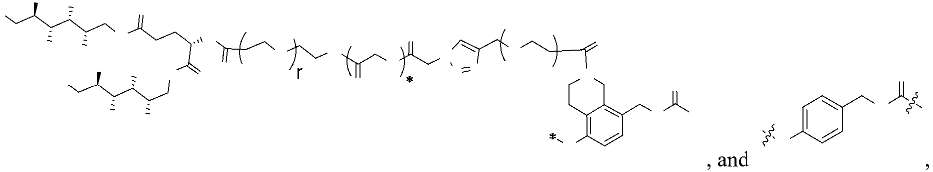 wherein r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50), v is an integer selected from 1-12 (e.g., 1, 2, 3, 4, 5, 6, 7, 8, 9, 10, 11, or 12), * represents linkage to a carboxyl group of L₃ via an amide bond, and ** represents linkage to the amino group of the bioactive molecule D.

In some embodiments, L is selected from the following structures: 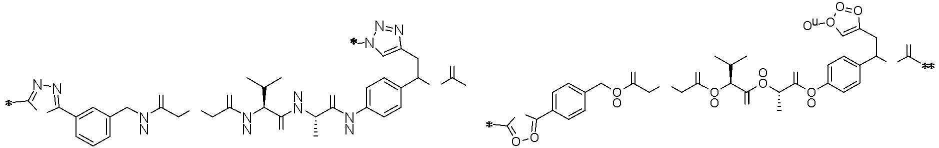 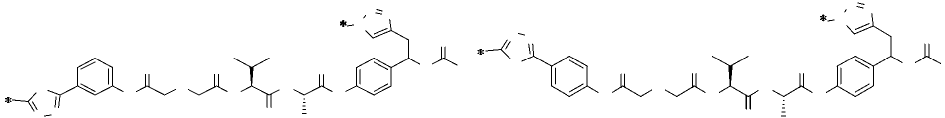 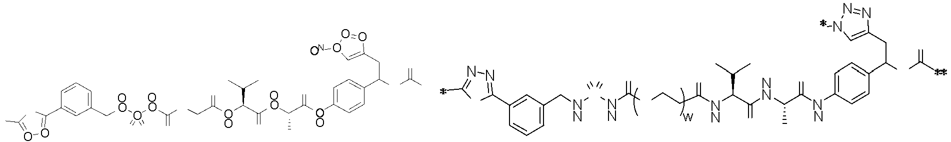 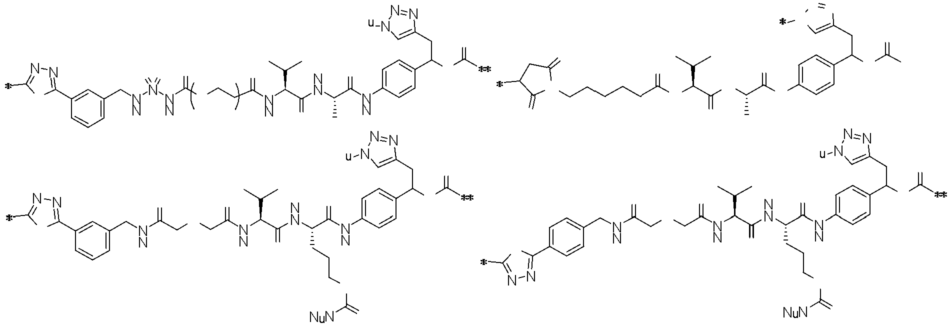 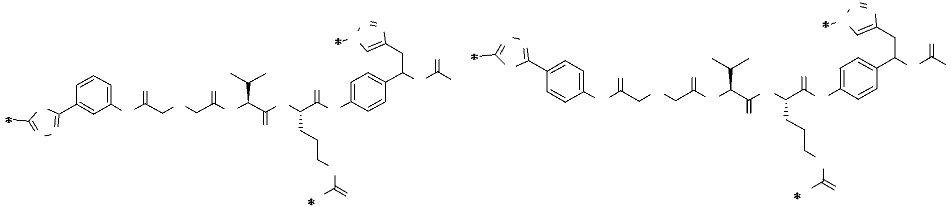 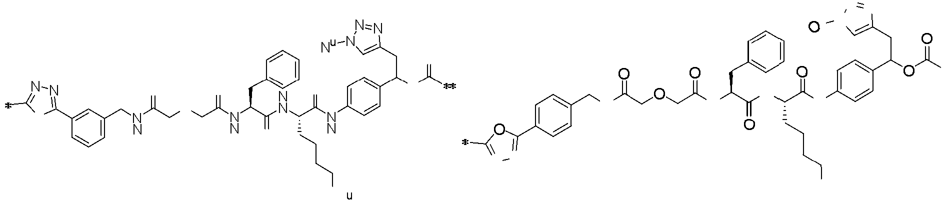 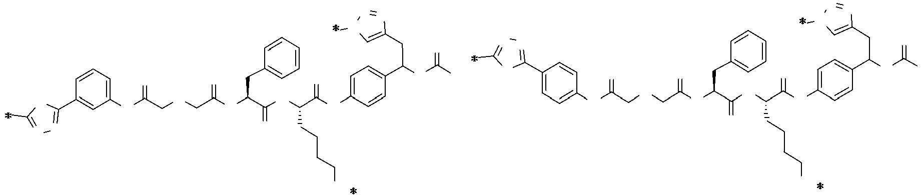 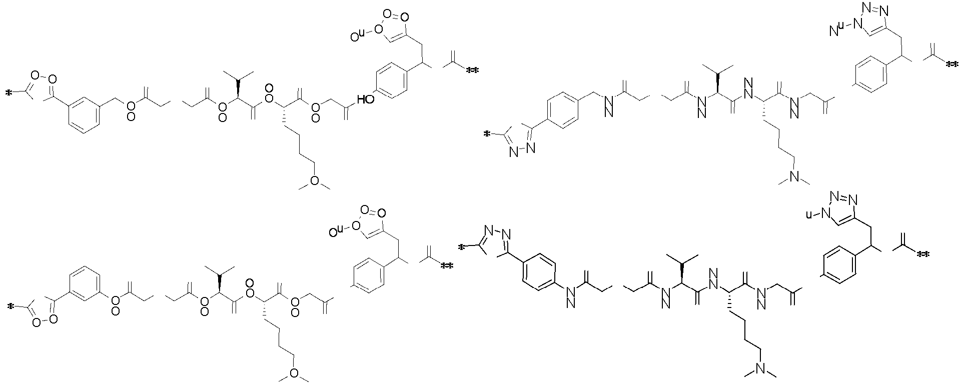 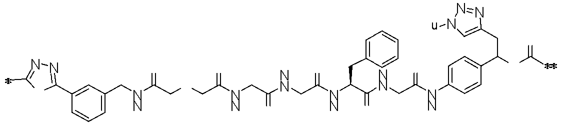 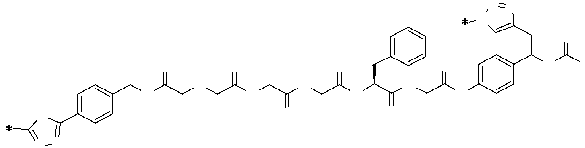 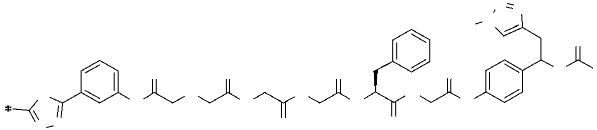 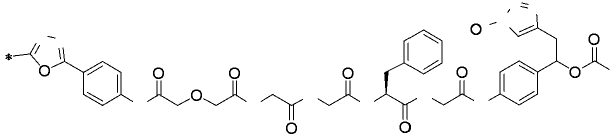 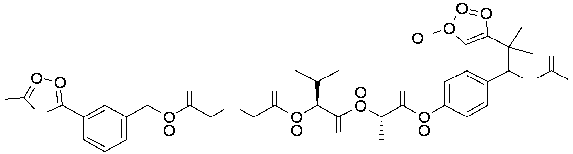 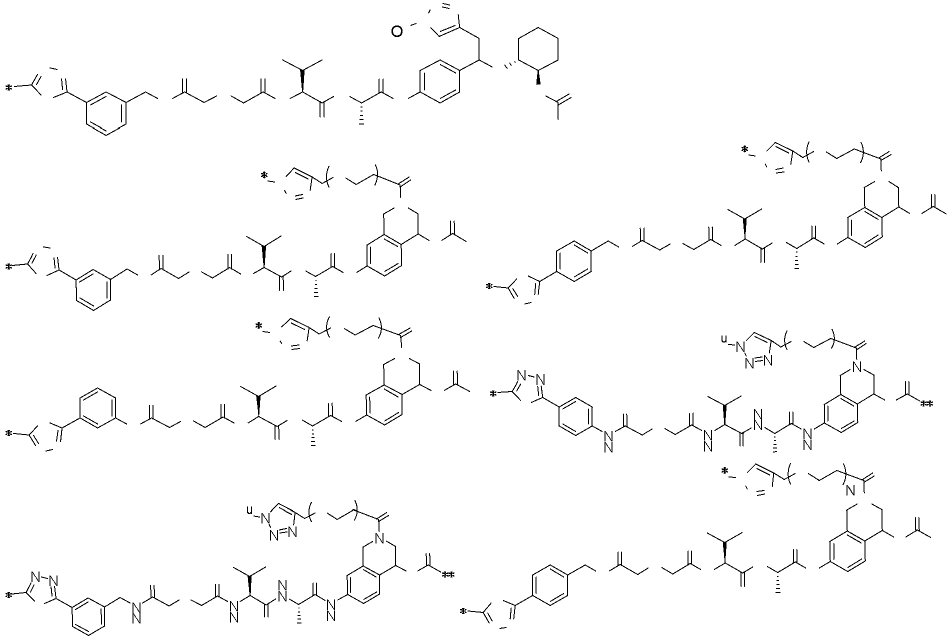 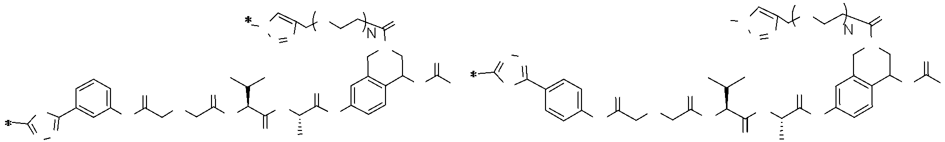 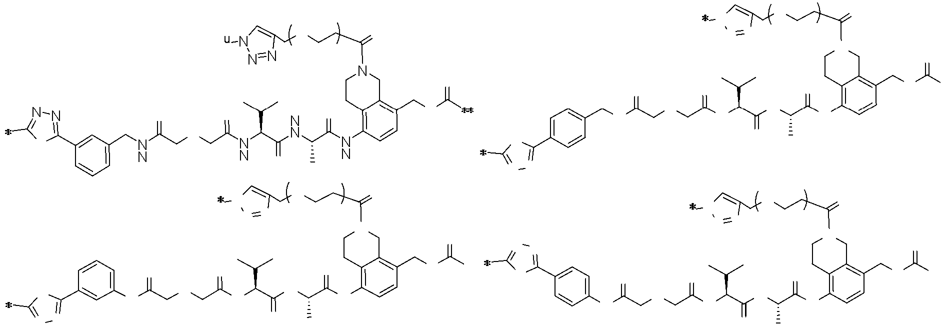 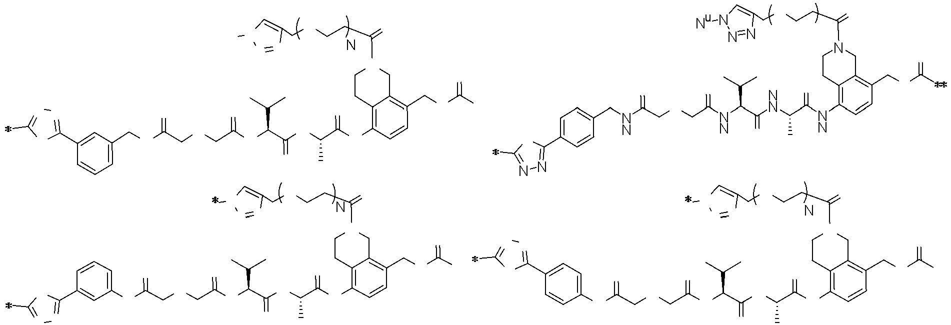 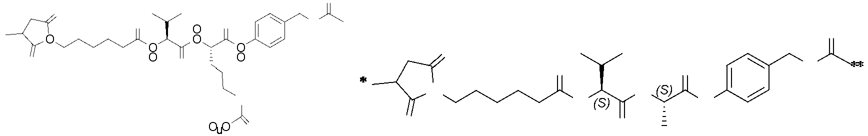 wherein v and w are each independently an integer selected from 1-12 (e.g., 1, 2, 3, 4, 5, 6, 7, 8, 9, 10, 11, or 12), * represents linkage to a sulfhydryl group of the antibody A, and ** represents linkage to the amino group of the bioactive molecule D; r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50).

In some embodiments, L is selected from the following structures: 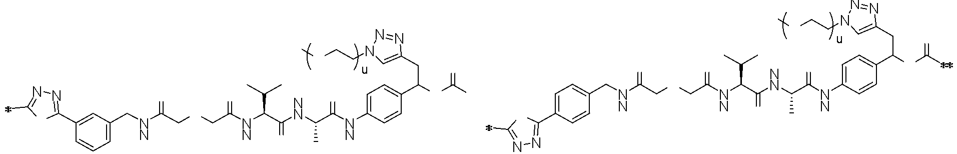 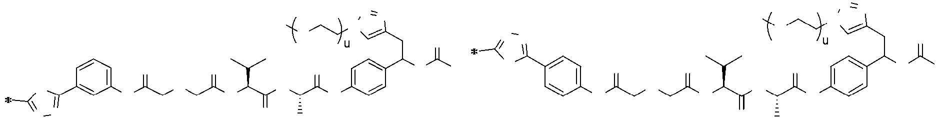 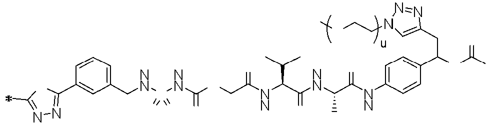 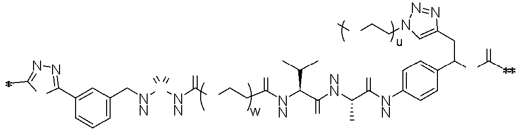 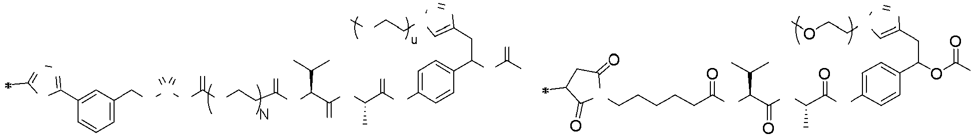 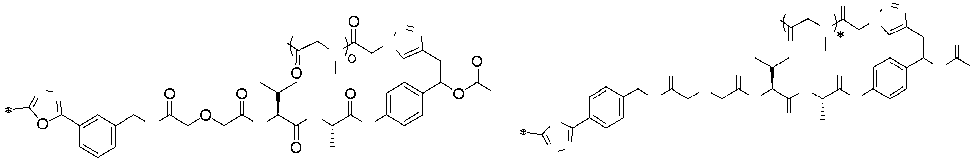 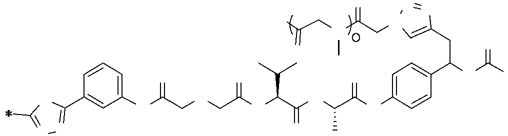 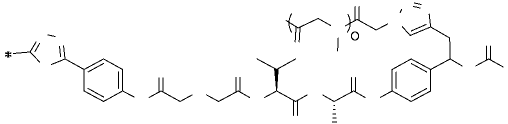 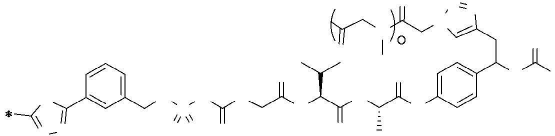 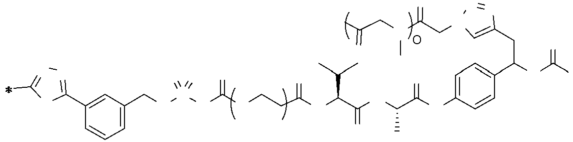 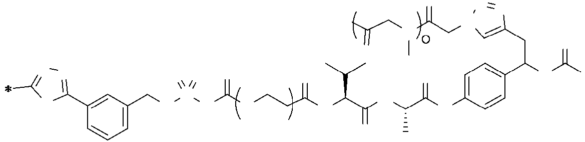 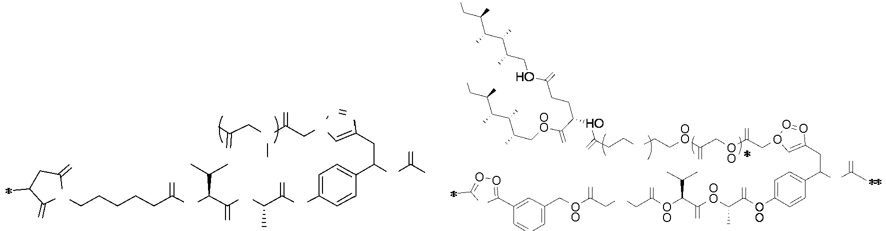 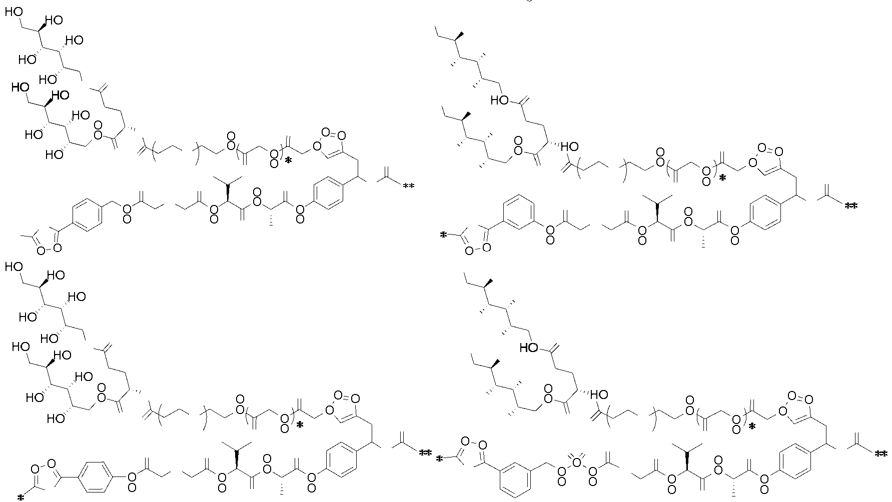 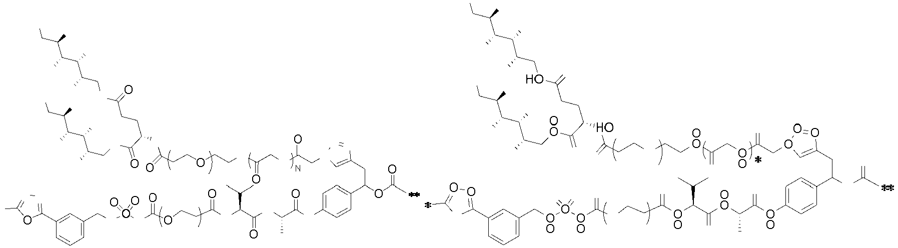 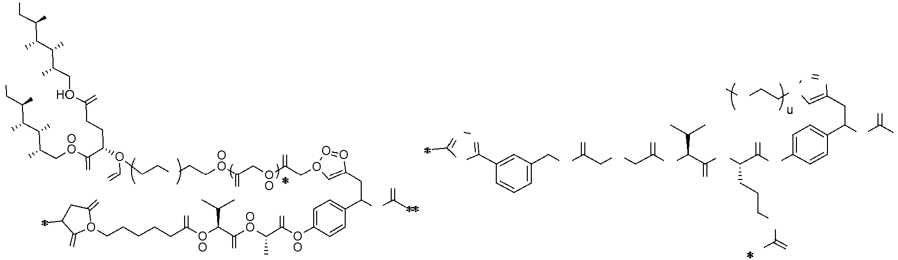 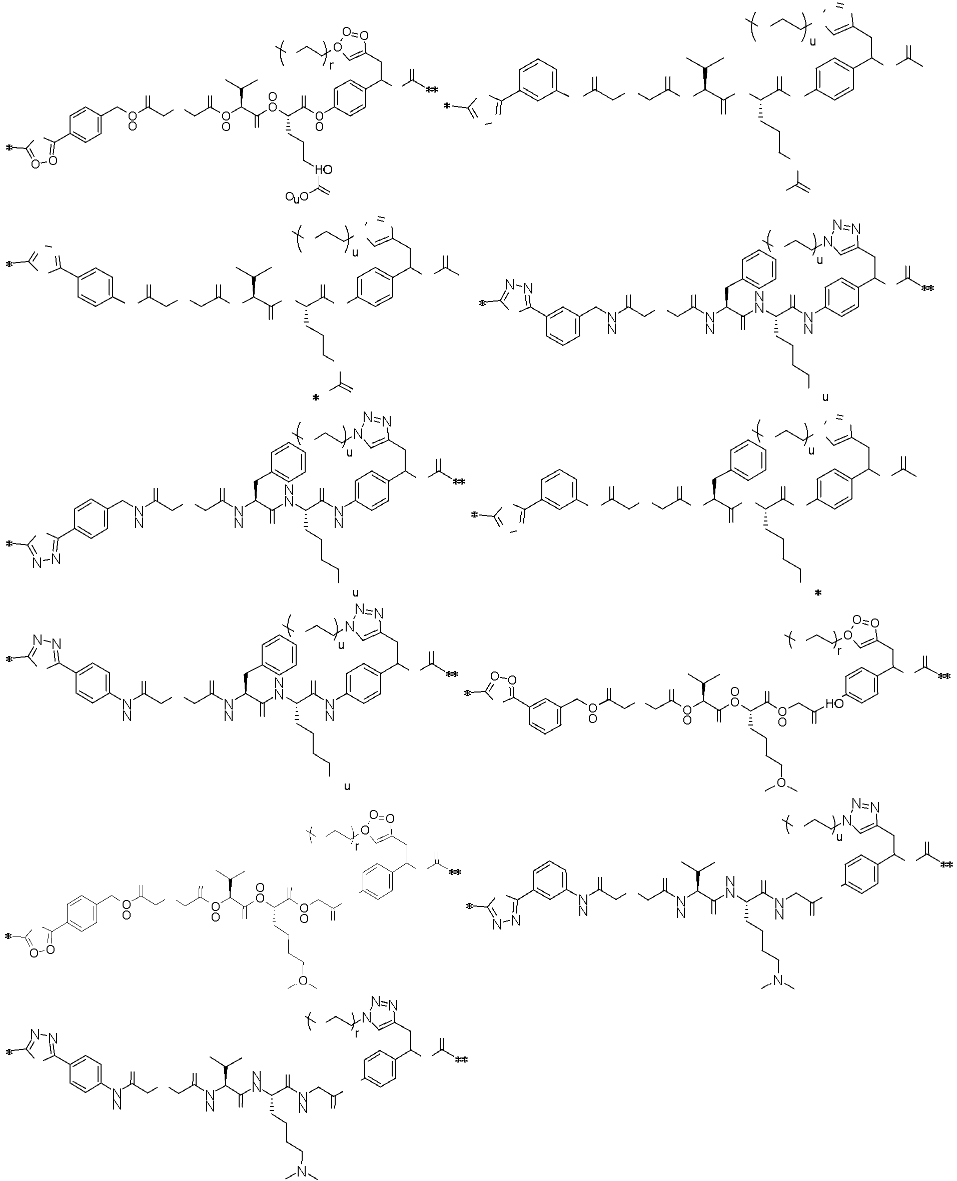 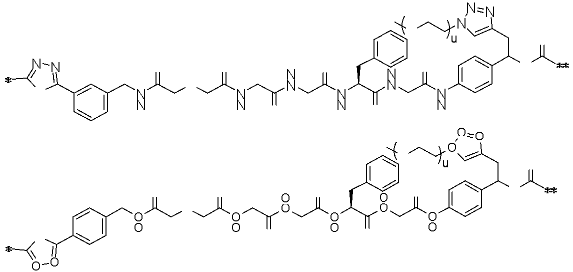 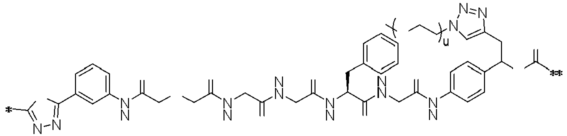 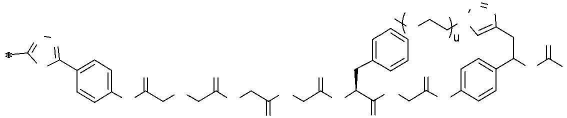 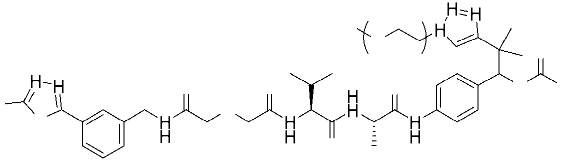 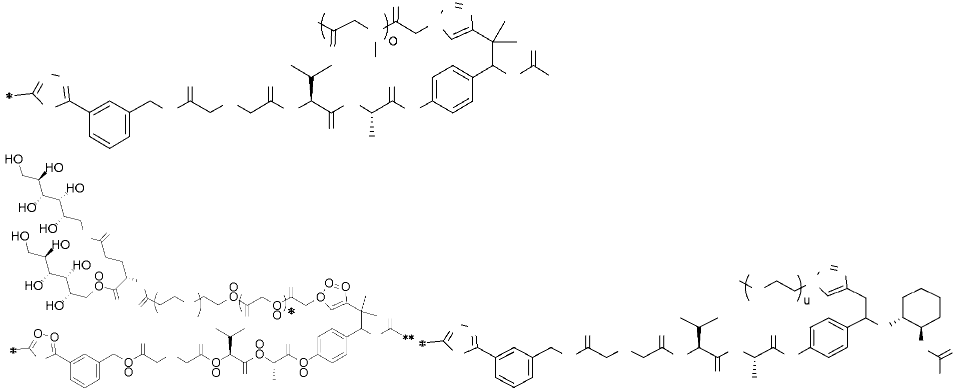 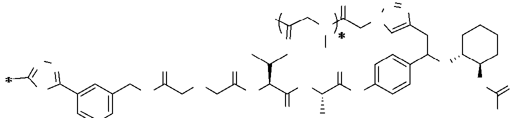 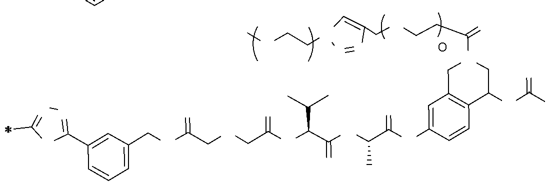 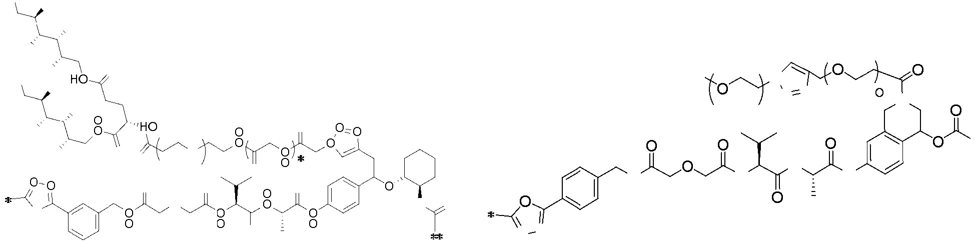 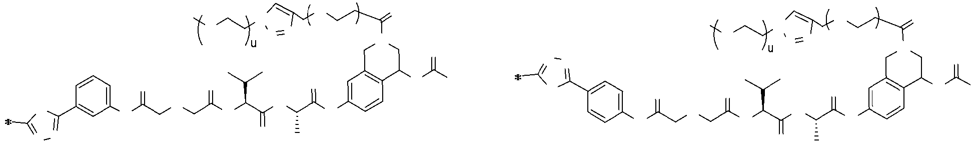 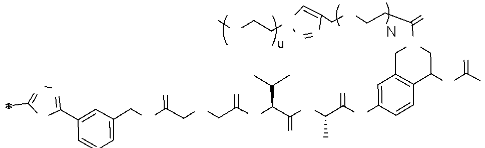 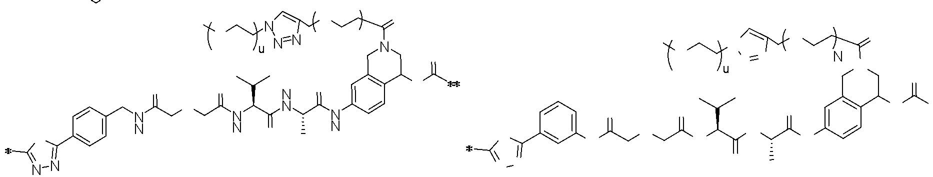 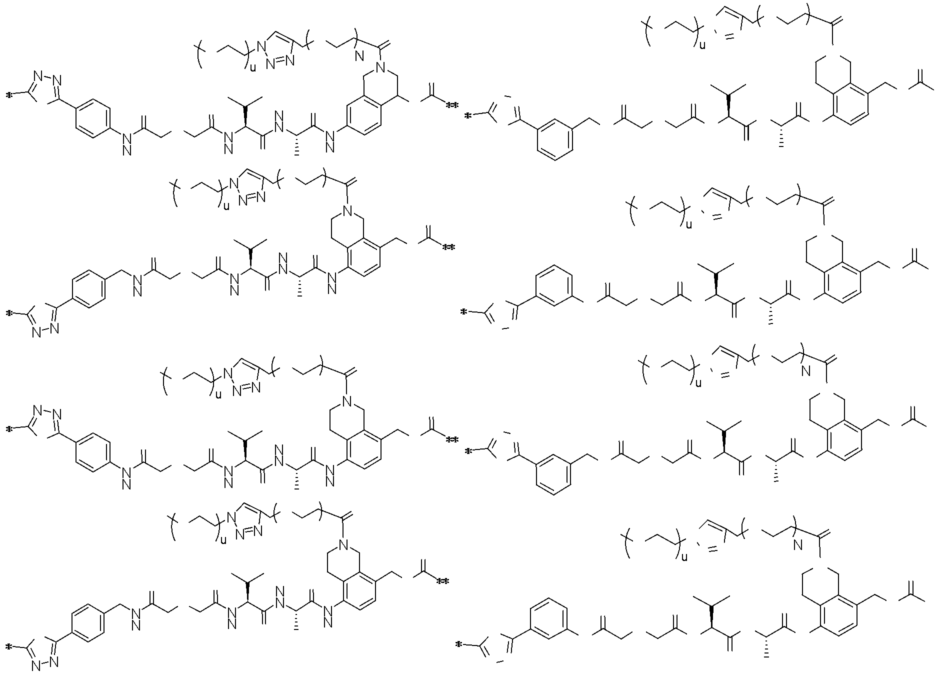 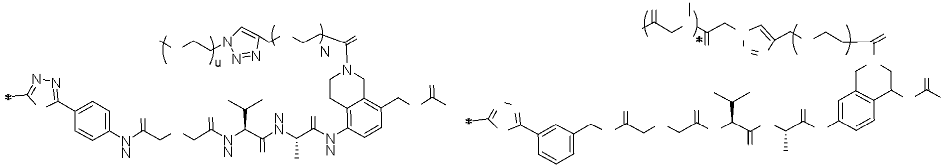 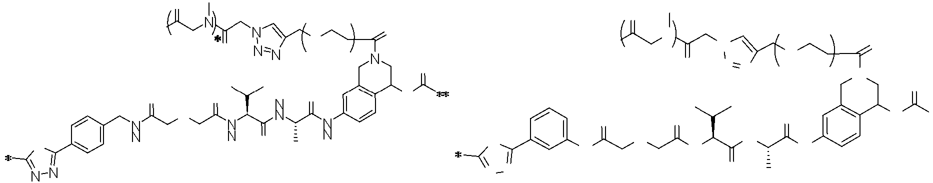 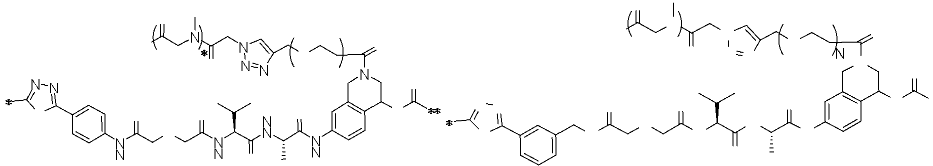 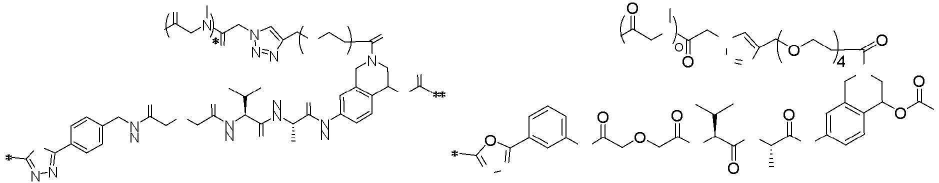 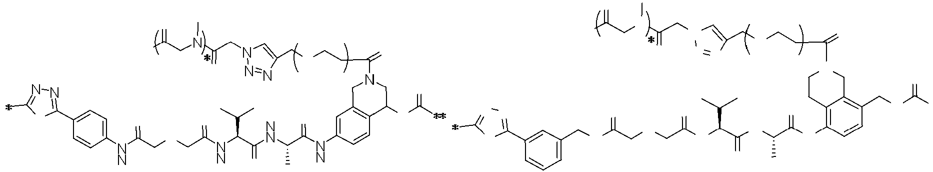 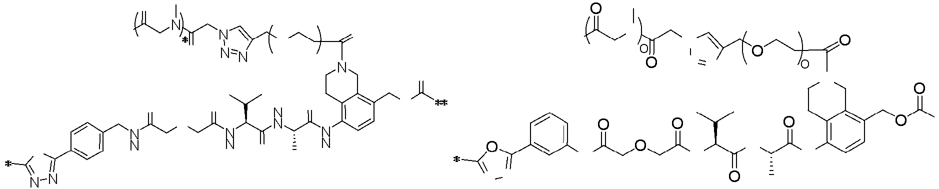 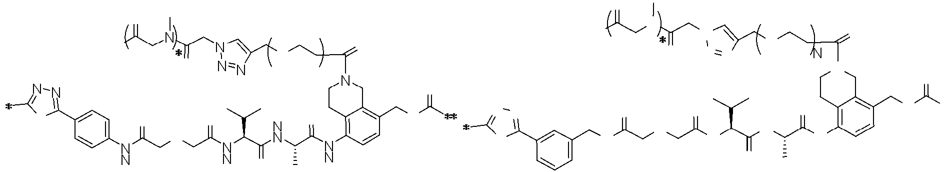 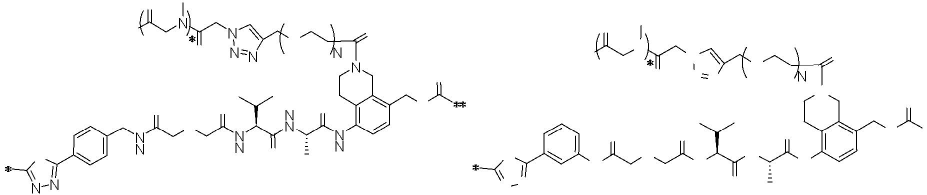 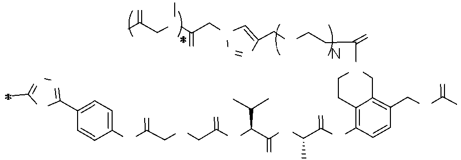 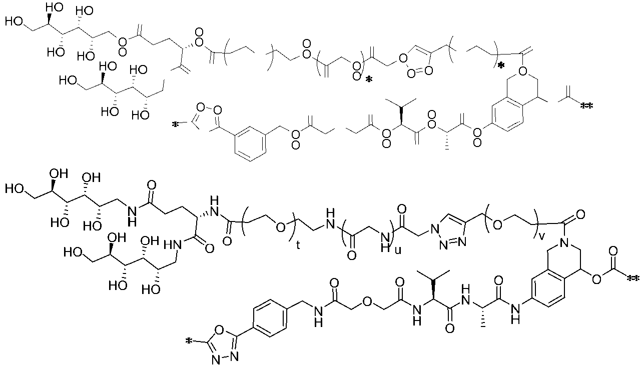 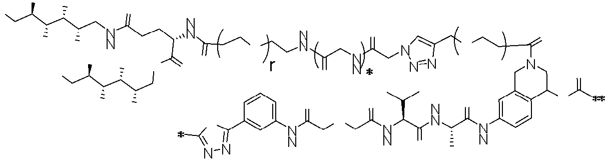 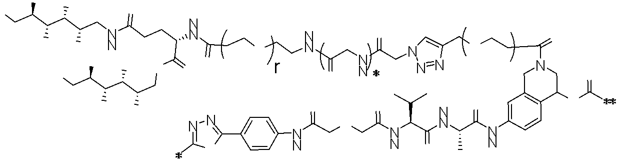 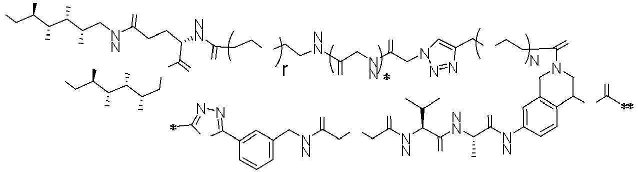 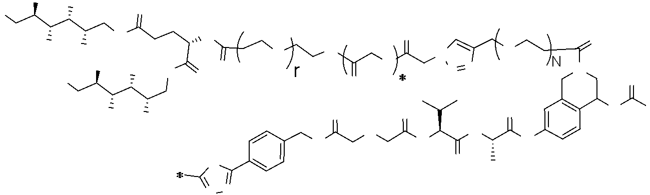 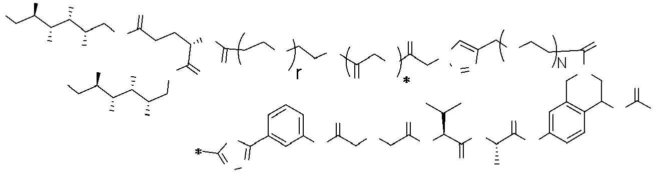 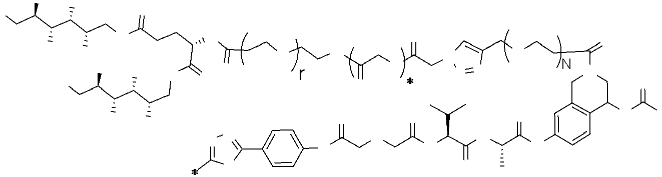 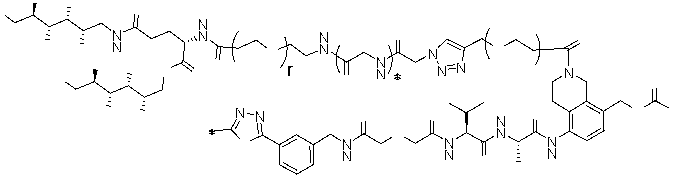 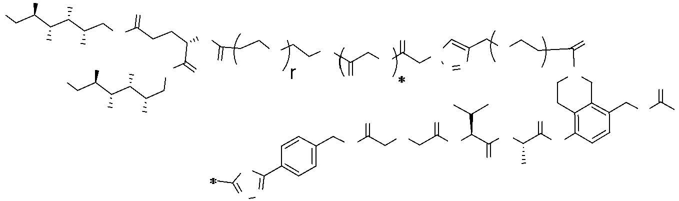 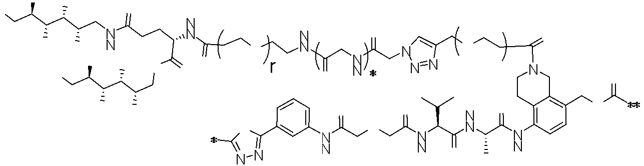 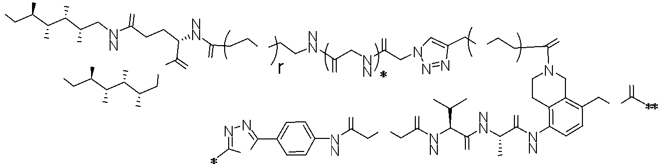 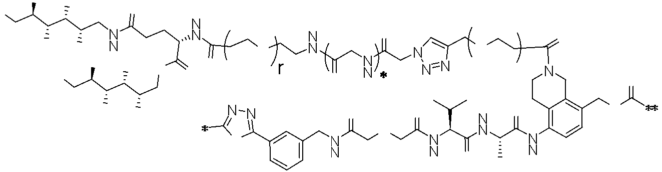 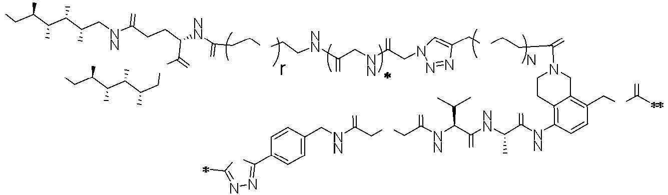 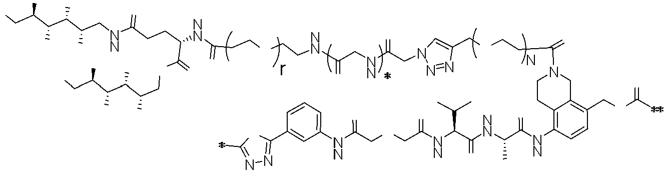 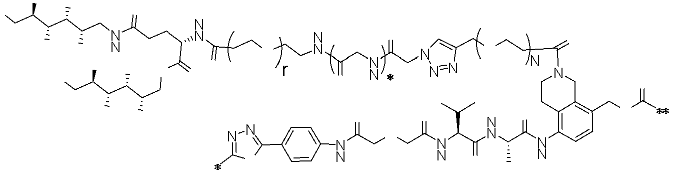 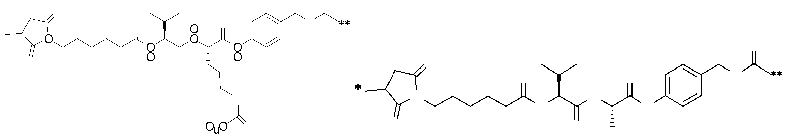 wherein v and w are each independently an integer selected from 1-12 (e.g., 1, 2, 3, 4, 5, 6, 7, 8, 9, 10, 11, or 12), * represents linkage to a sulfhydryl group of the antibody A, and ** represents linkage to the amino group of the bioactive molecule D; r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50).

In some embodiments, L is selected from the following structures: 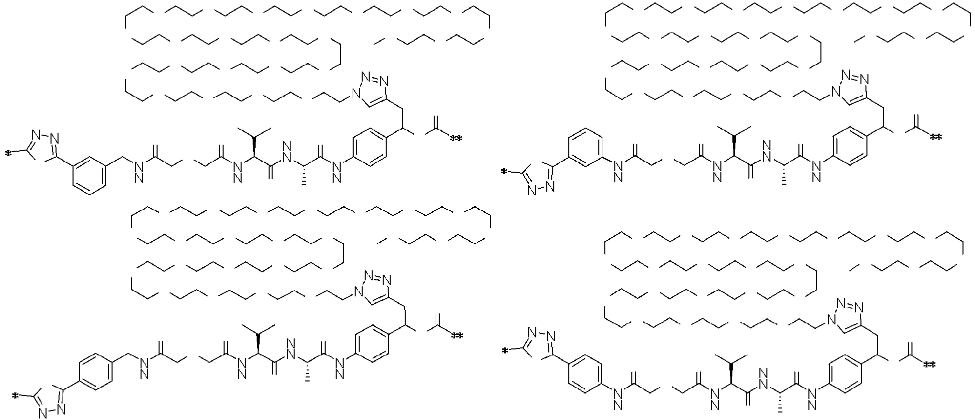 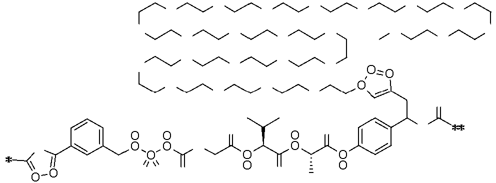 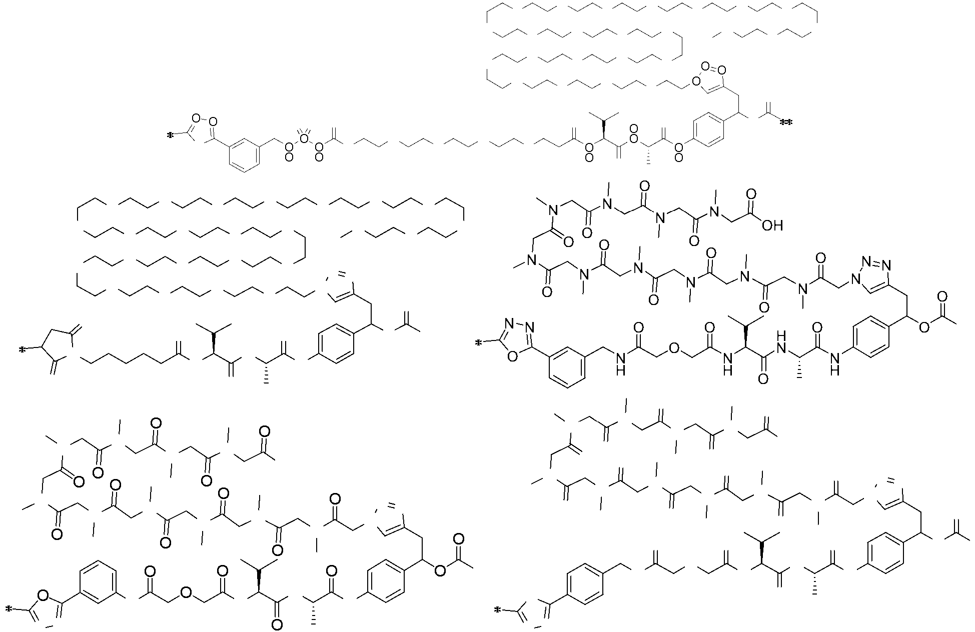 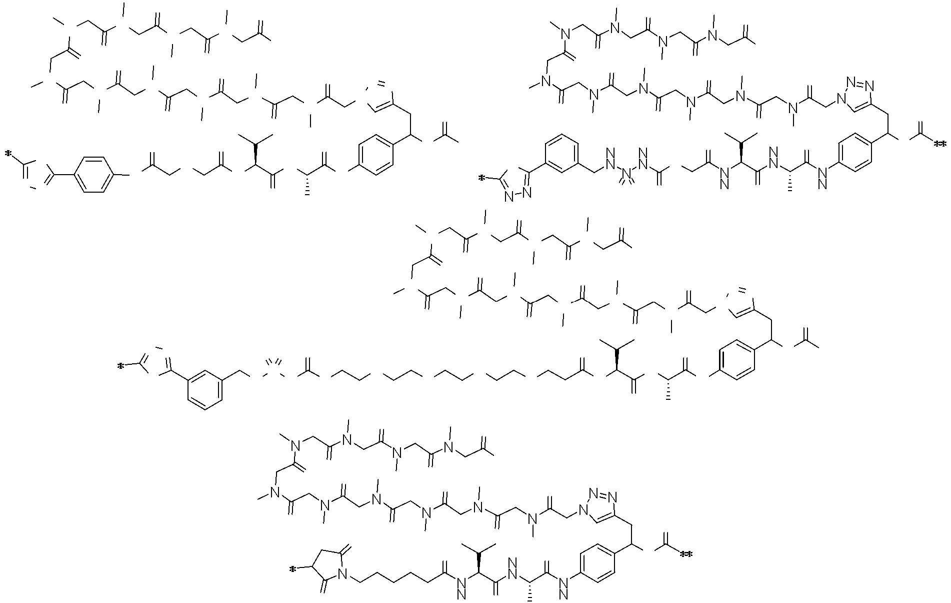 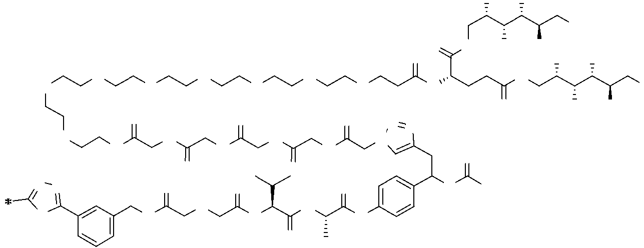 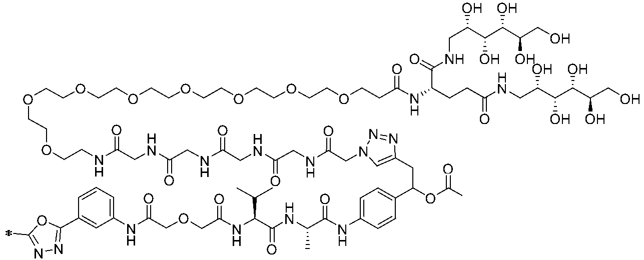 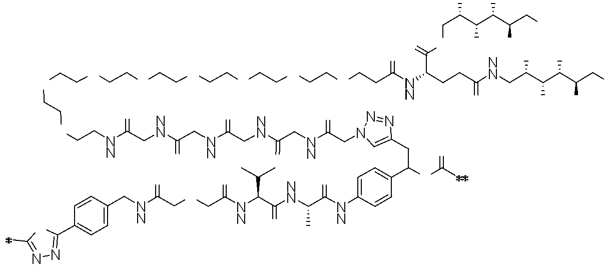 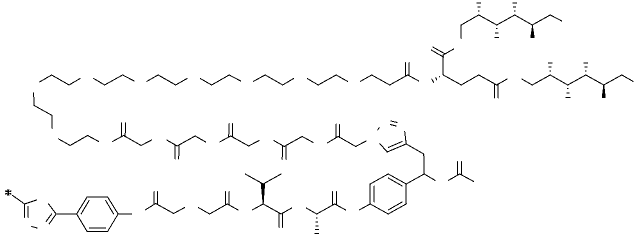 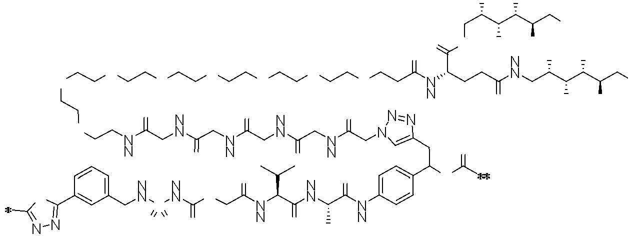 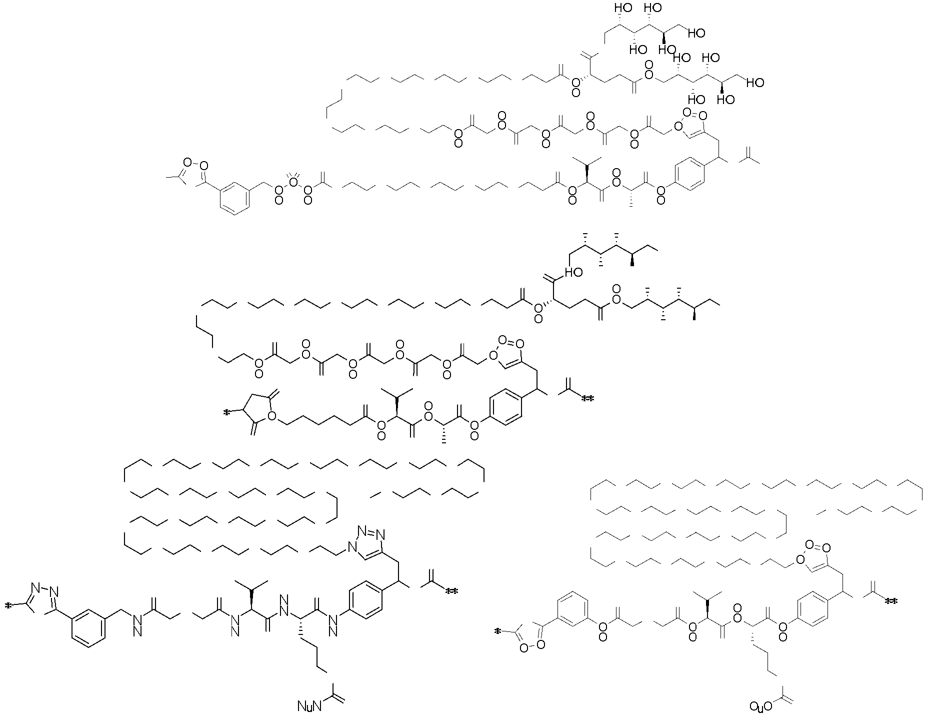 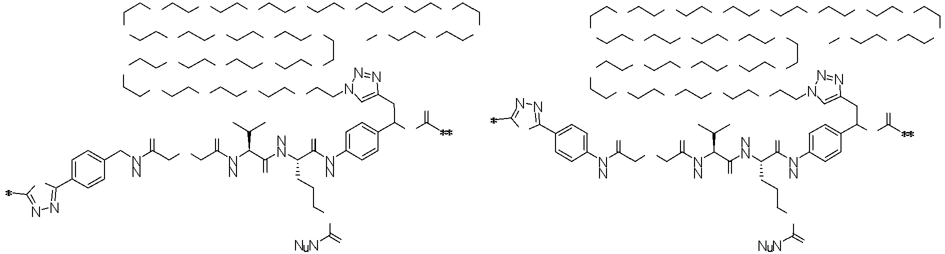 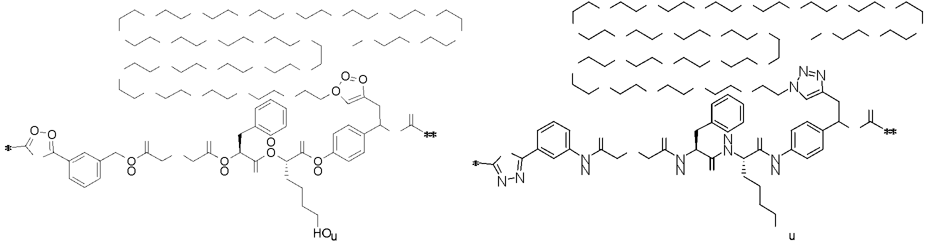 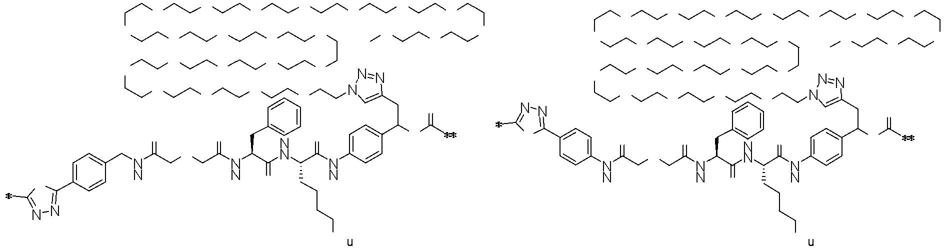 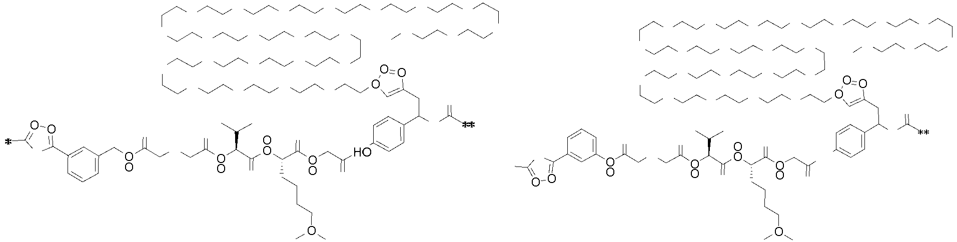 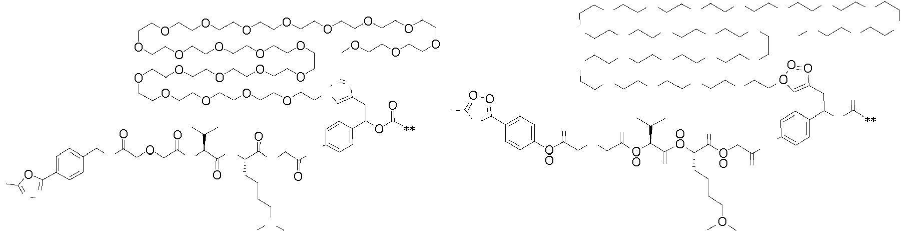 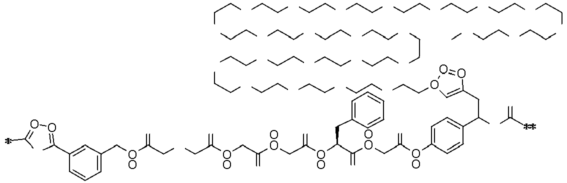 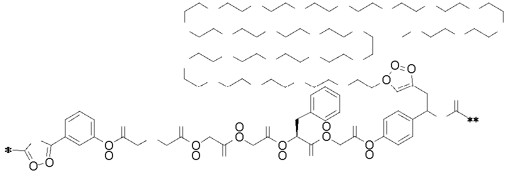 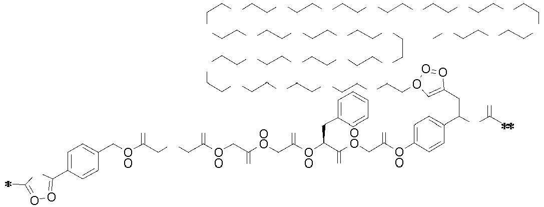 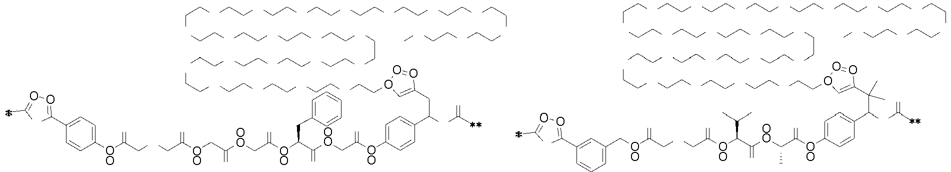 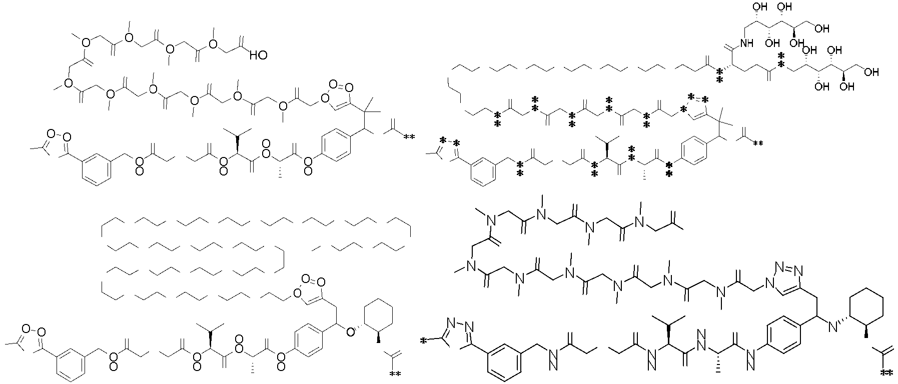 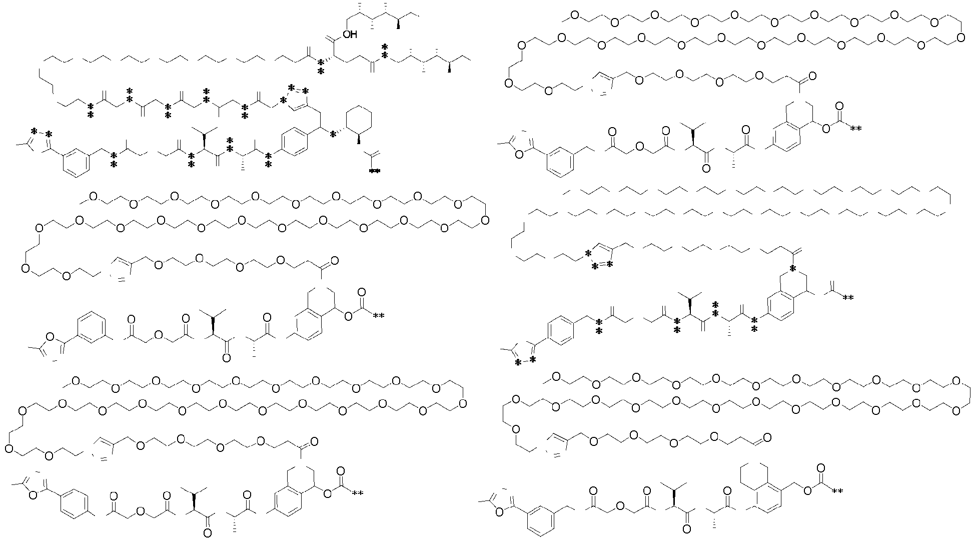 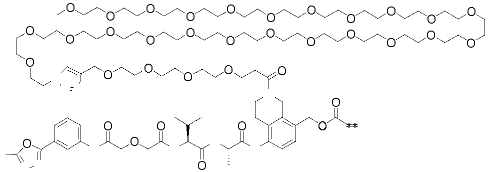 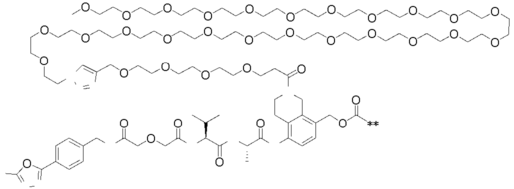 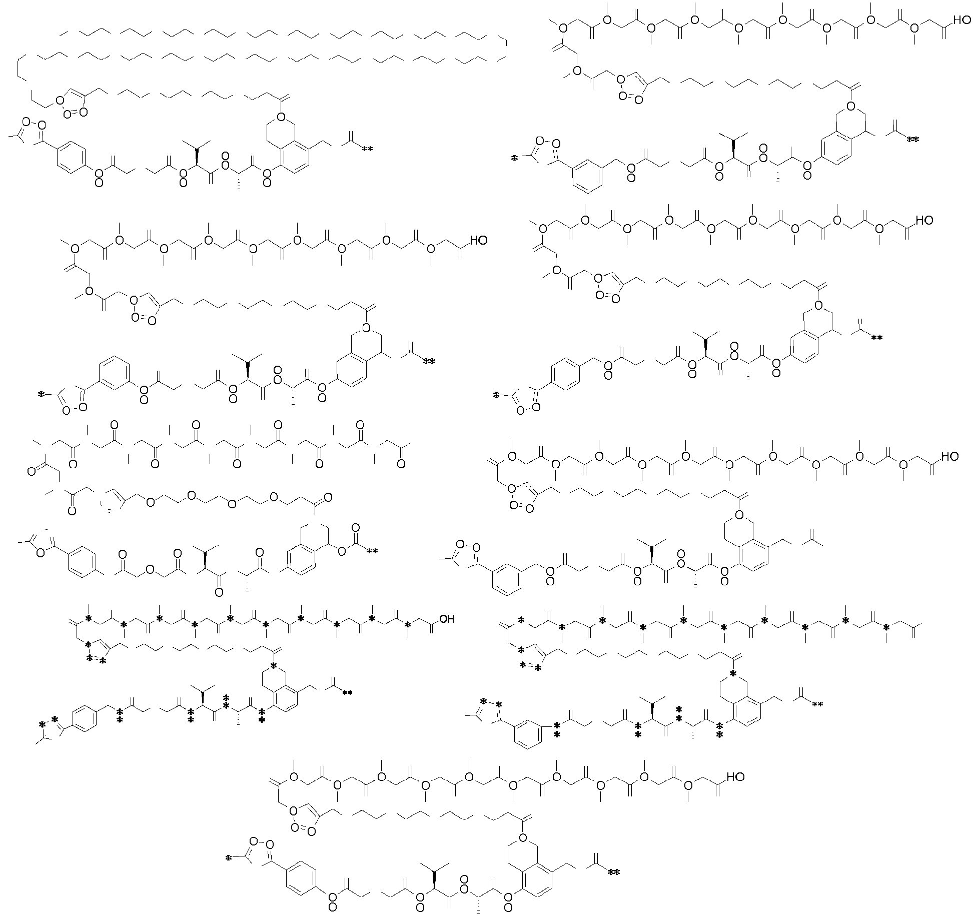 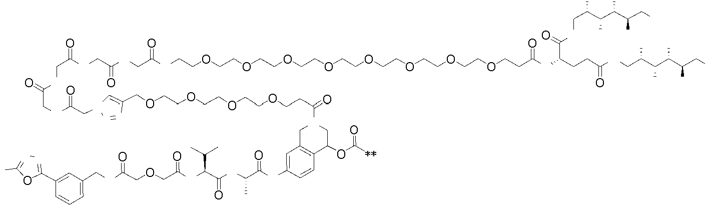 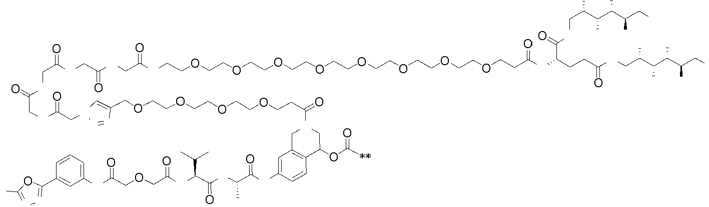 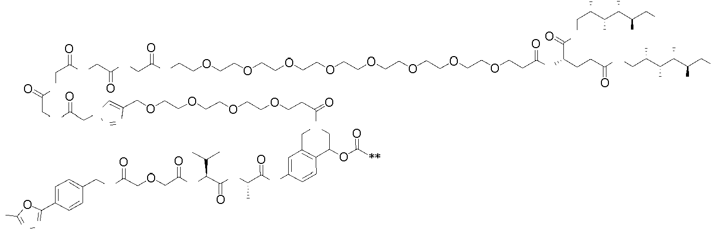 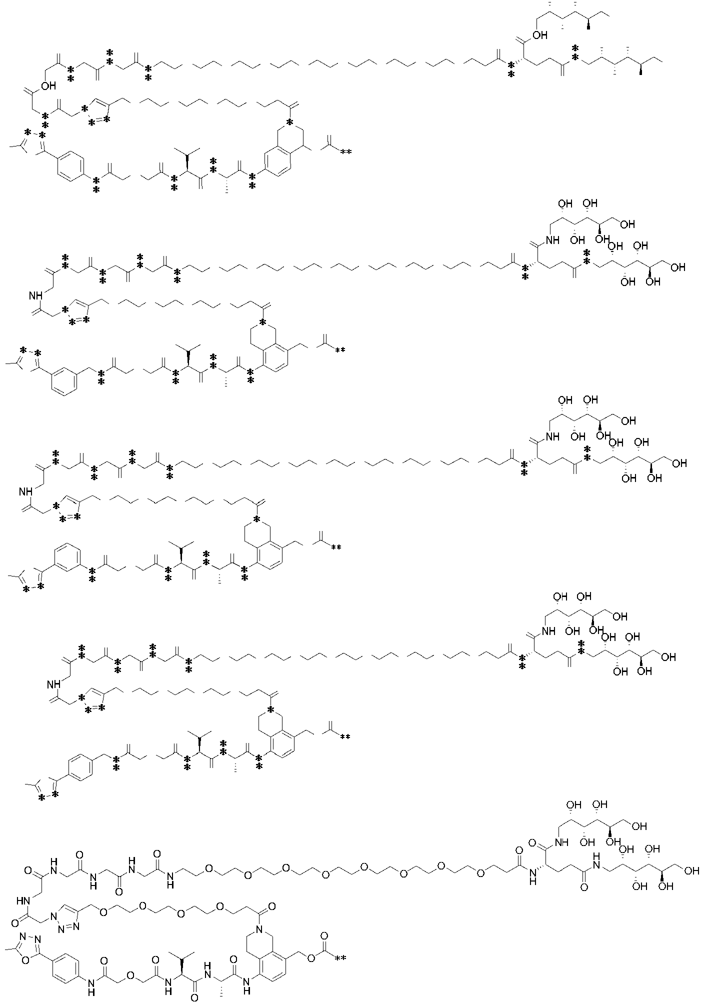 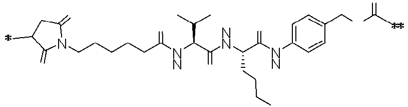 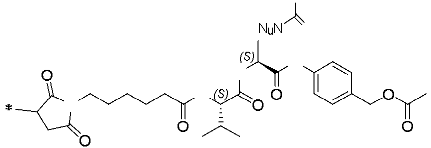 wherein * represents linkage to a sulfhydryl group of the antibody A, and ** represents linkage to the amino group of the bioactive molecule D.

In some embodiments, the bioactive molecule D is selected from a camptothecin derivative, eribulin, and a molecular glue.

In some embodiments, the bioactive molecule D is selected from a molecular glue compound, the molecular glue compound having a structure represented by formula (A-1): 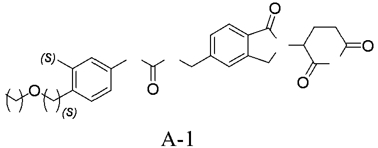 wherein RA is selected from optionally substituted 3- to 16-membered heterocyclyl, wherein the heteroatom in the heterocyclyl is selected from nitrogen; m1 and n1 are each independently an integer selected from 0-6, such as 0, 1, 2, 3, 4, 5, or 6.

In some embodiments of the present disclosure, RA is selected from the following optionally substituted substituents: 3- to 8-membered monocyclic heterocyclyl, 6- to 14-membered spiro heterocyclyl, 5- to 14-membered fused heterocyclyl, and 5- to 14-membered bridged heterocyclyl, wherein the heteroatom in the heterocyclyl is selected from nitrogen.

In some embodiments of the present disclosure, RA is selected from optionally substituted 5- to 6-membered monocyclic heterocyclyl.

In some embodiments of the present disclosure, RA is selected from the following optionally substituted substituents: azetidinyl, pyrrolidinyl, piperidinyl,

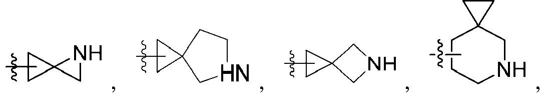

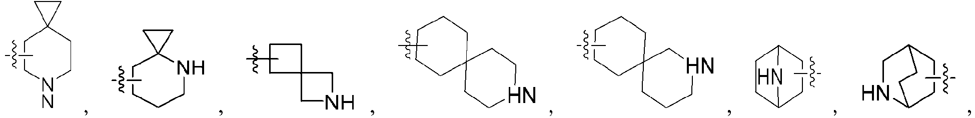

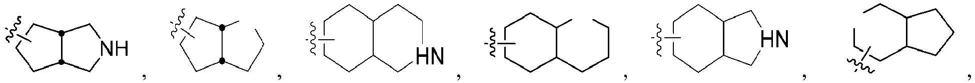

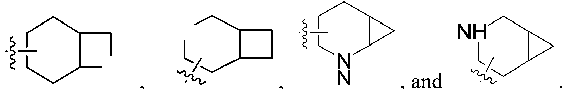

In some embodiments of the present disclosure, RA is selected from the following optionally substituted substituents: 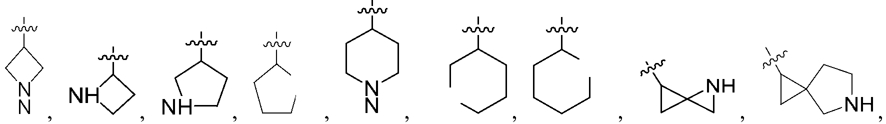 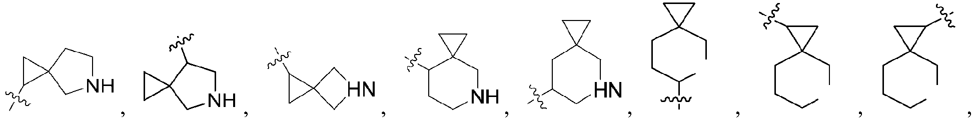 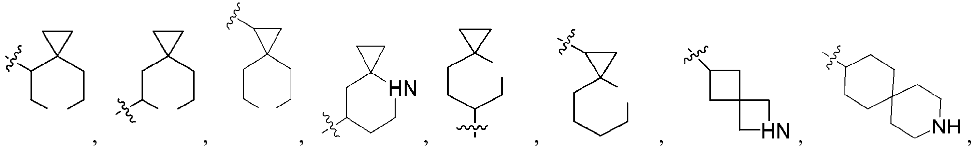 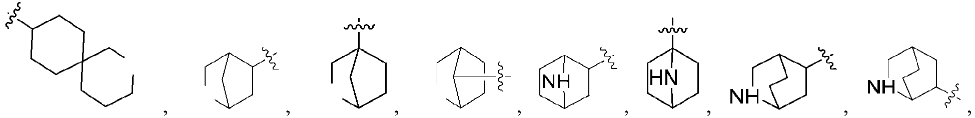 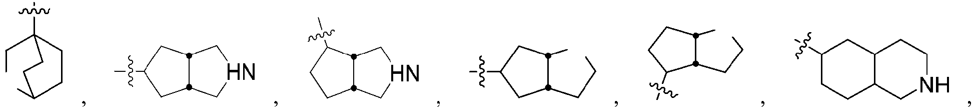 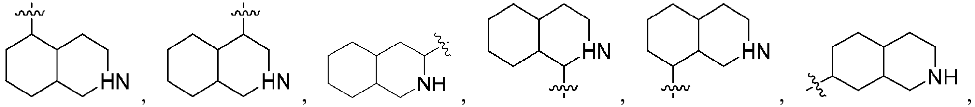 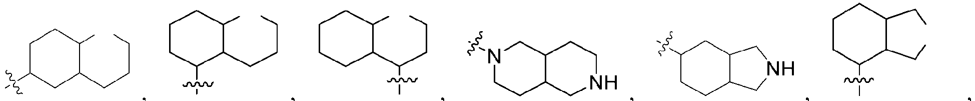 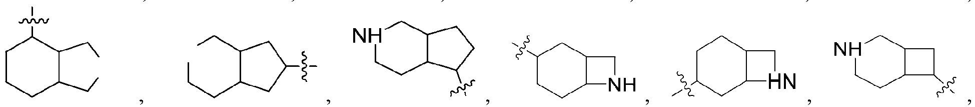 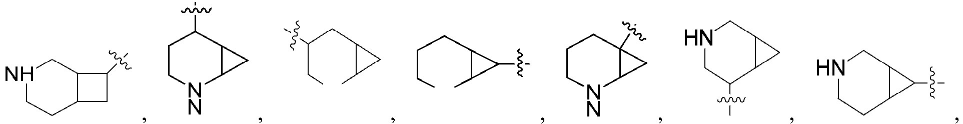 and

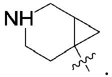

In some embodiments of the present disclosure, the optionally substituted substituent is selected from H and C₁₋₃ alkyl, such as methyl, ethyl, *n*-propyl, or isopropyl.

In some embodiments of the present disclosure, the compound having the structure represented by formula (A-1) has a structure represented by formula (A-2) or (A-3): 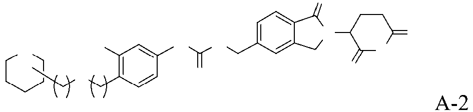 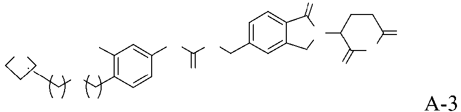 wherein m1 and n1 are each independently an integer selected from 0-6, such as 0, 1, 2, 3, 4, 5, or 6.

In some embodiments of the present disclosure, the compound having the structure represented by formula (A-1) has a structure represented by formula (A-4), (A-5), or (A-6):

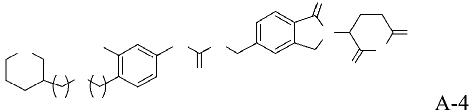

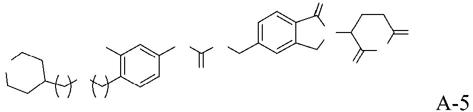

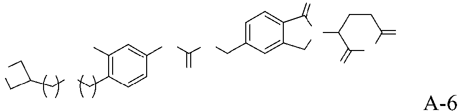

In some embodiments of the present disclosure, the structure represented by formula (A-1) is linked to L via a nitrogen atom on the amino group, further preferably via a nitrogen atom on RA,
for example: 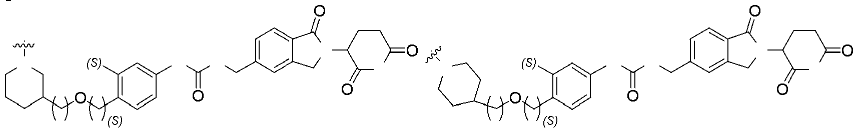 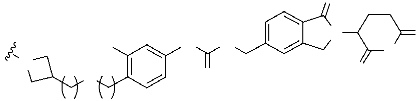 wherein the wavy line indicates a linking site of the bioactive molecule D to L.

In some embodiments, the bioactive molecule D is selected from compounds of the following formulas: 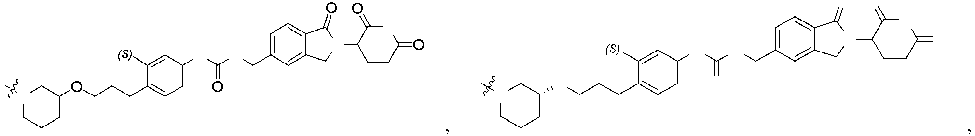 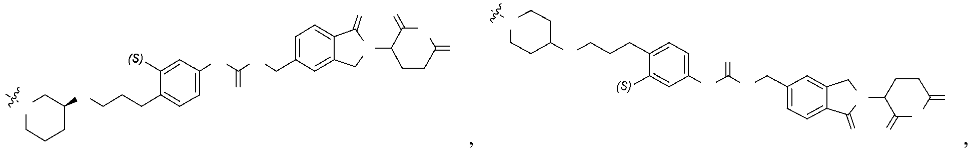 and

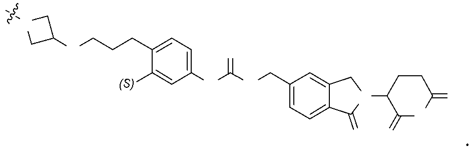

In some embodiments, the antibody-drug conjugate represented by formula I, and the tautomer, the mesomer, the racemate, the enantiomer, the diastereoisomer, the pharmaceutically acceptable salt, the hydrate, the solvate or the isotopically labeled compound thereof are selected from the following structures:

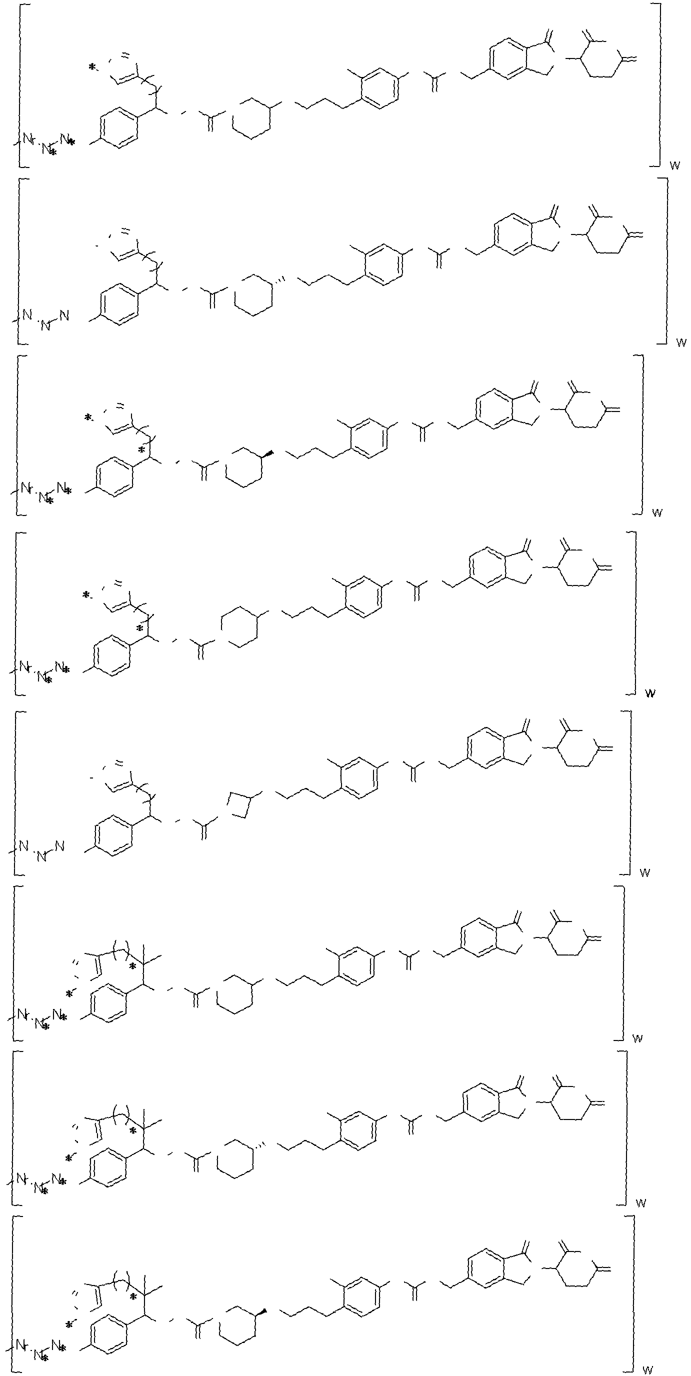

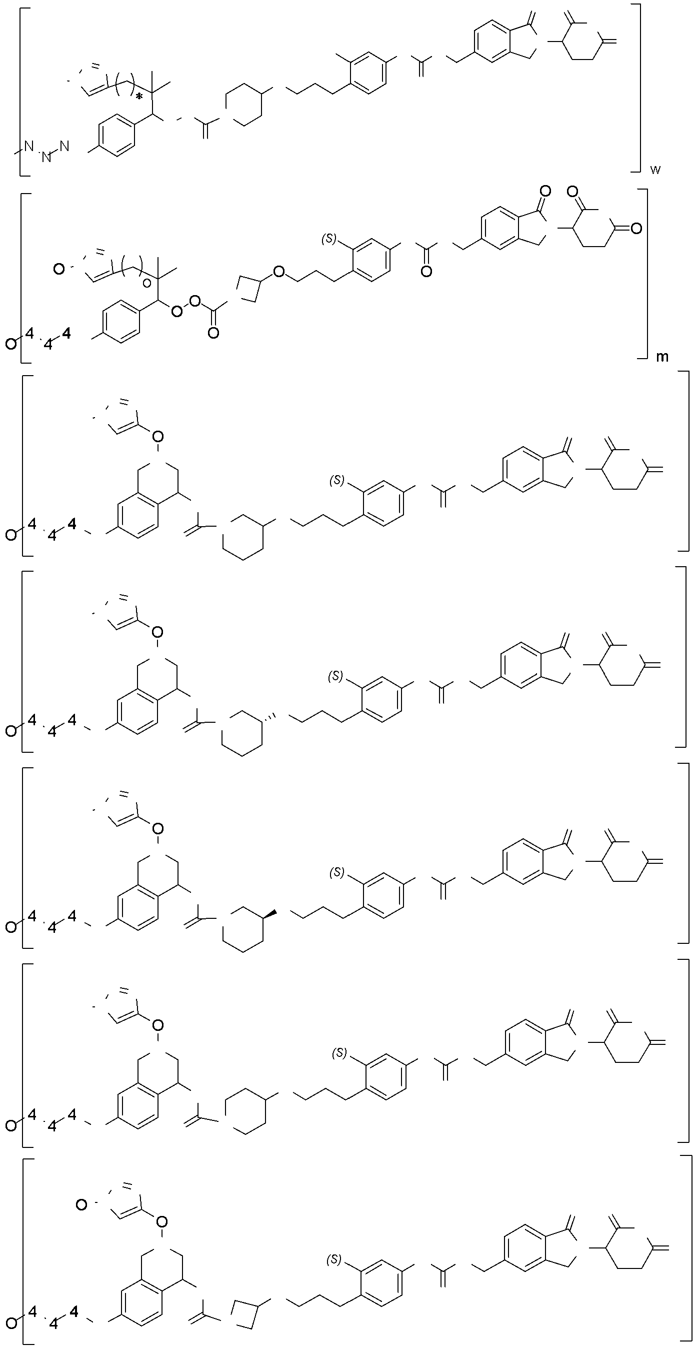

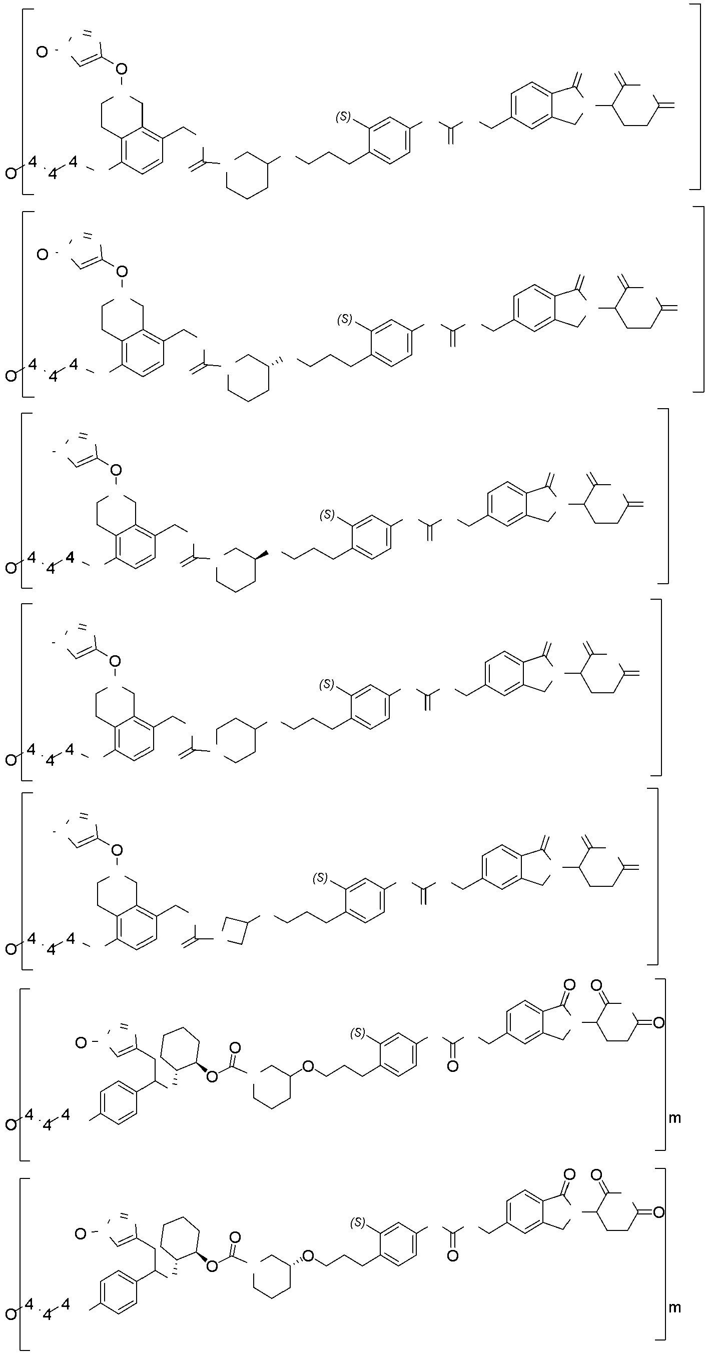

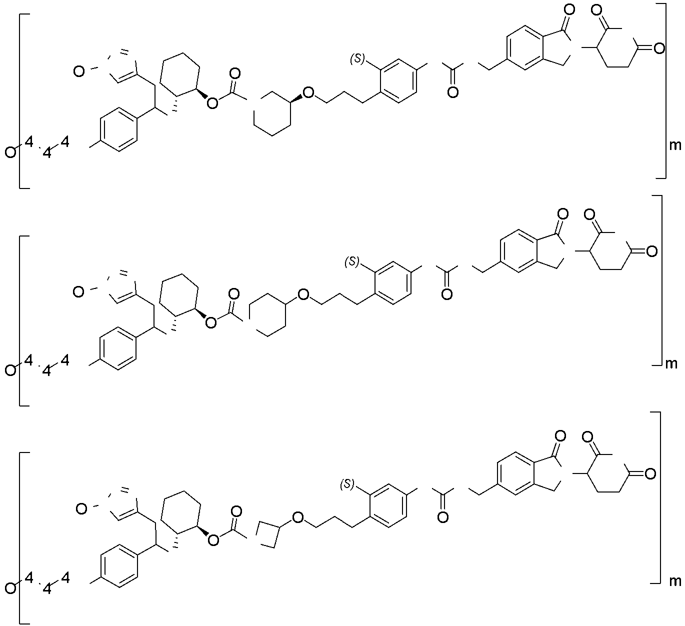

In some embodiments, the antibody-drug conjugate represented by formula I, and the tautomer, the mesomer, the racemate, the enantiomer, the diastereoisomer, the pharmaceutically acceptable salt, the hydrate, the solvate or the isotopically labeled compound thereof are selected from the following structures: 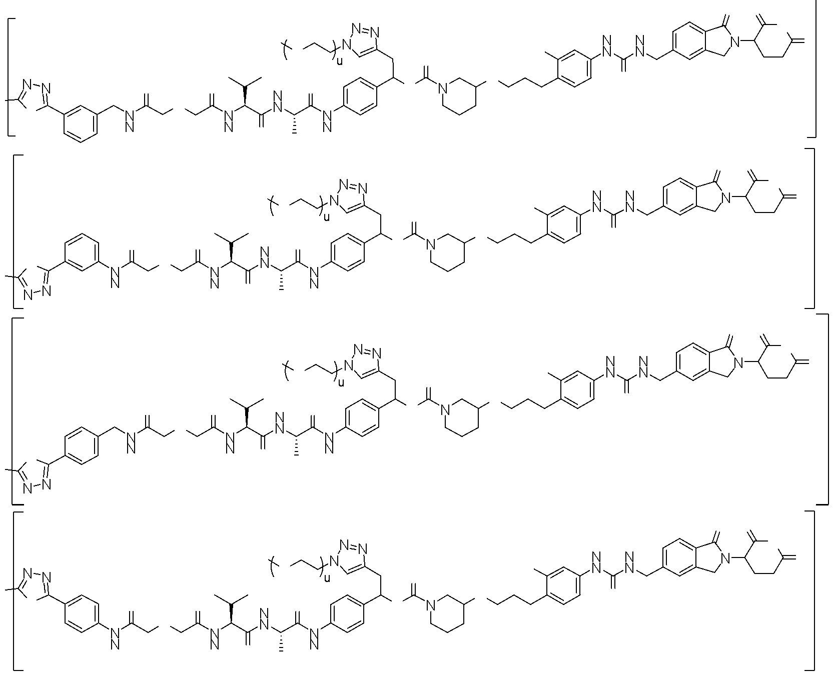 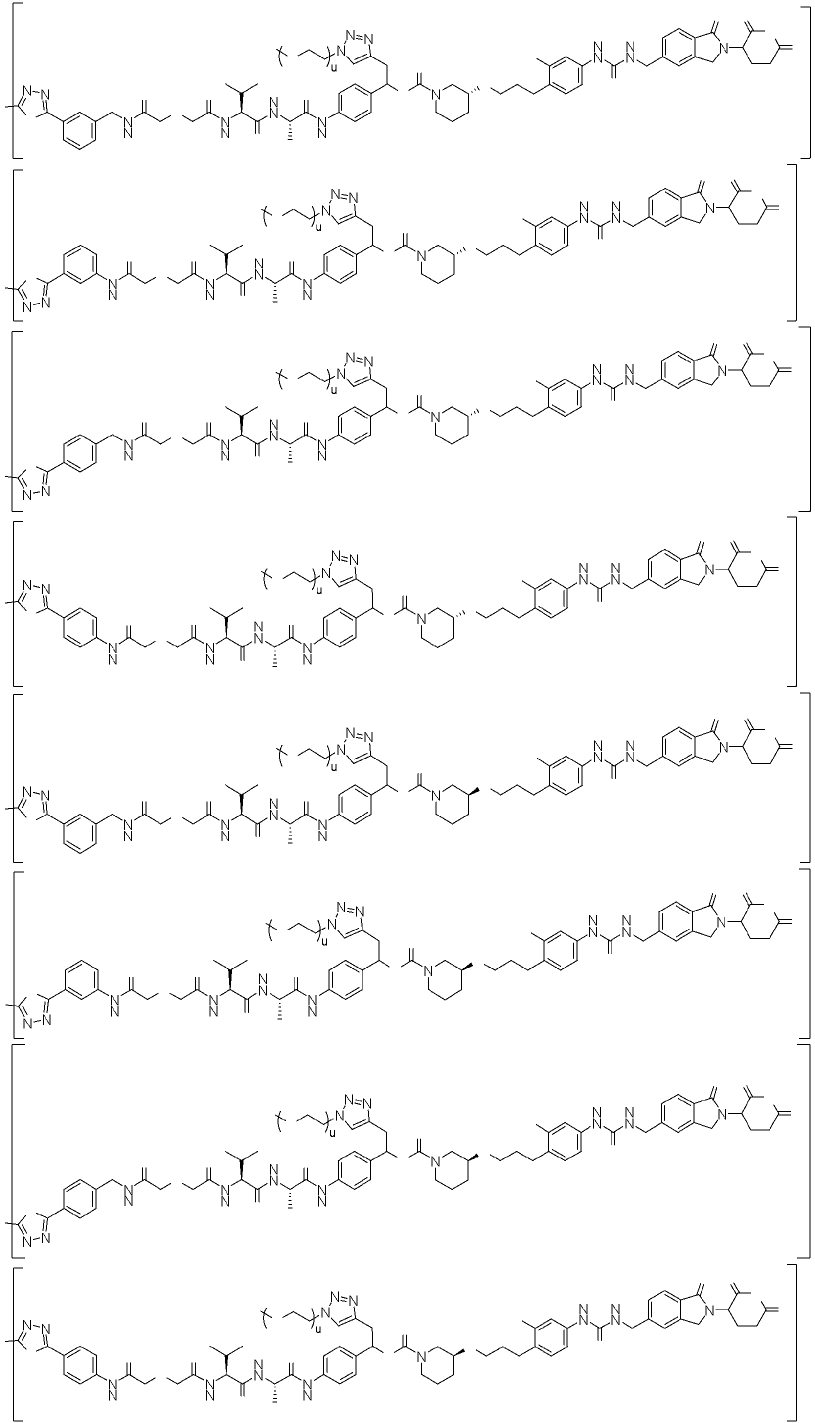 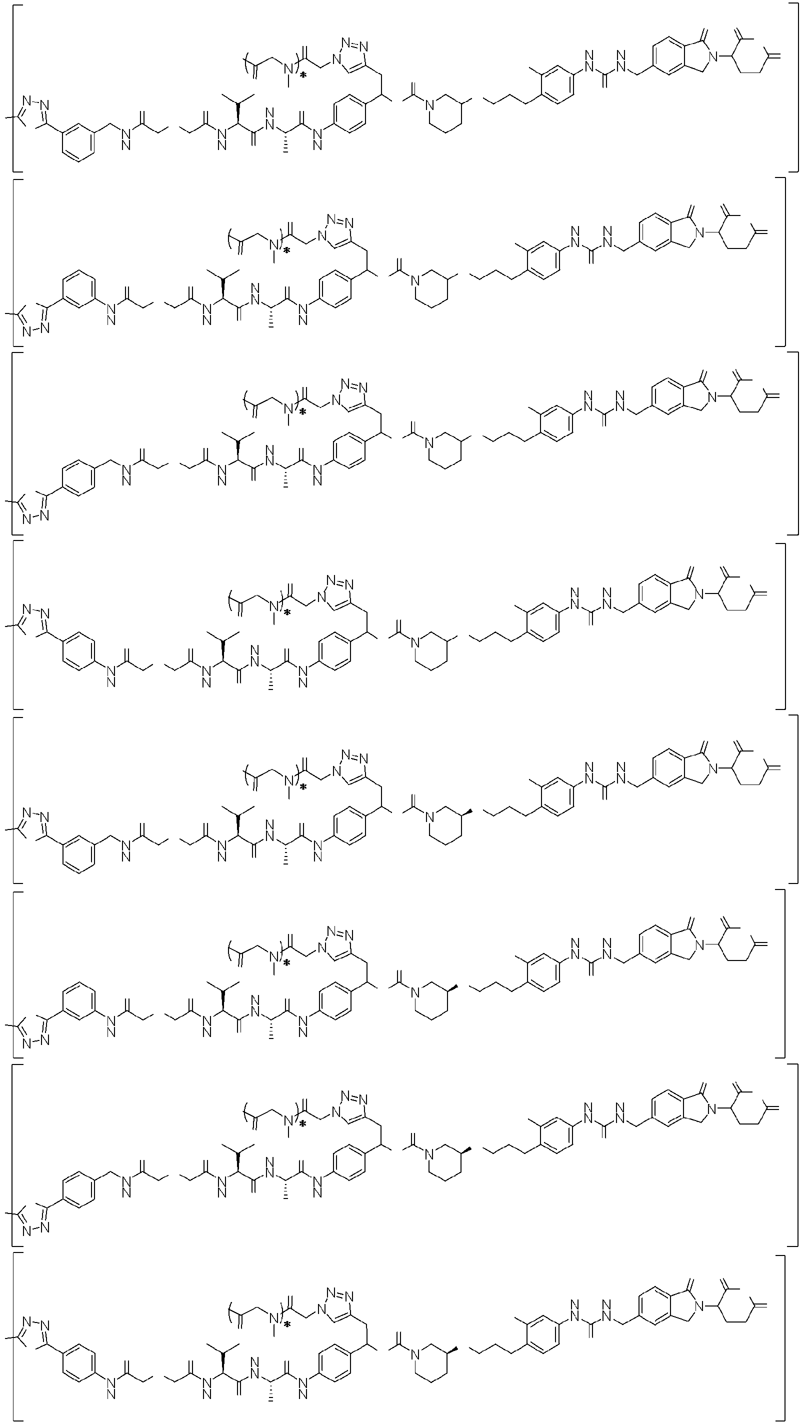 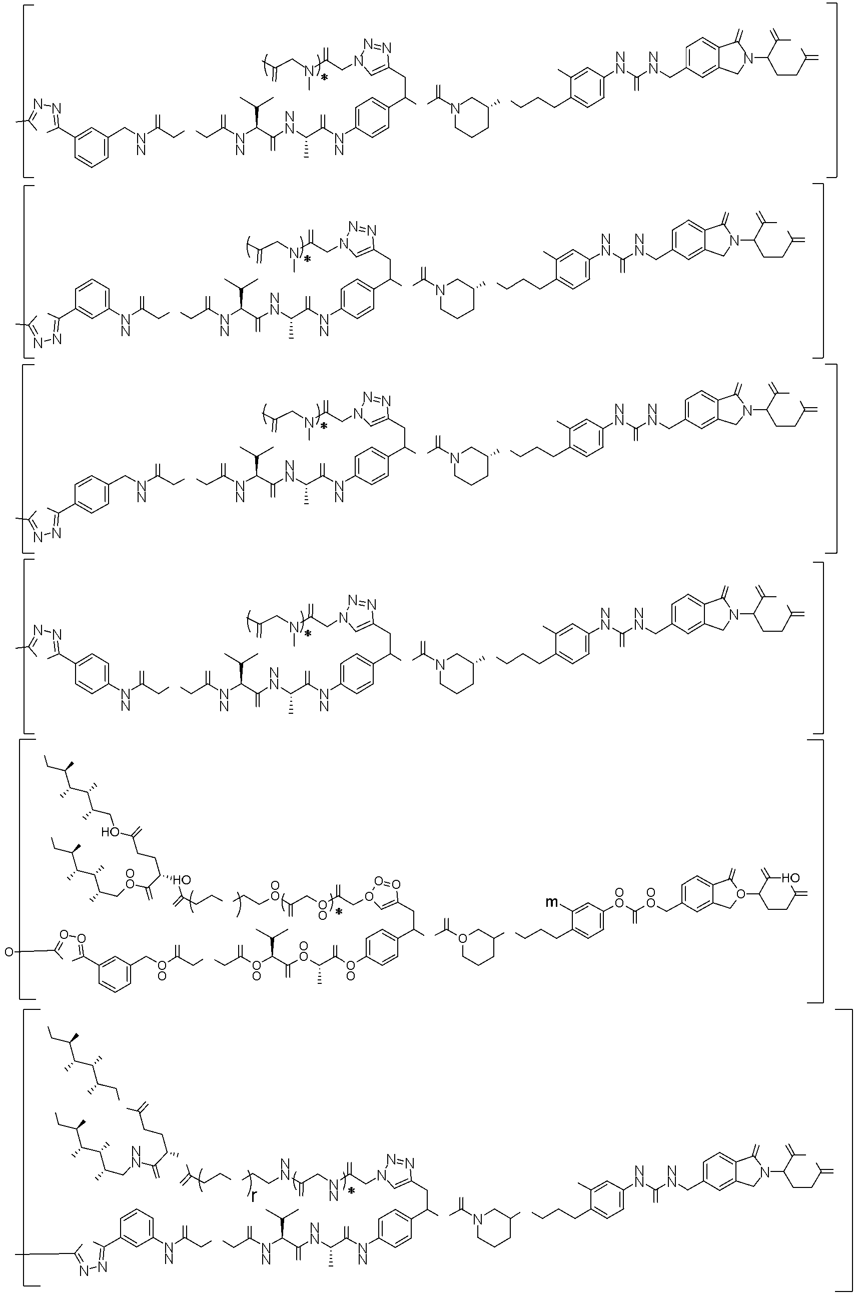 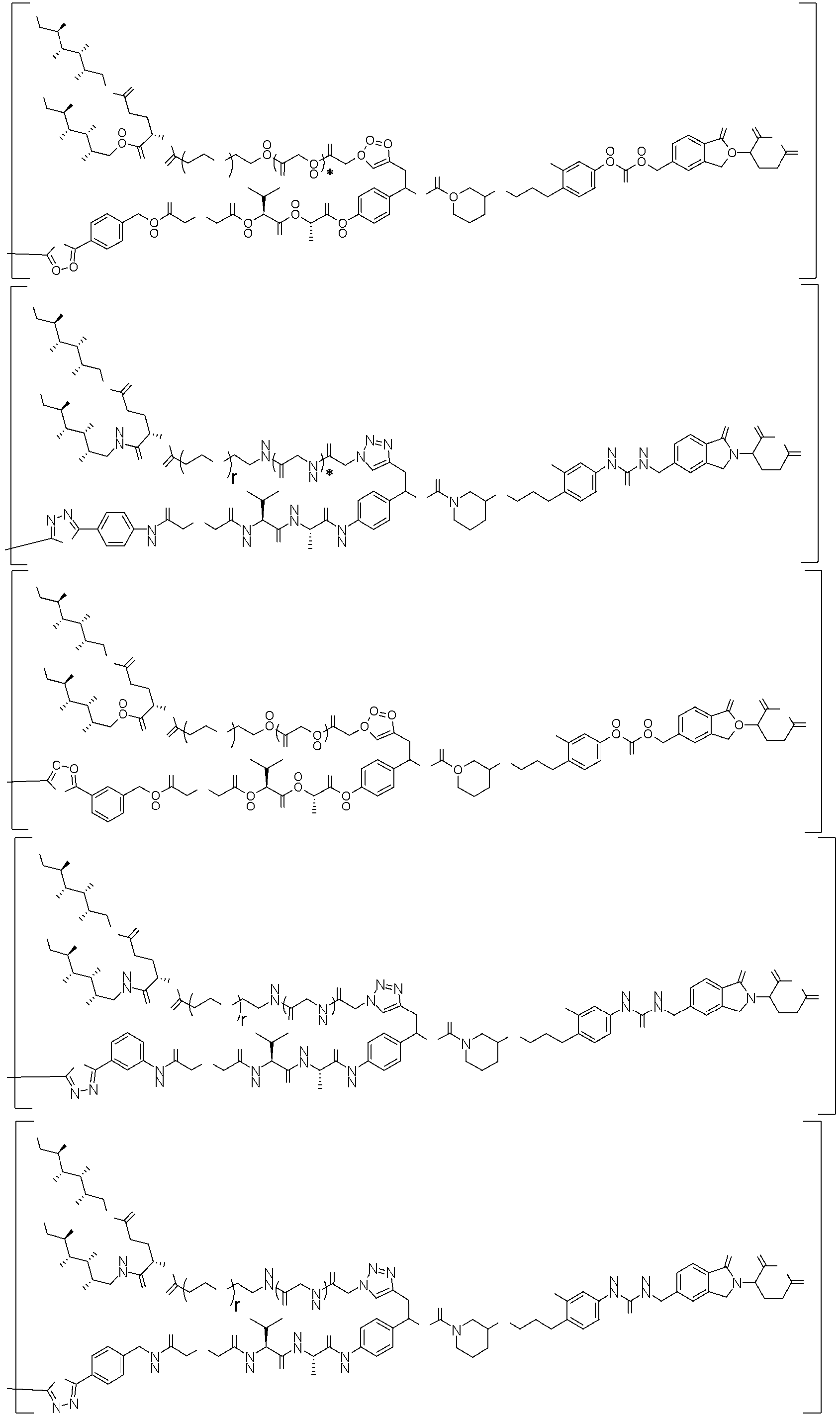 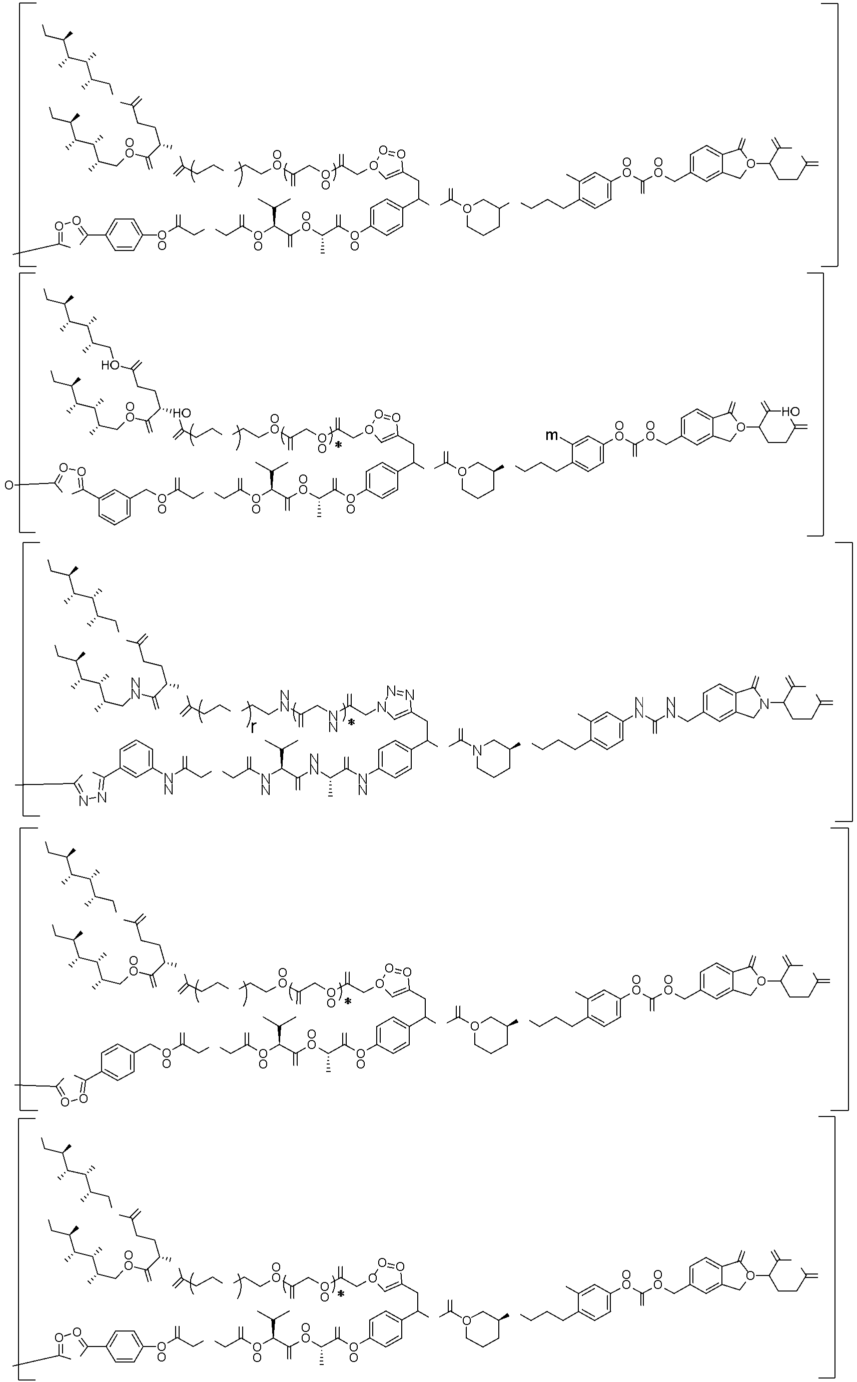 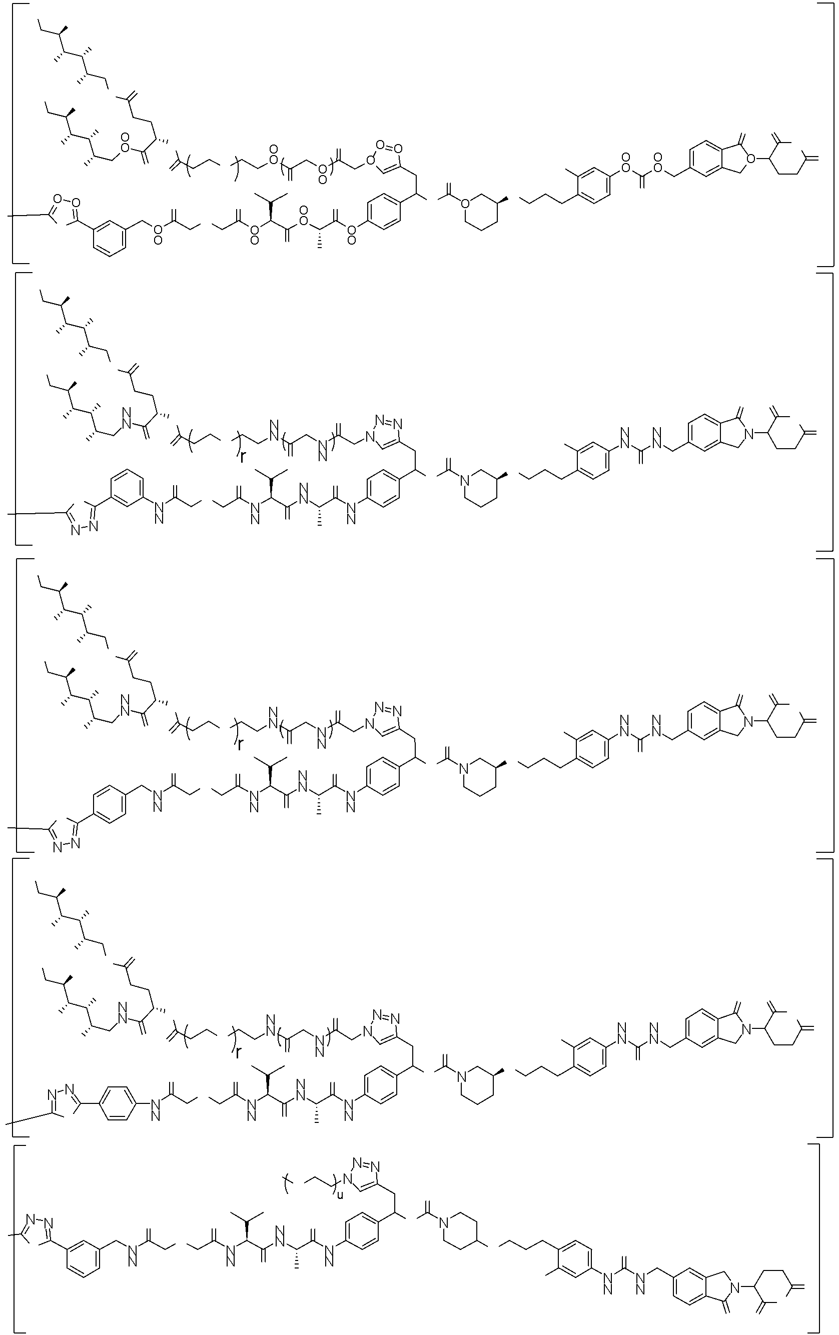 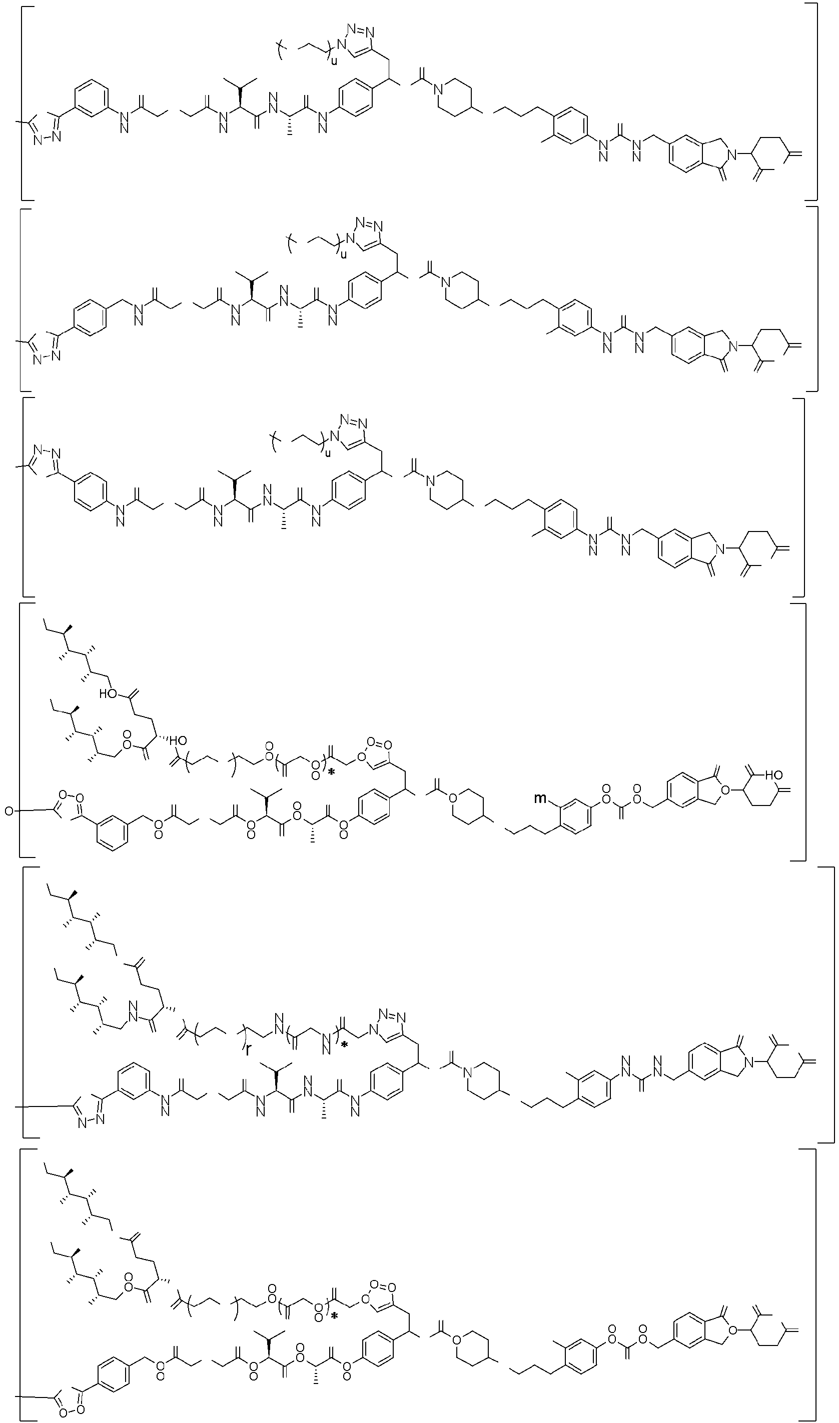 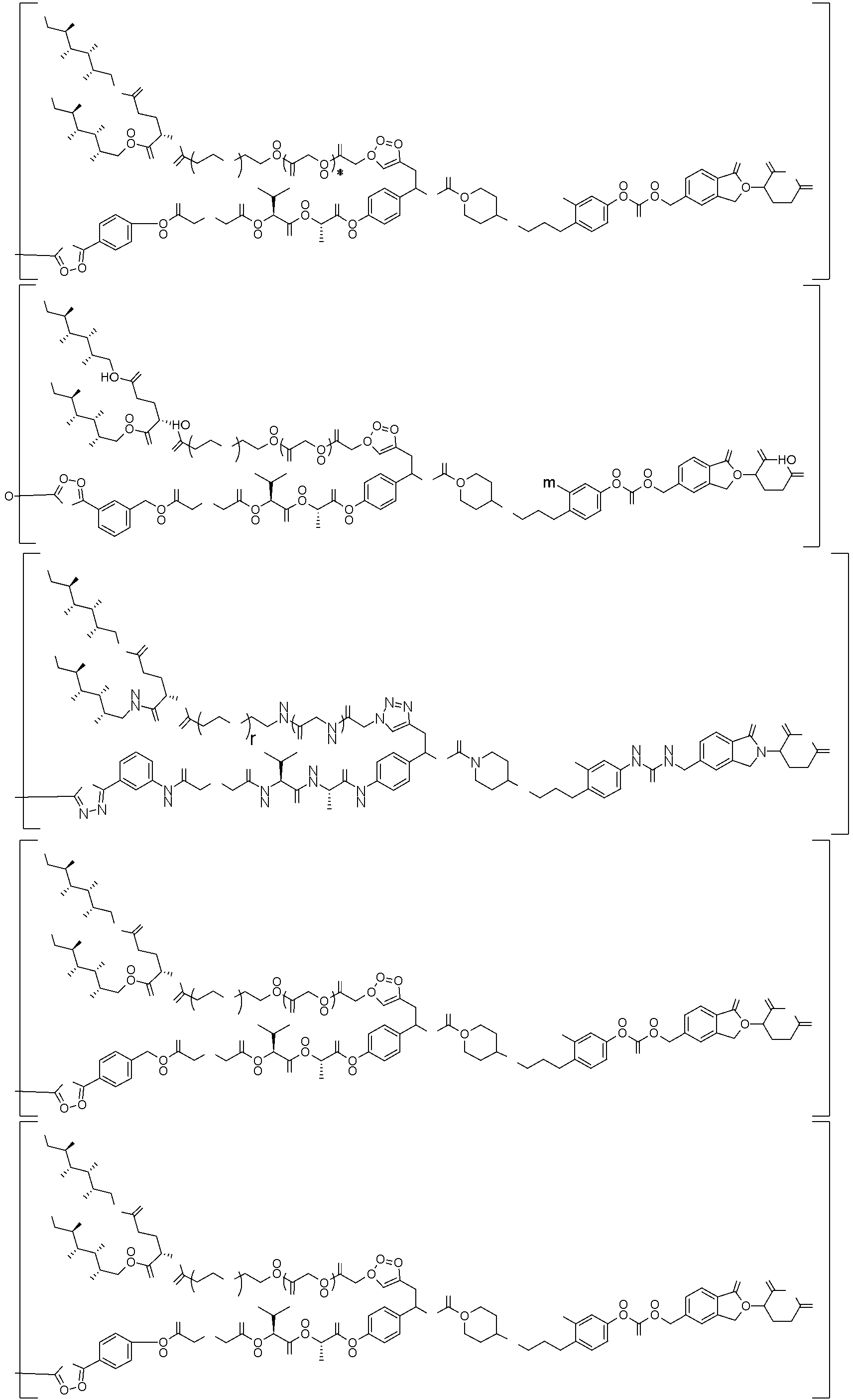 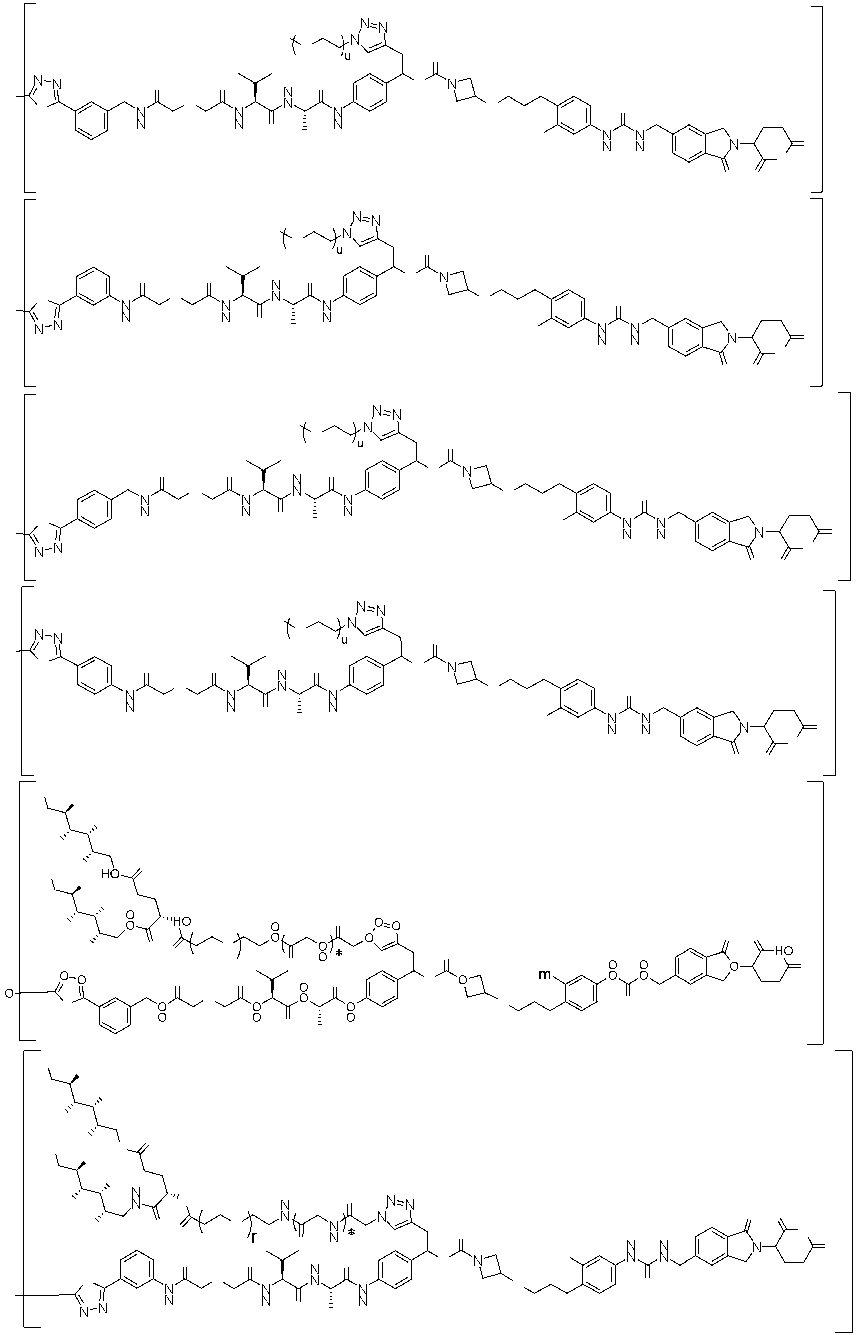 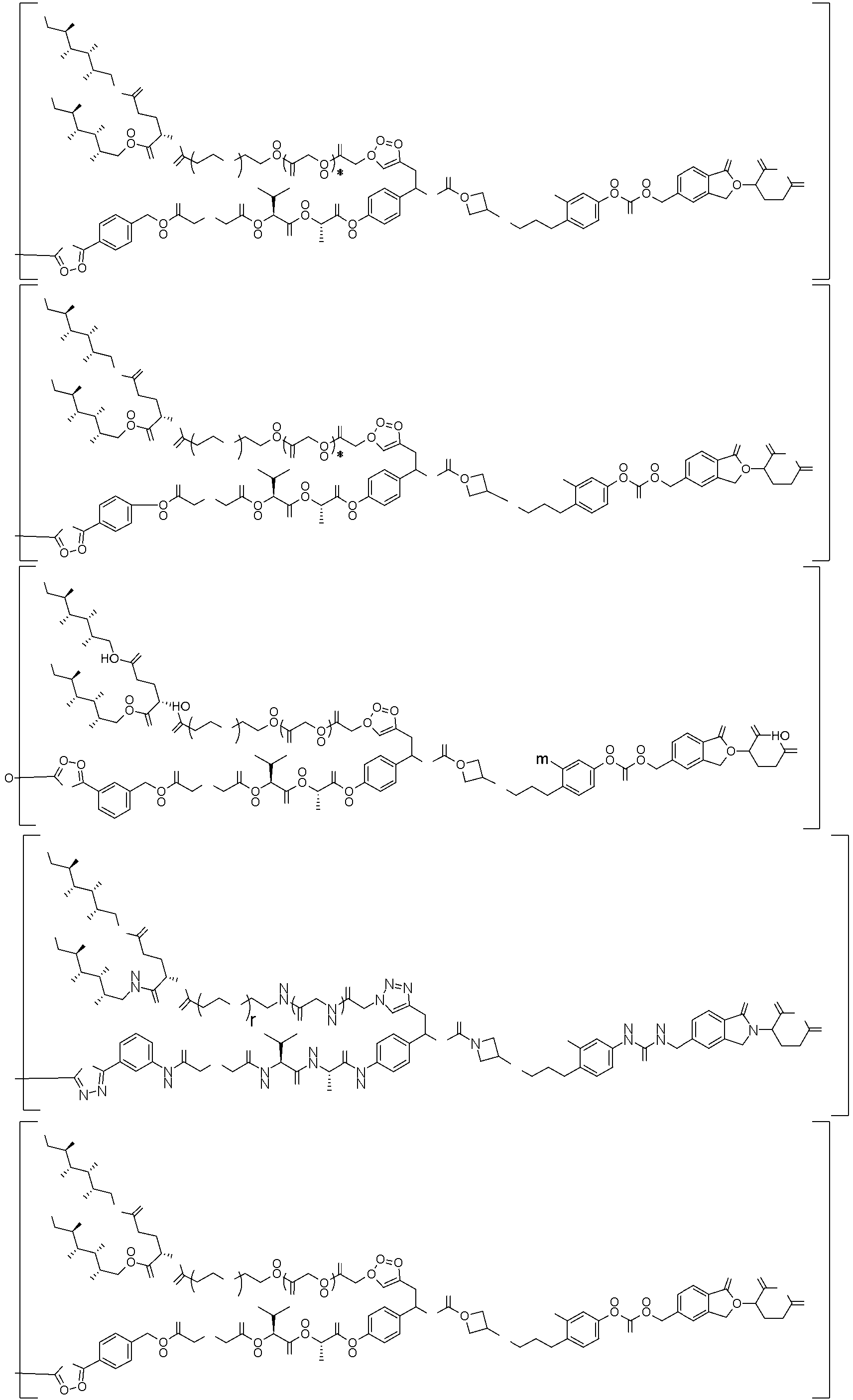 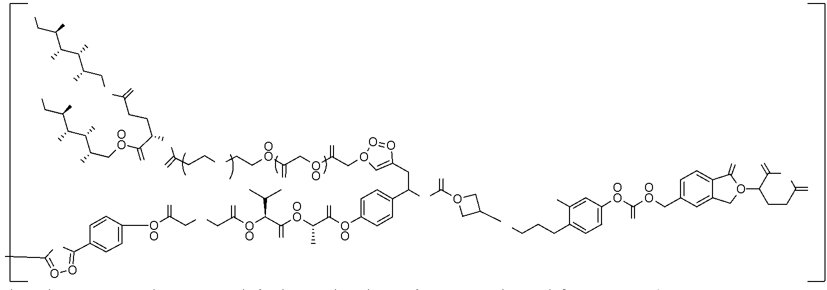 wherein r, s, t, and u are each independently an integer selected from 1-50 (e.g., 1, 2, 3, 4, , 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50), and v and w are each independently an integer selected from 1-12 (e.g., 1, 2, 3, 4, 5, 6, 7, 8, 9, 10, 11, or 12).

In some embodiments, the antibody-drug conjugate represented by formula I, and the tautomer, the mesomer, the racemate, the enantiomer, the diastereoisomer, the pharmaceutically acceptable salt, the hydrate, the solvate or the isotopically labeled compound thereof are selected from the following structures:

| No. | Structure |
|---|---|
| 1 | 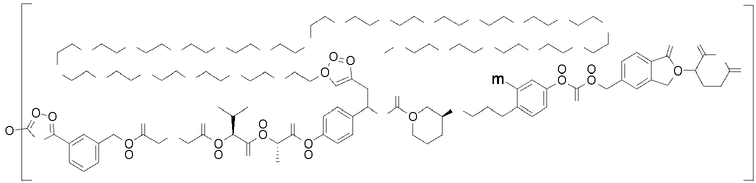 |
| 2 | 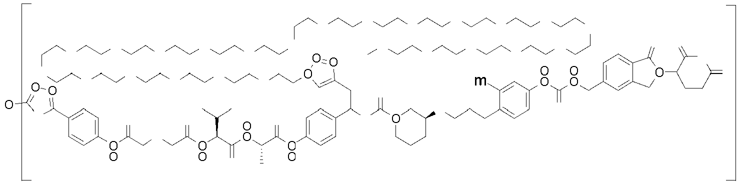 |
| 3 | 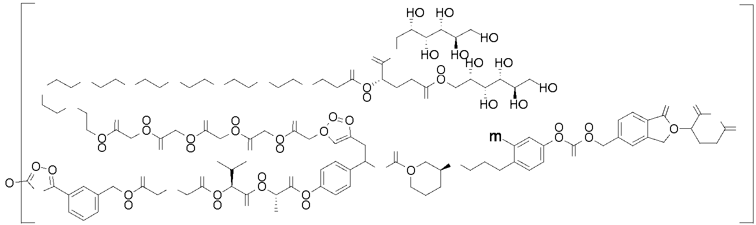 |
| 4 | 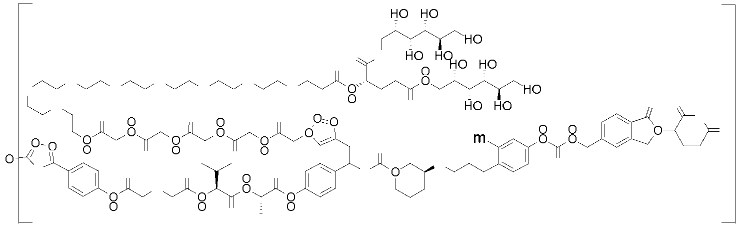 |
| 5 | 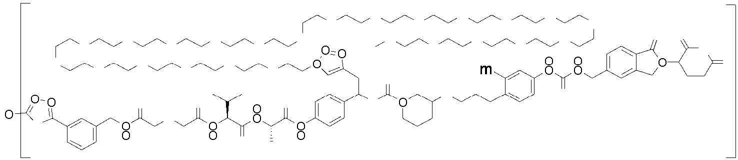 |
| 6 | 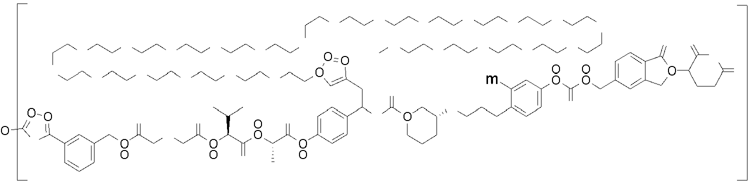 |
| 7 | 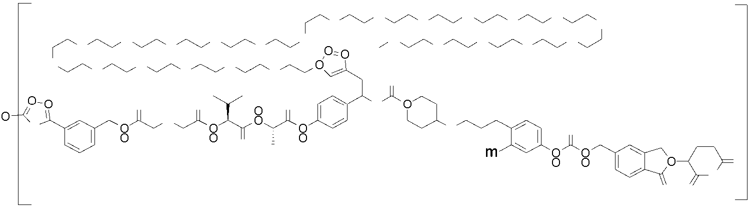 |
| 8 | 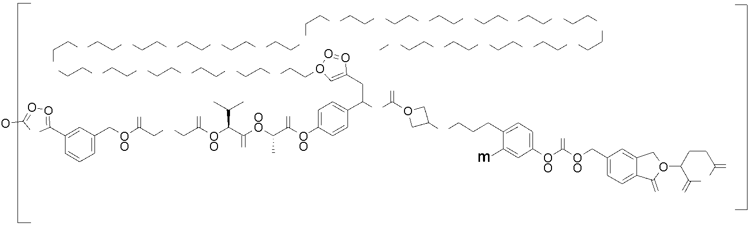 |
| 9 | 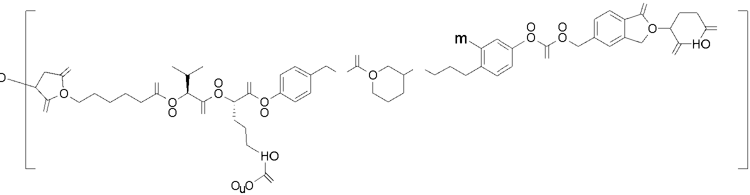 |
| 10 | 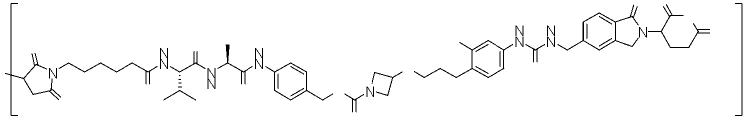 |
| 11 | 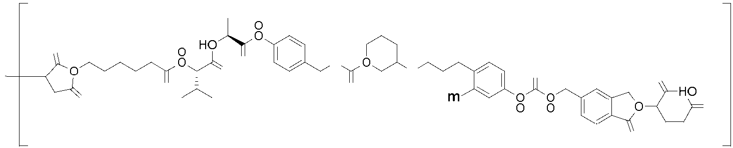 |

wherein preferably, the antibody A of structures 9-11 in the above table targets CD33 or CD123, and further preferably is 018, 019, 020, 021, 022, or 023.

In some embodiments, the present disclosure provides use of the antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof for the manufacturing of a medicament for the treatment of a cancer. The cancer includes liver cancer, kidney cancer, lung cancer (e.g., small cell lung cancer and non-small cell lung cancer), gastric cancer, esophageal cancer, urethral cancer, bladder cancer, colon cancer, rectal cancer, prostate cancer, breast cancer, ovarian cancer, pancreatic cancer, melanoma, hematological tumors or glioblastoma multiforme, lymphoma (e.g., Hodgkin lymphoma, non-Hodgkin lymphoma, or recurrent anaplastic large cell lymphoma), cervical cancer, uterine cancer, endometrial cancer, salivary gland cancer, glioma, neuroblastoma, sarcoma, colorectal cancer, leukemia (e.g., acute lymphoblastic leukemia, acute myeloid leukemia, acute promyelocytic leukemia, chronic myeloid leukemia, or chronic lymphocytic leukemia), bone cancer, skin cancer, thyroid cancer, etc.

In some embodiments, the present disclosure provides use of the antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof for the treatment of a cancer. The cancer includes liver cancer, kidney cancer, lung cancer (e.g., small cell lung cancer and non-small cell lung cancer), gastric cancer, esophageal cancer, urethral cancer, bladder cancer, colon cancer, rectal cancer, prostate cancer, breast cancer, ovarian cancer, pancreatic cancer, melanoma, hematological tumors or glioblastoma multiforme, lymphoma (e.g., Hodgkin lymphoma, non-Hodgkin lymphoma, or recurrent anaplastic large cell lymphoma), cervical cancer, uterine cancer, endometrial cancer, salivary gland cancer, glioma, neuroblastoma, sarcoma, colorectal cancer, leukemia (e.g., acute lymphoblastic leukemia, acute myeloid leukemia, acute promyelocytic leukemia, chronic myeloid leukemia, or chronic lymphocytic leukemia), bone cancer, skin cancer, thyroid cancer, etc.

In some embodiments, the cancer is a tumor expressing HER2 (HER2+), CD33 (CD33+), or CD123 (CD123+) on the surface of tumor cells.

In some embodiments, the present disclosure provides use of the antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof for the treatment of a cancer. The cancer is preferably a cancer associated with abnormal expression of HER2, CD33, or CD123. The cancer associated with abnormal expression of CD33 or CD123 includes leukemia (e.g., acute lymphoblastic leukemia, acute myeloid leukemia, acute promyelocytic leukemia, chronic myeloid leukemia, or chronic lymphocytic leukemia). The cancer associated with abnormal expression of HER2 includes lung cancer (e.g., small cell lung cancer and non-small cell lung cancer), breast cancer, ovarian cancer, endometrial cancer, gastric cancer, prostate cancer, etc. The cancer associated with abnormal expression of BCMA includes lymphoma (e.g., multiple myeloma).

In some embodiments, the present disclosure provides a pharmaceutical composition, which comprises the antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof, and one or more pharmaceutically acceptable auxiliary materials.

In another aspect of the present disclosure, provided is a linker-drug compound represented by formula (II), or a tautomer, a mesomer, a racemate, an enantiomer or a diastereoisomer thereof, and a pharmaceutically acceptable salt, a hydrate, a solvate or an isotopically labeled compound thereof:

L'-D (II)

wherein L' is a linker moiety;
D is the amino-containing bioactive molecule or the pharmaceutically acceptable salt thereof described in formula (I), covalently linked to the linker moiety L' via an amine group in its molecular structure;

In some embodiments, the linker moiety L' is represented by the following formula:

L₁'-L₂-L₃-L₄-,

wherein L₁' is selected from 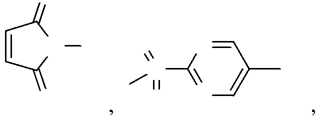 and 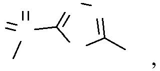 wherein ** represents linkage to L₂;
L₂, L₃, L₄, and D are as defined in formula (I).
L₂ is a spacer, preferably selected from -L₂ₐ-C(O)-, -L₂ₐ-L_{2b}-C(O)-, -L₂ₐ-NH-C(O)-, -L₂ₐ-C(O)-NH-, -L₂ₐ-L_{2b}-NH-C(O)-, -L₂ₐ-L_{2b}-C(O)-NH-, -L₂ₐ-C(O)-NH-L_{2b}-C(O)-, -L₂ₐ-NH-C(O)-L_{2b}-C(O)-, -L₂ₐ-C(O)-NH-L_{2b}-C(O)-NH-, - L₂ₐ-NH-C(O)-L_{2b}-NH-C(O)-, -L₂ₐ-NR¹-SO₂-NH-C(O)-O-L_{2b}-C(O)-, and -L₂ₐ-NR¹-SO₂-NH-C(O)-O-L_{2b}-NH-C(O)-, wherein L₂ₐ is selected from -C₁-C₈ alkylene-, -C₁-C₈ alkylene-C₃-C₈ cycloalkylene-, -C₆-C₁₄ arylene-, -C₆-C₁₄ arylene-C₁-C₈ alkylene-, -(5- to 6-membered heteroarylene)-, -(5- to 6-membered heteroarylene)-C₁-C₈ alkylene-, linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and (linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms)-(3- to 8-membered heterocyclylene), wherein the alkylene, cycloalkylene, arylene, heteroalkylene, heteroarylene, and heterocyclylene are each optionally substituted with one or more substituents independently selected from halogen, C₁-C₆ alkyl, heteroalkyl having 1-6 atoms, C₁-C₆ alkoxy, hydroxyl, amino, carboxyl, and C₃-C₈ cycloalkyl, the heteroalkylene, heterocyclylene, or heteroalkyl contains 1-12 (preferably 1-8, e.g., 1, 2, 3, 4, 5, 6, 7, 8, 9, 10, 11, or 12) heteroatoms, and the heteroatom in the heteroalkylene, heterocyclylene, heteroarylene, or heteroalkyl is selected from one or more of N, O, and S; L_{2b} is selected from - C₁-C₈ alkylene- and linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and R¹ is selected from hydrogen, C₁-C₆ alkyl, C₃-C₈ cycloalkyl, 3- to 8-membered heterocyclyl, C₁-C₆ haloalkyl, heteroalkyl having 2-8 atoms, C₆-C₁₄ aryl, and 5- to 6-membered heteroaryl, wherein the alkyl, heteroalkyl, aryl, heteroaryl, cycloalkyl, and heterocyclyl are each optionally substituted with one or more substituents independently selected from C₁-C₆ alkyl, heteroalkyl having 2-6 atoms, C₁-C₆ alkoxy, amino, and carboxyl, the heteroalkyl or heteroalkylene contains 1-12 (preferably 1-8, e.g., 1, 2, 3, 4, 5, 6, 7, 8, 9, 10, 11, or 12) heteroatoms, and the heteroatom in the heteroalkyl, heterocyclyl, heteroaryl, or heteroalkylene is selected from one or more of N, O, and S;
L₃ is a polypeptide sequence selected from a peptide residue consisting of 2-8 (e.g., 2, 3, 4, 5, 6, 7, or 8) natural or unnatural amino acids, wherein optionally, the amino acid is further substituted with one or more substituents selected from C₁-C₆ alkyl, heteroalkyl having 2-6 atoms, C₁-C₆ alkoxy, hydroxyl, amino, carboxyl, and C₃-C₈ cycloalkyl;
L₄ is a hydrophilic group-modified self-immolative fragment, and
the self-immolative fragment is selected from: 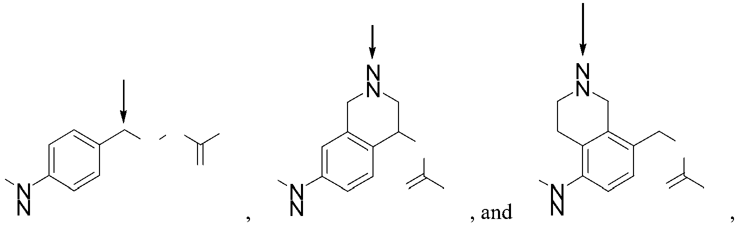 wherein * represents linkage to a carboxyl group of L₃ via an amide bond, ** represents linkage to the amino group of the bioactive molecule D, X is absent or 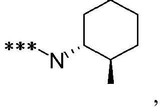 *** represents linkage to a carbon atom, **** represents linkage to an oxygen atom, and the arrow indicates a modification site of the hydrophilic group; further preferably, the self-immolative fragment, prior to modification by the hydrophilic group, is selected from the following structures: 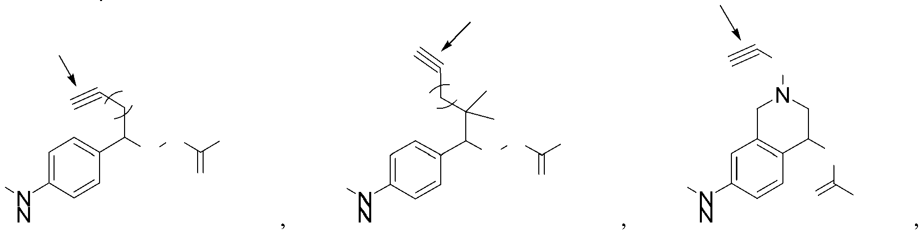 and 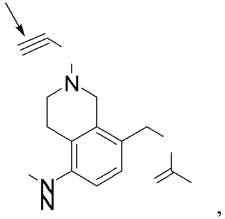
wherein Y is C₁-C₆ alkylene or -R³-C(O)-, wherein R³ is C₁-C₆ alkylene or heteroalkylene containing 1-8 - OCH₂CH₂- structural units; n is an integer from 0 to 6;
the hydrophilic group has at least one azido group and comprises a polyethylene glycol group, a poly-natural or unnatural amino acid group, a monosaccharide, an oligosaccharide, or a polysaccharide, or a combination thereof; preferably, L₄ is selected from: 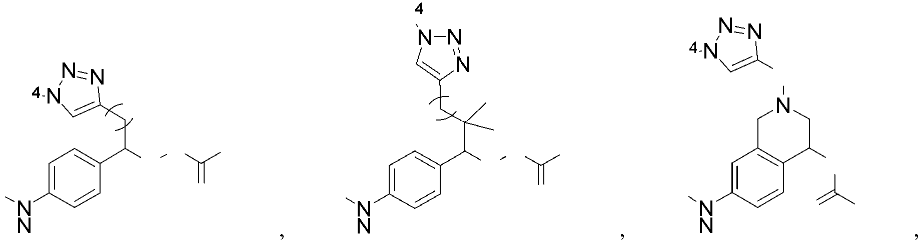 and 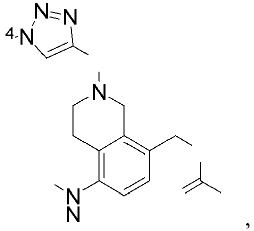
wherein * represents linkage to a carboxyl group of L₃ via an amide bond, and ** represents linkage to the amino group of the bioactive molecule D;
R² is selected from linear or branched heteroalkyl containing 4-50 (preferably 4-24, more preferably 6-24, and most preferably 8-24, e.g., 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) (OCH₂CH₂) structural units, a peptide chain containing 4-50 (preferably 4-24, more preferably 8-24, and most preferably 10-24, e.g., 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) natural or unnatural amino acids, and linear or branched heteroalkyl containing a monosaccharide, an oligosaccharide, or a polysaccharide; X is absent or 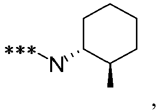 *** represents linkage to a carbon atom, and **** represents linkage to an oxygen atom; Y is C₁-C₆ alkylene or -R³-C(O)-, wherein R³ is C₁-C₆ alkylene or heteroalkylene containing 1-8 (e.g., 1, 2, 3, 4, 5, 6, 7, or 8) -OCH₂CH₂- structural units; n is an integer from 0 to 6 (e.g., 0, 1, 2, 3, 4, 5, or 6).

In some embodiments, R² is selected from the following structures: 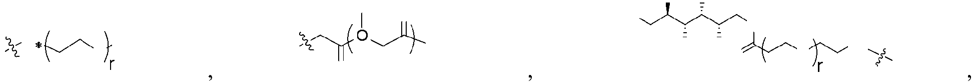 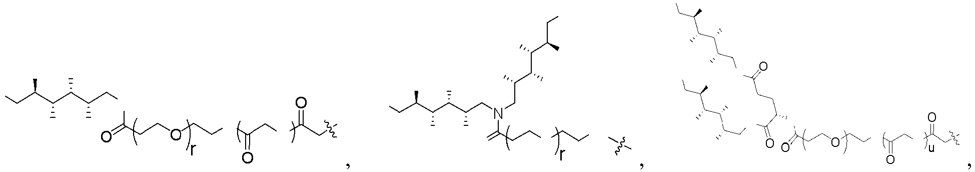 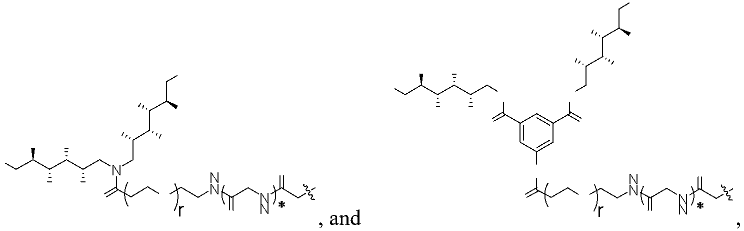 wherein r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50); Rₐ is selected from a bond and C₁-C₃ alkylene, such as methylene, ethylidene, n-propylidene, or isopropylidene; R_{b} is selected from C₁-C₃ alkyl, such as methyl, ethyl, n-propyl, or isopropyl.

In some embodiments, R² is selected from the following structures:

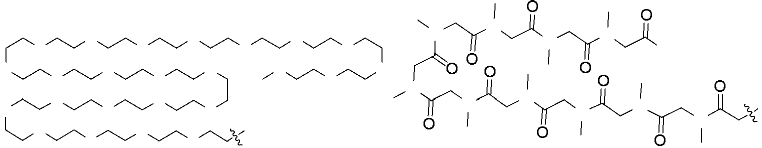

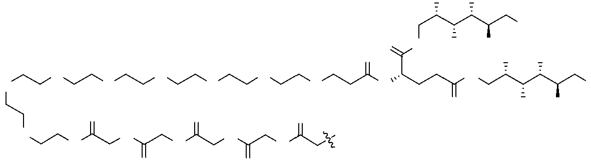

In some embodiments, Y is C₁-C₆ alkylene or -R³-C(O)-, wherein R³ is C₁-C₆ alkylene or -C₁-C₆ alkylene-(OCH₂CH₂)ₒ-, wherein o is an integer selected from 1-12 (e.g., 1, 2, 3, 4, 5, 6, 7, 8, 9, 10, 11, or 12).

In some embodiments, Y is C₁-C₃ alkylene or -R³-C(O)-, wherein R³ is C₁-C₃ alkylene or -C₁-C₃ alkylene-(OCH₂CH₂)ₒ-, wherein o is an integer selected from 1-12 (e.g., 1, 2, 3, 4, 5, 6, 7, 8, 9, 10, 11, or 12).

In some embodiments, Y is methylene, ethylidene, n-propylidene, isopropylidene, or -R³-C(O)-, wherein R³ is methylene-(OCH₂CH₂)ₒ-, ethylidene-(OCH₂CH₂)ₒ-, *n*-propylidene-(OCH₂CH₂)ₒ-, or isopropylidene-(OCH₂CH₂)ₒ-, wherein o is an integer selected from 1-12 (e.g., 1, 2, 3, 4, 5, 6, 7, 8, 9, 10, 11, or 12).

In some embodiments, Y is selected from -CH₂-(OCH₂CH₂)₄C(O)-.

In some embodiments, L₂ is selected from -L₂ₐ-C(O)-, -L₂ₐ-NH-C(O)-L_{2b}-C(O)-, and -L₂ₐ-NR¹-SO₂-NH-C(O)-O-L_{2b}-C(O)-, wherein L₂ₐ is selected from -C₁-C₆ alkylene-, -C₁-C₆ alkylene-C₃-C₆ cycloalkylene-, -C₆-C₁₀ arylene-, -C₆-C₁₀ arylene-C₁-C₆ alkylene-, -(5- to 6-membered heteroarylene)-, -(5- to 6-membered heteroarylene)-C₁-C₆ alkylene-, linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and (linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms)-(3- to 6-membered heterocyclylene), wherein the alkylene, cycloalkylene, arylene, heteroalkylene, heteroarylene, and heterocyclylene are each optionally substituted with one or more substituents independently selected from halogen, C₁-C₃ alkyl, heteroalkyl having 1-3 atoms, C₁-C₃ alkoxy, hydroxyl, amino, carboxyl, and C₃-C₆ cycloalkyl, the heteroalkylene, heterocyclylene, or heteroalkyl contains 1-12 (preferably 1-8, e.g., 1, 2, 3, 4, 5, 6, 7, 8, 9, 10, 11, or 12) heteroatoms, and the heteroatom in the heteroalkylene, heterocyclylene, heteroarylene, or heteroalkyl is selected from one or more of N, O, and S; L_{2b} is selected from - C₁-C₆ alkylene- and linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and R¹ is selected from hydrogen, C₁-C₃ alkyl, C₃-C₆ cycloalkyl, 3- to 6-membered heterocyclyl, C₁-C₃ haloalkyl, heteroalkyl having 2-6 atoms, C₆-C₁₀ aryl, and 5- to 6-membered heteroaryl, wherein the alkyl, heteroalkyl, aryl, heteroaryl, cycloalkyl, and heterocyclyl are each optionally substituted with one or more substituents independently selected from C₁-C₃ alkyl, heteroalkyl having 2-3 atoms, C₁-C₃ alkoxy, amino, and carboxyl, the heteroalkyl or heteroalkylene contains 1-12 (preferably 1-8, e.g., 1, 2, 3, 4, 5, 6, 7, 8, 9, 10, 11, or 12) heteroatoms, and the heteroatom in the heteroalkyl, heterocyclyl, heteroaryl, or heteroalkylene is selected from one or more of N, O, and S.

In some embodiments, L₂ is selected from -L₂ₐ-C(O)-, -L₂ₐ-NH-C(O)-L_{2b}-C(O)-, and -L₂ₐ-NR¹-SO₂-NH-C(O)-O-L_{2b}-C(O)-, wherein L₂ₐ is selected from methylene, ethylidene, *n*-propylidene, *n*-butylidene, *n*-pentylidene, *n-*hexylidene, -CH(CH₃)CH₂CH₂CH₂CH₂-, -C₁-C₃ alkylene-cyclohexylidene-, -phenylene-, -phenylene-C₁-C₃ alkylene-, -(5- to 6-membered heteroarylene)-C₁-C₃ alkylene-, linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and (linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms)-(3- to 6-membered heterocyclylene), wherein the alkylene, cycloalkylene, arylene, heteroalkylene, heteroarylene, and heterocyclylene are each optionally substituted with one or more substituents independently selected from halogen, methyl, ethyl, *n*-propyl, isopropyl, heteroalkyl having 1-3 atoms, methoxy, ethoxy, *n*-propoxy, isopropoxy, hydroxyl, amino, carboxyl or cyclopropyl, cyclobutyl, cyclopentyl, and cyclohexyl, the heteroalkylene, heterocyclylene, or heteroalkyl contains 1-12 (preferably 1-8, e.g., 1, 2, 3, 4, 5, 6, 7, 8, 9, 10, 11, or 12) heteroatoms, and the heteroatom in the heteroalkylene, heterocyclylene, heteroarylene, or heteroalkyl is selected from one or more of N, O, and S; L_{2b} is selected from methylene, ethylidene, *n*-propylidene, *n*-butylidene, *n*-pentylidene, *n-*hexylidene, -CH(CH₃)CH₂CH₂CH₂CH₂-, and linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and R¹ is selected from hydrogen, methyl, ethyl, *n*-propyl, isopropyl, cyclopropyl, cyclobutyl, cyclopentyl, cyclohexyl, 3- to 6-membered heterocyclyl, -CF₃, -CF₂CF₃, heteroalkyl having 2-3 atoms, phenyl, and 5- to 6-membered heteroaryl, wherein the alkyl, heteroalkyl, aryl, heteroaryl, cycloalkyl, and heterocyclyl are each optionally substituted with one or more substituents independently selected from methyl, ethyl, n-propyl, isopropyl, heteroalkyl having 2-3 atoms, methoxy, ethoxy, *n*-propoxy, isopropoxy, amino, and carboxyl, the heteroalkyl or heteroalkylene contains 1-12 (preferably 1-8, e.g., 1, 2, 3, 4, 5, 6, 7, 8, 9, 10, 11, or 12) heteroatoms, and the heteroatom in the heteroalkyl, heterocyclyl, heteroaryl, or heteroalkylene is selected from one or more of N, O, and S.

In some embodiments, L₂ₐ is selected from -C₁-C₈ alkylene-, -C₁-C₈ alkylene-C₃-C₈ cycloalkylene, C₆-C₁₄ arylene-C₁-C₈ alkylene, and linear or branched heteroalkylene having 1-50 (preferably 1-20, more preferably 1-12, and most preferably 1-8) atoms, wherein the alkylene, cycloalkylene, arylene, and heteroalkylene are each optionally substituted with one or more substituents independently selected from C₁-C₆ alkyl, heteroalkyl having 2-6 atoms, C₁-C₆ alkoxy, hydroxyl, amino, carboxyl, and C₃-C₈ cycloalkyl, the heteroalkylene or heteroalkyl contains 1-12 (preferably 1-8, e.g., 1, 2, 3, 4, 5, 6, 7, 8, 9, 10, 11, or 12) heteroatoms, and the heteroatom in the heteroalkyl, heterocyclyl, heteroaryl, heteroalkylene, or heteroarylene is selected from one or more of N, O, and S; L_{2b} is selected from -C₁-C₈ alkylene- and linear or branched heteroalkylene having 1-50 (preferably 1-20, more preferably 1-12, and most preferably 1-8) atoms, and R¹ is selected from hydrogen, C₁-C₆ alkyl, C₂-C₈ heteroalkyl, and C₆-C₁₄ aryl, wherein the alkyl, heteroalkyl, and aryl are each optionally substituted with one or more substituents independently selected from C₁-C₆ alkyl, heteroalkyl having 2-6 atoms, C₁-C₆ alkoxy, amino, and carboxyl, the heteroalkyl contains 1-12 (preferably 1-8, e.g., 1, 2, 3, 4, 5, 6, 7, 8, 9, 10, 11, or 12) heteroatoms, and the heteroatom in the heteroalkyl, heterocyclyl, heteroaryl, or heteroalkylene is selected from one or more of N, O, and S. In some embodiments, L₂ₐ is selected from -CH₂CH₂CH₂CH₂CH₂-, -CH₂CH₂(OCH₂CH₂)ₚ-, -(OCH₂CH₂)ₚ-, - CH₂(OCH₂CH₂)ₚ-, -CH₂CH₂(OCH₂CH₂)ₚCH₂-, -CH₂CH₂(OCH₂CH₂)ₚCH₂CH₂-, -CH₂(OCH₂CH₂)ₚCH₂CH₂-, 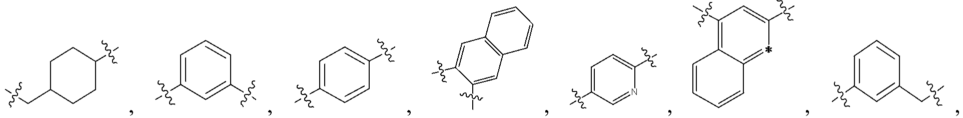 and 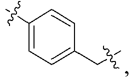 wherein p is an integer from 1 to 12 (preferably 2-8, e.g., 1, 2, 3, 4, 5, 6, 7, 8, 9, 10, 11, or 12); L_{2b} is selected from -CH₂OCH₂-, -CH₂CH₂OCH₂-, -CH₂CH₂OCH₂CH₂-, -CH₂O-, -CH₂CH₂O-, -CH₂-, -CH₂CH₂-, - CH₂CH₂CH₂-, -CH₂CH₂CH₂CH₂-, -CH₂CH₂CH₂CH₂CH₂-, -(OCH₂CH₂)_{q}-, -CH₂CH₂(OCH₂CH₂)_{q}-, - CH₂(OCH₂CH₂)_{q}-, -CH₂CH₂(OCH₂CH₂)_{q}CH₂-, -CH₂CH₂(OCH₂CH₂)_{q}CH₂CH₂-, and -CH₂(OCH₂CH₂)_{q}CH₂CH₂-, and R¹ is selected from hydrogen, methyl, ethyl, n-propyl, isopropyl, and cyclopropyl, wherein q is an integer from 1 to 12 (preferably 2-8, e.g., 1, 2, 3, 4, 5, 6, 7, 8, 9, 10, 11, or 12).

In some embodiments, L₂ is selected from the following structures:

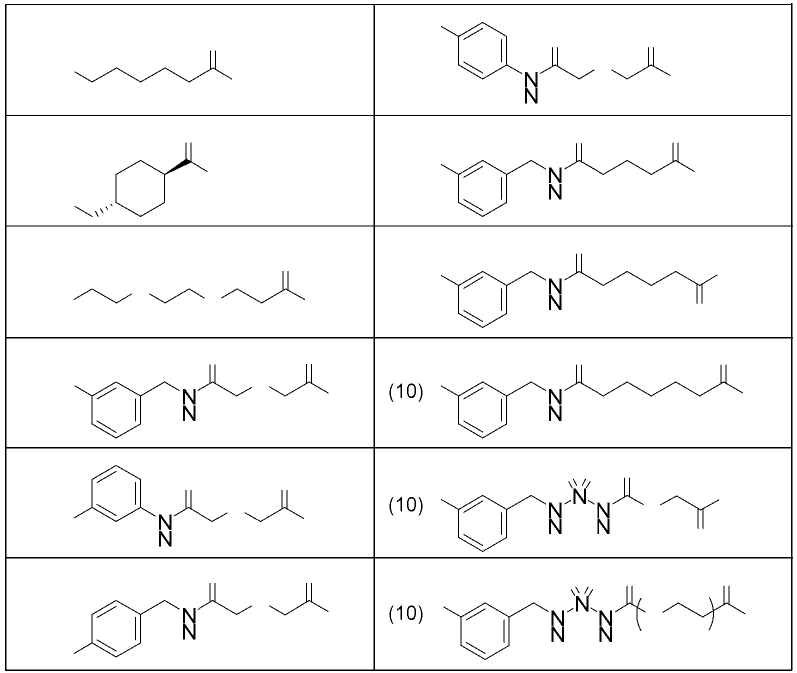

wherein w is an integer selected from 1-12 (e.g., 1, 2, 3, 4, 5, 6, 7, 8, 9, 10, 11, or 12), * represents linkage to L₁, and ** represents linkage to L₃.

In some embodiments, L₃ is selected from a peptide residue formed from 2-8 amino acids selected from phenylalanine (F), glycine (G), valine (V), citrulline (C), glutamic acid (E), alanine (A), lysine (K), C₁-C₆ alkyl-substituted lysine (e.g., C₁-C₃ alkyl-substituted lysine, such as methyl-substituted lysine, ethyl-substituted lysine, *n-*propyl-substituted lysine, or isopropyl-substituted lysine), etc.

In some embodiments, L₃ is selected from the following structures: 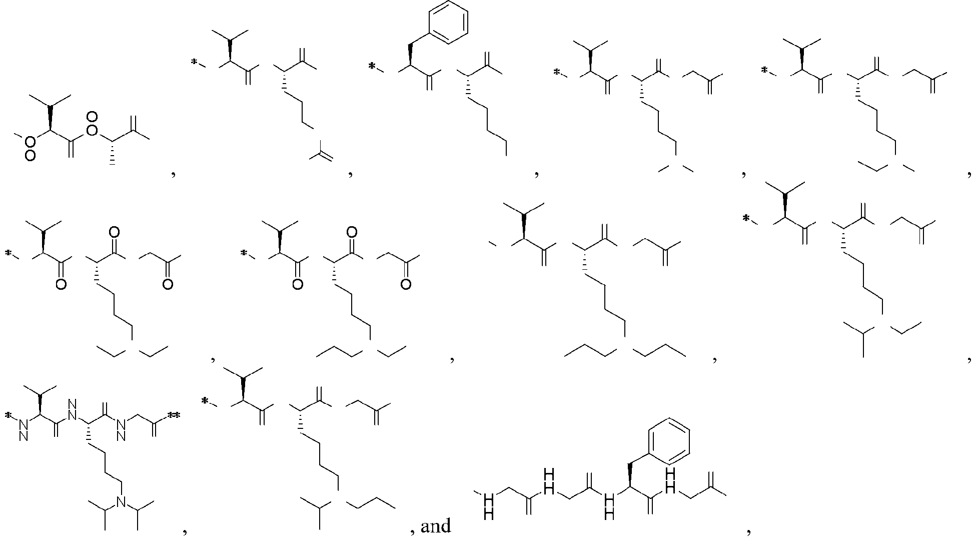 wherein * represents linkage to L₂, and ** represents linkage to L₄.

In some embodiments, L₄ is selected from: 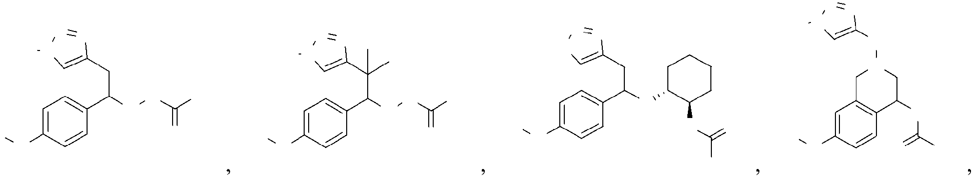 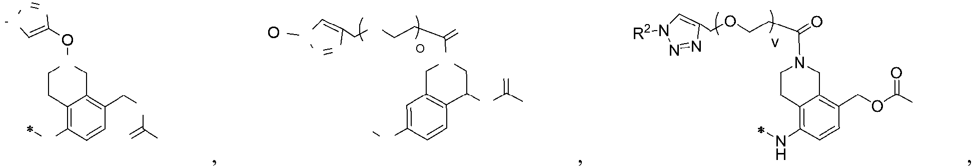 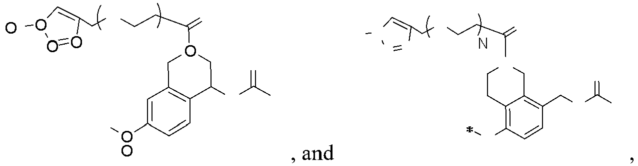 wherein v is an integer selected from 1-12 (e.g., 1, 2, 3, 4, 5, 6, 7, 8, 9, 10, 11, or 12), * represents linkage to a carboxyl group of L₃ via an amide bond, and ** represents linkage to the amino group of the bioactive molecule D. In some embodiments, L₄ is selected from: 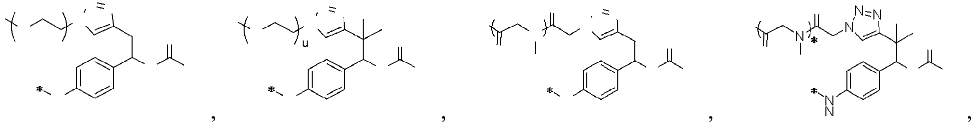 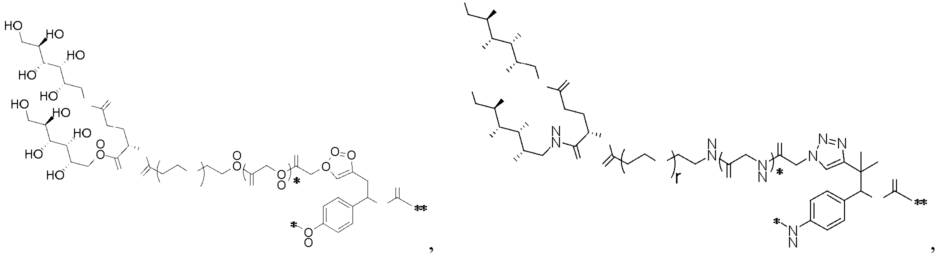 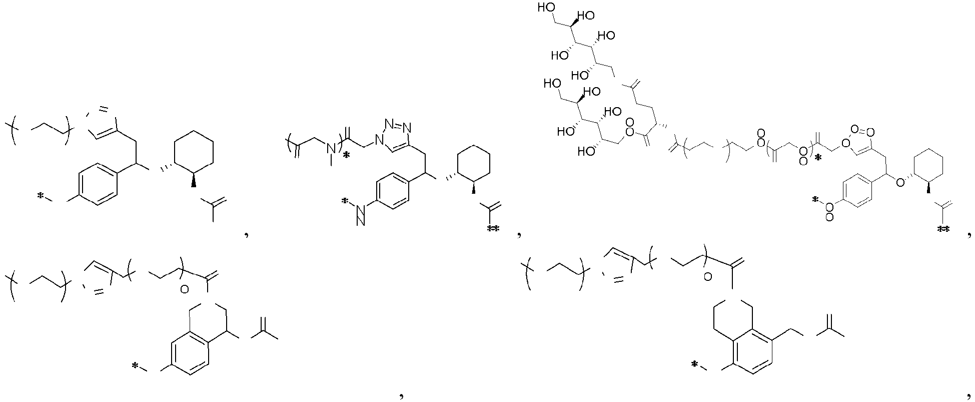 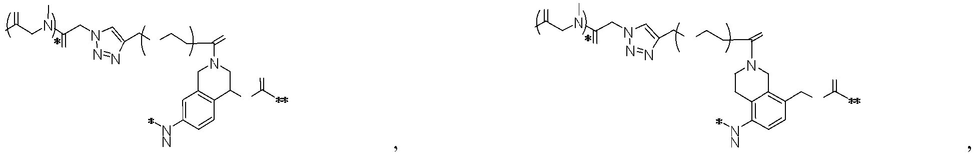 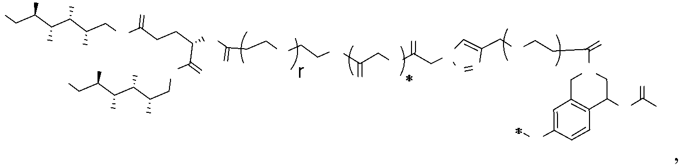 and 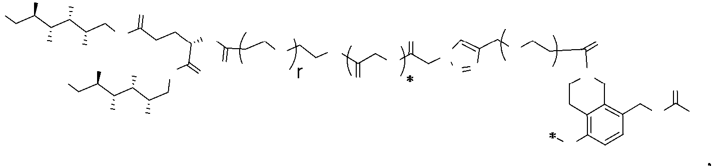 wherein r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50), v is an integer selected from 1-12 (e.g., 1, 2, 3, 4, 5, 6, 7, 8, 9, 10, 11, or 12), * represents linkage to a carboxyl group of L₃ via an amide bond, and ** represents linkage to the amino group of the bioactive molecule D.

In some embodiments, L' is selected from the following structures: 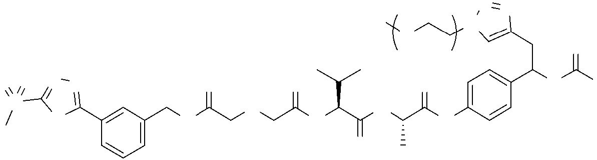 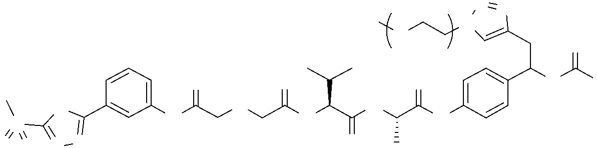 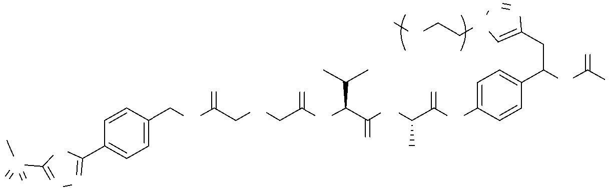 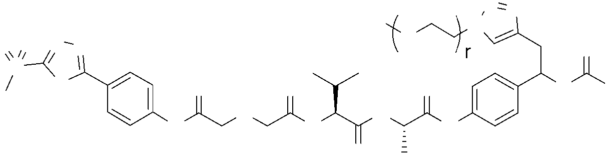 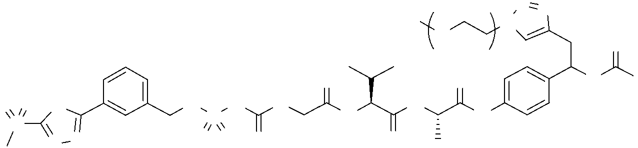 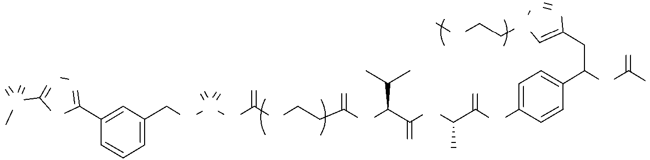 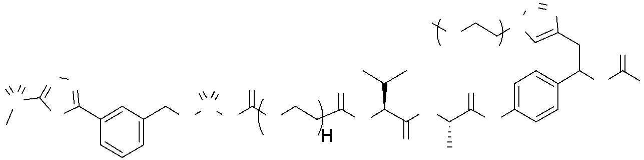 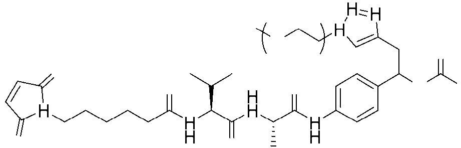 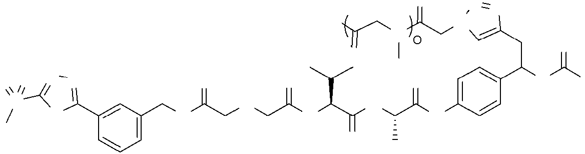 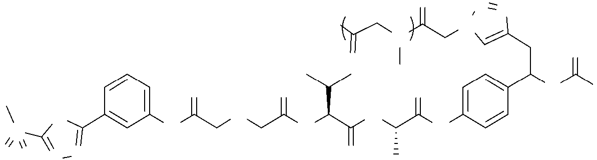 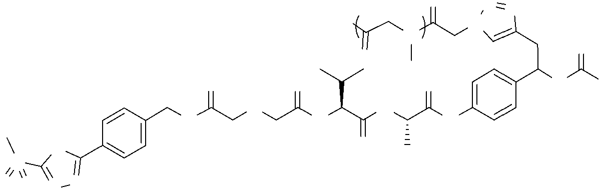 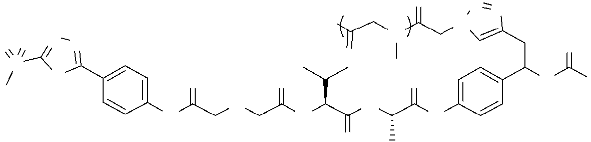 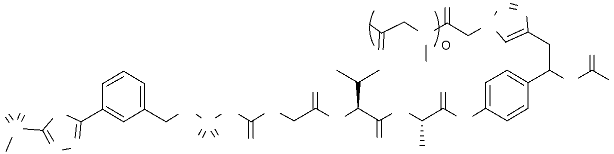 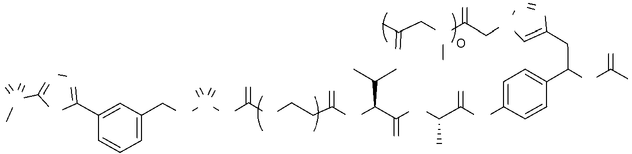 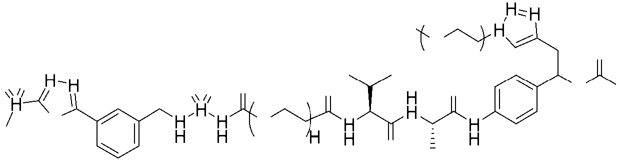 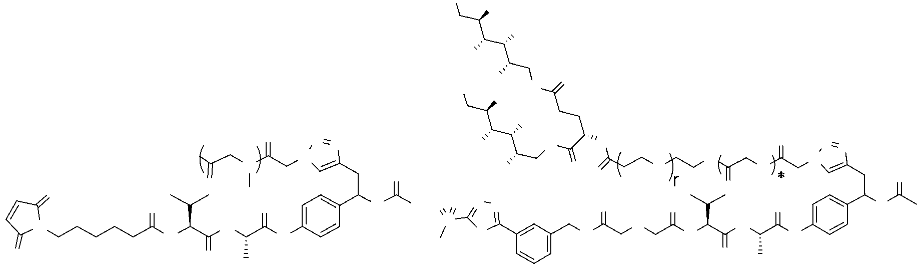 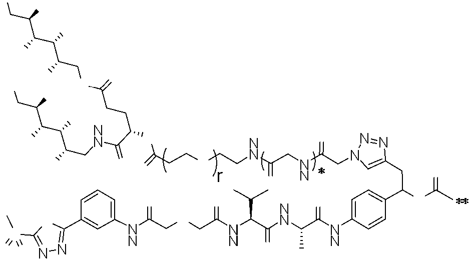 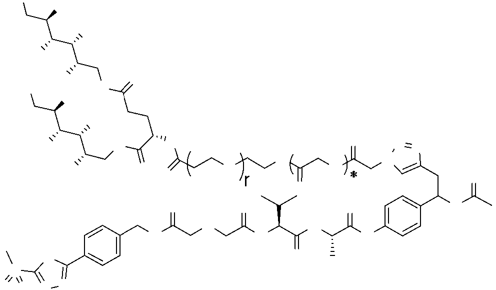 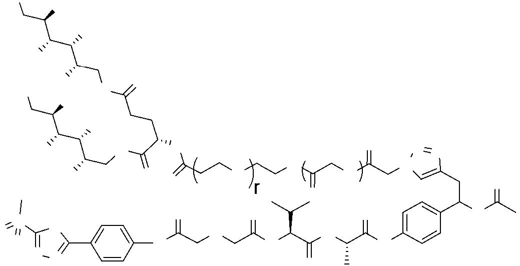 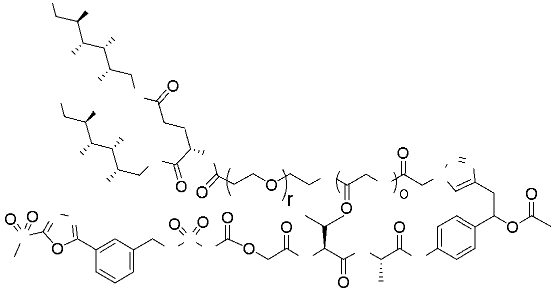 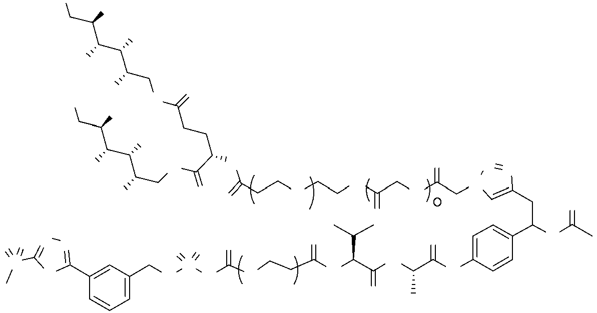 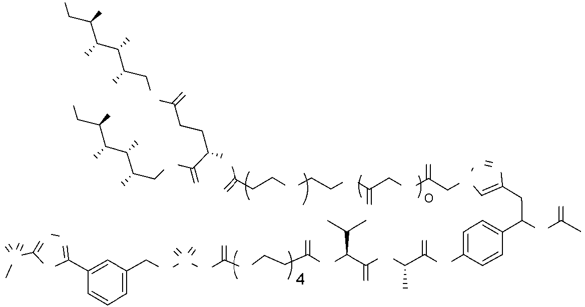 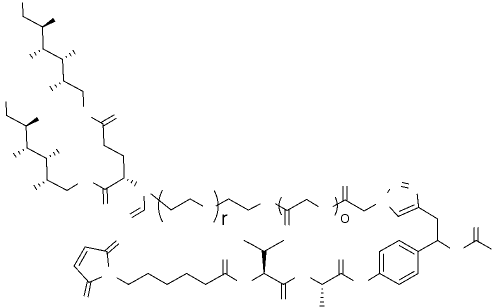 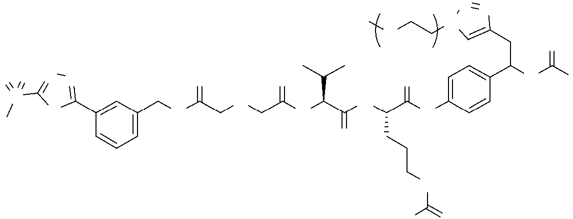 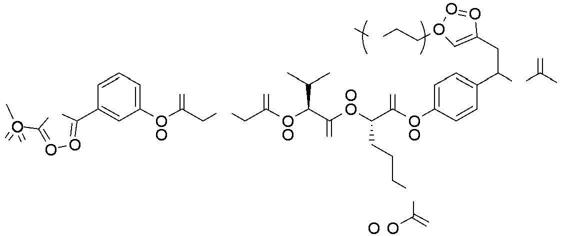 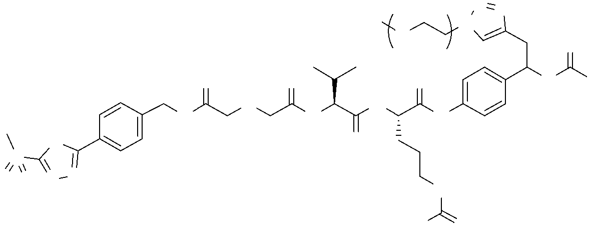 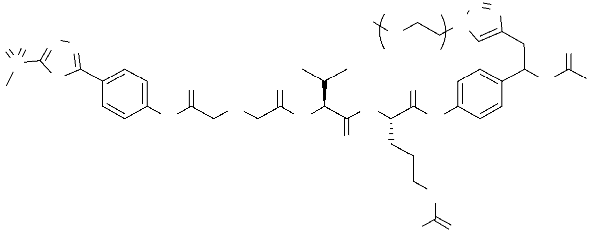 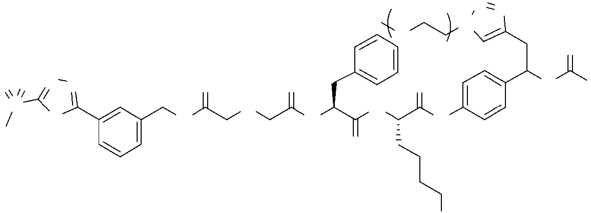 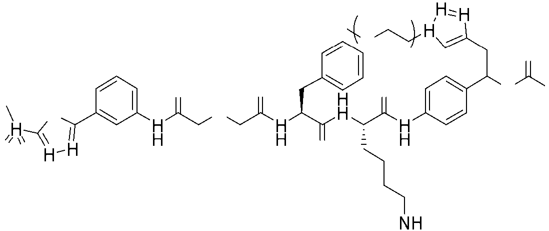 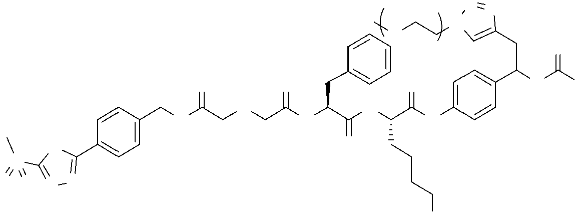 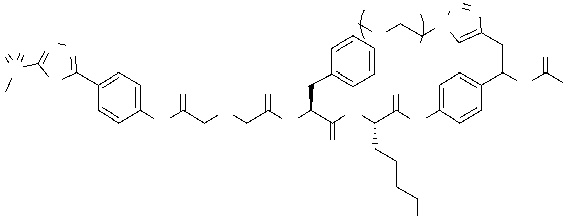 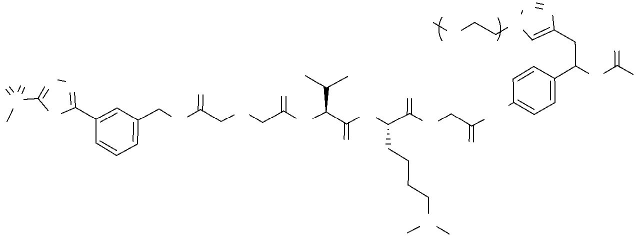 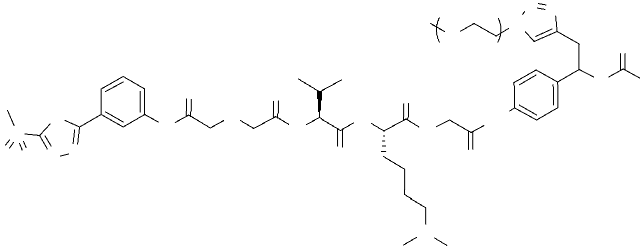 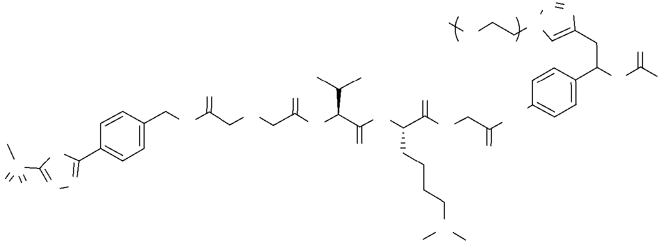 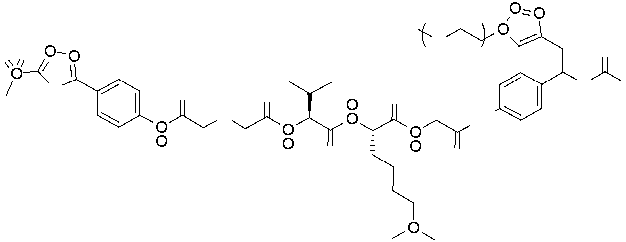 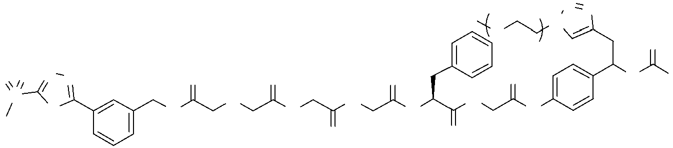 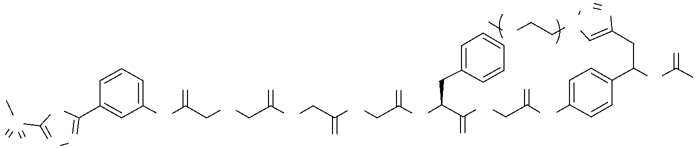 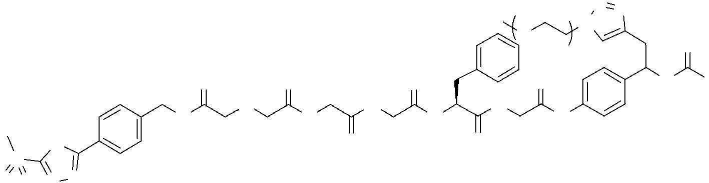 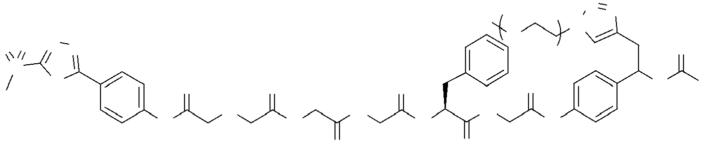 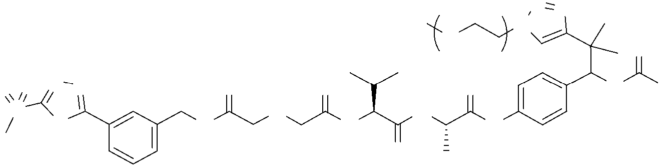 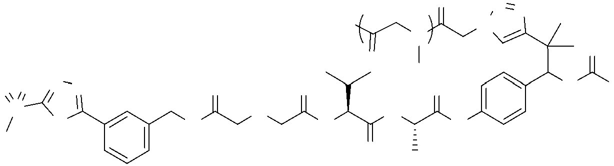 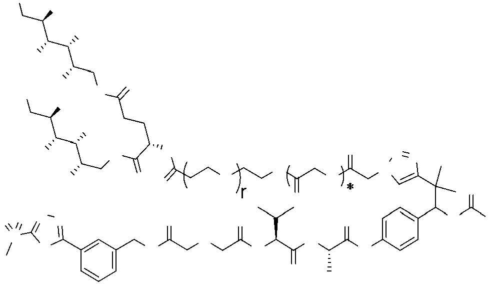 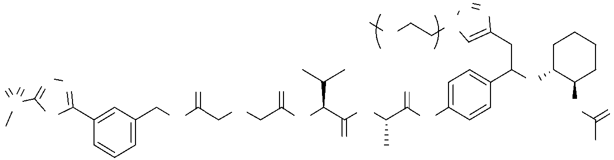 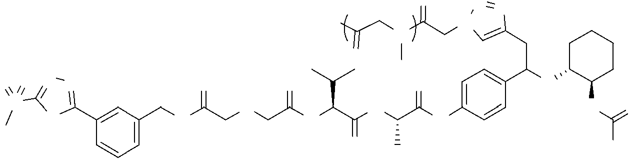 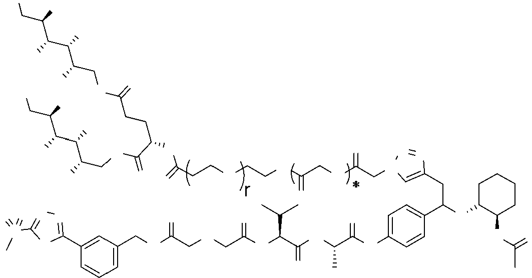 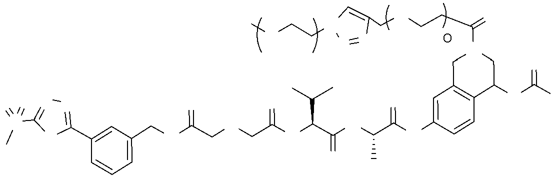 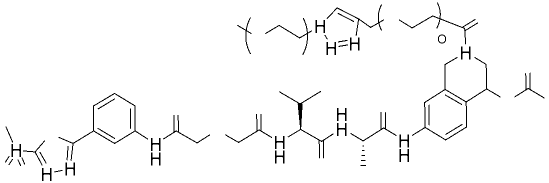 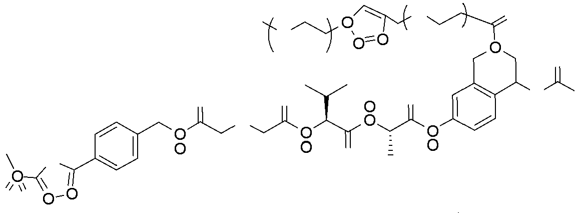 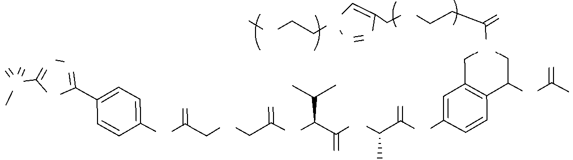 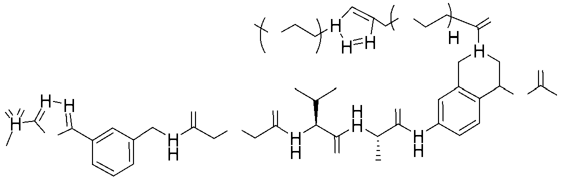 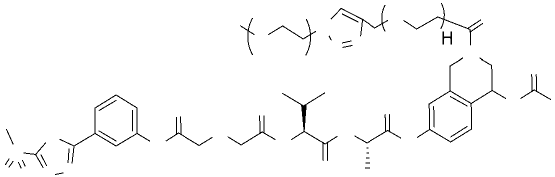 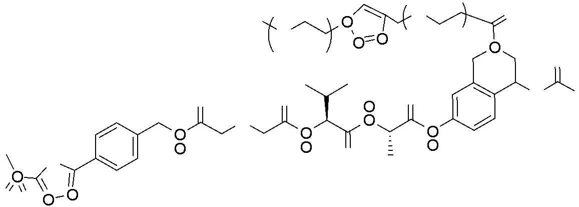 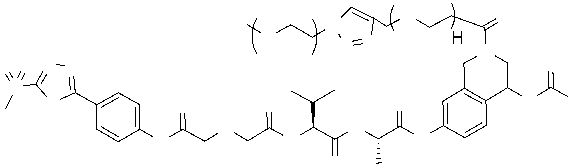 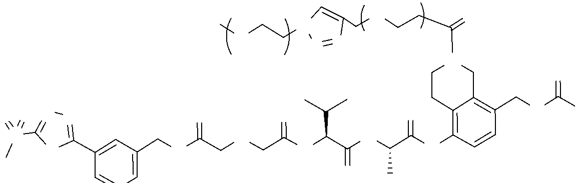 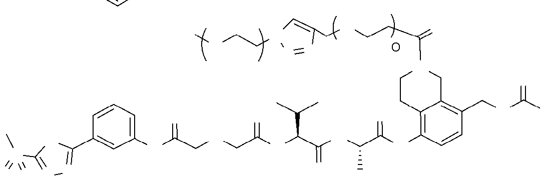 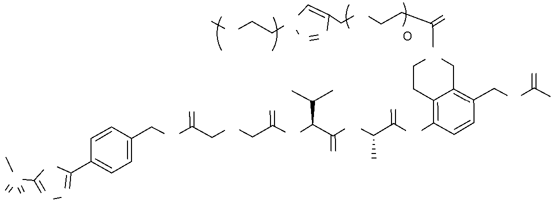 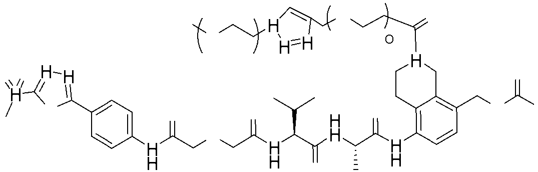 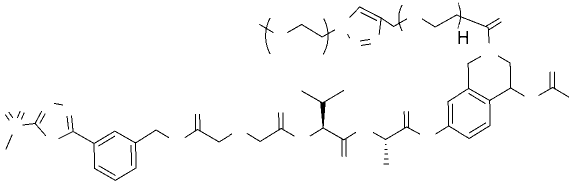 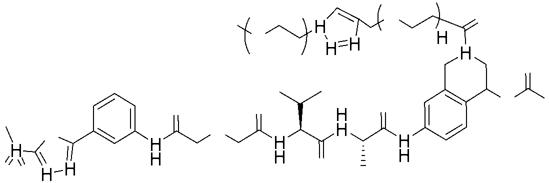 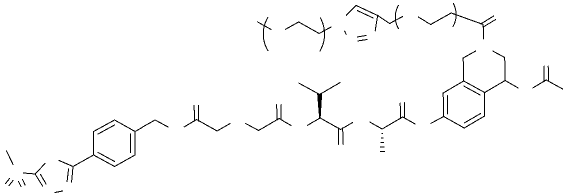 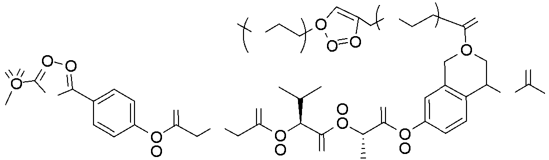 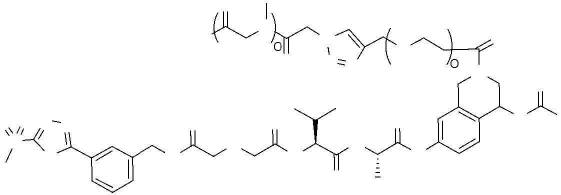 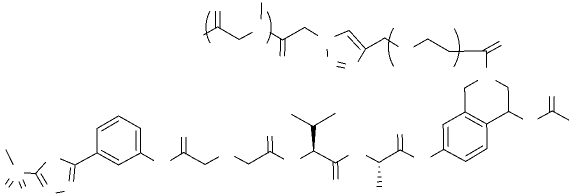 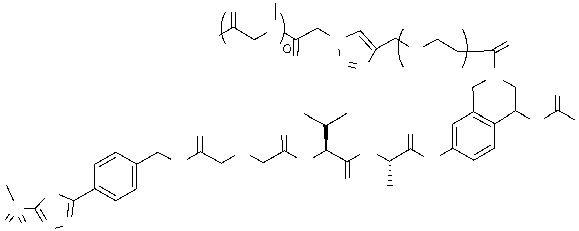 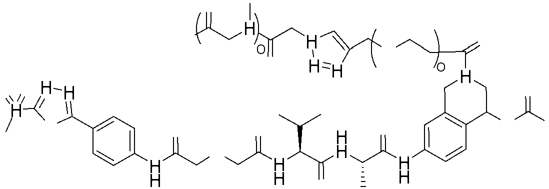 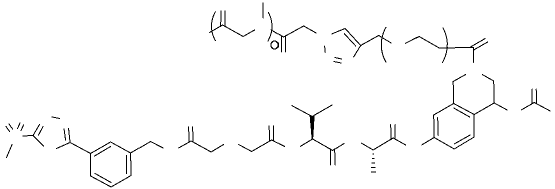 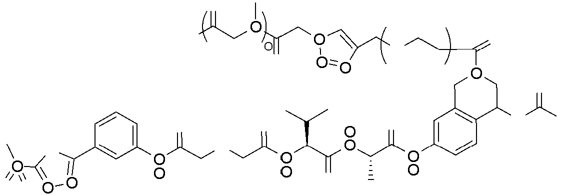 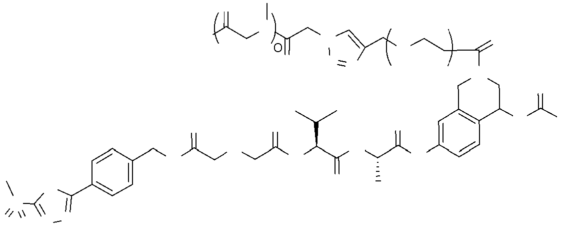 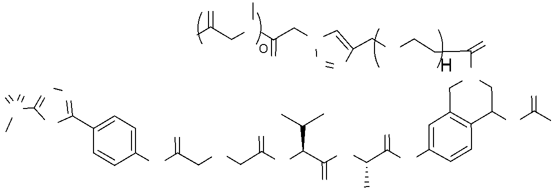 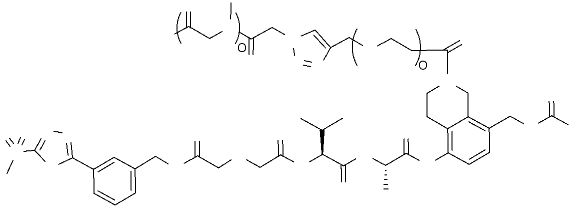 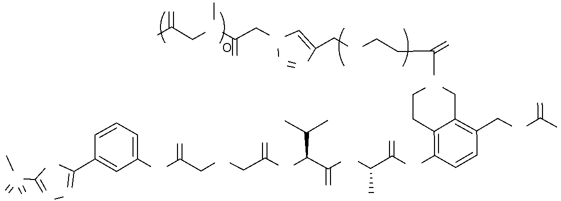 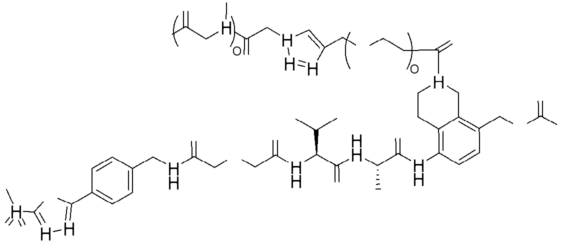 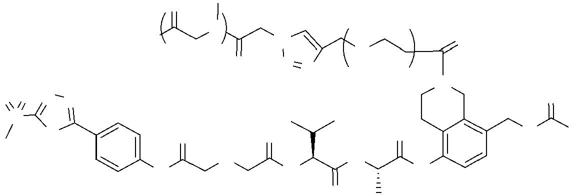 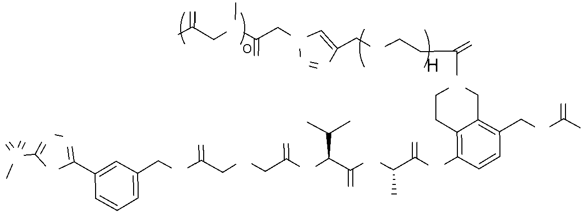 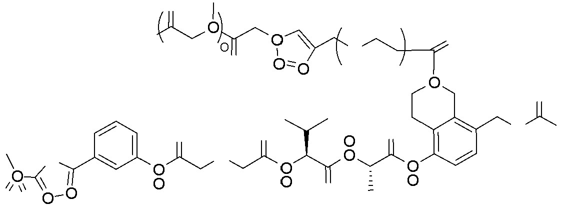 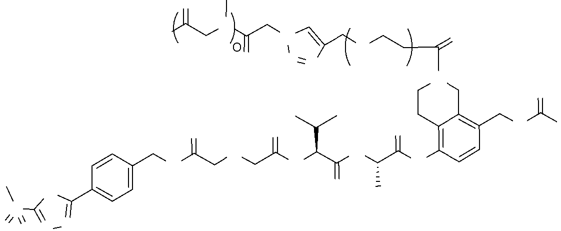 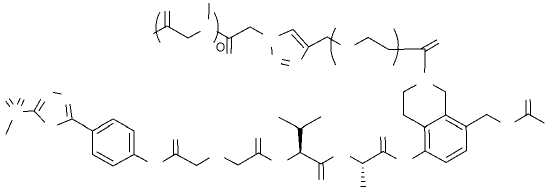 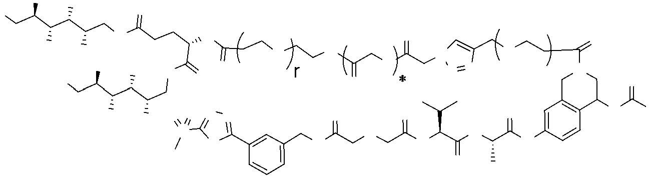 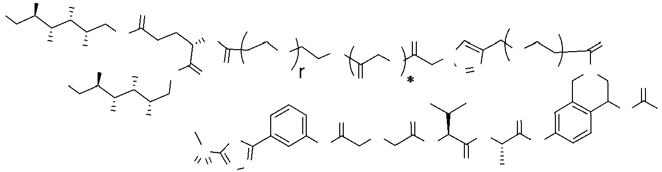 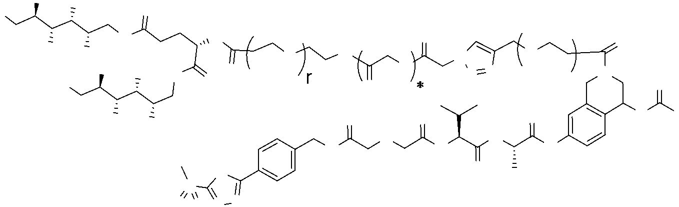 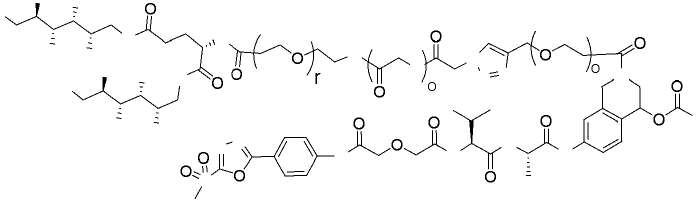 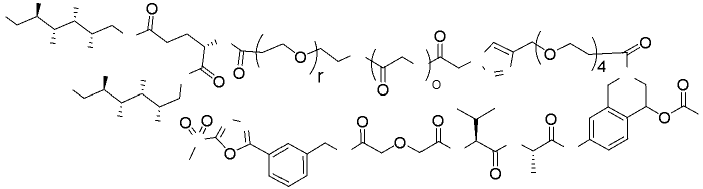 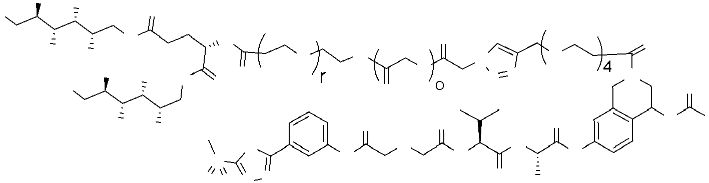 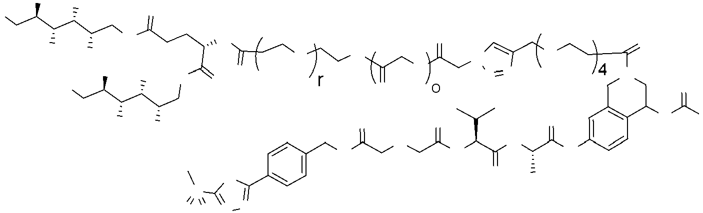 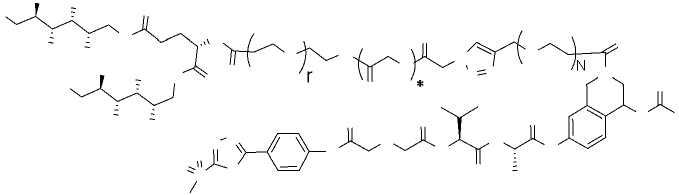 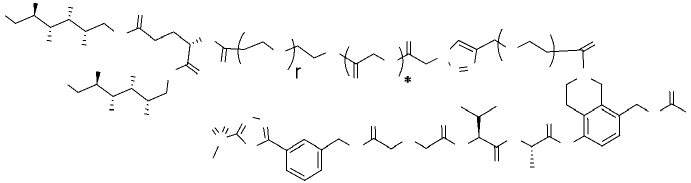 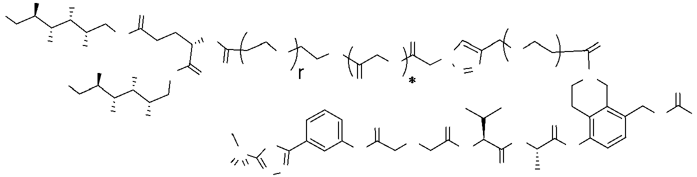 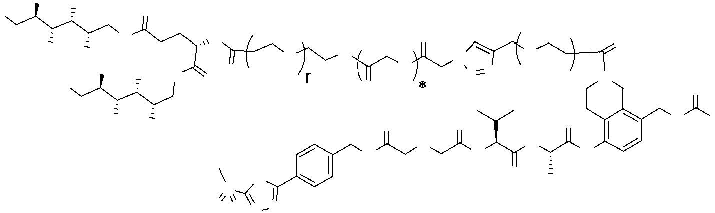 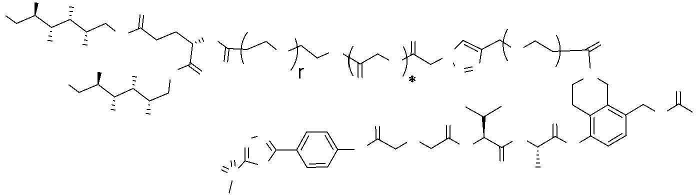 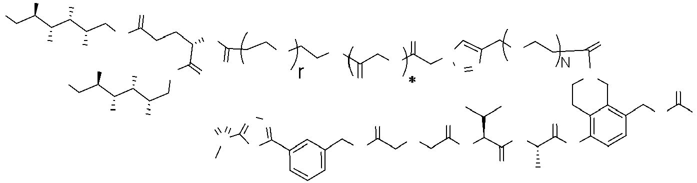 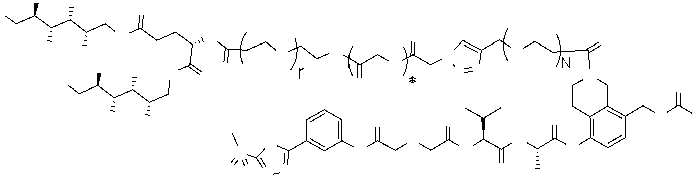 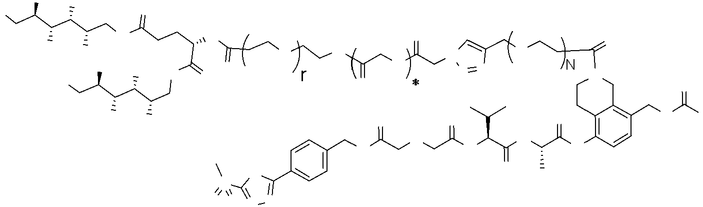 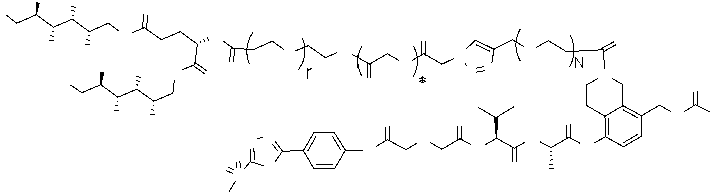 wherein v and w are each independently an integer selected from 1-12 (e.g., 1, 2, 3, 4, 5, 6, 7, 8, 9, 10, 11, or 12), and ** represents linkage to the amino group of the bioactive molecule D; r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50).

In some embodiments, L' is selected from the following structures: 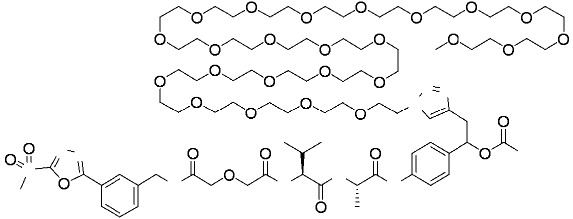 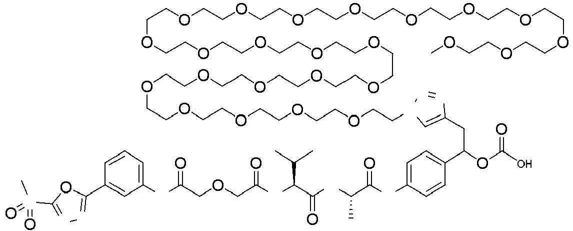 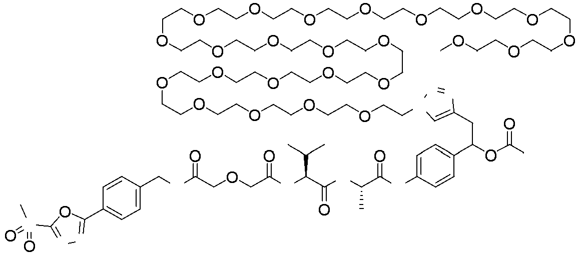 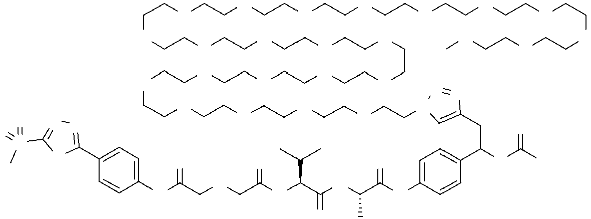 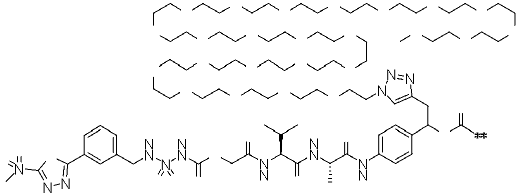 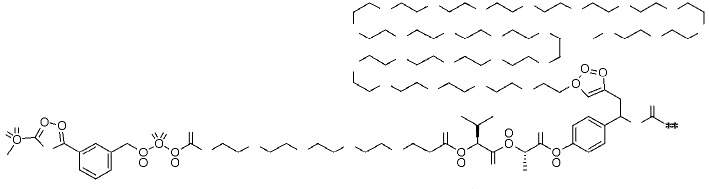 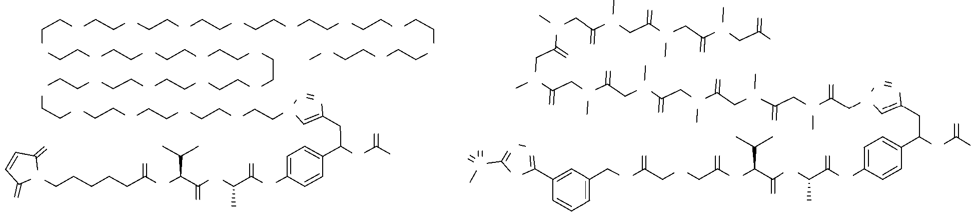 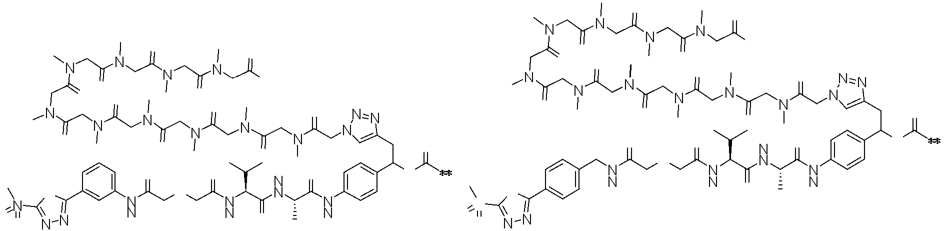 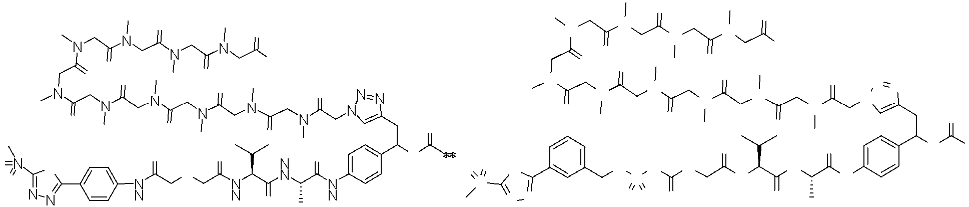 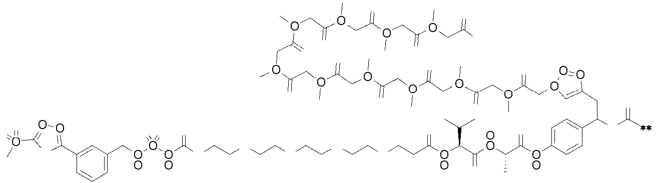 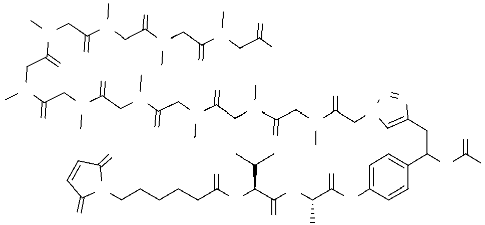 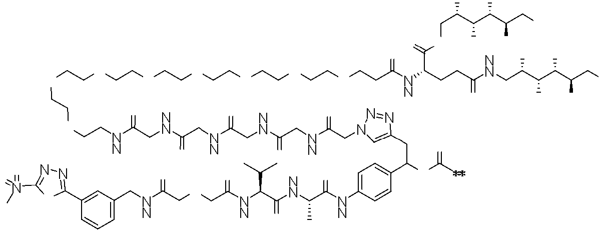 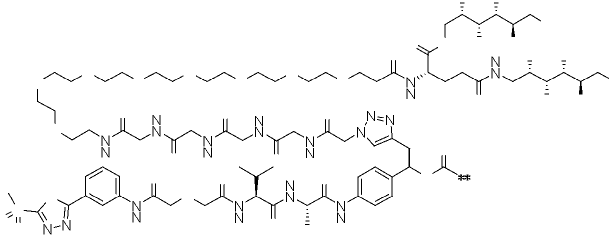 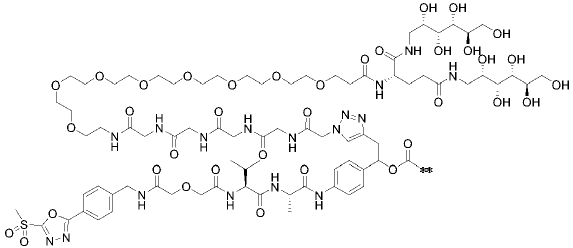 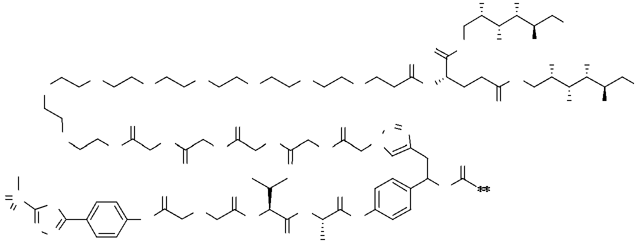 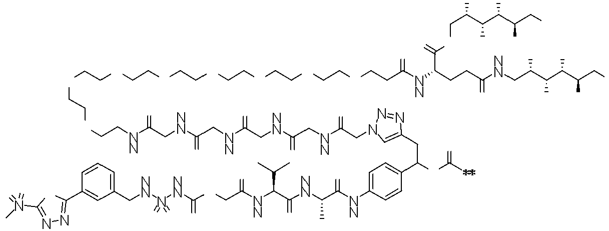 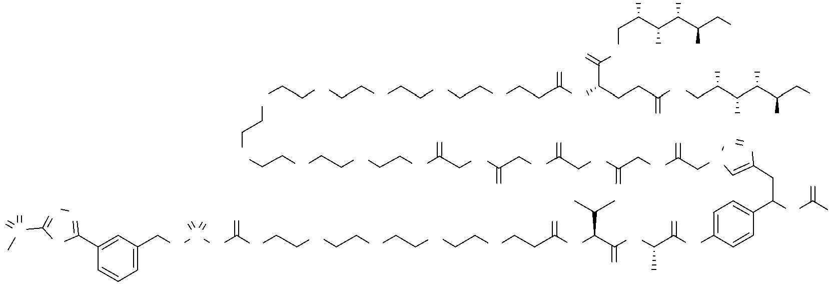 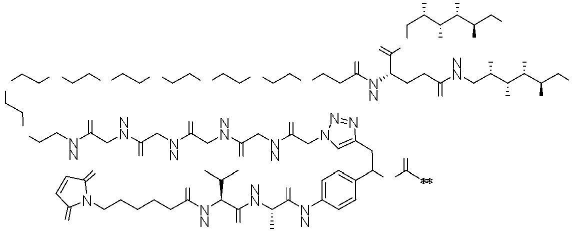 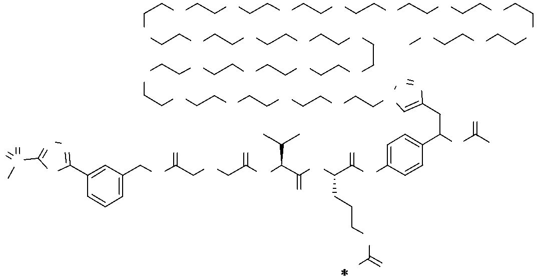 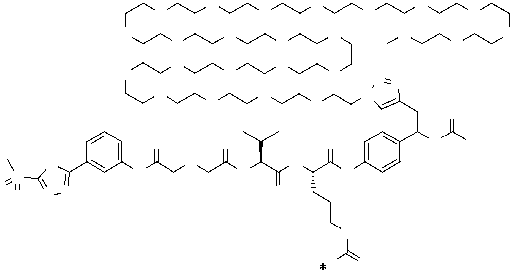 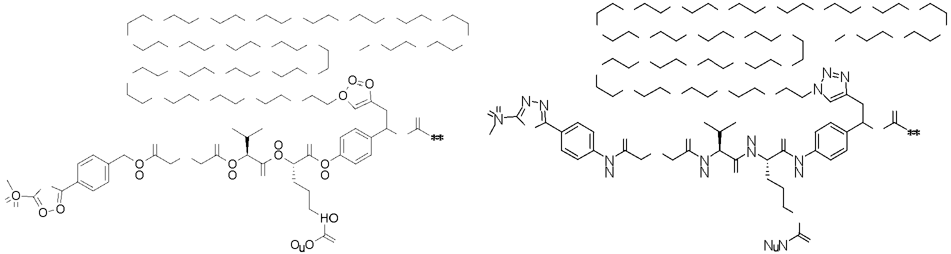 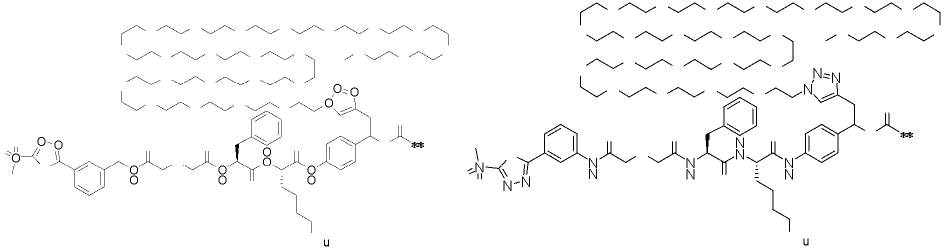 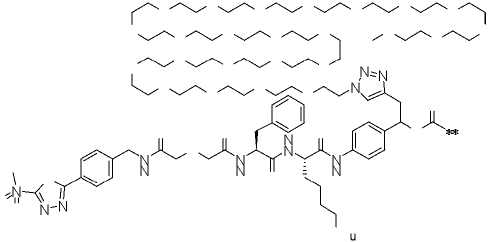 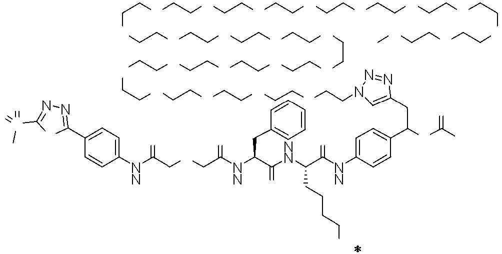 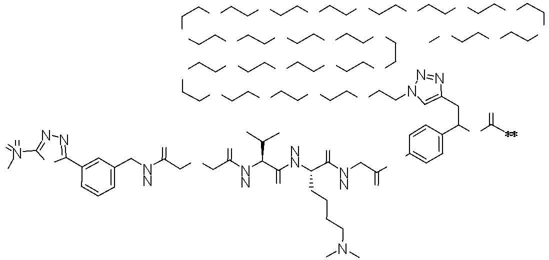 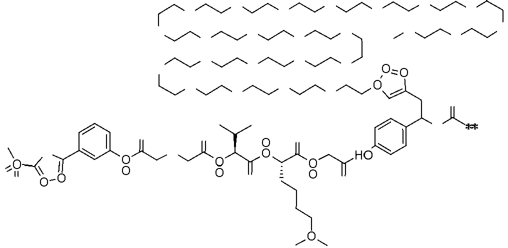 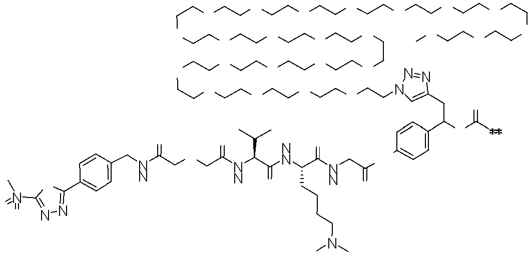 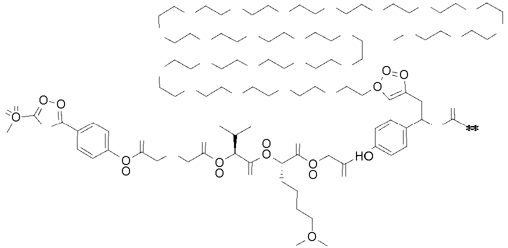 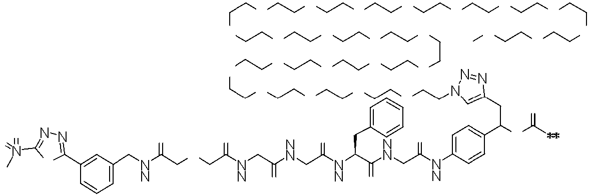 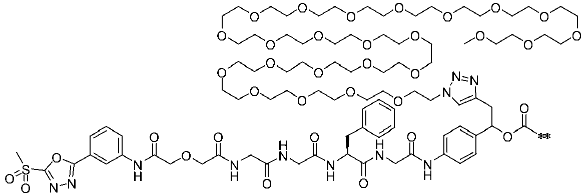 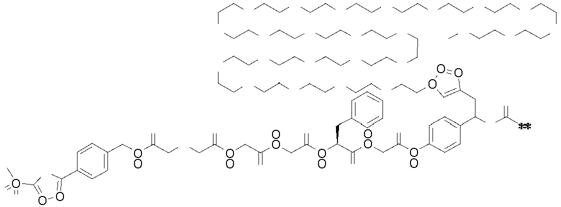 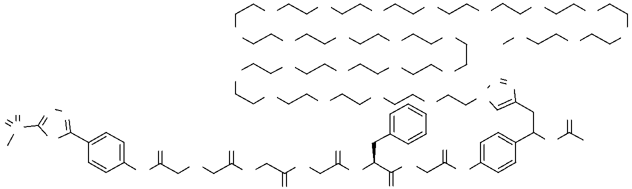 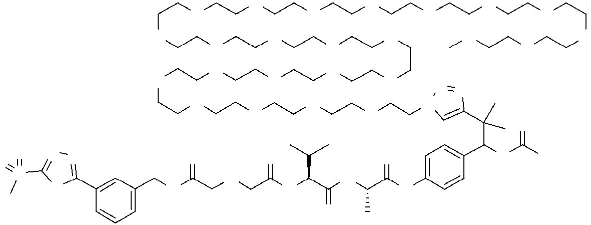 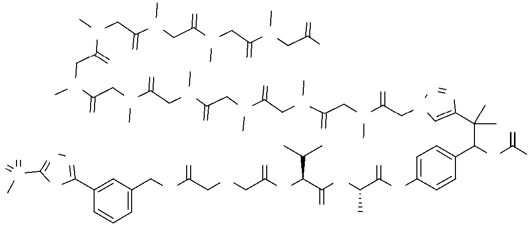 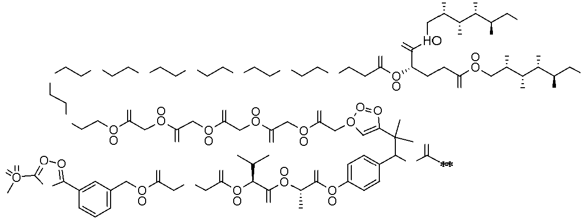 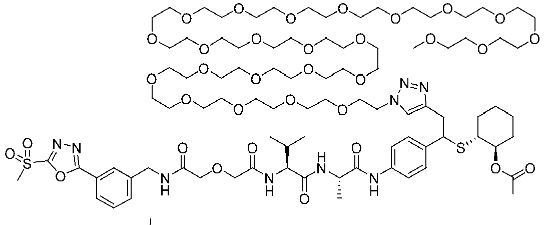 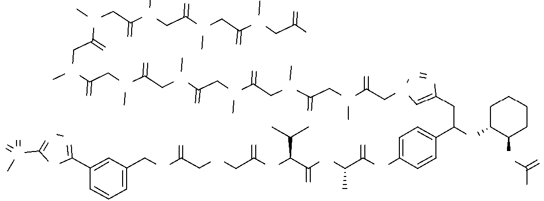 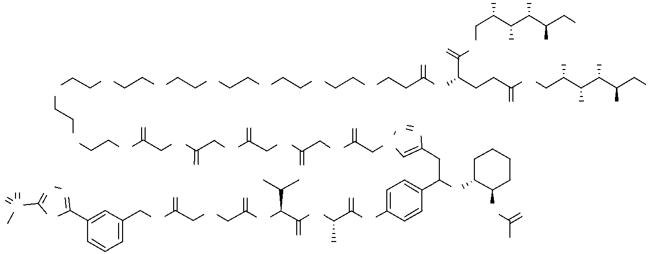 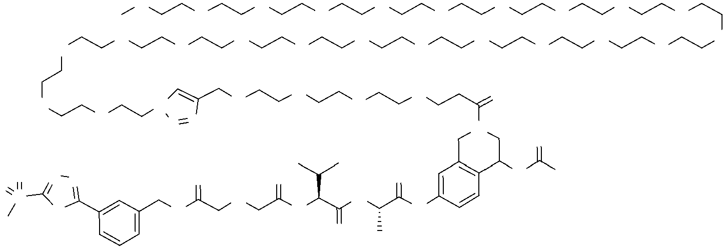 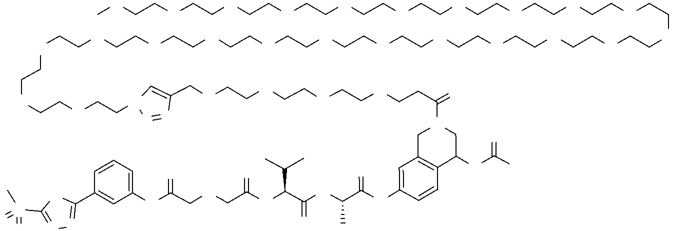 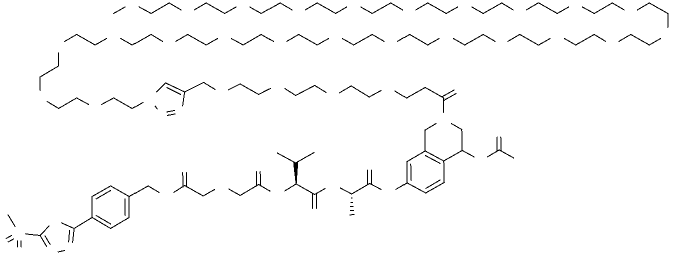 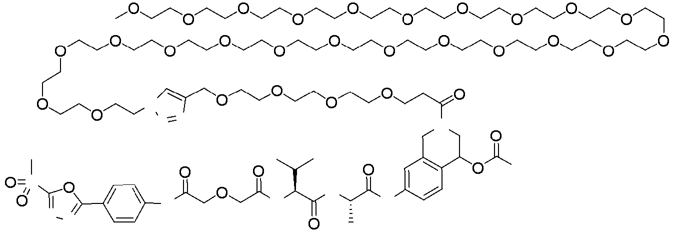 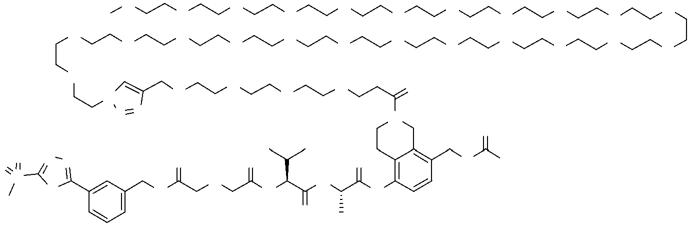 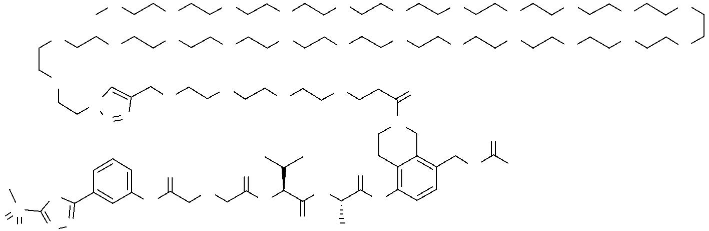 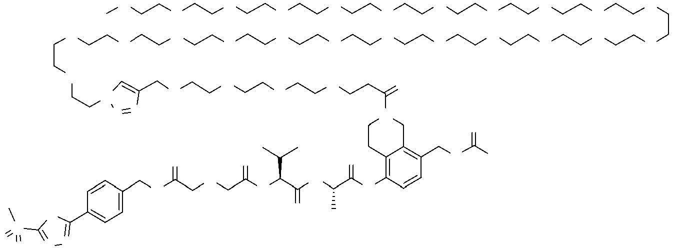 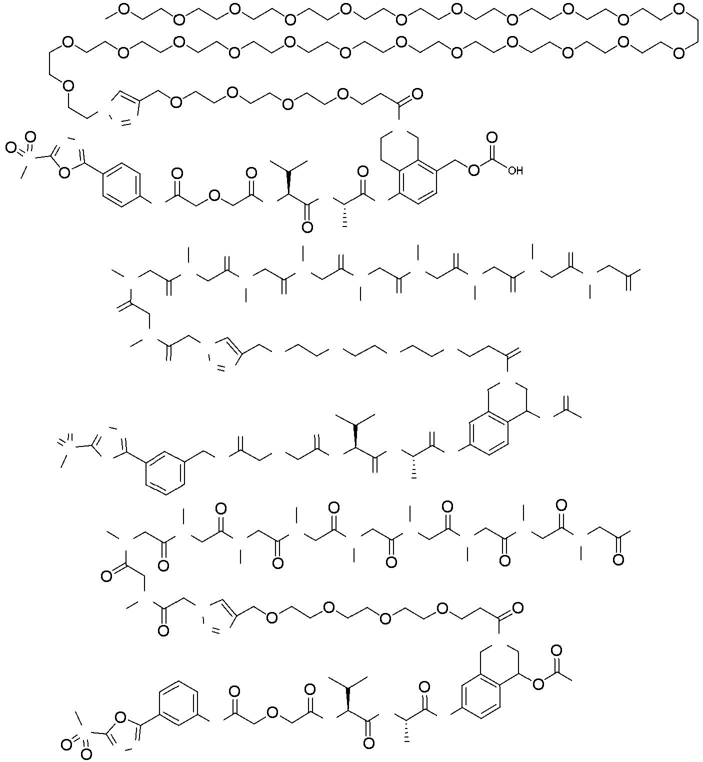 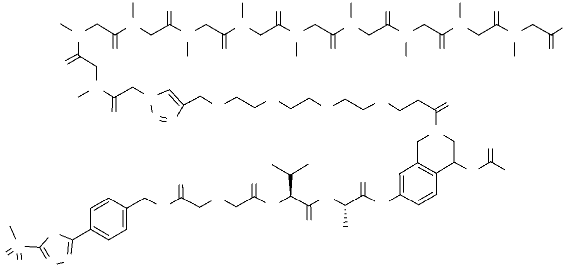 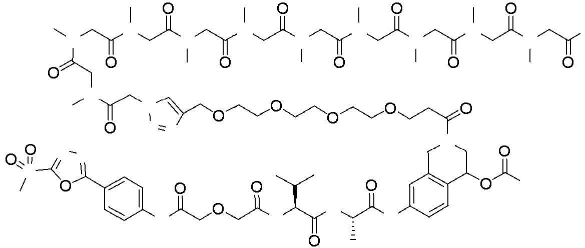 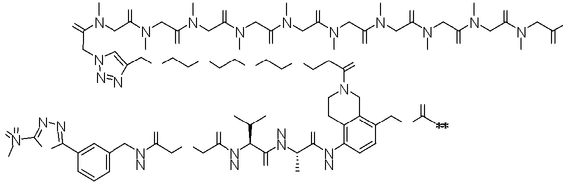 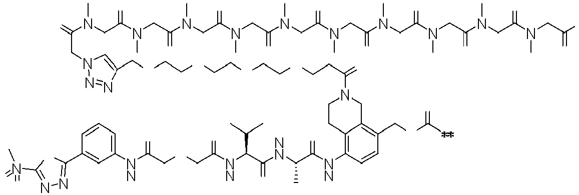 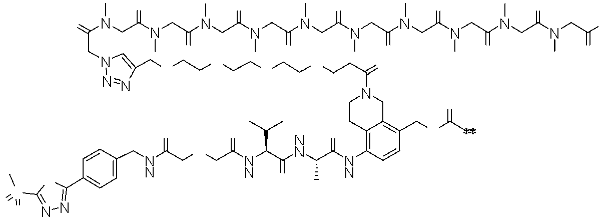 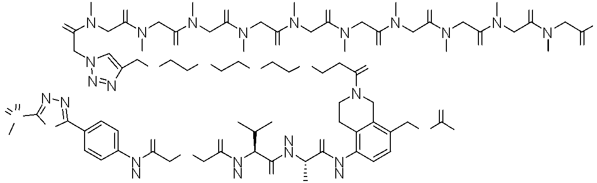 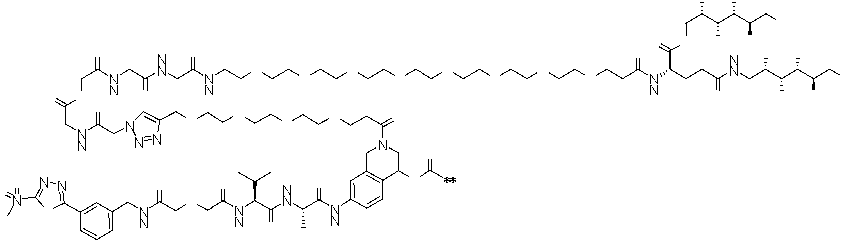 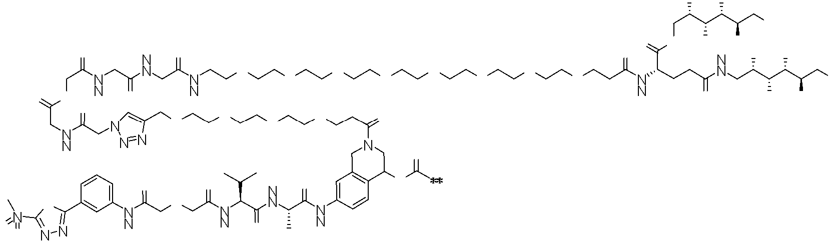 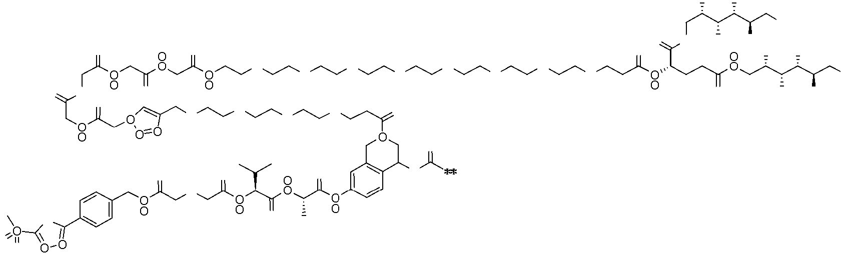 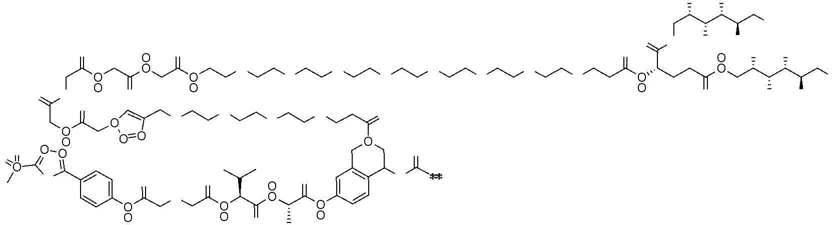 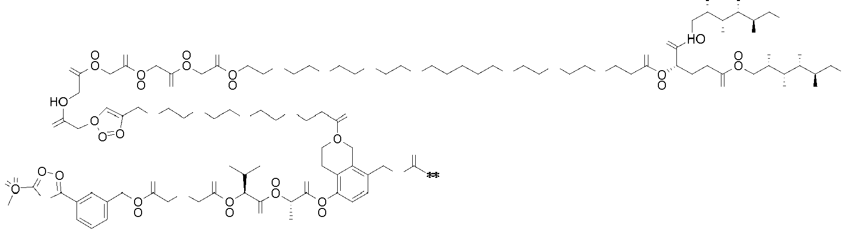 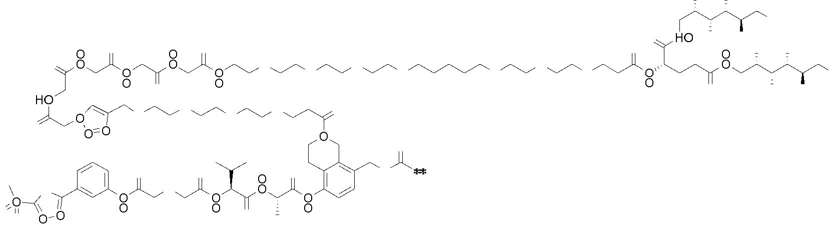 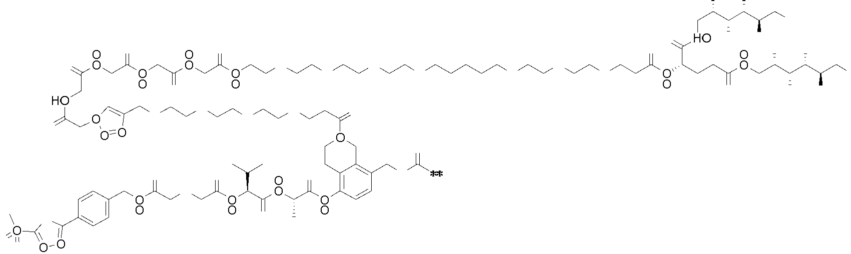 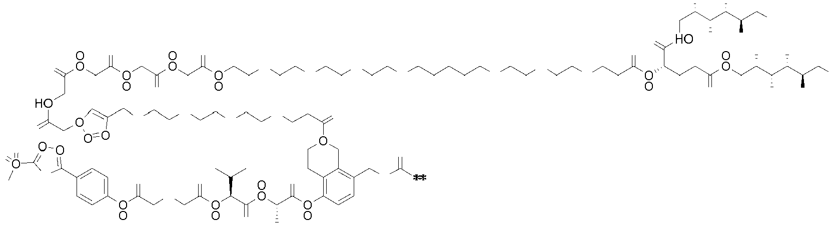 wherein ** represents linkage to the amino group of the bioactive molecule D.

In some embodiments, in the linker-drug compound represented by formula (II), or the tautomer, the mesomer, the racemate, the enantiomer or the diastereoisomer thereof, and the pharmaceutically acceptable salt, the hydrate, the solvate or the isotopically labeled compound thereof, the bioactive molecule D is selected from a camptothecin derivative, eribulin, and a molecular glue.

In some embodiments, in the linker-drug compound represented by formula (II), or the tautomer, the mesomer, the racemate, the enantiomer or the diastereoisomer thereof, and the pharmaceutically acceptable salt, the hydrate, the solvate or the isotopically labeled compound thereof, the bioactive molecule D is selected from formulas (A-1) to (A-6) according to the first aspect.

In some embodiments, in the linker-drug compound represented by formula (II), or the tautomer, the mesomer, the racemate, the enantiomer or the diastereoisomer thereof, and the pharmaceutically acceptable salt, the hydrate, the solvate or the isotopically labeled compound thereof, the bioactive molecule D is selected from compounds of the following formulas:

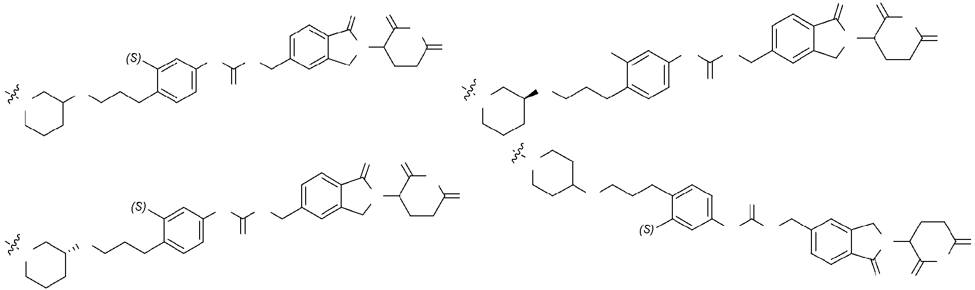

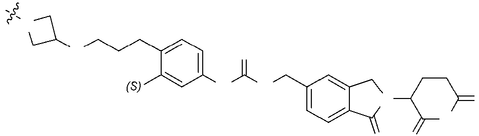

In some embodiments, the linker-drug compound represented by formula (II), or the tautomer, the mesomer, the racemate, the enantiomer or the diastereoisomer thereof, and the pharmaceutically acceptable salt, the hydrate, the solvate or the isotopically labeled compound thereof have a structure shown as follows:

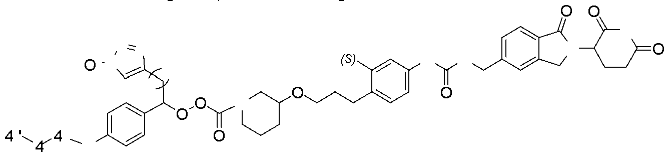

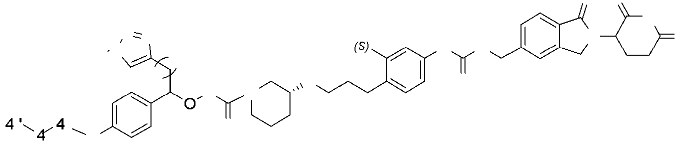

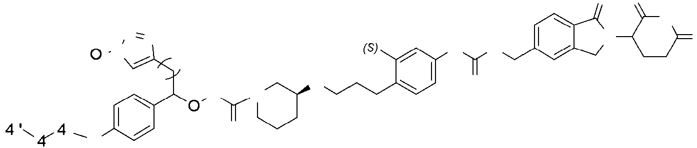

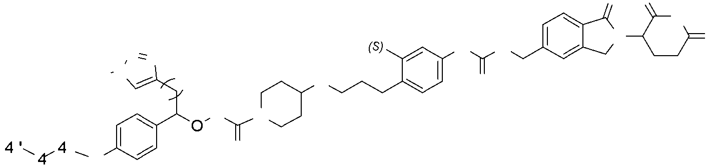

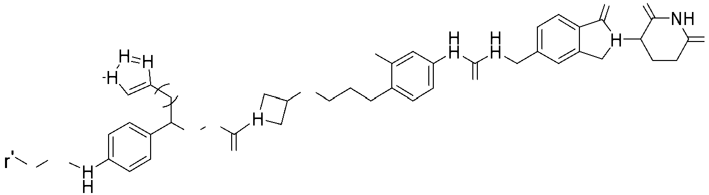

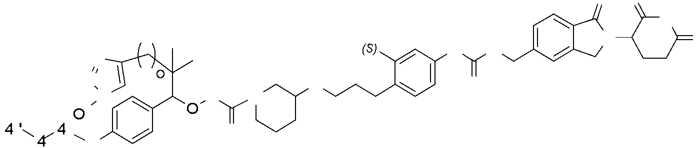

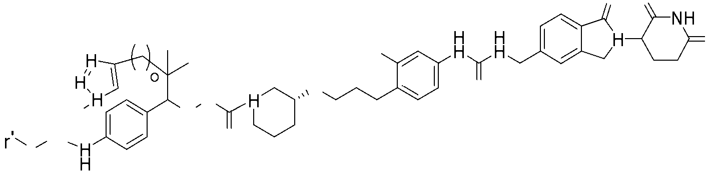

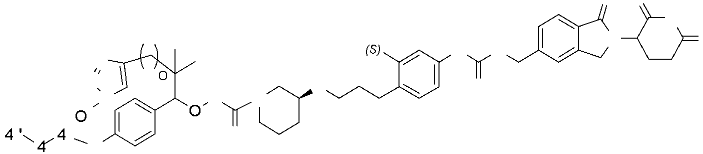

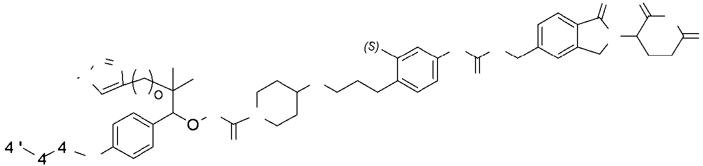

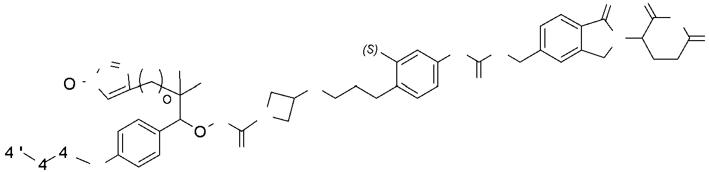

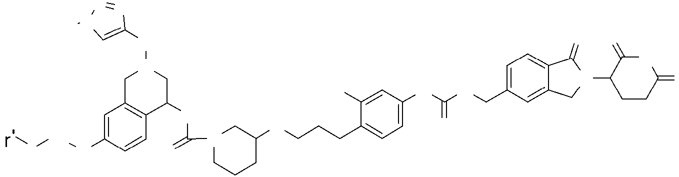

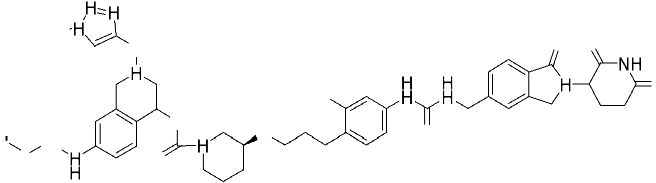

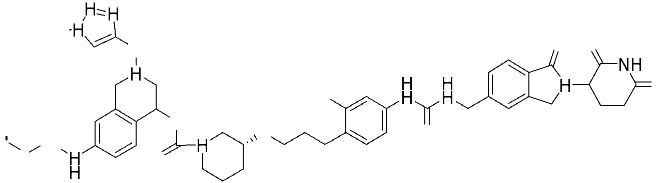

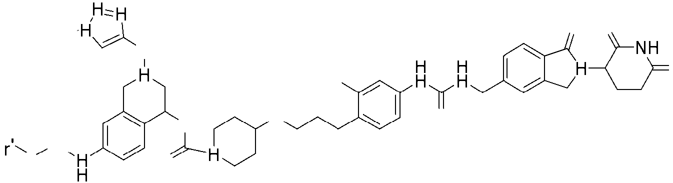

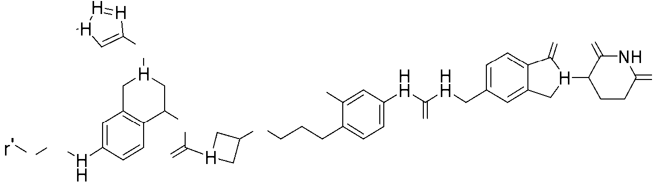

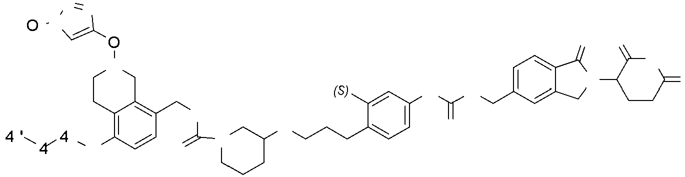

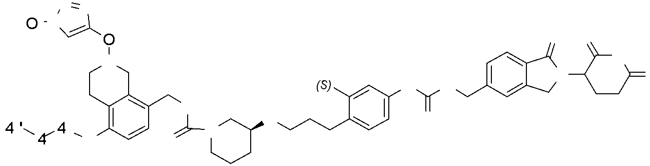

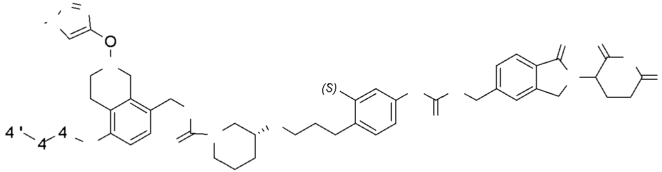

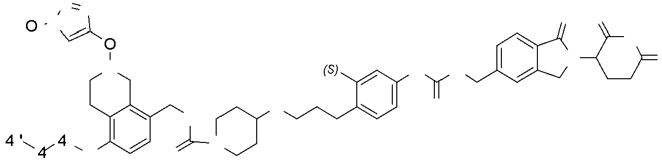

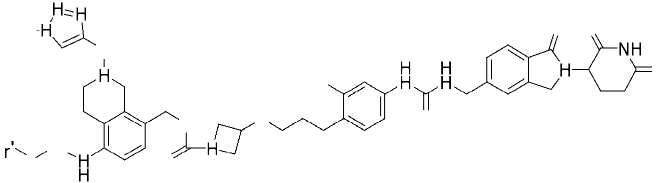

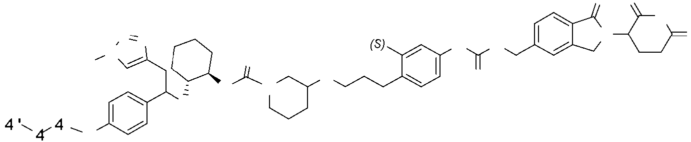

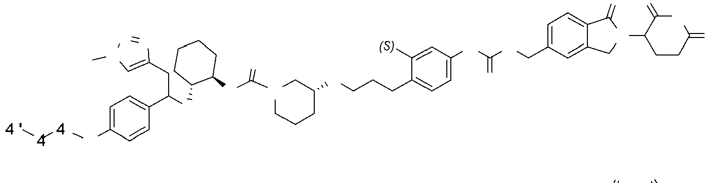

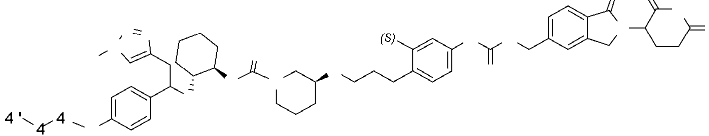

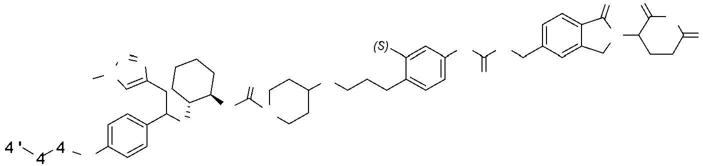

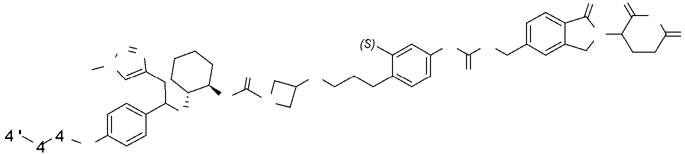

In some embodiments, the linker-drug compound represented by formula (II), or the tautomer, the mesomer, the racemate, the enantiomer or the diastereoisomer thereof, and the pharmaceutically acceptable salt, the hydrate, the solvate or the isotopically labeled compound thereof have a structure shown as follows: 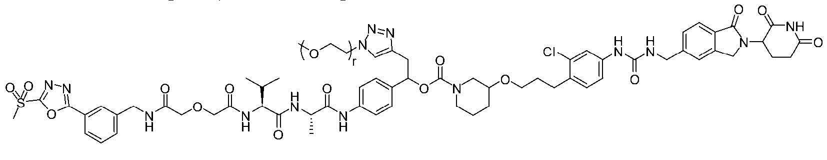 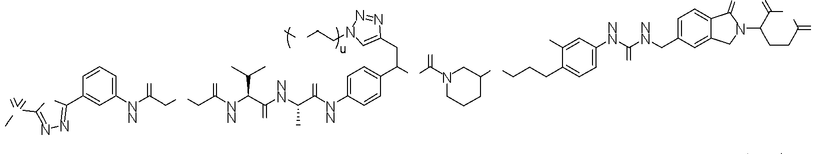 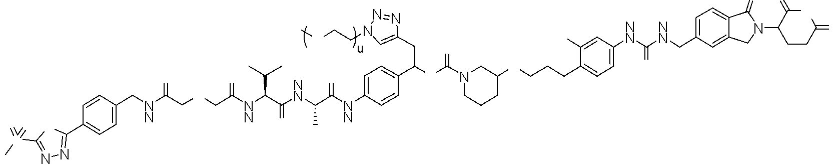 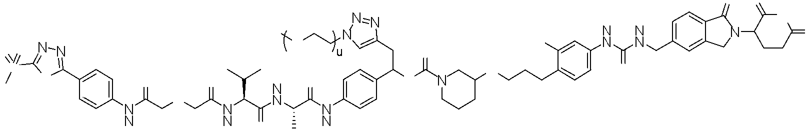 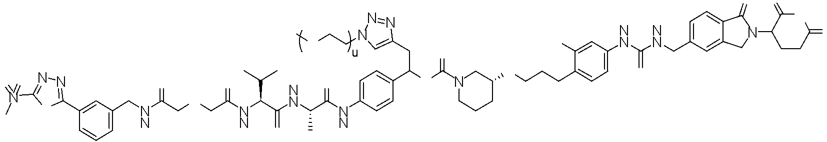 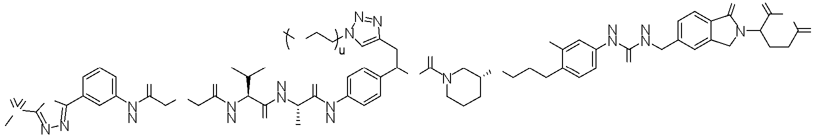 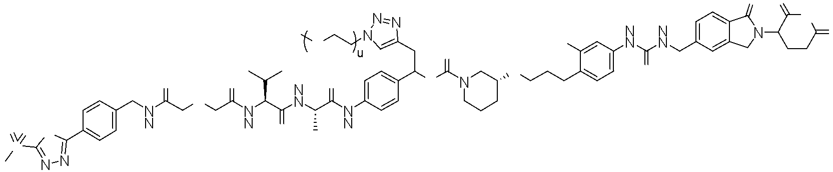 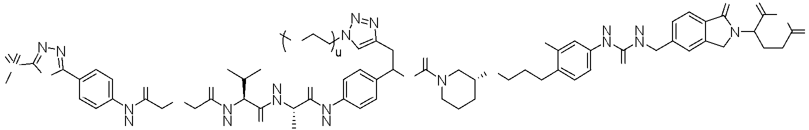 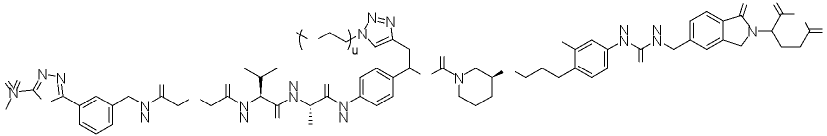 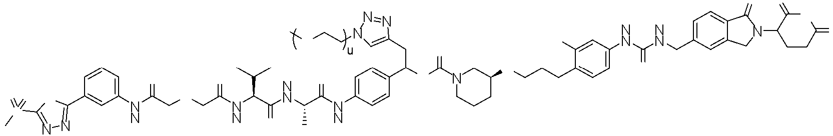 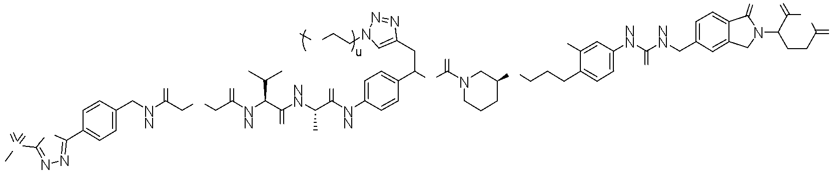 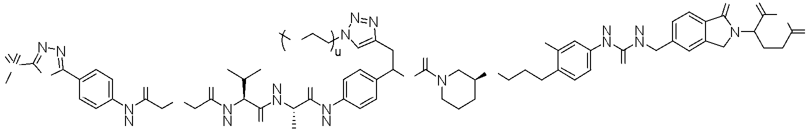 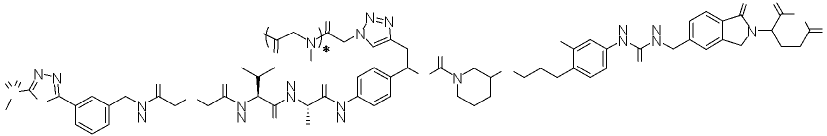 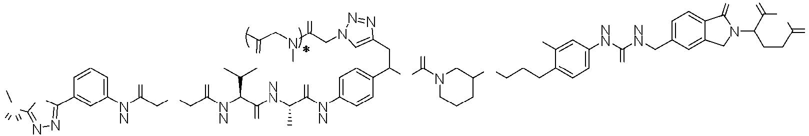 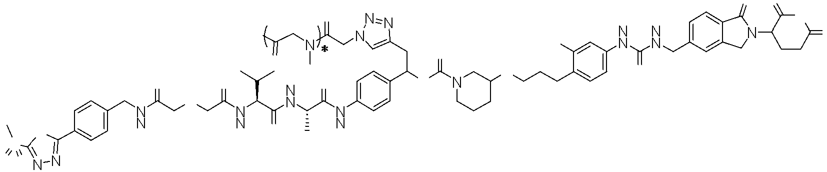 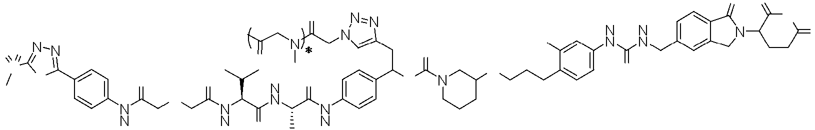 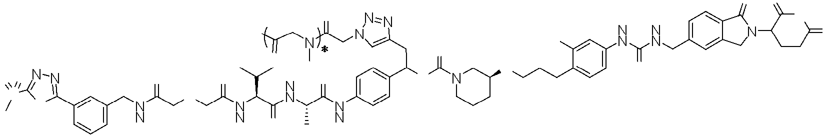 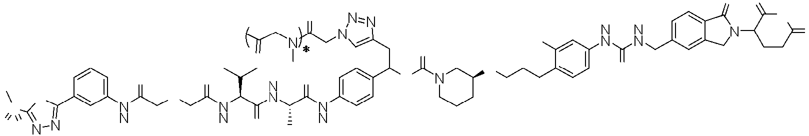 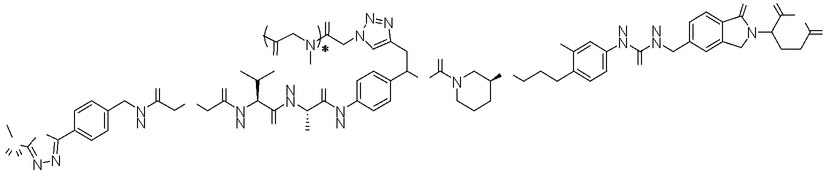 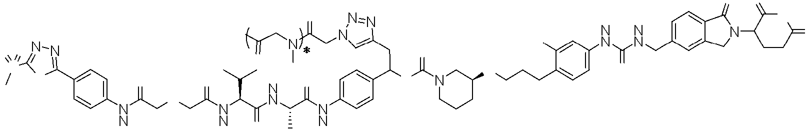 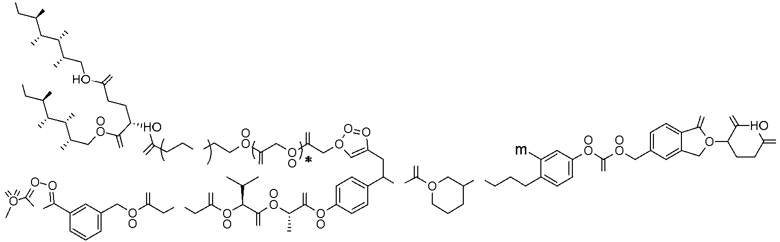 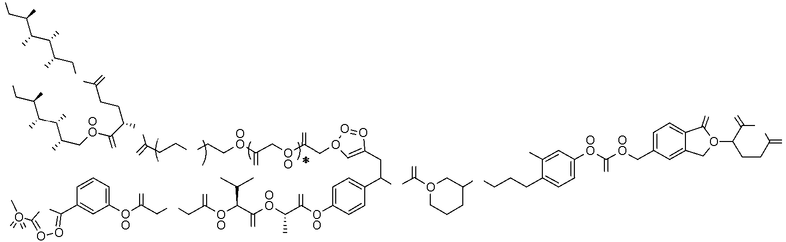 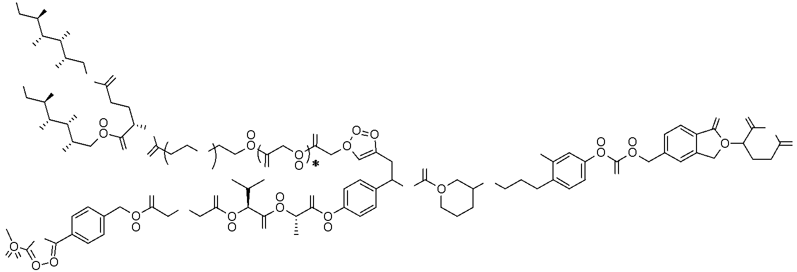 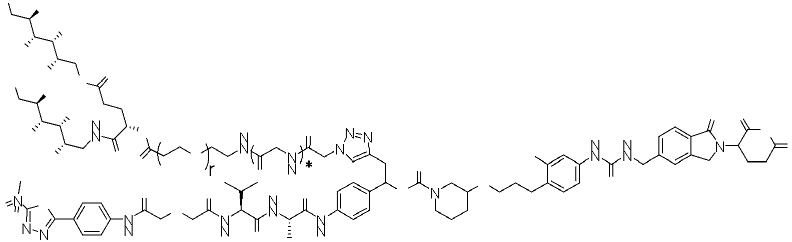 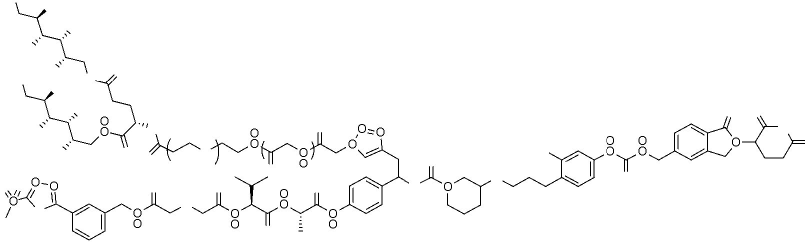 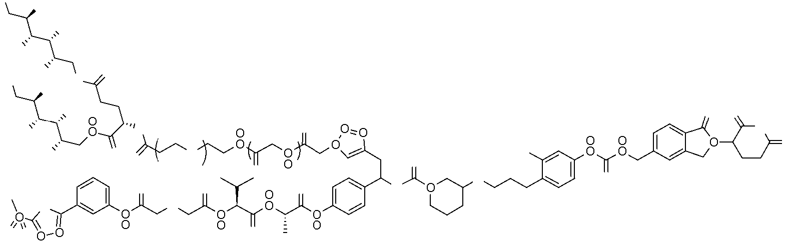 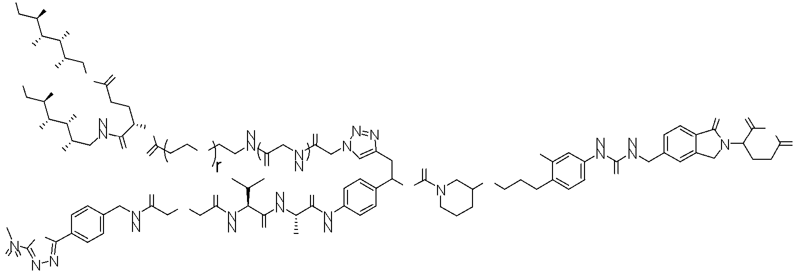 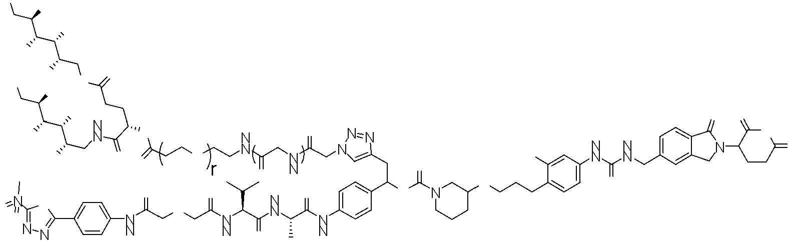 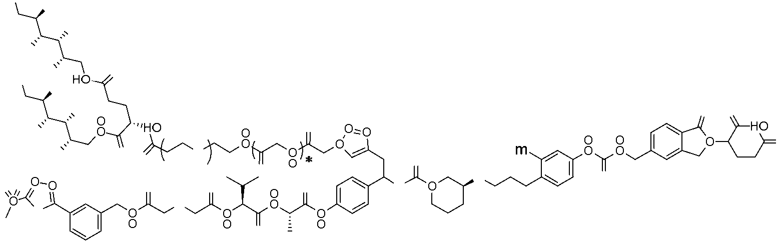 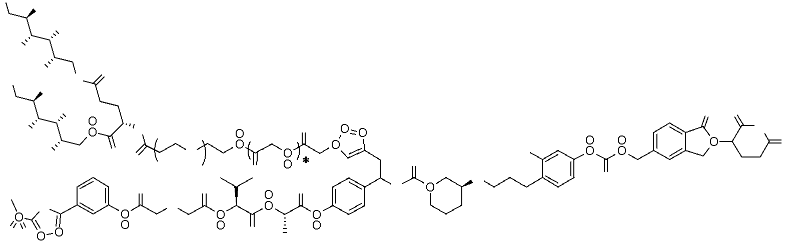 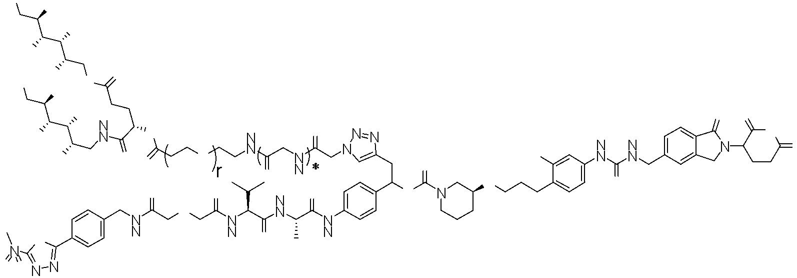 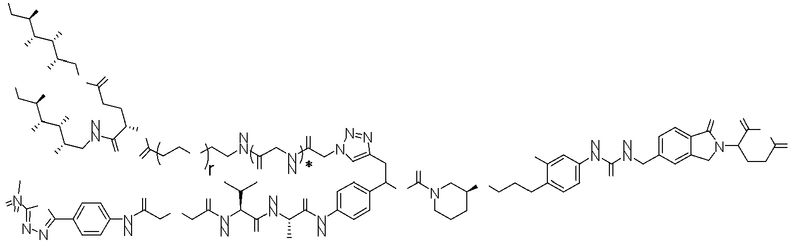 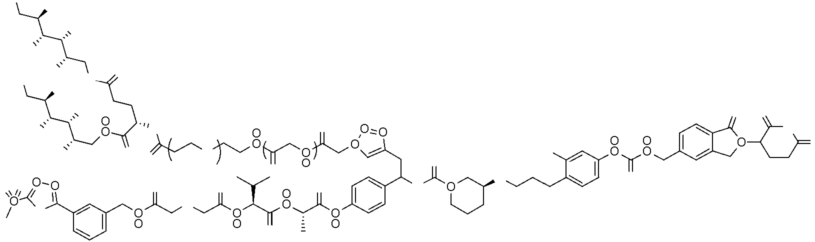 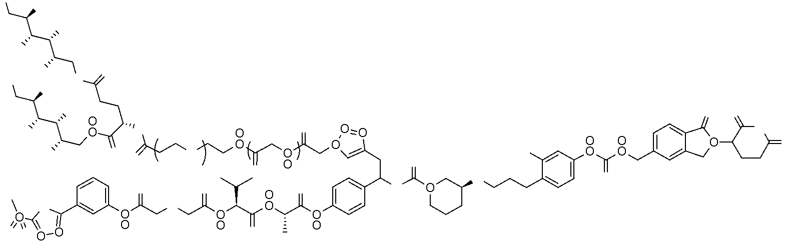 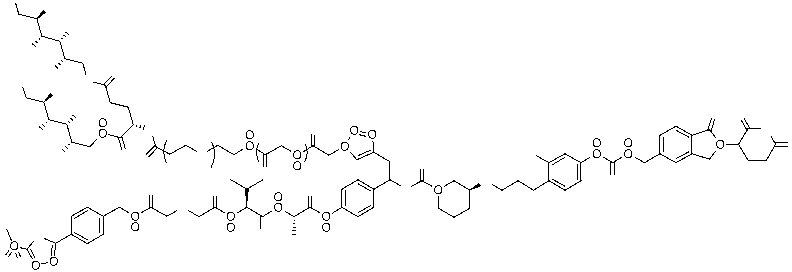 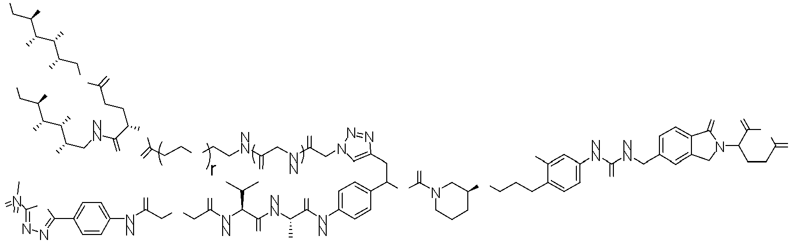 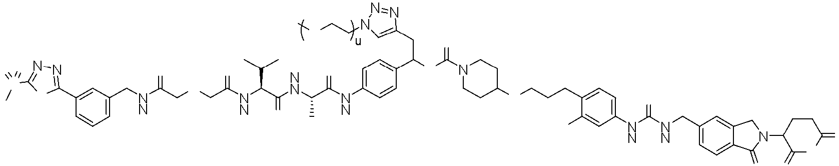 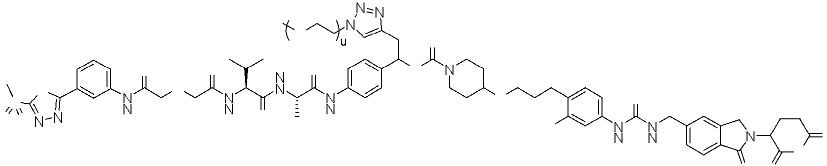 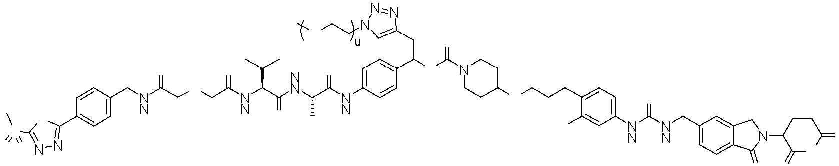 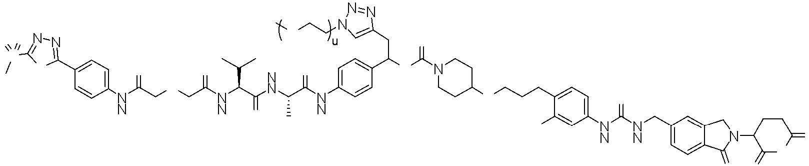 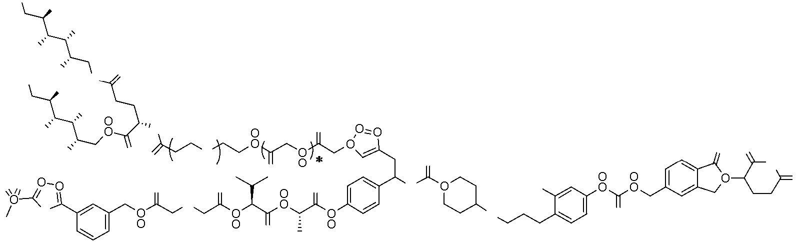 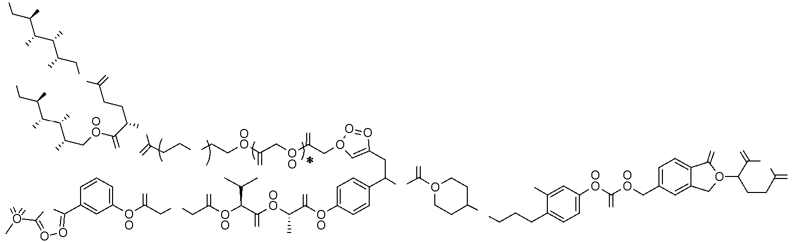 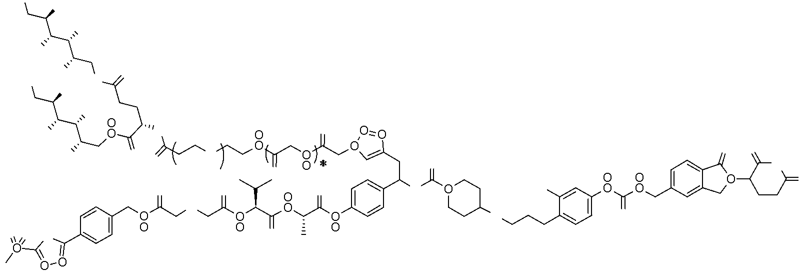 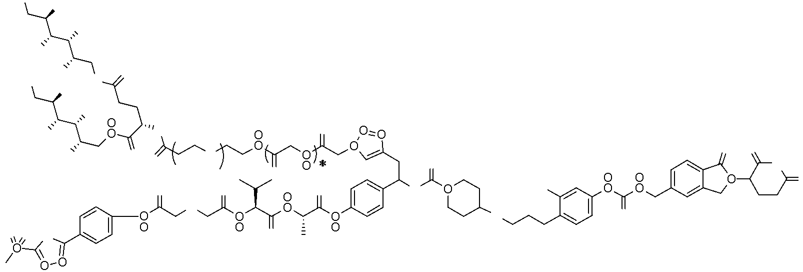 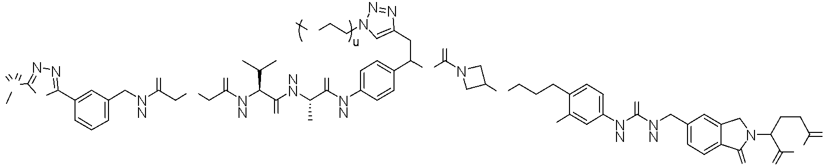 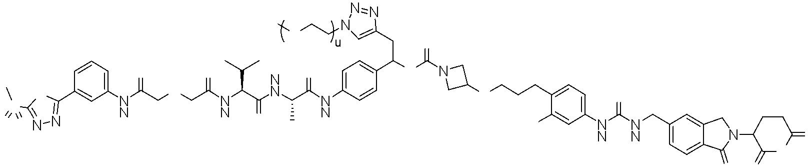 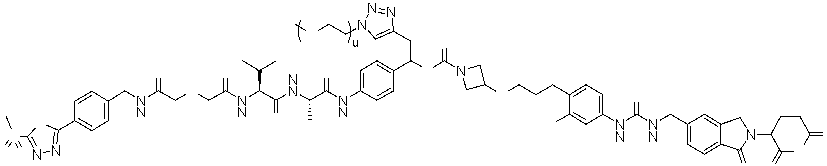 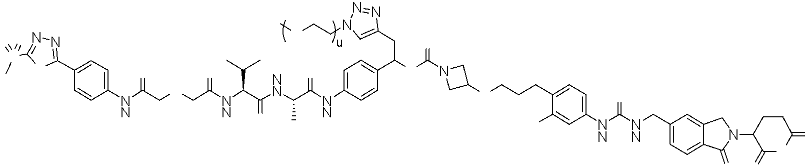 wherein r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50), and v and w are each independently an integer selected from 1-12 (e.g., 1, 2, 3, 4, 5, 6, 7, 8, 9, 10, 11, or 12).

In some embodiments, the linker-drug compound represented by formula (II), or the tautomer, the mesomer, the racemate, the enantiomer or the diastereoisomer thereof, and the pharmaceutically acceptable salt, the hydrate, the solvate or the isotopically labeled compound thereof have a structure shown as follows:

| No. | Structure |
|---|---|
| LD38 | 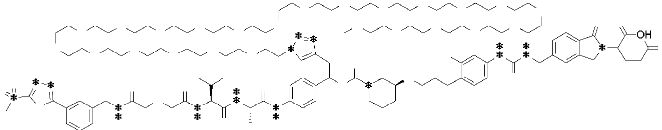 |
| LD39 | 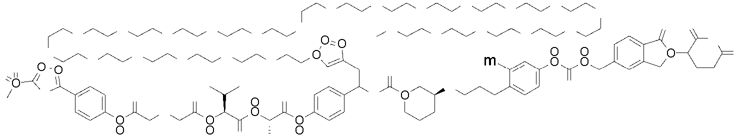 |
| LD40 | 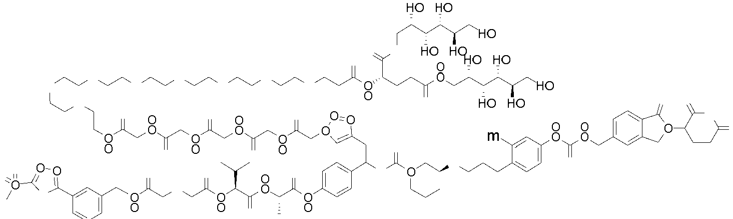 |
| LD41 | 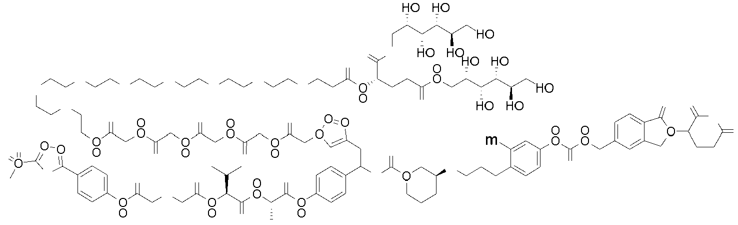 |
| LD42 | 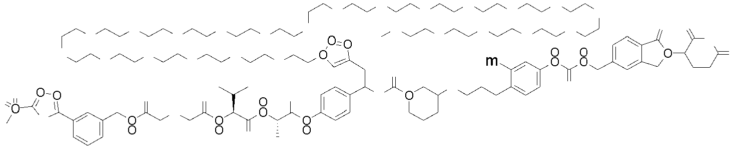 |
| LD43 | 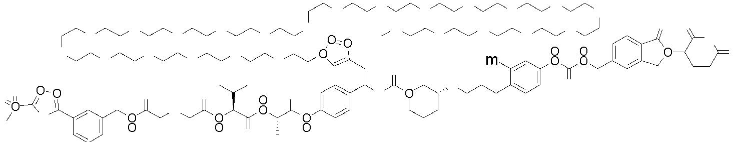 |
| LD44 | 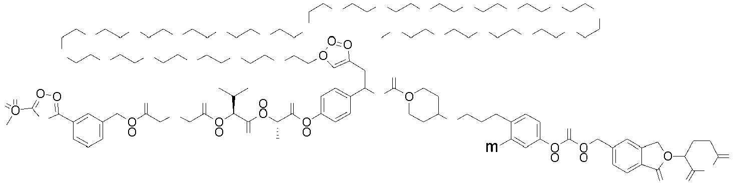 |
| LD45 | 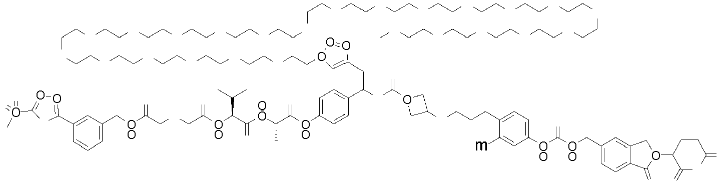 |

### Beneficial Effects

The present disclosure provides an antibody-drug conjugate based on a molecular glue with good activity. In addition, the introduction of a hydrophilic linker into the ADC can meet the requirements of most of the linkages to the active molecules described above, so that the obtained ADC has excellent efficacy, while having relatively good stability and hydrophilicity as well as good pharmacokinetic properties.

### Abbreviations and Definitions

Unless otherwise stated, the following terms used in the present application have the following meanings.

When a trade name is used in the present application, the trade name includes the product formula, generic drug, and active pharmaceutical ingredient of the product under the trade name, unless otherwise indicated in the context. The term "antibody-drug conjugate" or "ADC" means that a targeting ligand such as an antibody (e.g., a monoclonal antibody) or an antibody fragment is linked to a bioactive molecule with biological activity via a stable chemical linker compound.

The term "linker-drug compound" or "linker-drug" refers to a partial structure in an "antibody-drug conjugate" consisting of a linker compound and a bioactive compound.

The term "linker" refers to a chemical structural fragment, represented by L, which is linked to an antibody at one end and to a cytotoxic drug at the other end. It is formed by an "linker compound" connecting an antibody and a bioactive compound. In certain embodiments of the present disclosure, one part of the linker compound is first linked to the antibody and the other part is linked to the bioactive compound, and then the "linker" is formed through chemical reactions between different portions of the linker. The linker in the present disclosure comprises a linking moiety to the antibody, a spacer, a polypeptide sequence, and a self-immolative fragment.

The linker-drug compound in the present disclosure is linked to the antibody by conventional conjugation methods in the art, including: lysine conjugation, reductive disulfide bond conjugation between heavy and light chains, and site-specific conjugation (Beck A, Reichert JM. Antibody-drug conjugates: Present and future. MAbs, 2014, 6: 15-17; McCombs J R, Owen S C. Antibody drug conjugates: design and selection of linker, payload and conjugation chemistry. The AAPS journal, 2015, 17: 339-351). The present disclosure preferably provides conjugation via reductive disulfide bonds between the light and heavy chains, i.e., linkage via a reaction with thiol groups (sulfur atoms of cysteine residues) formed by reduction of one or more of disulfide bond sites between the light and heavy chains (two sites between the heavy chains and two sites between the heavy and light chains).

In the present disclosure, the linking moiety of the linker L to the antibody is represented by L₁, which is formed by the reaction of the L₁' group in the linker compound with the antibody, e.g., 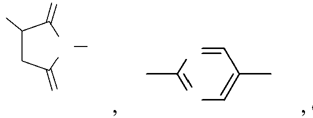 or 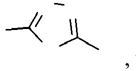 wherein * represents linkage to a sulfhydryl group of the antibody, and ** represents linkage to a spacer.

The term "spacer" refers to a linking group between the linking moiety of the linker L to the antibody and the polypeptide sequence, which may be any divalent organic group, such as a chemical bond, C₁₋₁₀ alkylene, C₂₋₁₀ alkenylene, C₂₋₁₀ alkynylene, C₃₋₁₀ cycloalkyl, C₆₋₁₂ aryl, 5- to 12-membered heteroaryl, 5- to 12-membered heterocyclyl, or a combination of two or more thereof; the C₁₋₁₀ alkylene, C₂₋₁₀ alkenylene, C₂₋₁₀ alkynylene, C₃₋₁₀ cycloalkyl, C₆₋₁₂ aryl, 5- to 12-membered heteroaryl, 5- to 12-membered heterocyclyl, or the combination of two or more thereof is optionally interrupted by a carbonyl group or an O, S or N atom; the C₁₋₁₀ alkylene, C₂₋₁₀ alkenylene, C₂₋₁₀ alkynylene, C₃₋₁₀ cycloalkyl, C₆₋₁₂ aryl, 5- to 12-membered heteroaryl, or 5- to 12-membered heterocyclyl may be optionally substituted with C₁₋₆ alkyl, C₃₋₆ cycloalkyl, halogen, or halogenated C₁₋₆ alkyl; preferably, as defined in L₂.

The term "polypeptide sequence" is well known in the art and is selected from a divalent peptide group comprising 2 to 8 optionally substituted natural or unnatural, L-type or D-type amino acid residues, wherein each of the amino acid residues is identical or different and is independently selected from residues of the following amino acids: alanine (Ala), cysteine (Cys), aspartic acid (Asp), glutamic acid (Glu), phenylalanine (Phe), glycine (Gly), histidine (His), isoleucine (Ile), lysine (Lys), leucine (Leu), methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucine (Nle), and analogs of the amino acids described above.

The term "self-immolative fragment" is well known in the art, such as the *p*-aminobenzyl alcohol carbonate fragment conventionally used in the art. In specific embodiments of the present disclosure, the "self-immolative fragment" of the linker of the present disclosure comprises a modification with a "hydrophilic fragment".

The term "hydrophilic fragment" is well known in the art, and the hydrophilic fragment may comprise a polyethylene glycol group, such as a polyethylene glycol group terminated with a methoxy group, or may be further linked to other hydrophilic fragments via the polyethylene glycol group. The hydrophilic fragment may also include a polyamino acid fragment, such as polyglycine or polysarcosine; the polyamino acid fragment may be combined with a polyethylene glycol group. The hydrophilic fragment may also be a monosaccharide, a disaccharide, or an oligosaccharide; the monosaccharide, the disaccharide, or the oligosaccharide may be a saccharide of a chain-form molecule or a cyclic molecule, and may comprise a glycosylamine, a sugar acid, or a phosphorylated sugar. Preferably, the saccharide group is combined with a polyethylene glycol fragment or a polyamino acid fragment; also preferably, at least two saccharide groups are introduced by polyvalent linking groups, such as aspartic acid-, glutamic acid- or lysine-based linking groups. The hydrophilic fragment may also comprise a polycarboxylic acid group, a polysulfonic acid group, a chelating group, etc.; the structure of the polycarboxylic acid group is, for example: 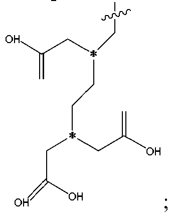 the structure of the polysulfonic acid group is, for example: 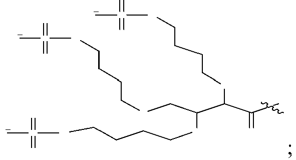 the chelating group may be, for example, a DOTA group or a NOTA group.

The term "solvate" means a physical association of the compound of the present disclosure with one or more solvent molecules (whether organic or inorganic). The physical association includes hydrogen bonding. In certain cases, the solvate can be isolated, for example, when one or more solvent molecules are incorporated into the crystal lattice of a crystalline solid. The solvent molecules in the solvate may be present in a regular arrangement and/or a disordered arrangement. The solvate may contain a stoichiometric or non-stoichiometric amount of solvent molecules. The "solvate" encompasses both solution phase and isolatable solvates. Exemplary solvates include, but are not limited to, hydrates, ethanolates, methanolates, and isopropanolates. Solvation methods are well known in the art.

The term "stereoisomer" refers to compounds having the same chemical constitution but different spatial arrangements of the atoms or groups. Stereoisomers include enantiomers, diastereoisomers, conformational isomers (rotamers), geometric isomers (cis/trans isomers), atropisomers, etc. Any resulting mixture of stereoisomers may be separated into pure or substantially pure geometric isomers, enantiomers, or diastereoisomers depending on differences in the physicochemical properties of the components, for example, by chromatography and/or fractional crystallization.

The term "tautomer" refers to isomers having different energies that are interconvertible by a low energy barrier. If tautomerism is possible (e.g., in a solution), the chemical equilibrium of the tautomers can be achieved. For example, proton tautomers (also known as prototropic tautomers) include tautomers that undergo interconversion by proton migration, such as keto-enol isomerization and imine-enamine isomerization. Valence tautomers include tautomers that undergo interconversion by recombination of bonding electrons.

The "more" in the term "one or more" includes two or more (e.g., 3, 4, 5, etc.).

The term "heteroatom" refers to a nitrogen, oxygen, sulfur, or halogen atom.

The term "Cₘ-Cₙ" means that the moiety has an integer number of carbon atoms ranging from m to n. For example, the "C₁-C₃" means that the group may have 1 carbon atom, 2 carbon atoms, or 3 carbon atoms.

The term "-(CH₂)ₙ-" means that the moiety has a group in which n -CH₂- are linked. For example, when the number of a linking group is 0, such as "-(CH)₀-", it means that the linking group is a covalent bond.

The term "alkyl" refers to a monovalent saturated aliphatic hydrocarbyl group, i.e., a linear or branched group containing 1-20 carbon atoms, preferably containing 1-10 carbon atoms (i.e., C₁₋₁₀ alkyl), further preferably containing 1-8 carbon atoms (C₁₋₈ alkyl), and more preferably containing 1-6 carbon atoms (i.e., C₁₋₆ alkyl). For example, "C₁₋₆ alkyl" means that the group is alkyl and the number of carbon atoms on the carbon chain is between 1 and 6 (specifically 1, 2, 3, 4, 5, or 6), and examples thereof include, but are not limited to, methyl, ethyl, *n*-propyl, isopropyl, *n*-butyl, isobutyl, *tert*-butyl*, sec*-butyl*, n*-pentyl, neopentyl, 1,1-dimethylpropyl, 1,2-dimethylpropyl, 2,2-dimethylpropyl, 1-ethylpropyl, 2-methylbutyl, 3-methylbutyl, *n*-hexyl, etc.

The term "alkoxy" refers to -O-alkyl, wherein the alkyl is as defined above, i.e., the alkyl contains 1-20 carbon atoms, preferably 1-10 carbon atoms, more preferably 1-8 carbon atoms, and further more preferably 1-6 (specifically 1, 2, 3, 4, 5, or 6) carbon atoms. Representative examples of the alkoxy include, but are not limited to, methoxy, ethoxy, propoxy, isopropoxy, butoxy, 1-methylpropoxy, 2-methylpropoxy, *tert*-butoxy, pentyloxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, etc.

The term "halogen", "halogenated", or "halo" refers to F, Cl, Br, or I.

The term "haloalkyl" refers to alkyl as defined above in which one, two, or more hydrogen atoms or all hydrogen atoms are replaced by halogen. Representative examples of the haloalkyl include CCl₃, CHCl₂, CH₂Cl, CF₃, CHF₂, CH₂F, CBr₃, CHBr₂, CH₂Br, Cl₃, CHI₂, CH₂I, CH₂CF₃, CF₂CF₃, etc.

The term "aryl" refers to a monocyclic, bicyclic, or tricyclic aromatic carbocyclic system containing 6-16 carbon atoms, or 6-14 carbon atoms, or 6-12 carbon atoms, or 6-10 carbon atoms, preferably 6-10 carbon atoms. Examples of the aryl group may include, but are not limited to, phenyl, naphthyl, anthryl, phenanthryl, pyrenyl, etc. The term "heteroaryl" refers to an aromatic monocyclic or polycyclic ring system containing a 5- to 14-membered structure, or preferably a 5- to 10-membered structure or a 5- to 8-membered structure, and more preferably a 5- to 6-membered structure, wherein 1, 2, 3, or more ring atoms are heteroatoms and the remaining atoms are carbon, the heteroatoms are independently selected from O, N, and S, and the number of the heteroatoms is preferably 1, 2, or 3. Examples of the heteroaryl include, but are not limited to, furanyl, thienyl, oxazolyl, thiazolyl, isoxazolyl, oxadiazolyl, thiadiazolyl, pyrrolyl, pyrazolyl, imidazolyl, triazolyl, tetrazolyl, pyridinyl, pyrimidinyl, pyrazinyl, pyridazinyl, thiadiazolyl, triazinyl, phthalazinyl, quinolinyl, isoquinolinyl, pteridinyl, purinyl, indolyl, isoindolyl, indazolyl, benzofuranyl, benzothienyl, benzopyridinyl, benzopyrimidinyl, benzopyrazinyl, benzimidazolyl, benzophthalazinyl, pyrrolo[2,3-*b*]pyridinyl, imidazo[1,2-*a*]pyridinyl, pyrazolo[1,5-*a*]pyridinyl, pyrazolo[1,5-*a*]pyrimidinyl, imidazo[1,2-*b*]pyridazinyl, [1,2,4]triazolo[4,3-*b*]pyridazinyl, [1,2,4]triazolo[1,5-*a*]pyrimidinyl, [1,2,4]triazolo[1,5-*a*]pyridinyl, etc.

The term "cycloalkyl" refers to a fully saturated carbocyclic ring that may exist as a monocyclic, bridged cyclic, or spiro cyclic structure. Preferably, the cycloalkyl contains 3-12 carbon atoms (i.e., C3-12 cycloalkyl), more preferably 3-10 carbon atoms (C3-10 cycloalkyl), and further preferably 3-7 carbon atoms (C3-7 cycloalkyl), 4-6 carbon atoms (C4-6 cycloalkyl), or 5-6 carbon atoms (C5-6 cycloalkyl). Examples of the cycloalkyl include, but are not limited to, cyclopropyl, cyclobutyl, cyclopentyl, cyclohexyl, cycloheptyl, methylcyclopropyl, 2-ethylcyclopentyl, dimethylcyclobutyl, norbornyl (bicyclo[2.2.1]heptyl), bicyclo[2.2.2]octyl, adamantyl, etc.

The term "-alkyl-" or "alkylene" refers to saturated linear or branched divalent hydrocarbyl. For example, C1-C8 alkylene refers to linear or branched alkylene having 1-8 carbon atoms.

The term "heterocyclyl" refers to a fully saturated or partially unsaturated (non-fully unsaturated heteroaromatic) nonaromatic ring that may exist as a monocyclic, bridged cyclic, or spiro cyclic structure. Unless otherwise specified, the heterocycle is generally a 3- to 7-membered ring containing 1 to 3 heteroatoms independently selected from sulfur, oxygen, and/or nitrogen (preferably 1 or 2 heteroatoms, but excluding -O-O-, -O-S-, or -S-S- moieties). Non-limiting examples of the heterocyclyl include, but are not limited to, oxiranyl, tetrahydrofuranyl, dihydrofuranyl, pyrrolidinyl, N-methylpyrrolidinyl, dihydropyrrolyl, piperidinyl, piperazinyl, pyrazolidinyl, 4*H-*pyranyl, morpholinyl, thiomorpholinyl, tetrahydrothienyl, etc.

The term "heteroalkyl", by itself or in combination with another term, refers to a stable linear or branched alkyl atomic group or a composition thereof, consisting of a certain number of carbon atoms and at least one heteroatom. The number of the carbon atoms may be 1-50 (preferably 1-20, more preferably 1-12, and most preferably 1-8), such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50. The heteroalkyl may optionally contain one, two, or more (e.g., 3, 4, 5, 6, 7, 8, 9, 10, 11, or 12) heteroatoms selected from N, O, and S (or may be construed as optional insertion of heteroatoms into alkyl at any C-C bond or C-H bond). The heteroatoms O, N, and S may be located at any internal position of the heteroalkyl or at the position where the alkyl is attached to the rest of the molecule. Examples of the heteroalkyl include, but are not limited to, -CH₂-CH₂-O-CH₃, -CH₂-CH₂-NH-CH₃, -CH₂-CH₂-N(CH₃)-CH₃, -CH₂-S-CH₂-CH₃, -CH₂-CH₂-S(O)-CH₃, and -CH₂-CH₂-S(O)₂-CH₃. Up to two heteroatoms may be consecutive, such as -CH₂-NH-OCH₃.

Unless otherwise indicated, the definitions of the terms herein are also applicable to groups containing the term; for example, the definition of C₁₋₆ alkyl is also applicable to C₁₋₆ alkyloxy.

In various sections of the present disclosure, linking substituents are described. When a linking group is clearly required by the structure, the Markush variables listed for the group should be understood as linking groups. For example, if a linking group is required by the structure and "heteroalkyl", "aryl", and the like are listed for the Markush group definition of the variable, it should be understood that the "heteroalkyl" or "aryl" respectively represents a linked heteroalkylene group or arylene group. Moreover, it should also be understood that when a Markush variable listed for a group is to be understood as a linking group, regardless of whether it is defined as "alkylene" or "heteroalkylene", the scope of definitions applicable to both is identical. Therefore, for example, the definitions of the terms "alkylene", "cycloalkylene", "arylene", "heteroalkylene", "heteroarylene", and "heterocyclylene" herein are equivalent to or referenced as the "alkyl", "cycloalkyl", "aryl", "heteroalkyl", "heteroaryl", and "heterocyclyl" as defined above.

The term "derivative" refers to a compound formed by replacing an atom or atomic group in the molecule of a parent compound with another atom or atomic group, which is referred to as a derivative of the parent compound. The phrase "pharmaceutically acceptable salt" refers to a pharmaceutically acceptable organic or inorganic salt of a compound (e.g., a drug, a drug-linker, or an antibody-linker-drug conjugate). The compound may contain at least one amino, imino, hydroxyl, or carboxyl group, and thus may form addition salts with the corresponding acids or bases. Exemplary salts include, but are not limited to: sulfate, trifluoroacetate, citrate, acetate, oxalate, hydrochloride, hydrobromide, hydroiodide, nitrate, bisulfate, phosphate, acidic phosphate (-H₂PO₄), phosphite, isonicotinate, lactate, salicylate, acidic citrate, tartrate, oleate, tannate, pantothenate, bitartrate, ascorbate, formate, benzoate, glutamate, methanesulfonate, ethanesulfonate, benzenesulfonate, *p*-toluenesulfonate, potassium salts, sodium salts, ammonium salts, calcium salts, etc. In addition, the pharmaceutically acceptable salt has more than one charged atom in the structure. Examples in which multiple charged atoms are part of the pharmaceutically acceptable salt can have multiple counter ions. For example, the pharmaceutically acceptable salt has one or more charged atoms and/or one or more counter atoms.

The term "amino acid" refers to a naturally occurring amino acid or a non-naturally occurring amino acid, represented by NH₂-C(R'R")-C(=O)OH, wherein R' and R" are each independently hydrogen, optionally substituted linear, branched, or cyclic alkyl, alkenyl, or alkynyl having 1 to 10 carbon atoms, aryl, heteroaryl, or heterocyclyl, or R" and the N-terminal nitrogen atom may be combined to form a heterocyclic ring, for example, proline.

The term "amino acid residue" refers to the corresponding residue obtained after a hydrogen atom is removed from the amine of an amino acid and/or a hydroxyl group is removed from the carboxyl terminus, for example, -NH-C(R'R")-C(O)-.

The term "peptide" refers to a short chain of amino acid monomers linked by peptide (amide) bonds.

The term "tumor" refers to a neoplasm formed by clonal abnormal proliferation of a certain cell in a local tissue due to the loss of normal regulation of its growth at the genetic level under the action of various carcinogenic factors in the body.

The term "linker" refers to a chemical structural fragment, represented by L, which is linked to an antibody at one end and to a cytotoxic drug at the other end.

The term "antibody" refers to an immunoglobulin molecule that recognizes and specifically binds to a target, e.g., a protein, a polypeptide, a peptide, a carbohydrate, a polynucleotide, a lipid, or a combination of the foregoing, via at least one antigen recognition site located in a variable region of the immunoglobulin molecule, represented by A. The term encompasses intact polyclonal antibodies, intact monoclonal antibodies, antibody fragments (e.g., Fab, Fab', F(ab')₂, and Fv fragments), single-chain Fv (scFv) mutants, multispecific antibodies (e.g., bispecific antibodies, biparatopic antibodies, etc.), multivalent antibodies (e.g., trivalent, tetravalent, or higher-valency antibodies having three, four, or more antigen-binding sites), chimeric antibodies, humanized antibodies, human antibodies, fusion proteins containing an antigen determination portion of an antibody, and any other modified immunoglobulin molecule containing an antigen recognition site, so long as the antibody exhibit the desired biological activity.

The CDRs disclosed herein may also include variants. Generally, the amino acid identity between individual variant CDRs is at least 90%, 91%, 92%, 93%, 94%, 95%, 96%, 97%, 98%, 99%, or 100%. Therefore, the "variant CDR" is one that has a specific identity to, and shares a biological function with, the parent or reference CDR of the present disclosure, including but not limited to at least 80%, 81%, 82%, 83%, 84%, 85%, 86%, 87%, 88%, 89%, 90%, 91%, 92%, 93%, 94%, 95%, 96%, 97%, 98%, or 99% of the specificity and/or activity of the parent CDR. For example, the "variant CDR" may be a sequence that contains 1, 2, 3, or 4 amino acid changes compared to the parent or reference CDR of the present disclosure and shares or improves the biological function, specificity, and/or activity of the parent CDR.

### BRIEF DESCRIPTION OF THE DRAWINGS

FIG. 1: Total Flux [p/s] detection in experimental mice in Efficacy Example 5 of the present disclosure.
FIG. 2: Tumor growth curves of experimental mice in Efficacy Example 5 of the present disclosure.

### DETAILED DESCRIPTION

The present application is further illustrated by examples, which, however, are not intended to limit the scope of the present application. Experimental procedures without specified conditions in the following examples are generally conducted according to conventional conditions or according to conditions recommended by the manufacturers. Unless otherwise stated, all percentages, proportions, ratios, or parts are by weight.

### Description of abbreviations

**Table 1**

| | | | |
|---|---|---|---|
| DMF | *N,N*-Dimethylformamide | TBAF | Tetrabutylammonium fluoride |
| TEA | Triethylamine | DMSO | Dimethylsulfoxide |
| THF | Tetrahydrofuran | DIBAL-H | Diisobutylaluminium hydride |
| DIPEA | *N,N-*Diisopropylethylamine | TFAA | Trifluoroacetic anhydride |
| DBU | 1,8-Diazabicyclo[5.4.0]undec-7-ene | Boc | *tert*-Butoxycarbonyl |
| EEDQ | 2-Ethoxy-1-ethoxycarbonyl-1,2-dihydroquinoline | Fmoc | 9-Fluorenylmethyloxycarbonyl |
| PPTS | Pyridinium *p*-toluenesulfonate | TECP | Tris(2-carboxyethyl)phosphine |
| THPTA | Tris(3-hydroxypropyltriazolylmethyl)amine | DCA | Dichloroacetic acid |
| EA | Ethyl acetate | Py | Pyridine |
| EDCI | 1-(3-Dimethylaminopropyl)-3-ethylcarbodiimide hydrochloride | HOBT | 1-Hydroxybenzotriazole |
| 9-BBN | 9-Borabicyclo[3.3.1]nonane | NH₄FA | Ammonium formate |
| tBuOH | *tert*-Butanol | DCM | Dichloromethane |
| NPCCl | *p*-Nitrobenzoyl chloride | MTBE | Methyl *tert-*butyl ether |
| ACN | Acetonitrile | | |
| PE | Petroleum Ether | | |

### Example 1: Synthesis of Compound 8-11

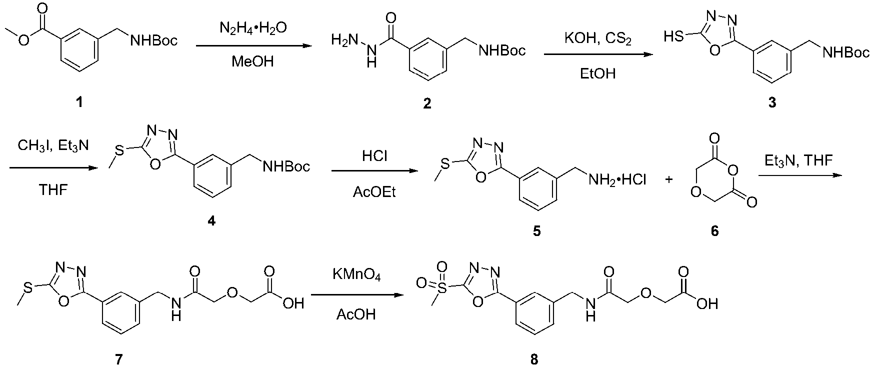

Compound **1** (50 g, 189 mmol) and methanol (250 mL) were added to a reaction flask, and 80% hydrazine hydrate (35.4 g, 567 mmol) was slowly added at room temperature. The mixture was warmed to 70 °C and refluxed for 6 h. After cooling, a white crystal was precipitated, and the mixture was subjected to suction filtration. The remaining solid was washed with methanol (20 mL × 3) to give **2** (50 g, yield: 100%) as a white solid. MS (ESI): (M+H)⁺, calculated 266.1, found 266.2.

Compound **2** (50 g, 189 mmol), potassium hydroxide (12.7 g, 227 mmol), and ethanol (400 mL) were added to a reaction flask, and the mixture was stirred for dissolution at room temperature. Carbon disulfide (17 g, 283 mmol) was slowly added, and the mixture was warmed to 100 °C and refluxed for 5 h. The solvent was removed by concentration under reduced pressure, and water (50 mL) was added. The mixture was adjusted to pH 6 with diluted hydrochloric acid and extracted with dichloromethane (100 mL × 3). The organic phases were combined, washed with saturated brine, dried over anhydrous sodium sulfate, and filtered. The filtrate was then concentrated under reduced pressure, and the residue was purified by silica gel chromatography to give compound 3 (54 g, yield: 93%).

MS (ESI): (M+H)⁺, calculated 308.1, found 308.2.

Compound **3** (5 g, 16.3 mmol), triethylamine (2 g, 19.6 mmol), and tetrahydrofuran (30 mL) were added to a reaction flask, and iodomethane (2.5 g, 17.9 mmol) was added to the reaction liquid described above. The reaction was completed after the mixture was stirred for reaction at 25 °C for 1.5 h. The solvent was removed by concentration under reduced pressure, and the residue was purified by silica gel chromatography to give compound **4** (3.4 g, yield: 65%). MS (ESI): (M+H)⁺, calculated 322.1, found 322.2.

Compound **4** (3.4 g, 10.6 mmol) and ethyl acetate (20 mL) were added to a reaction flask, and a solution of hydrogen chloride in ethyl acetate (2.7 mL, 4 M) was added dropwise at 25 °C. The mixture was reacted for 6 h, and the solvent was removed by concentration under reduced pressure to give a crude product of **5,** which was directly used in the next step.

The crude product of **5,** diglycolic anhydride **6** (1.35 g, 11.7 mmol), triethylamine (2.14 g, 21.2 mmol), and tetrahydrofuran (30 mL) were added to a reaction flask. The mixture was reacted at 25 °C for 1.5 h, and the solvent was removed by concentration under reduced pressure. Diethyl ether was added to the residue, and the mixture was subjected to suction filtration and then washed with water and diethyl ether to give compound **7** (3.5 g, yield: 99%). MS (ESI): (M+H)⁺, calculated 338.0, found 338.2.

Compound **7** (3.5 g, 10.5 mmol) and glacial acetic acid (20 mL) were added to a reaction flask. After dissolution, potassium permanganate (2.48 g, 15.7 mmol) was added at 0 °C. The mixture was warmed to 25 °C and reacted for 1 h. A saturated sodium sulfite solution was added until the solution became colorless, and then the solution was extracted with ethyl acetate (100 mL × 3). The organic phases were combined, washed with saturated brine, dried over anhydrous sodium sulfate, and filtered. The filtrate was then concentrated under reduced pressure, and the residue was purified by silica gel chromatography to give compound **8** (2.3 g, yield: 60%). MS (ESI): (M+H)⁺, calculated 370.0, found 370.1.

**Table 2**

| No. | Structural formula | MS(ESI) : (M+H)⁺ | |
|---|---|---|---|
| | | Calculated | Found |
| **9** | 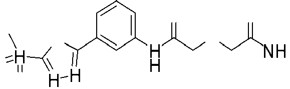 | 356.0 | 356.1 |
| **10** | 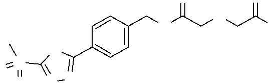 | 370.0 | 370.2 |
| **11** | 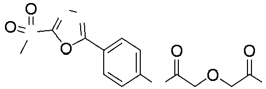 | 356.0 | 356.0 |

The synthesis of compounds **9-11** was carried out with reference to the synthetic route of compound 8.

### Example 2: Synthesis of Compound 14

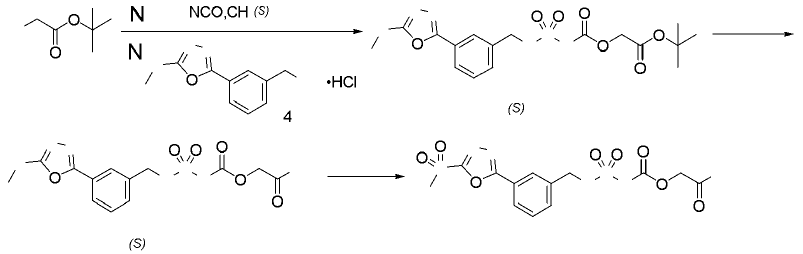

*tert*-Butyl glycolate (2.57 g, 19.5 mmol) was slowly added dropwise to a solution of chlorosulfonyl isocyanate (2.75 g, 19.5 mmol) in dichloromethane (40 mL) at 0 °C. The mixture was warmed to 25 °C and reacted for 1 h. A solution of compound **5** (5 g, 19.5 mmol) in dichloromethane (10 mL) was added dropwise to the reaction system described above. After 6 h of reaction, water (40 mL) was added, and the mixture was extracted with ethyl acetate (100 mL × 3). The organic phases were combined, washed with saturated brine, dried over anhydrous sodium sulfate, and filtered. The filtrate was then concentrated under reduced pressure, and the residue was purified by silica gel chromatography to give compound **12** (7.68 g, yield: 86%). MS (ESI): (M+H)⁺, calculated 459.1, found 459.0.

Compound **12** (7.68 g, 16.8 mmol) and ethyl acetate (20 mL) were added to a reaction flask, and a solution of hydrogen chloride in ethyl acetate (4.2 mL, 4 M) was added dropwise at 25 °C. The mixture was reacted for 6 h, and the solvent was removed by concentration under reduced pressure to give a crude product of **13,** which was directly used in the next step.

The crude product of **13** and glacial acetic acid (20 mL) were added to a reaction flask. After dissolution, potassium permanganate (4 g, 25.2 mmol) was added at 0 °C. The mixture was warmed to 25 °C and reacted for 1 h. A saturated sodium sulfite solution was added until the solution became colorless, and then the solution was extracted with ethyl acetate (100 mL × 3). The organic phases were combined, washed with saturated brine, dried over anhydrous sodium sulfate, and filtered. The filtrate was then concentrated under reduced pressure, and the residue was purified by silica gel chromatography to give compound **14** (3.28 g, yield: 45%). MS (ESI): (M+H)⁺, calculated 435.0, found 435.1.

### Example 3: Synthesis of Compound 18

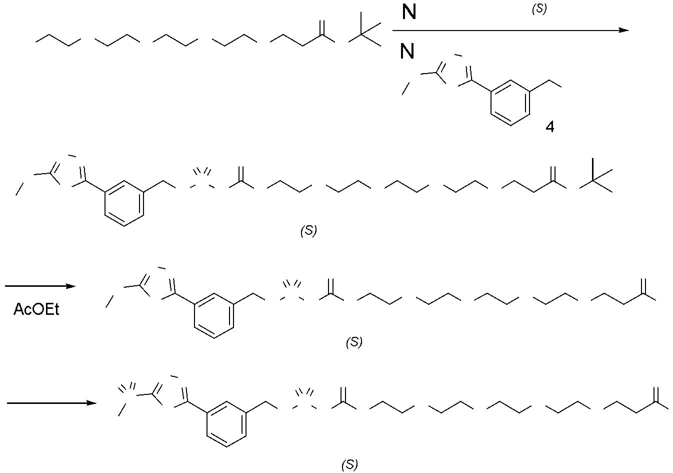

The synthesis of compound **18** was carried out with reference to the synthesis method of compound **14.**

### Example 4: Synthesis of Compounds 20, 22, and 37

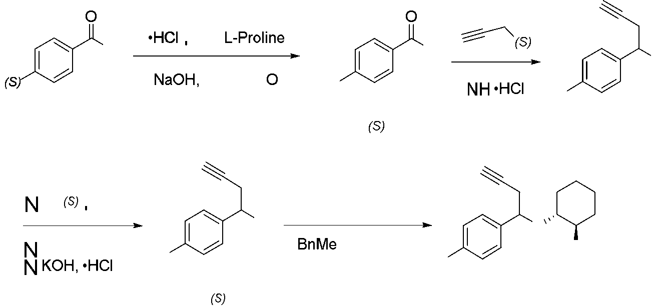

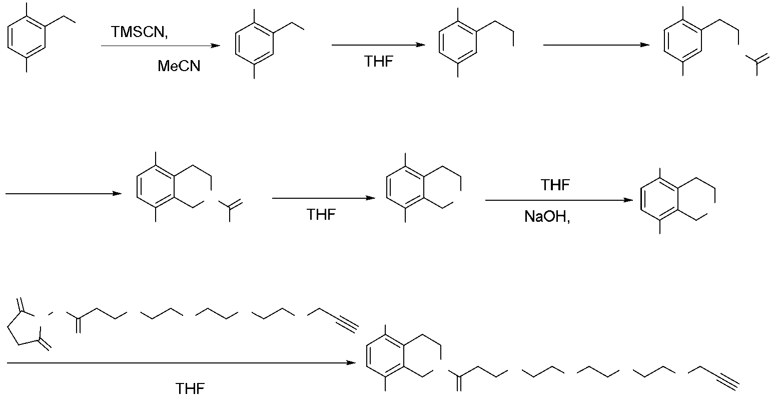

*p*-Bromobenzaldehyde (7.97 g, 43 mmol), sodium azide (5.57 g, 86 mmol), copper(I) iodide (817 mg, 4.3 mmol), L-proline (1.48 g, 12.9 mmol), sodium hydroxide (515 mg, 12.9 mmol), ethanol (70 mL), and water (20 mL) were added to a reaction flask, and the mixture was reacted at 95 °C for 24 h. After the reaction was completed, the mixture was cooled, and water was added. The mixture was extracted with ethyl acetate (100 mL × 3). The organic phases were combined, washed with saturated brine, dried over anhydrous sodium sulfate, and filtered. The filtrate was then concentrated under reduced pressure, and the residue was purified by silica gel chromatography to give compound **19** (5.5 g, yield: 87%). MS (ESI): (M+H)⁺, calculated 148.0, found 148.1.

Zinc powder (7.3 g, 113 mmol) and tetrahydrofuran (50 mL) were added to a reaction flask, and a solution of propargyl bromide (14.9 g, 125.5 mmol) in tetrahydrofuran (30 mL) was added dropwise at 10 °C. The mixture was reacted at 10 °C for 2 h, and a solution of **19** (2.75 g, 18.7 mmol) in tetrahydrofuran (30 mL) was slowly added dropwise. The mixture was then stirred for 2 h. The reaction liquid was poured into a 1 N aqueous HCl solution at 0 °C and extracted with ethyl acetate (100 mL × 3). The organic phases were combined, washed with saturated brine, dried over anhydrous sodium sulfate, and filtered. The filtrate was then concentrated under reduced pressure, and the residue was purified by silica gel chromatography to give compound **20** (3.15 g, yield: 90%). MS (ESI): (M+H)⁺, calculated 188.0, found 188.1.

Compound **20** (1 g, 5.3 mmol) and dichloromethane (5 mL) were added to a reaction flask, and thionyl chloride (1.9 g, 16 mmol) was added dropwise at 0 °C. After the addition, the mixture was warmed to 25 °C and reacted for 2 h. The solvent and the remaining thionyl chloride were removed by concentration under reduced pressure, and potassium thioacetate (906 mg, 8 mmol) and *N,N*-dimethylformamide (10 mL) were added. The mixture was reacted at 25 °C for 12 h. Water was then added, and the mixture was extracted with ethyl acetate (100 mL × 3). The organic phases were combined, washed with saturated brine, dried over anhydrous sodium sulfate, and filtered. The filtrate was then concentrated under reduced pressure, and a solution of tetrahydrofuran (10 mL) and potassium hydroxide (594 mg, 10.6 mmol) was added. The mixture was reacted at 25 °C for 2 h. Water was then added, and the mixture was extracted with ethyl acetate (20 mL × 3). The organic phases were combined, washed with saturated brine, dried over anhydrous sodium sulfate, and filtered. The filtrate was then concentrated under reduced pressure, and the residue was purified by silica gel chromatography to give compound **21** (612 mg, yield: 56%). MS (ESI): (M+H)⁺, calculated 204.0, found 204.1.

Cyclohexene oxide (1.47 g, 15 mmol) and 40% trimethylbenzylammonium hydroxide (2.5 g, 6 mmol) were added to a reaction flask. Compound **21** (612 mg, 3 mmol) was dissolved in *N,N*-dimethylformamide (6 mL), and the resulting solution was added dropwise to the reaction system described above. The mixture was reacted at 25 °C for 3 h. Water was then added, and the mixture was extracted with ethyl acetate (20 mL × 3). The organic phases were combined, washed with saturated brine, dried over anhydrous sodium sulfate, and filtered. The filtrate was then concentrated under reduced pressure, and the residue was purified by silica gel chromatography to give compound **22** (813 mg, yield: 90%). MS (ESI): (M+H)⁺, calculated 302.1, found 302.1.

Trimethylsilyl cyanide (3.86 g, 39 mmol) was added to tetrahydrofuran (20 mL), and tetrabutylammonium fluoride (35.7 mL, 1 M) was added dropwise to the mixture at 0 °C. After 1 h of reaction, a solution of compound **30** (10 g, 32.5 mmol) in acetonitrile (30 mL) was added to the reaction system described above. The mixture was reacted at 80 °C for 1 h. After cooling, the solvent was removed, and the residue was purified by silica gel chromatography to give compound **31** (5.4 g, yield: 66%). MS (ESI): (M+H)⁺, calculated 254.0, found 254.1.

Compound **31** (5.4 g, 21.5 mmol) was added to tetrahydrofuran, and a solution of borane in tetrahydrofuran (21.5 mL, 1 M) was added dropwise to the mixture at 0 °C. The mixture was then reacted at 25 °C for 16 h. Methanol was added to quench the reaction, and the solvent was removed by concentration under reduced pressure. The residue was purified by silica gel chromatography to give compound **32** (4.1 g, yield: 74%). MS (ESI): (M+H)⁺, calculated 258.0, found 258.1.

Compound **32** (4.1 g, 15.9 mmol) and triethylamine (3.2 g, 31.8 mmol) were added to dichloromethane (30 mL), and trifluoroacetic anhydride (5 g, 23.8 mmol) was added dropwise to the mixture at 0 °C. The mixture was then reacted at 25 °C for 1 h, added with water for dilution, and extracted with dichloromethane (20 mL × 3). The organic phases were combined, washed with saturated brine, dried over anhydrous sodium sulfate, and filtered. The filtrate was then concentrated under reduced pressure, and the residue was purified by silica gel chromatography to give compound **33** (5.6 g, yield: 60%). MS (ESI): (M+H)⁺, calculated 354.0, found 354.1.

Compound **33** (5.6 g, 9.5 mmol) and paraformaldehyde (0.37 g, 12.4 mmol) were added to sulfuric acid, and the mixture was reacted at 25 °C for 1 h. The reaction liquid was then poured into ice water and extracted with ethyl acetate (20 mL × 3). The organic phases were combined, washed with saturated brine, dried over anhydrous sodium sulfate, and filtered. The filtrate was then concentrated under reduced pressure, and the residue was purified by silica gel chromatography to give compound **34** (2.77 g, yield: 80%). MS (ESI): (M+H)⁺, calculated 366.0, found 366.1.

Compound **34** (2.77 g, 7.6 mmol) was added to tetrahydrofuran, and diisobutylaluminium hydride was added dropwise to the mixture at 0 °C. The mixture was then reacted at 25 °C for 2 h. Water was added to quench the reaction, and the mixture was filtered. The filtrate was then concentrated under reduced pressure, and the residue was purified by silica gel chromatography to give compound **35** (1.65 g, yield: 90%). MS (ESI): (M+H)⁺, calculated 242.0, found 242.1.

Compound **35** (1.65 g, 6.8 mmol), sodium azide (3.5 g, 54.4 mmol), copper(I) iodide (130 mg, 0.68 mmol), L-proline (0.4 g, 2 mmol), and sodium hydroxide (80 mg, 2 mmol) were added to ethanol (20 mL) and water (10 mL), and the mixture was reacted at 95 °C for 24 h. After cooling, water was added, and the mixture was extracted with ethyl acetate (20 mL × 3). The organic phases were combined, washed with saturated brine, dried over anhydrous sodium sulfate, and filtered. The filtrate was then concentrated under reduced pressure, and the residue was purified by silica gel chromatography to give compound **36** (0.97 g, yield: 70%). MS (ESI): (M+H)⁺, calculated 205.1, found 205.1.

Compound **36** (0.97 g, 4.8 mmol), compound **28** (1.8 g, 5.04 mmol), and triethylamine (0.76 g, 7.2 mmol) were added to tetrahydrofuran (10 mL), and the mixture was reacted at 25 °C for 12 h. The solvent was removed by concentration under reduced pressure, and the residue was purified by silica gel chromatography to give compound **37** (1.7 g, yield: 80%). MS (ESI): (M+H)⁺, calculated 447.2, found 447.1.

### Example 5: Synthesis of Compound 45

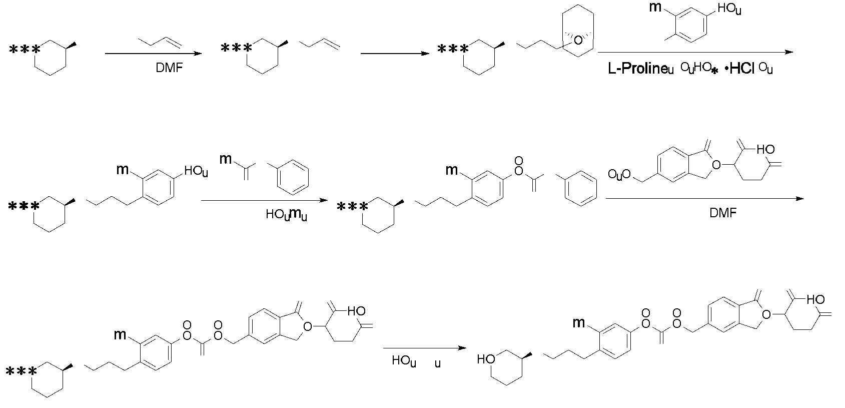

Compound **38** (10 g, 50 mmol) was dissolved in tetrahydrofuran (120 mL), and sodium hydride (4 g, 100 mmol) was added at 0 °C. The mixture was stirred for 0.5 h. Allyl bromide (12.1 g, 100 mmol) was then added, and the mixture was reacted for 2 h. A saturated ammonium chloride solution was added to quench the reaction. The mixture was extracted with ethyl acetate (10 mL × 3). The organic phases were combined, dried over anhydrous sodium sulfate, and then filtered. The filtrate was concentrated under reduced pressure, and the residue was purified by a silica gel column to give compound **39** (4.65 g, yield: 66%). MS (ESI): (M+H)⁺, calculated 142.2, found 142.3.

Compound **39** (4.65 g, 33 mmol) was added to tetrahydrofuran (100 mL), and a solution of 9-borabicyclo[3.3.1]nonane (80 mL, 0.5 M) in tetrahydrofuran was added. The mixture was reacted at 80 °C for 2 h and then cooled to room temperature. The reaction liquid was directly used in the next step.

*N,N*-Dimethylformamide (100 mL) and an aqueous solution of potassium phosphate (8.7 g, 41 mmol) were added to the reaction liquid of compound **40,** and the mixture was stirred for 10 min. 3-Chloro-4-bromoaniline (6.8 g, 33 mmol) and [1,1'-bis(diphenylphosphino)ferrocene]dichloropalladium(II) (2.4 g, 3.3 mmol) were added, and the mixture was reacted at 90 °C for 3 h, cooled to room temperature, and extracted with ethyl acetate (10 mL × 3). The organic phases were combined, dried over anhydrous sodium sulfate, and then filtered. The filtrate was concentrated under reduced pressure, and the residue was purified by a silica gel column to give compound **41** (3.7 g, yield: 42%). MS (ESI): (M+H)⁺, calculated 269.1, found 269.1.

Compound **41** (3.7 g, 13.8 mmol) was added to dichloromethane (90 mL), and diisopropylethylamine (3.5 g, 27 mmol) and phenyl chloroformate (2.5 g, 16 mmol) were sequentially added to the solution. The mixture was reacted at 25 °C for 0.5 h. The reaction liquid was then concentrated under reduced pressure, and the residue was purified by a silica gel column to give compound **42** (4.66 g, yield: 89%). MS (ESI): (M+H)⁺, calculated 389.2, found 389.2. Compound **42** (4.66 g, 12 mmol) was dissolved in N,N-dimethylformamide (50 mL), and then compound **43** (4.08 g, 13.2 mmol) was added. The solid was dispersed in the solution by ultrasonication, and diisopropylethylamine (48 mmol) was then added. The mixture was reacted at 50 °C for 3 h and cooled to room temperature. Water was then added, and the mixture was extracted with ethyl acetate (20 mL × 3). The organic phases were combined, dried over anhydrous sodium sulfate, and then filtered. The filtrate was concentrated under reduced pressure, and the residue was purified by a silica gel column to give compound **44** (6.4 g, yield: 94%). MS (ESI): (M+H)⁺, calculated 568.2, found 568.4.

Compound **44** (1.6 g, 2.8 mmol) was added to dichloromethane (19 mL), and a solution of hydrogen chloride in ethyl acetate (11 mL, 4 M) was added dropwise to the mixture at 0 °C. The mixture was then reacted for 2 h, and the solvent was removed by concentration under reduced pressure. The residue was dried under vacuum to give compound **45** (1.6 g, yield: 94%). MS (ESI): (M+H)⁺, calculated 568.2, found 568.3.

### Example 6: Synthesis of Compounds 46-49

**Table 3**

| No. | Structural formula | MS(ESI) : (M+H)⁺ | |
|---|---|---|---|
| | | Calculated | Found |
| **46** | 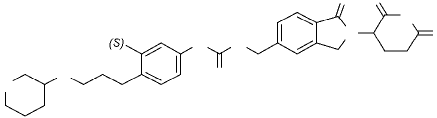 | 568.2 | 568.3 |
| **47** | 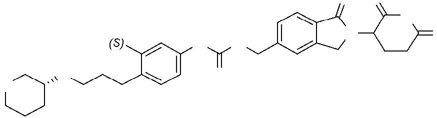 | 568.2 | 568.3 |
| **48** | 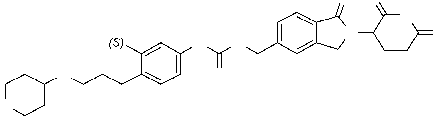 | 568.2 | 568.4 |
| **49** | 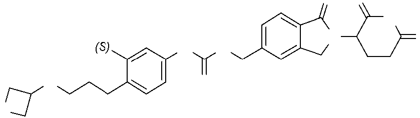 | 540.2 | 540.4 |

The synthesis of compounds **46-49** was carried out with reference to the synthetic route of compound **45.**

### Example 7: Synthesis of Compounds 52 and 55

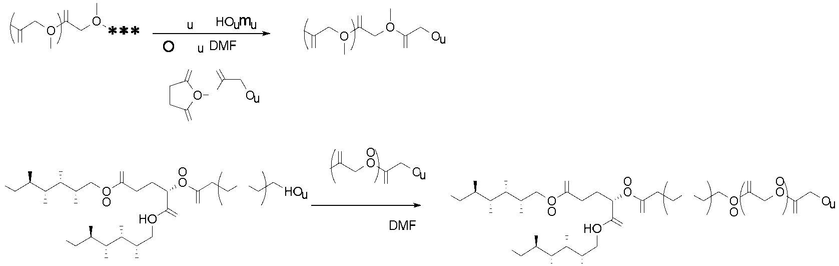

Compound **50** (1.1 g, 1.06 mmol, obtained from solid-phase peptide synthesis) and diethylamine (3.89 g, 53.24 mmol) were dissolved in dichloromethane (5 mL), and the reaction liquid was purged 3 times with N₂ and then reacted at 25 °C for 1 h. After the starting materials were completely converted, the reaction liquid was concentrated under reduced pressure to remove the solvent, and then the low-boiling compounds were removed under high vacuum. Compound **51** (3.15 g, 1.6 mmol) and *N,N*-dimethylformamide (4 mL) were then added, and the mixture was stirred. Triethylamine (3.23 g, 3.2 mmol) was added dropwise to the mixture at 25 °C. After 6 h of reaction, no starting material remained as detected by LCMS, and the excess triethylamine was removed by concentration under reduced pressure. The residue was directly separated by reversed-phase chromatography to give compound **52** (810 mg, yield: 10%). MS (ESI): (M+H)⁺, calculated 812.4, found 812.3.

Compound **53** (1.2 g, 1.36 mmol, synthesized with reference to the synthetic route reported in patent CN 111757757 A) and compound **54** (447 mg, 1.36 mmol, obtained from solid-phase peptide synthesis) were added to *N,N-*dimethylformamide (40 mL), and then 1-hydroxybenzotriazole (40 mg, 0.26 mmol) and 1-(3-dimethylaminopropyl)-3-ethylcarbodiimide hydrochloride (285 mg, 1.46 mmol) were added. The mixture was stirred for reaction at 25 °C for 16 h. After the reaction was stopped, the solvent was removed by concentration under reduced pressure, and the residue was separated by reversed-phase chromatography to give compound **55** (820 mg, yield: 50%). MS (ESI): (M+H)⁺, calculated 1208.5, found 1208.6.

### Example 8:

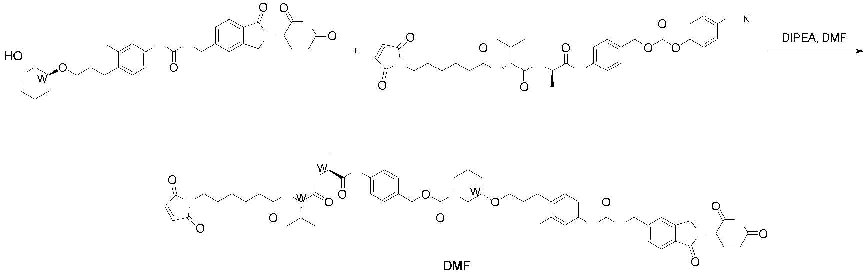

Compound 45 (0.1 mmol) was dissolved in DMF (2 mL), and linker-04 (0.11 mmol) was added to the solution, followed by the dropwise addition of DIPEA (0.3 mmol). The mixture was stirred for reaction. The reaction progress was detected by TLC and LCMS. After the reaction was completed, the mixture was added with ethyl acetate and a saturated sodium chloride solution for extraction. The organic phase was collected, dried over anhydrous sodium sulfate, and then filtered. The filtrate was concentrated under reduced pressure, and the residue was purified by silica gel column chromatography to give compound LD02 (0.085 mmol, 85%). LC-MS (ESI): (M+H)⁺, calculated 1080.4, found 1080.9.

### Example 9:

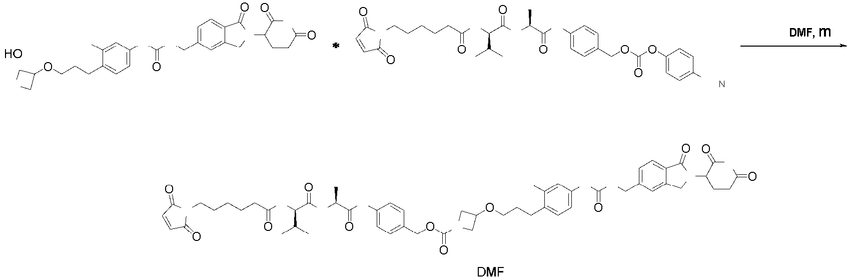

Compound 57 (0.1 mmol) was dissolved in DMF (2 mL), and linker-04 (0.11 mmol) was added to the solution, followed by the dropwise addition of DIPEA (0.3 mmol). The mixture was stirred for reaction. The reaction progress was detected by TLC and LCMS. After the reaction was completed, the mixture was added with ethyl acetate and a saturated sodium chloride solution for extraction. The organic phase was collected, dried over anhydrous sodium sulfate, and then filtered. The filtrate was concentrated under reduced pressure, and the residue was purified by silica gel column chromatography to give compound LD06 (0.083 mmol, 83%). LC-MS (ESI): (M+H)⁺, calculated 1052.4, found 1052.7.

### Example 10: Synthesis of Compound LD38

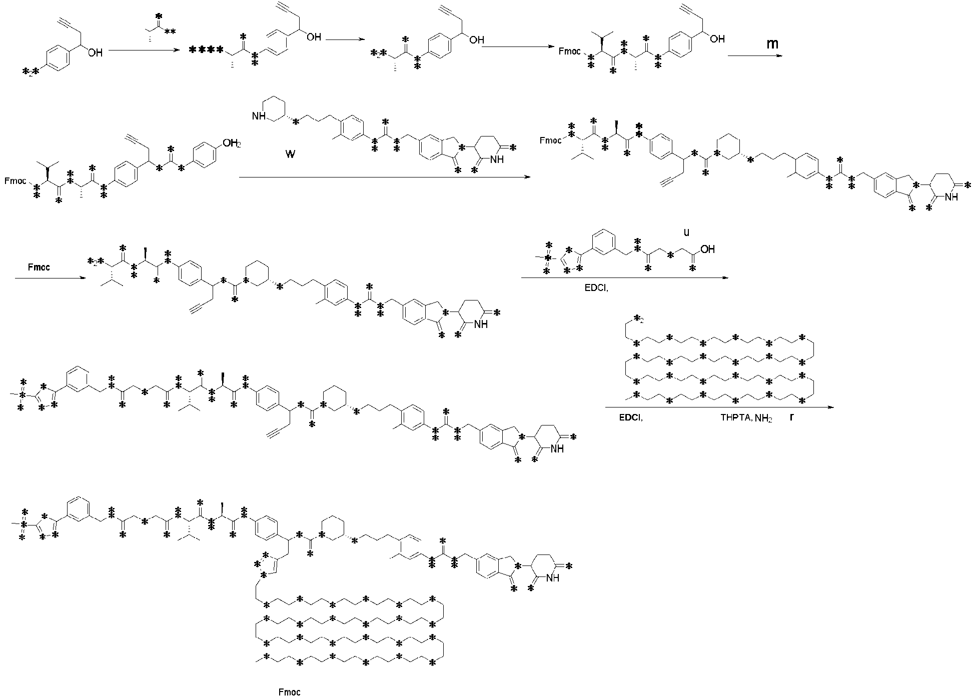

1-(4-Aminophenyl)-3-butyn-1-ol (4997 mg, 31 mmol), Fmoc-Ala-OH (9651 mg, 31 mmol), EEDQ (11498 mg, 46.5 mmol), and anhydrous DCM (90 mL) were added to a reaction flask at 0 °C. After 3 h of reaction, the solvent was removed by concentration under reduced pressure, and the residue was added with MTBE (500 mL) for slurrying to give compound **56** (9007 mg, yield: 64%, *dr =* 1:1). MS (ESI): (M+H)⁺, calculated 455.2, found 455.2. **56** (4000 mg, 8.8 mmol, *dr =* 1:1) and 100 mL of THF were added to a reaction flask at 0 °C. The mixture was stirred while DBU (1337 mg, 8.8 mmol) was slowly added. After half an hour of reaction, the mixture was warmed to room temperature. After the starting materials disappeared as monitored by TLC, the solvent was removed by concentration under reduced pressure, and the residue was purified by silica gel chromatography (DCM/MeOH 95:5) to give compound **57** (1776 mg, yield: 87%, *dr =* 1:1). MS (ESI): (M+H)⁺, calculated 233.1, found 233.2.

Compound **57** (1624 mg, 7 mmol, *dr =* 1:1), Fmoc-Val-OSu (362 mg, 8.4 mmol, CASRN: 688585-20-8), and *N,N-*dimethylformamide (150 mL) were added to a reaction flask. The mixture was stirred while DIEA (1158 µL, 7 mmol) was slowly added dropwise. The mixture was reacted at 25 °C for 12 h. The solvent was removed by concentration under reduced pressure, and 50 mL of EA and 50 mL of PE were added for slurrying, resulting in the precipitation of a white solid. The procedures were repeated three times to give compound **58** (*dr =* 1:1). MS (ESI): (M+H)⁺, calculated 554.3, found 554.4.

Compound **58** (1303 mg, 2.4 mmol, *dr =* 1:1), 4-nitrophenyl chloroformate (964.8 mg, 4.8 mmol), and THF (120 mL) were added to a reaction flask, and the mixture was stirred while Py (382.8 µL, 4.8 mmol) was added dropwise. The mixture was reacted at 65 °C for 1 h. The solvent was removed by concentration under reduced pressure, and the residue was purified by silica gel chromatography (DCM/MeOH 98:2) to give compound **59** (1430 mg, yield: *83%, dr =* 1:1). MS (ESI): (M+Na)⁺, calculated 741.3, found 741.5.

Compound **59** (3.72 g, 5.174 mmol), compound **45** (2.6 g, 4.311 mmol), and *N,N*-dimethylformamide (100 mL) were added to a reaction flask, and the mixture was stirred while DIPEA (1.668 g, 2.254 mL, 12.933 mmol) was slowly added dropwise. The mixture was reacted at 25 °C for 2 h. The solvent was removed by concentration under reduced pressure, and the residue was purified by silica gel chromatography to give compound **60** (3.86 g, yield: 79.52%). MS (ESI): (M+H)⁺, calculated 1147.5, found 1147.8.

Compound **60** (3.85 g, 3.419 mmol) and *N,N*-dimethylformamide (50 mL) were added to a reaction flask, and DBU (417.3 mg, 409.5 µL, 2.735 mmol) was added dropwise. The mixture was reacted at 0 °C for 0.5 h. After the reaction was completed as monitored by LCMS, methanesulfonic acid (590.8 mg, 399 µL, 6.154 mmol) was added to neutralize the reaction system, and the pH was measured to be about 7. The solvent was removed by concentration under reduced pressure, and the residue was slurried sequentially with MTBE and DCM. The slurry was filtered, and the solid was collected and dried to give compound 61 (2.728 g, yield: 78.22%). MS (ESI): (M+H)⁺, calculated 925.4, found 925.8.

Compound **61** (1.15 g, 1.125 mmol), compound 8 (622 mg, 1.687 mmol), and HOBT (30 mg, 0.225 mmol) were dissolved in *N,N*-dimethylformamide (22.5 mL), and EDC (262 mg, 298 µL, 1.687 mmol) was added. The mixture was reacted at 25 °C for 2 h. After the reaction was completed as monitored by LCMS, 110 mL of deionized water was placed in an ice bath, and the reaction liquid was slowly added dropwise to the deionized water. The mixture was stirred, resulting in the precipitation of a white solid. The mixture was then filtered, and the filter cake was washed with deionized water and n-hexane. The solid was collected and dried to give compound **62** (1.256 g, yield: 87.44%) as an off-white solid. MS (ESI): (M+H)⁺, calculated 1276.4, found 1276.8.

Compound **62** (896.3 mg, 0.7025 mmol), N₃-24PEG-OMe (860.9 mg, 0.7728 mmol), sodium ascorbate (27.8 mg, 0.1405 mmol), tris(3-hydroxypropyltriazolylmethyl)amine (61.0 mg, 0.1405 mmol), and copper(I) bromide (20.1 mg, 0.1405 mmol) were added to a reaction flask, and the mixture was purged three times with nitrogen. *tert*-Butanol (40 mL) and acetonitrile (40 mL) were added, and the mixture was reacted at 60 °C for 18 h. After the reaction was completed as monitored by LCMS, the solid was removed by filtration, and the solvent was removed by concentration under reduced pressure. Water (20 mL) was added for dilution, and formic acid was added for acidification until the pH was close to 5. The mixture was subjected to purification by reversed-phase chromatography, concentrated under reduced pressure to remove the solvent, and lyophilized to give compound **LD38** (703.4 mg, yield: 41.91%). MS (ESI): (M+2H)²⁺, calculated 1195.5, found 1195.9.

### Example 11: Synthesis of Compounds LD39-54

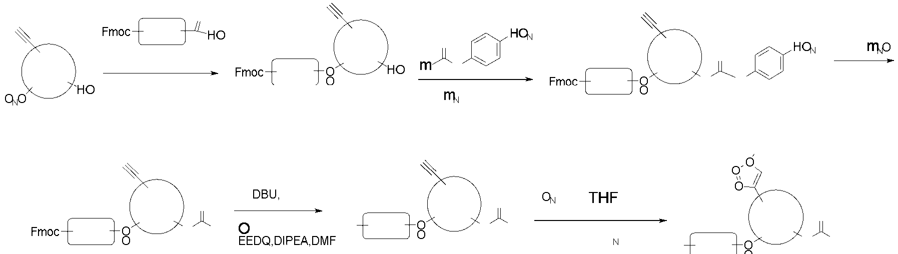

**Table 4**

| No. | **Con** | **Pep** | **Trig** | **HDP** | **Pay** | MS(ESI):(M+H)⁺ | |
|---|---|---|---|---|---|---|---|
| | | | | | | Calculated | Found |
| **LD39** | **Con2** | **Pep1** | **Trig1** | **HDP1** | **Pay8** | 2376.1 | 2376.1 |
| **LD40** | **Con1** | **Pep1** | **Trig1** | **HDP3** | **Pay8** | 2484.0 | 2484.0 |
| **LD41** | **Con2** | **Pep1** | **Trig1** | **HDP3** | **Pay8** | 2470.0 | 2470.1 |
| **LD42** | **Con1** | **Pep1** | **Trig1** | **HDP1** | **Pay9** | 2390.1 | 2390.1 |
| **LD43** | **Con1** | **Pep1** | **Trig1** | **HDP1** | **Pay10** | 2390.1 | 2390.1 |
| **LD44** | **Con1** | **Pep1** | **Trig1** | **HDP1** | **Pay11** | 2390.1 | 2390.2 |
| **LD45** | **Con1** | **Pep1** | **Trig1** | **HDP1** | **Pay12** | 2362.0 | 2362.2 |

For the synthetic routes of compounds **LD39-45,** reference was made to the synthesis of compound **LD38. Con1-5, Pep1-5, Trig1-3, HDP1-3, and Pay8-12** have structures shown as follows: 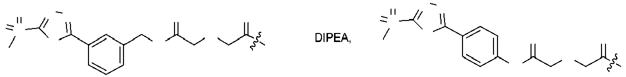 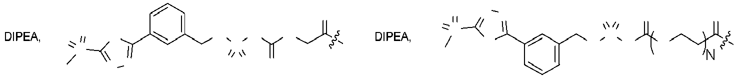 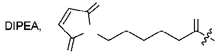 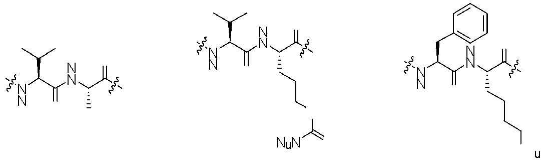 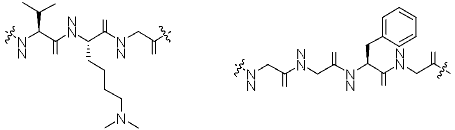 wherein the N-terminus is linked to Fmoc. 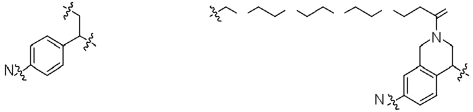 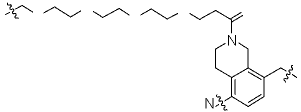 wherein position # is linked to -N₃, position ## is linked to -OH, and position ### is linked to alkynyl.

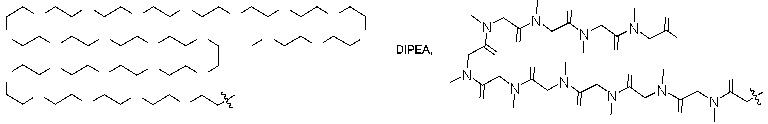

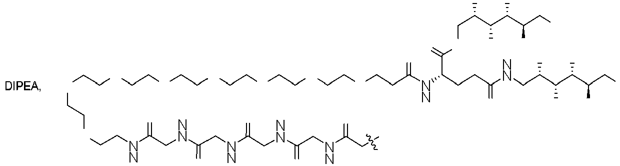

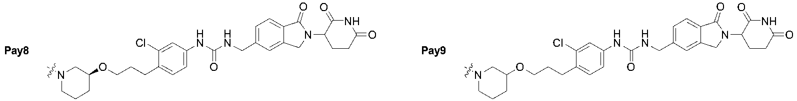

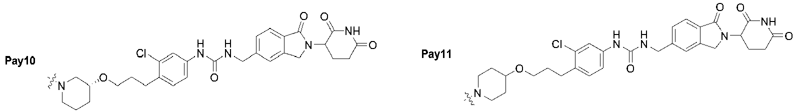

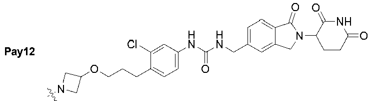

### Example 12: General Method for ADC Sample Preparation and DAR Determination

For an antibody with a known sequence, a protein expression method well known to those skilled in the art could be used to obtain a fermentation broth, which was then subjected to steps such as affinity chromatography and ion chromatography to obtain a sample with relatively high purity.

The antibody sample was diluted to about 10 mg/mL with a suitable buffer (consistent with the sample buffer), and an appropriate amount of a reducing agent TCEP was added, with the number of equivalents adjusted according to a target DAR (8-10 equivalents for a target DAR of 8). The pH was then adjusted to 7-7.4 with a Tris buffer, and the antibody was reduced at room temperature for 1-1.5 h. The reduced intermediate state of the antibody could be monitored by CE-SDS. After the antibody was completely reduced, an appropriate amount of a saturated citric acid solution was first added to adjust the pH to about 6.5, and then an excess of a solution of the linker-drug in DMSO was added to make the equivalents of the linker-drug 15-20 times those of the antibody. The conjugation reaction was performed at room temperature for about 30 min. After the conjugation was completed, the reaction liquid was first filtered, and then subjected to buffer exchange by ultrafiltration using a centrifugal concentration tube to remove excess linker-drug and other small-molecule impurities. After the purification was completed, the resulting sample was subjected to DAR determination by mass spectrometry. The statistical data for all ADC samples are shown in the table below.

**Table 5**

| **ADC drug No.** | **Linker-drug No.** | **Antibody A (target)** | **DAR** |
|---|---|---|---|
| **ADC38** | **LD38** | Trastuzumab (HER2) | 7.6 |
| **ADC39** | **LD39** | Trastuzumab (HER2) | 7.8 |
| **ADC40** | **LD40** | Trastuzumab (HER2) | 7.7 |
| **ADC41** | **LD41** | Trastuzumab (HER2) | 7.9 |
| **ADC42** | **LD42** | Trastuzumab (HER2) | 7.6 |
| **ADC43** | **LD43** | Trastuzumab (HER2) | 7.5 |
| **ADC44** | **LD44** | Trastuzumab (HER2) | 7.8 |
| **ADC45** | **LD45** | Trastuzumab (HER2) | 7.9 |
| **ADC55** | **LD38** | 018 (CD33) | 4.85 |
| **ADC56** | **LD38** | 019 (CD33) | 3.04 |
| **ADC57** | **LD38** | 020 (CD33) | 4.59 |
| **ADC58** | **LD38** | 021 (CD33) | 4.62 |
| **ADC59** | **LD38** | 022 (CD33) | 4.83 |
| **ADC60** | **LD38** | 023 (CD123) | 4.34 |
| **ADC62-4** | **LD38** | 008 (BCMA) | 3.54 |
| **ADC62** | **LD38** | 008 (BCMA) | 7.61 |
| **ADC64-4** | **LD40** | 008 (BCMA) | 4.01 |
| **ADC64** | **LD40** | 008 (BCMA) | 7.45 |
| **ADC65** | **LD06** | 019 (CD33) | 4.71 |
| **ADC66** | **LD06** | 022 (CD33) | 3.87 |
| **ADC67** | **LD02** | 023 (CD123) | 3.49 |

The amino acid sequences of the antibodies used for conjugation are as follows:
HER2
Trastuzumab
> Heavy chain (SEQ ID NO. 1)

EVQLVESGGGLVQPGGSLRLSCAASGFNIKDTYIHWVRQAPGKGLEWVARIYPTNGYTRYADSVKGR
FTISADTSKNTAYLQMNSLRAEDTAVYYCSRWGGDGFYAMDYWGQGTLVTVSSASTKGPSVFPLAPSS
KSTSGGTAALGCLVKDYFPEPVTVSWNSGALTSGVHTFPAVLQSSGLYSLSSVVTVPSSSLGTQTYICNV
NHKPSNTKVDKKVEPKSCDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKALPAPIEKTISKAK
GQPREPQVYTLPPSREEMTKNQVSLTCLVKGFYPSDIAVEWESNGQPENNYKTTPPVLDSDGSFFLYSKL
TVDKSRWQQGNVFSCSVMHEALHNHYTQKSLSLSPGK

The heavy chain variable region (VH) of the antigen-binding protein may comprise the following CDRs or variants of these CDRs (as defined by Kabat (Kabat et al.: Sequences of proteins of Immunological Interest, NIH, 1987)):
VHCDR1 is provided as SEQ ID NO. 21: GFNIKDTYIH
VHCDR2 is provided as SEQ ID NO. 22: RIYPTNGYTRYADSVKG
VHCDR3 is provided as SEQ ID NO. 23: WGGDGFYAMDY
> Light chain (SEQ ID NO. 2)

DIQMTQSPSSLSASVGDRVTITCRASQDVNTAVAWYQQKPGKAPKLLIYSASFLYSGVPSRFSGSRSGTD
FTLTISSLQPEDFATYYCQQHYTTPPTFGQGTKVEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFN
RGEC

The light chain variable region (VL) of the antigen-binding protein may comprise the following CDRs or variants of these CDRs (as defined by Kabat (Kabat et al.: Sequences of proteins of Immunological Interest, NIH, 1987)):
VLCDR1 is provided as SEQ ID NO. 24: RASQDVNTAVA
VLCDR2 is provided as SEQ ID NO. 25: SASFLYS
VLCDR3 is provided as SEQ ID NO. 26: QQHYTTPPT
Pertuzumab
> Heavy chain (SEQ ID NO. 3)

EVQLVESGGGLVQPGGSLRLSCAASGFTFTDYTMDWVRQAPGKGLEWVADVNPNSGGSIYNQRFKG
RFTLSVDRSKNTLYLQMNSLRAEDTAVYYCARNLGPSFYFDYWGQGTLVTVSSASTKGPSVFPLAPSSK
STSGGTAALGCLVKDYFPEPVTVSWNSGALTSGVHTFPAVLQSSGLYSLSSVVTVPSSSLGTQTYICNVN
HKPSNTKVDKKVEPKSCDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKALPAPIEKTISKAKG
QPREPQVYTLPPSREEMTKNQVSLTCLVKGFYPSDIAVEWESNGQPENNYKTTPPVLDSDGSFFLYSKLT
VDKSRWQQGNVFSCSVMHEALHNHYTQKSLSLSPG
The heavy chain variable region (VH) of the antigen binding protein comprises the following CDR sequences[?]

The heavy chain variable region (VH) of the antigen-binding protein may comprise the tollowing CDRs or variants of these CDRs (as defined by Kabat (Kabat et al.: Sequences of proteins of Immunological Interest, NIH, 1987)):
VHCDR1 is provided as SEQ ID NO. 27: GFTFTDYTMD
VHCDR2 is provided as SEQ ID NO. 28: DVNPNSGGSIYNQRFKG
VHCDR3 is provided as SEQ ID NO. 29: NLGPSFYFDY
> Light chain (SEQ ID NO. 4)

DIQMTQSPSSLSASVGDRVTITCKASQDVSIGVAWYQQKPGKAPKLLIYSASYRYTGVPSRFSGSGSGTD
FTLTISSLQPEDFATYYCQQYYIYPYTFGQGTKVEIKRTVAAPSVFIFPPSDEQLKSGTASVVCLLNNFYPR

EAKVQWKVDNALQSGNSQESVTEQDSKDSTYSLSSTLTLSKADYEKHKVYACEVTHQGLSSPVTKSFNR
GEC

The light chain variable region (VL) of the antigen-binding protein may comprise the following CDRs or variants of these CDRs (as defined by Kabat (Kabat et al.: Sequences of proteins of Immunological Interest, NIH, 1987)):
VLCDR1 is provided as SEQ ID NO. 30: KASQDVSIGVA
VLCDR2 is provided as SEQ ID NO. 31: SASYRYT
VLCDR3 is provided as SEQ ID NO. 32: QQYYIYPYT
CD33
014 (Gemtuzumab)
> Heavy chain (SEQ ID NO. 5)

EVQLVQSGAEVKKPGSSVKVSCKASGYTITDSNIHWVRQAPGQSLEWIGYIYPYNGGTDYNQKFKNRA
TLTVDNPTNTAYMELSSLRSEDTAFYYCVNGNPWLAYWGQGTLVTVSSASTKGPSVFPLAPCSRSTSES
TAALGCLVKDYFPEPVTVSWNSGALTSGVHTFPAVLQSSGLYSLSSVVTVPSSSLGTKTYTCNVDHKPSN
TKVDKRVESKYGPPCPPCPAPEFLGGPSVFLFPPKPKDTLMISRTPEVTCVVVDVSQEDPEVQFNWYVDG
VEVHNAKTKPREEQFNSTYRVVSVLTVLHQDWLNGKEYKCKVSNKGLPSSIEKTISKAKGQPREPQVYT
LPPSQEEMTKNQVSLTCLVKGFYPSDIAVEWESNGQPENNYKTTPPVLDSDGSFFLYSRLTVDKSRWQEG
NVFSCSVMHEALHNHYTQKSLSLS LGK

The heavy chain variable region (VH) of the antigen-binding protein may comprise the following CDRs or variants of these CDRs (as defined by Kabat (Kabat et al.: Sequences of proteins of Immunological Interest, NIH, 1987)):
VHCDR1 is provided as SEQ ID NO. 33: DSNIH
VHCDR2 is provided as SEQ ID NO. 34: YIYPYNGGTDYNQKFKN
VHCDR3 is provided as SEQ ID NO. 35: GNPWLAY
> Light chain (SEQ ID NO. 6)

DIQLTQSPSTLSASVGDRVTITC**RASESLDNYGIRFLT**WFQQKPGKAPKLLMY**AASNQGSG**VPSRFSGSG
SGTEFTLTISSLQPDDFATYYC**QQTKEVPWS**FGQGTKVEVKRTVAAPSVFIFPPSDEQLKSGTASVVCLL
NNFYPREAKVQWKVDNALQSGNSQESVTEQDSKDSTYSLSSTLTLSKADYEKHKVYACEVTHQGLSSP
VTKSFNRGEC

The light chain variable region (VL) of the antigen-binding protein may comprise the following CDRs or variants of these CDRs (as defined by Kabat (Kabat et al.: Sequences of proteins of Immunological Interest, NIH, 1987)):
VLCDR1 is provided as SEQ ID NO. 36: RASESLDNYGIRFLT
VLCDR2 is provided as SEQ ID NO. 37: AASNQGSG
VLCDR3 is provided as SEQ ID NO. 38: QQTKEVPWS
018 (CD33AB)
> Heavy chain (SEQ ID NO. 7)

EVQLVQSGAEVKKPGSSVKVSCKASGYTITDSNIHWVRQAPGQSLEWIGYIYPYNGGTDYNQKFKNRA
TLTVDNPTNTAYMELSSLRSEDTAFYYCVNGNPWLAYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSN
TKVDKKVEPKSCDKTHTCPPCPAPELLGGPSVFLFPPKPKDTLMISRTPEVTCVVVDVSHEDPEVKFNWY
VDGVEVHNAKTKPREEQYASTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSKLTVDKSR
WQQGNVFSCSVMHEALHNHYTQKSLSLSPGK

The heavy chain variable region (VH) of the antigen-binding protein may comprise the following CDRs or variants of these CDRs (as defined by Kabat (Kabat et al.: Sequences of proteins of Immunological Interest, NIH, 1987)):
VHCDR1 is provided as SEQ ID NO. 39: DSNIH
VHCDR2 is provided as SEQ ID NO. 40: YIYPYNGGTDYNQKFKN
VHCDR3 is provided as SEQ ID NO. 41: GNPWLAY
> Light chain (SEQ ID NO. 8)

DIQLTQSPSTLSASVGDRVTITCRASESLDNYGIRFLTWFQQKPGKAPKLLMYAASNQGSGVPSRFSGSG
SGTEFTLTISSLQPDDFATYYCQQTKEVPWSFGQGTKVEVKRTVAAPSVFIFPPSDEQLKSGTASVVCLL
NNFYPREAKVQWKVDNALQSGNSQESVTEQDSKDSTYSLSSTLTLSKADYEKHKVYACEVTHQGLSSP
VTKSFNRGEC

The light chain variable region (VL) of the antigen-binding protein may comprise the following CDRs or variants of these CDRs (as defined by Kabat (Kabat et al.: Sequences of proteins of Immunological Interest, NIH, 1987)):
VLCDR1 is provided as SEQ ID NO. 42: RASESLDNYGIRFLT
VLCDR2 is provided as SEQ ID NO. 43: AASNQGSG
VLCDR3 is provided as SEQ ID NO. 44: QQTKEVPWS
019
> Heavy chain (SEQ ID NO. 9)

QVQLQQPGAEVVKPGASVKMSCKASGYTFTSYYIHWIKQTPGQGLEWVGVIYPGNDDISYNQKFQGK
ATLTADKSSTTAYMQLSSLTSEDSAVYYCAREVRLRYFDVWGQGTTVTVSSASTKGPSVFPLAPCSRST
SESTAALGCLVKDYFPEPVTVSWNSGALTSGVHTFPAVLQSSGLYSLSSVVTVPSSSLGTKTYTCNVDHK
PSNTKVDKRVESKYGPPCPPCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPVLDSDGSFFLYSRLTVDKSRW
QEGNVFSCSVMHEALHNHYTQKSLSLSLGK

The heavy chain variable region (VH) of the antigen-binding protein may comprise the following CDRs or variants of these CDRs (as defined by Kabat (Kabat et al.: Sequences of proteins of Immunological Interest, NIH, 1987)):
VHCDR1 is provided as SEQ ID NO. 45: SYYIH
VHCDR2 is provided as SEQ ID NO. 46: VIYPGNDDISYNQKFQG
VHCDR3 is provided as SEQ ID NO. 47: EVRLRYFDV
> Light chain (SEQ ID NO. 10)

EIVLTQSPGSLAVSPGERVTMSCKSSQSVFFSSSQKNYLAWYQQIPGQSPRLLIYWASTRESGVPDRFTG
SGSGTDFTLTISSVQPEDLAIYYCHQYLSSRTFGQGTKLEIKRTVAAPSVFIFPPSDEQLKSGTASVVCLLN
NFYPREAKVQWKVDNALQSGNSQESVTEQDSKDSTYSLSSTLTLSKADYEKHKVYACEVTHQGLSSPVT
KSFNRGEC

The light chain variable region (VL) of the antigen-binding protein may comprise the following CDRs or variants of these CDRs (as defined by Kabat (Kabat et al.: Sequences of proteins of Immunological Interest, NIH, 1987)):
VLCDR1 is provided as SEQ ID NO. 48: KSSQSVFFSSSQKNYLA
VLCDR2 is provided as SEQ ID NO. 49: WASTRES
VLCDR3 is provided as SEQ ID NO. 50: HQYLSSRT
020
> Heavy chain (SEQ ID NO. 11)

QVQLVQSGAEVKKPGASVKVSCKASGYTFTNYDINWVRQAPGQGLEWIGWIYPGDGSTKYNEKFKAK
ATLTADTSTSTAYMELRSLRSDDTAVYYCASGYEDAMDYWGQGTTVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKP
SNTKVDKKVEPKSCDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALAASIEKTISKAKGQP
REPQVYTLPPSRDELTKNQVSLTCLVKGFYPSDIAVEWESNGQPENNYKTTPPVLDSDGSFFLYSKLTVD
KSRWQQGNVFSCSVMHEALHNHYTQKSLSLSPGK

The heavy chain variable region (VH) of the antigen-binding protein may comprise the following CDRs or variants of these CDRs (as defined by Kabat (Kabat et al.: Sequences of proteins of Immunological Interest, NIH, 1987)):
VHCDR1 is provided as SEQ ID NO. 51: NYDIN
VHCDR2 is provided as SEQ ID NO. 52: WIYPGDGSTKYNEKFKA
VHCDR3 is provided as SEQ ID NO. 53: GYEDAMDY
> Light chain SEQ ID NO. 12)

DIQMTQSPSSLSASVGDRVTINCKASQDINSYLSWFQQKPGKAPKTLIYRANRLVDGVPSRFSGSGSGQD
YTLTISSLQPEDFATYYCLQYDEFPLTFGGGTKVEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFN
RGEC

The light chain variable region (VL) of the antigen-binding protein may comprise the following CDRs or variants of these CDRs (as defined by Kabat (Kabat et al.: Sequences of proteins of Immunological Interest, NIH, 1987)):
VLCDR1 is provided as SEQ ID NO. 54: KASQDINSYLS
VLCDR2 is provided as SEQ ID NO. 55: RANRLVD
VLCDR3 is provided as SEQ ID NO. 56: LQYDEFPLT
021
> Heavy chain (SEQ ID NO. 13)

QVQLVQSGAEVKKPGSSVKVSCKASGYTFTDYNMHWVRQAPGQGLEWIGYIYPYNGGTGYNQKFKS
KATITADESTNTAYMELSSLRSEDTAVYYCARGRPAMDYWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKP
SNTKVDKKVEPKSCDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALAASIEKTISKAKGQP
REPQVYTLPPSRDELTKNQVSLTCLVKGFYPSDIAVEWESNGQPENNYKTTPPVLDSDGSFFLYSKLTVD
KSRWQQGNVFSCSVMHEALHNHYTQKSLSLSPGK

The heavy chain variable region (VH) of the antigen-binding protein may comprise the following CDRs or variants of these CDRs (as defined by Kabat (Kabat et al.: Sequences of proteins of Immunological Interest, NIH, 1987)):
VHCDR1 is provided as SEQ ID NO. 57: DYNMH
VHCDR2 is provided as SEQ ID NO. 58: YIYPYNGGTGYNQKFK
VHCDR3 is provided as SEQ ID NO. 59: GRPAMDY
> Light chain (SEQ ID NO. 14)

DIQMTQSPSSLSASVGDRVTITCRASESVDNYGISFMNWFQQKPGKAPKLLIYAASNQGSGVPSRFSGSG
SGTDFTLTISSLQPDDFATYYCQQSKEVPWTFGQGTKVEIKRTVAAPSVFIFPPSDEQLKSGTASVVCLLN
NFYPREAKVQWKVDNALQSGNSQESVTEQDSKDSTYSLSSTLTLSKADYEKHKVYACEVTHQGLSSPVT
KSFNRGEC

The light chain variable region (VL) of the antigen-binding protein may comprise the following CDRs or variants of these CDRs (as defined by Kabat (Kabat et al.: Sequences of proteins of Immunological Interest, NIH, 1987)):
VLCDR1 is provided as SEQ ID NO. 60: RASESVDNYGISFMN
VLCDR2 is provided as SEQ ID NO. 61: AASNQGS
VLCDR3 is provided as SEQ ID NO. 62: QQSKEVPWT
022
> Heavy chain (SEQ ID NO. 15)

QVQLQQPGAEVVKPGASVKMSCKASGYTFTSYYIHWIKQTPGQGLEWVGVIYPGNDDISYNQKFQGK
ATLTADKSSTTAYMQLSSLTSEDSAVYYCAREVRLRYFDVWGQGTTVTVSSASTKGPSVFPLAPSSKST
SGGTAALGCLVKDYFPEPVTVSWNSGALTSGVHTFPAVLQSSGLYSLSSVVTVPSSSLGTQTYICNVNHK
PSNTKVDKKVEPKSCDKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALAASIEKTISKAKGQ
PREPQVYTLPPSRDELTKNQVSLTCLVKGFYPSDIAVEWESNGQPENNYKTTPPVLDSDGSFFLYSKLTV
DKSRWQQGNVFSCSVMHEALHNHYTQKSLSLSPGK

The heavy chain variable region (VH) of the antigen-binding protein may comprise the following CDRs or variants of these CDRs (as defined by Kabat (Kabat et al.: Sequences of proteins of Immunological Interest, NIH, 1987)):
VHCDR1 is provided as SEQ ID NO. 63: SYYIH
VHCDR2 is provided as SEQ ID NO. 64: VIYPGNDDISYNQKFQG
VHCDR3 is provided as SEQ ID NO. 65: EVRLRYFDV
> Light Chain (SEQ ID NO. 16)

EIVLTQSPGSLAVSPGERVTMSCKSSQSVFFSSSQKNYLAWYQQIPGQSPRLLIYWASTRESGVPDRFTG
SGSGTDFTLTISSVQPEDLAIYYCHQYLSSRTFGQGTKLEIKRTVAAPSVFIFPPSDEQLKSGTASVVCLLN
NFYPREAKVQWKVDNALQSGNSQESVTEQDSKDSTYSLSSTLTLSKADYEKHKVYACEVTHQGLSSPVT
KSFNRGEC

The light chain variable region (VL) of the antigen-binding protein may comprise the following CDRs or variants of these CDRs (as defined by Kabat (Kabat et al.: Sequences of proteins of Immunological Interest, NIH, 1987)):
VLCDR1 is provided as SEQ ID NO. 66: KSSQSVFFSSSQKNYLA
VLCDR2 is provided as SEQ ID NO. 67: WASTRES
VLCDR3 is provided as SEQ ID NO. 68: HQYLSSRT
CD123
023
> Heavy chain (SEQ ID NO. 17)

QVQLVQSGAEVKKPGASVKVSCKASGYIFTSSIMHWVRQAPGQGLEWIGYIKPYNDGTKYNEKFKGR
ATLTSDRSTSTAYMELSSLRSEDTAVYYCAREGGNDYYDTMDYWGQGTLVTVSSASTKGPSVFPLAPSS
KSTSGGTAALGCLVKDYFPEPVTVSWNSGALTSGVHTFPAVLQSSGLYSLSSVVTVPSSSLGTQTYICNV
NHKPSNTKVDKKVEPKSCDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKALPAPIEKTISKAK

GQPREPQVYTLPPSRDELTKNQVSLTCLVKGFYPSDIAVEWESNGQPENNYKTTPPVLDSDGSFFLYSKL
TVDKSRWQQGNVFSCSVMHEALHNHYTQKSLCLSPG

The heavy chain variable region (VH) of the antigen-binding protein may comprise the following CDRs or variants of these CDRs (as
defined by Kabat (Kabat et al.: Sequences of proteins of Immunological Interest, NIH, 1987)):
VHCDR1 is provided as SEQ ID NO. 69: SSIMH
VHCDR2 is provided as SEQ ID NO. 70: YIKPYNDGTKYNEKFKG
VHCDR3 is provided as SEQ ID NO. 71: EGGNDYYDTMDY
> Light chain (SEQ ID NO. 18)

DIQMTQSPSSLSASVGDRVTITCRASQDINSYLSWFQQKPGKAPKTLIYRVNRLVDGVPSRFSGSGSGND
YTLTISSLQPEDFATYYCLQYDAFPYTFGQGTKVEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFN
RGEC

The light chain variable region (VL) of the antigen-binding protein may comprise the following CDRs or variants of these CDRs (as defined by Kabat (Kabat et al.: Sequences of proteins of Immunological Interest, NIH, 1987)):
VLCDR1 is provided as SEQ ID NO. 72: RASQDINSYLS
VLCDR2 is provided as SEQ ID NO. 73: RVNRLVD
VLCDR3 is provided as SEQ ID NO. 74: LQYDAFPYT
BCMA
008 (belantamab)
> Heavy chain (SEQ ID NO. 19)

QVQLVQSGAEVKKPGSSVKVSCKASGGTFSNYWMHWVRQAPGQGLEWMGATYRGHSDTYYNQKFK
GRVTITADKSTSTAYMELSSLRSEDTAVYYCARGAIYDGYDVLDNWGQGTLVTVSSASTKGPSVFPLAP
SSKSTSGGTAALGCLVKDYFPEPVTVSWNSGALTSGVHTFPAVLQSSGLYSLSSVVTVPSSSLGTQTYICN
VNHKPSNTKVDKKVEPKSCDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPPVLDSDGSFFLYSK
LTVDKSRWQQGNVFSCSVMHE ALHNHYTQKSLSLSPGK

The heavy chain variable region (VH) of the antigen-binding protein may comprise the following CDRs or variants of these CDRs (as defined by Kabat (Kabat et al.: Sequences of proteins of Immunological Interest, NIH, 1987)):
VLCDR1 is provided as SEQ ID NO. 75: NYWMH
VLCDR2 is provided as SEQ ID NO. 76: ATYRGHSDTYYNQKFKG
VLCDR3 is provided as SEQ ID NO. 77: GAIYDGYDVLDN
> Light chain (SEQ ID NO. 20)

DIQMTQSPSSLSASVGDRVTITCSASQDISNYLNWYQQKPGKAPKLLIYYTSNLHSGVPSRFSGSGSGTDF
TLTISSLQPEDFATYYCQQYRKLPWTFGQGTKLEIKRTVAAPSVFIFPPSDEQLKSGTASVVCLLNNFYPR
EAKVQWKVDNALQSGNSQESVTEQDSKDSTYSLSSTLTLSKADYEKHKVYACEVTHQGLSSPVTKSFNR
GEC

The light chain variable region (VL) of the antigen-binding protein may comprise the following CDRs or variants of these CDRs (as defined by Kabat (Kabat et al.: Sequences of proteins of Immunological Interest, NIH, 1987)):
VLCDR1 is provided as SEQ ID NO. 78: SASQDISNYLN
VLCDR2 is provided as SEQ ID NO. 79: YTSNLHS
VLCDR3 is provided as SEQ ID NO. 80: QQYRKLPWT

### Example 13: Preparation of Control ADC Samples

ORM-5029 was obtained with reference to the method in the document WO 2021/198965 A1, and ORM-6151 (using 018) was obtained with reference to the method in the document WO 2022/254376 A1. They served as control examples of the pay8-pay11 series of ADCs, and their DARs were 3.5 as determined by LC-MS. Moreover, low-DAR samples of ADC38-ADC44 were obtained under the same conjugation conditions and designated as ADC38-4 to ADC44-4, respectively, and their DARs were also all 3.5±0.1 as determined by LC-MS. In particular, when the linker-drug used in ORM-5029 was employed for antibody conjugation, qualified ADC samples with high Dar values (>7) could not be successfully obtained due to the relatively high aggregate ratio. Enhertu is a commercially available product.

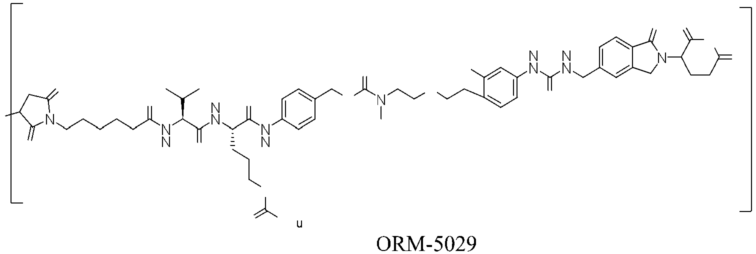

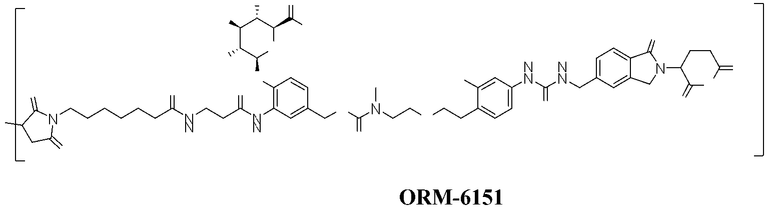

### Efficacy Example 1: Assay for In Vitro Activity of ADC Samples

1.1. SK-BR-3 cells with high HER2 expression were selected as test cells. The cells were cultured according to the general method and then seeded into a 96-well plate at an appropriate density. After the cell state was observed to be normal, the sample to be tested was added at a concentration gradient of 0-100 nM. After 144 h of culture, the cell viability was determined by the CTG method, and then the IC₅₀ value was calculated. See Table 6 below:

**Table 6**

| ADC sample No. | IC₅₀ |
|---|---|
| Enhertu | C |
| ORM-5029 | C |
| ADC38 | A |
| ADC38-4 | B |
| ADC39 | A |
| ADC39-4 | B |
| ADC40 | A |
| ADC40-4 | B |
| ADC41 | A |
| ADC41-4 | B |
| ADC42 | A |
| ADC42-4 | B |
| ADC43 | A |
| ADC43-4 | B |
| ADC44 | A |
| ADC44-4 | B |

| | |
|---|---|
| Note: In the table, A represents an IC₅₀ value of <15 pM, B represents an IC₅₀ value between 15 pM and 25 pM, and C represents an IC₅₀ value of >25 pM. | |

It can be seen from the activity assay results that the ADCs of the present disclosure exhibited a significant improvement in activity compared to the same-type ADC ORM-5029, and also exhibited a significant improvement in activity compared to Enhertu with the same target.

### In vitro killing activity against other HER2-positive cells

BT-474 and NCI-N87 cells with high HER2 expression and T-47D cells with low-to-moderate HER2 expression were selected as test cells. The cells were cultured according to the general method and then seeded into a 96-well plate at an appropriate density. After the cell state was observed to be normal, the sample to be tested was added at a concentration gradient of 0-100 nM. After 144 h of culture, the cell viability was determined by the CTG method, and then the IC₅₀ value was calculated.

**Table 7: BT-474**

| ADC sample No. | IC₅₀ (pM) |
|---|---|
| ADC38 | 0.8 |

**Table 8: NCI-N87**

| ADC sample No. | IC₅₀ (nM) |
|---|---|
| ADC38 | 0.4645 |

**Table 9: T-47D**

| ADC sample No. | IC₅₀ (nM) |
|---|---|
| ADC38 | 0.01676 |

It can be seen from the activity assay results that the ADC of the present disclosure had excellent *in vitro* killing activity against multiple cell lines.

1.2. NCI-H929 cells with high BCMA expression were selected as test cells. The cells were cultured according to the general method and then seeded into a 96-well plate at an appropriate density. After the cell state was observed to be normal, the sample to be tested was added at a concentration gradient of 0-100 nM. After 144 h of culture, the cell viability was determined by the CTG method, and then the IC₅₀ value was calculated.

**Table 10**

| Sample No. | IC₅₀ (nM) |
|---|---|
| ADC62-4 | 0.21 |
| ADC62 | 0.12 |
| ADC64-4 | 0.29 |
| ADC64 | 0.14 |

It can be seen from the activity assay results that the ADCs of the present disclosure all had good activity.

1.3. MV-4-11 cells with high CD33 expression were selected as test cells. The cells were cultured according to the general method and then seeded into a 96-well plate at an appropriate density. After the cell state was observed to be normal, the sample to be tested was added at a concentration gradient of 0-100 nM. After 144 h of culture, the cell viability was determined by the CTG method, and then the IC₅₀ value was calculated.

**Table 11: Cell activity of ADC samples**

| ADC sample No. | IC₅₀ (pM) |
|---|---|
| ORM-6151 | 20.77 |
| ADC55 | 1.475 |
| ADC56 | 1.404 |
| ADC57 | 5.355 |
| ADC59 | 2.486 |
| ADC60 | 0.2666 |
| ADC65 | 6.699 |
| ADC66 | 6.613 |
| ADC67 | 12.3 |

It can be seen from the activity assay results that the ADCs of the present disclosure had very excellent activity in such cells, and most of the samples exhibited a significant improvement in activity compared to the control sample ORM-6151.

### Efficacy Example 2: Assay for In Vivo Activity of ADC Samples

JIMT-1 cells with low-to-moderate HER2 expression were selected and inoculated into NUNU mice, and after tumorigenesis, mice with a tumor volume of about 150 mm³ were selected and divided into different groups, with 5 mice in each group. Different drugs were administered via tail vein injection at a preset dose. After administration, the tumor size and mouse body weight were observed and measured periodically. At the experimental endpoint, the final tumor size and mouse body weight were measured, and the tumor growth inhibition rate was calculated. The assay results are shown in Table 12 below:

**Table 12: In vivo efficacy of ADCs with non-hydrophilic linkers**

| **Group** | **Administration dose (mg/kg)** | **Administration frequency** | **TGI%** | | | | | | | |
|---|---|---|---|---|---|---|---|---|---|---|
| | | | **D0** | **D4** | **D7** | **D11** | **D14** | **D18** | **D21** | **D26** |
| Vehicle | - | D0, D11, D18 | | - | - | - | - | - | - | - |
| Enhertu | 3 | | | 44.8 | 71.9 | 81.4 | 95.6 | 96.0 | 86.0 | 74.5 |
| ORM-5029 | 3 | | | 86.8 | 47.8 | 24.6 | 30.8 | 27.9 | 23.7 | 22.4 |
| T-ADC | 3 | | | 119.8 | 97.3 | 61.3 | 79.3 | 75.6 | 69.0 | 65.0 |

T-ADC was prepared with reference to the method in the document WO 2021/198965 A1. The following linker-drug compound was first prepared: 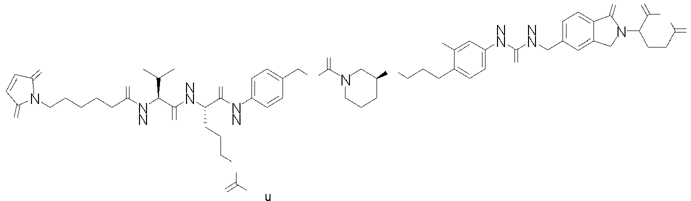 which was then conjugated to an antibody to give the target ADC:

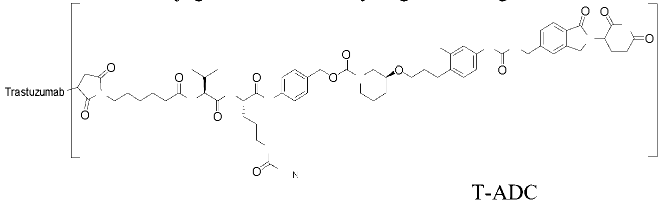

**Table 13: In vivo efficacy of ADCs with hydrochilic linkers**

| **Group** | **Administration dose (mg/kg)** | **Administration frequency** | **(1-T/C)% (D13)** |
|---|---|---|---|
| Vehicle | - | Single administration | - |
| Enhertu | 5 | | 76.5 |
| ADC38 | 5 | | 95 |
| ADC40 | 5 | | 90.1 |

It can be seen from the *in vivo* efficacy results in Table 12 that among the ADCs using non-hydrophilic linkers, when the linkers were identical, T-ADC exhibited significantly superior efficacy compared to ORM-5029 and slightly lower efficacy compared to Enhertu. It can be seen from the *in vivo* efficacy results in Table 13 that the ADCs using hydrophilic linkers exhibited superior efficacy compared to Enhertu. Based on the results in Tables 12 and 13, it can be concluded that ADCs using hydrophilic linkers exhibit significantly superior efficacy compared to ADCs using non-hydrophilic linkers.

### Efficacy Example 3: Assay for In Vivo Activity of ADC Samples

HCC-1954 cells were selected and inoculated into NUNU mice, and after tumorigenesis, mice with a tumor volume of about 100 mm³ were selected and divided into different groups, with 5 mice in each group. Different drugs were administered via tail vein injection at a preset dose. After administration, the tumor size and mouse body weight were observed and measured periodically. At the experimental endpoint, the tumor size and mouse body weight were measured, and the tumor growth inhibition rate was calculated. The assay results are shown in Table 14 below:

**Table 14: In vivo efficacy of ADCs with hydrophilic linkers**

| **Group** | **Administration dose (mg/kg)** | **Administration frequency** | **(1-T/C)% (D21)** |
|---|---|---|---|
| Vehicle | - | Single administration | - |
| ADC38 | 3 | | 88.0 |
| ADC44 | 3 | | 79.6 |

It can be seen from the *in vivo* efficacy results in Table 14 that the ADCs using hydrophilic linkers exhibited good efficacy.

### Efficacy Example 4: Assay for In Vivo Activity of ADC Samples

MV-4-11 cells were selected and inoculated into NOD SCID mice, and after tumorigenesis, mice with a tumor volume of about 100 mm³ were selected and divided into different groups, with 6 mice in each group. Different drugs were administered via tail vein injection at preset doses. After administration, the tumor volume and mouse body weight were observed and measured periodically. The assay results are shown in Table 15 below:

**Table 15: In vivo efficacy of ADCs with hydrophilic linkers**

| **Group** | **Administration dose (mg/kg)** | **Administration frequency** | **(1-T/C)% (D19)** |
|---|---|---|---|
| Vehicle | - | Single administration | - |
| ADC55 | 1 | | 108.6 |
| ADC57 | 1 | | 108.7 |
| ADC59 | 1 | | 108.3 |
| Azacitidine (IV) + Venetoclax (PO) | 8 / 50 | QW*3 / QD*14 | 75.9 |
| Vehicle | - | Single administration | - |
| ADC60 (CD123) | 5 | | 63.0 |

It can be seen from the *in vivo* efficacy results in Table 15 that the ADCs using hydrophilic linkers exhibited excellent therapeutic efficacy, and some of the samples showed significant advantages over the positive control therapy for this indication, and could achieve complete cure.

### Efficacy Example 5: Assay for In Vivo Activity of ADC Samples

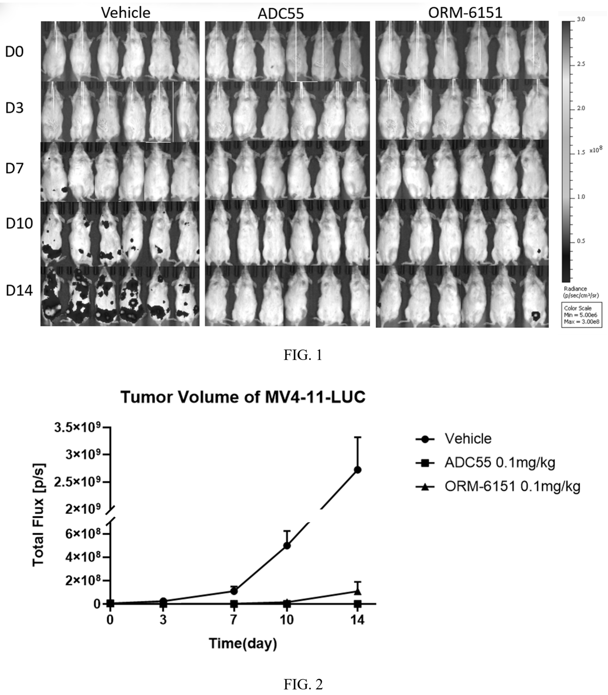

MV-4-11-luc cells were selected and inoculated into NCG mice via tail vein administration. With a fixed exposure time, when the Total Flux [p/s] in the mice was detected to be about 5E6, the mice were randomly divided into different groups, with 6 mice in each group. Different drugs were administered via tail vein injection at a preset dose. After administration, the mouse body weight and the Total Flux [p/s] in the mice were observed and measured periodically (FIGs. 1 and 2). At the experimental endpoint, the final fluorescence intensity and mouse body weight were measured, and the tumor growth inhibition rate was calculated. The assay results are shown in Table 16 below:

**Table 16: In vivo efficacy of ADCs with hydrophilic linkers**

| **Group** | **Administration dose (mg/kg)** | **Administration frequency** | **TGI% (D14)** |
|---|---|---|---|
| Vehicle | - | Single administration | - |
| ADC55 | 0.1 | | 100.2 |
| ORM-6151 | 0.1 | | 96.2 |

It can be seen from the *in vivo* efficacy results in Table 16 that the ADC using the hydrophilic linker exhibited excellent therapeutic efficacy, could achieve complete cure without relapse, and showed significant advantages over the control sample ORM-6151.

Efficacy Example 6: Evaluation of *In Vivo* PK Properties of Antibody-Drug Conjugates of the Present Disclosure T-ADC-2 was prepared with reference to the method in the document WO 2021/198965 A1. The following linker-drug compound was first prepared: 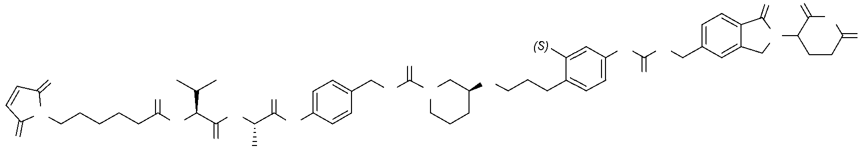 which was then conjugated to an antibody to give the target T-ADC-2:

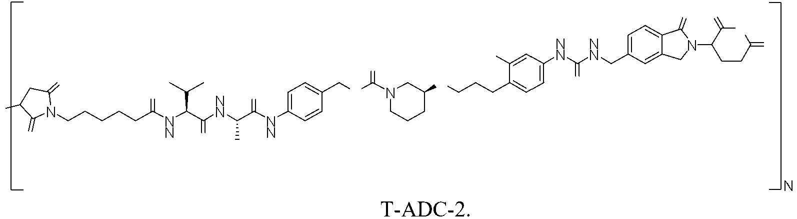

The pharmacokinetic behavior of the compounds ADC38-4, ADC38, T-ADC-2, and Enhertu in rats after a single intravenous injection at 6 mg/kg was studied. According to the experimental protocol, the rats were divided into 3 groups, with 3 male rats in each group. The assignment was performed randomly. The ADC compounds were injected into the rats via the tail vein at a dose of 6 mg/kg. The designed protocol is shown in Table 17. The animals were fasted for 2 h before administration (water was accessible). On the day of the experiment, the administration solutions were weighed according to the body weight of the animals.

**Table 17: Experimental protocol for in vivo PK of ADCs with hydrophilic linkers**

| Group | Administration dose | Administration route | Survival/Enrollment |
|---|---|---|---|
| ADC38-4 | 6 mg/kg | i.v. | 3/3 |
| ADC38 | | | 3/3 |
| T-ADC-2 | | | 3/3 |
| Enhertu | | | 3/3 |

Before administration and at 0.5 h, 18 h, 24 h, 48 h, 96 h and 168 h after administration, blood was collected via retro-orbital bleeding into citrate tubes and processed into plasma. The content of antibody-drug conjugate in the blood samples was determined using the enzyme-linked immunosorbent assay method, and the pharmacokinetic parameters were calculated by a non-compartmental model. The content of shed small molecules in the blood samples was determined using the LC-MS/MS method, and the pharmacokinetic parameters were calculated. The results are shown in Table 18.

The areas under the curves for the antibody-drug conjugate and the shed small molecules were converted to the same unit, and then the small molecule shedding rate was calculated based on the DAR value of the sample. The results are shown in Table 18.

**Table 18. Small molecule shedding rates of hydrophilic ADCs in rat PK model**

| Drug | AUC_{7day} (h*ug/mL) ADC | AUC_{7day} (h*pg/mL) Payload | AUC_{7day} (h*nmol/mL) ADC | AUC_{7day} (h*nmol/mL) Payload | Small molecule shedding rate (%) |
|---|---|---|---|---|---|
| ADC38-4 | 9222±319 | 6469±845 | 61.48 | 0.011 | 0.005 |
| ADC38 | 8282±511 | 18492±3469 | 55.21 | 0.033 | 0.007 |
| T-ADC-2 | 5306±427 | 28690±7387 | 35.37 | 0.051 | 0.018 |

By comparing the PK parameters of ADC38 and T-ADC-2, it can be found that the AUC of ADC38 was significantly higher than that of T-ADC-2 with the same high DAR value. By comparing ADC38 and ADC38-4, it can be found that increasing the DAR value from 4 to 8 did not significantly reduce the AUC of the ADC. This indicates that the introduction of a hydrophilic side chain can significantly improve the metabolic stability of ADCs with high DAR values. By comparing the small molecule shedding rates of ADC38 and T-ADC-2, it can be found that the use of the linker technology and hydrophilic side chain of the present disclosure can improve the *in vivo* stability of the ADC linker.

## Claims

1. An antibody-drug conjugate having a structure of formula (I), or a tautomer, a mesomer, a racemate, an enantiomer, a diastereoisomer, a pharmaceutically acceptable salt, a hydrate, a solvate or an isotopically labeled compound thereof:
A-(L-D)ₘ (I)
wherein A is a targeting ligand selected from an antibody (e.g., a monoclonal antibody) or an antigen-binding fragment, a small-molecule ligand, and a polypeptide;
L is a linker moiety, with one end linked to the ligand A and the other end linked to a bioactive molecule D;
D is an amino-containing bioactive molecule or a tautomer, a mesomer, a racemate, an enantiomer, a diastereoisomer or an isotopically labeled compound thereof, covalently linked to the linker moiety L via the amino group in its molecular structure;
m is an integer or a decimal from 1 to 12;
the linker moiety -L- is represented by the following formula:
-L₁-L₂-L₃-L₄-,
wherein L₁ is selected from 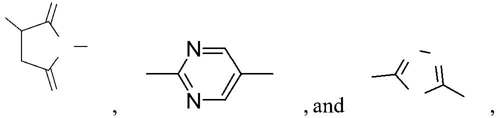 wherein * represents linkage to a sulfhydryl group of A (e.g., a monoclonal antibody), and ** represents linkage to L₂;
L₂ is a spacer selected from -L₂ₐ-C(O)-, -L₂ₐ-L₂ₚ-C(O)-, -L₂ₐ-NH-C(O)-, -L₂ₐ-L_{2b}-NH-C(O)-, -L₂ₐ-L_{2b}-C(O)-NH-, - L₂ₐ-C(O)-NH-L_{2b}-C(O)-, -L₂ₐ-NH-C(O)-L_{2b}-C(O)-, -L₂ₐ-C(O)-NH-L_{2b}-C(O)-NH-, -L₂ₐ-NH-C(O)-L_{2b}-NH-C(O)-, - L₂ₐ-NR¹-SO₂-NH-C(O)-O-L_{2b}-C(O)-, and -L₂ₐ-NR¹-SO₂-NH-C(O)-O-L_{2b}-NH-C(O)-, wherein L₂ₐ is selected from -C₁-C₈ alkylene-, -C₁-C₈ alkylene-C₃-C₈ cycloalkylene-, -C₆-C₁₄ arylene-, -C₆-C₁₄ arylene-C₁-C₈ alkylene-, -(5- to 6-membered heteroarylene)-, -(5- to 6-membered heteroarylene)-C₁-C₈ alkylene-, linear or branched heteroalkylene having 1-50 atoms, and (linear or branched heteroalkylene having 1-50 atoms)-(3- to 8-membered heterocyclylene), wherein the alkylene, cycloalkylene, arylene, heteroalkylene, heteroarylene, and heterocyclylene are each optionally substituted with one or more substituents independently selected from halogen, C₁-C₆ alkyl, heteroalkyl having 1-6 atoms, C₁-C₆ alkoxy, hydroxyl, amino, carboxyl, and C₃-C₈ cycloalkyl, the heteroalkylene, heterocyclylene, or heteroalkyl contains 1-12 heteroatoms, and the heteroatom in the heteroalkylene, heterocyclylene, heteroarylene, or heteroalkyl is selected from one or more of N, O, and S; L_{2b} is selected from -C₁-C₈ alkylene- and linear or branched heteroalkylene having 1-50 atoms, and R¹ is selected from hydrogen, C₁-C₆ alkyl, C₃-C₈ cycloalkyl, 3- to 8-membered heterocyclyl, C₁-C₆ haloalkyl, heteroalkyl having 2-8 atoms, C₆-C₁₄ aryl, and 5- to 6-membered heteroaryl, wherein the alkyl, heteroalkyl, aryl, heteroaryl, cycloalkyl, and heterocyclyl are each optionally substituted with one or more substituents independently selected from C₁-C₆ alkyl, heteroalkyl having 2-6 atoms, C₁-C₆ alkoxy, amino, and carboxyl, the heteroalkyl or heteroalkylene contains 1-12 heteroatoms, and the heteroatom in the heteroalkyl, heterocyclyl, heteroaryl, or heteroalkylene is selected from one or more of N, O, and S;
L₃ is a polypeptide sequence selected from a peptide residue consisting of 2-8 natural or unnatural amino acids, wherein optionally, the amino acid is further substituted with one or more substituents selected from C₁-C₆ alkyl, heteroalkyl having 2-6 atoms, C₁-C₆ alkoxy, hydroxyl, amino, carboxyl, and C₃-C₈ cycloalkyl;
L₄ is a self-immolative fragment, and
further preferably, L₄ is a hydrophilic group-modified self-immolative fragment;
the self-immolative fragment is selected from: 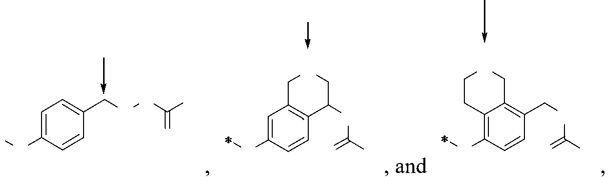
wherein * represents linkage to a carboxyl group of L₃ via an amide bond, ** represents linkage to the amino group of the bioactive molecule D, X is absent or 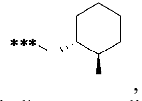 *** represents linkage to a carbon atom, **** represents linkage to an oxygen atom, and the arrow indicates a modification site of the hydrophilic group;
further preferably, the self-immolative fragment, prior to modification by the hydrophilic group, is selected from the following structures: 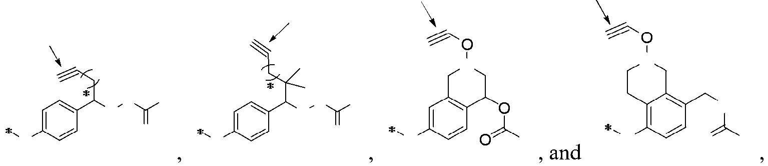
wherein Y is C₁-C₆ alkylene or -R³-C(O)-, wherein R³ is C₁-C₆ alkylene or heteroalkylene containing 1-8 - OCH₂CH₂- structural units; n is an integer from 0 to 6;
the hydrophilic group has at least one azido group and comprises a polyethylene glycol group, a poly-natural or unnatural amino acid group, a monosaccharide, an oligosaccharide, or a polysaccharide, or a combination thereof; preferably, L₄ is selected from: 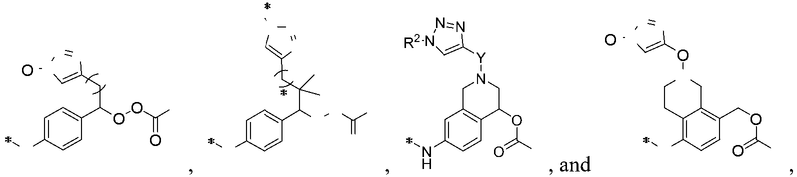
wherein * represents linkage to a carboxyl group of L₃ via an amide bond, and ** represents linkage to the amino group of the bioactive molecule D;
R² is a hydrophilic fragment selected from linear or branched heteroalkyl containing 4-50 -OCH₂CH₂- structural units, a peptide chain containing 4-50 proteinogenic amino acids or non-proteinogenic amino acids, and linear or branched heteroalkyl containing a monosaccharide, an oligosaccharide, or a polysaccharide; X is absent or 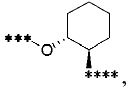 *** represents linkage to a carbon atom, and **** represents linkage to an oxygen atom; Y is C₁-C₆ alkylene or -R³-C(O)-, wherein R³ is C₁-C₆ alkylene or heteroalkylene containing 1-8 -OCH₂CH₂- structural units; n is an integer from 0 to 6;
when A is selected from an antibody targeting CD33 or CD123, L₄ may also be: 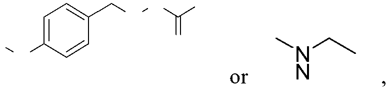
wherein * represents linkage to a carboxyl group of L₃ via an amide bond, ** represents linkage to the amino group or a hydroxyl group of the bioactive molecule D, X is absent or 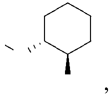 *** represents linkage to a carbon atom, and **** represents linkage to an oxygen atom;
the bioactive molecule D is selected from a molecular glue compound, the molecular glue compound having a structure represented by the following formula (A-1): 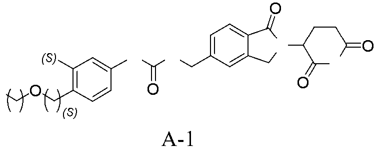
wherein RA is selected from optionally substituted 3- to 16-membered heterocyclyl, wherein the heteroatom in the heterocyclyl is selected from nitrogen; m1 and n1 are each independently an integer selected from 0-6.

2. The antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to claim 1, wherein A is selected from an antibody or an antigen-binding fragment thereof targeting HER2 (ErbB2), HER3 (ErbB3), HER4 (ErbB4), EGFR, DLL3, TROP2, B7-H3, c-Met, CD20, CD22, CD30, CD33, CD123, CD44, CD47, CD56, CD70, CD73, CD79b, CD105, CEA, A33, Cripto, EphA2, G250, MUC1, Lewis Y, VEGFR, VEGF, PD-1, PD-L1, MET, RET, GPNMB, Integrin, PSMA, Tenascin-C, SLC44A4, or Mesothelin.

3. The antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to any one of claims 1-2, wherein L₂ is selected from the following structures: 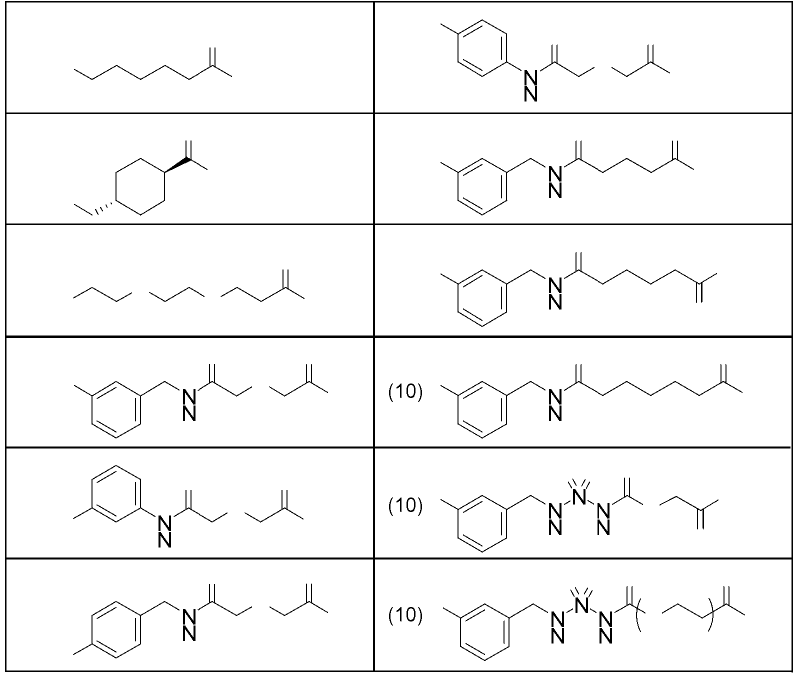 wherein w is an integer selected from 1-12, * represents linkage to L₁, and ** represents linkage to L₃.

4. The antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to any one of claims 1-3, wherein L₃ is selected from the following structures: 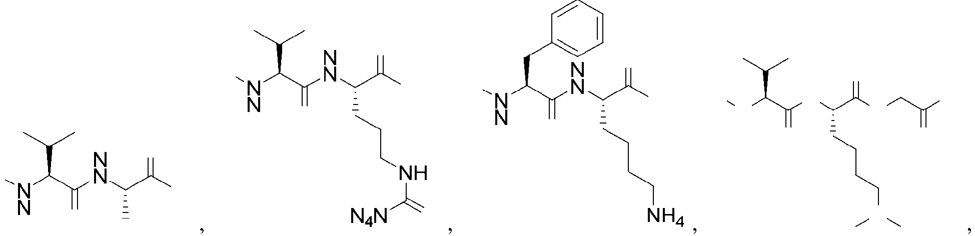 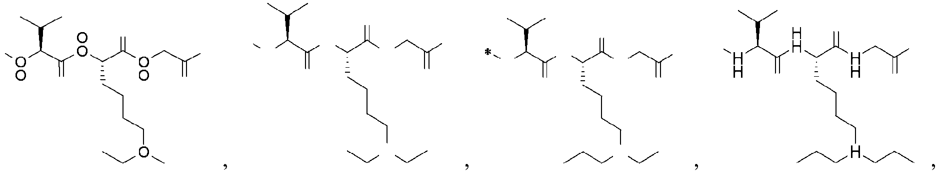 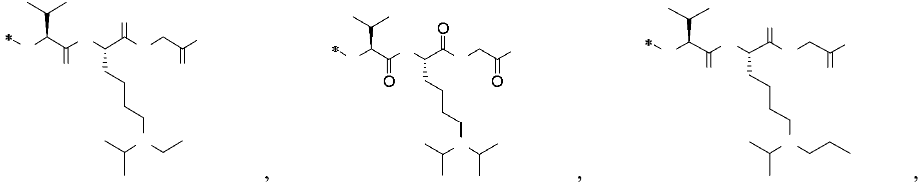 and 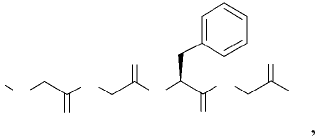 wherein * represents linkage to L₂, and ** represents linkage to L₄.

5. The antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to any one of claims 1-4, wherein L₄ is selected from the following structures: 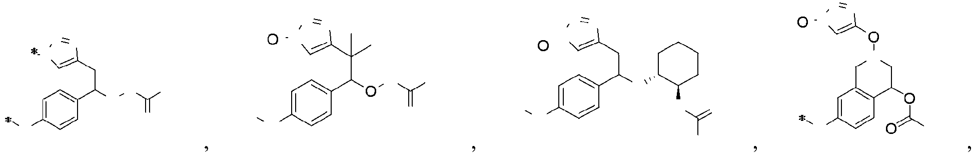 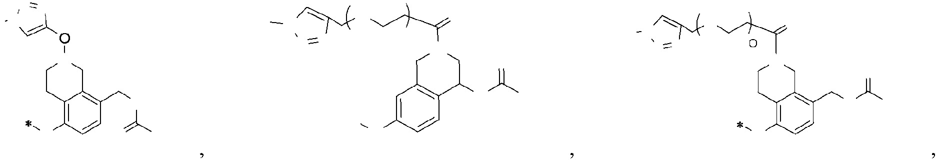 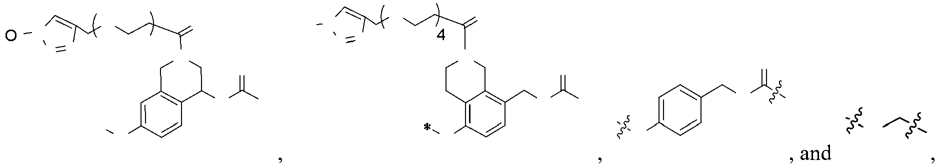 wherein v is an integer selected from 1-12, * represents linkage to a carboxyl group of L₃ via an amide bond, and ** represents linkage to the amino group of the bioactive molecule D.

6. The antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to any one of claims 1-5, wherein R² is selected from the following structures: 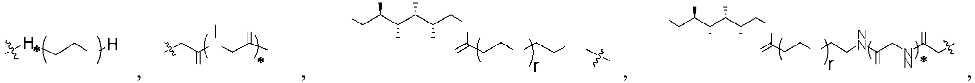 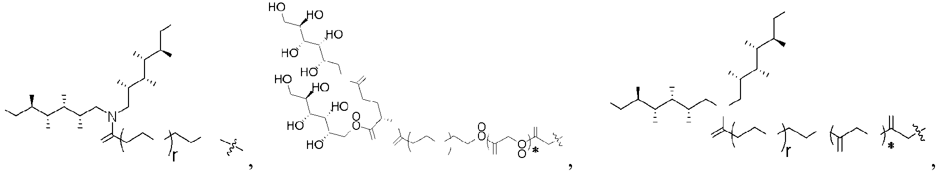 and 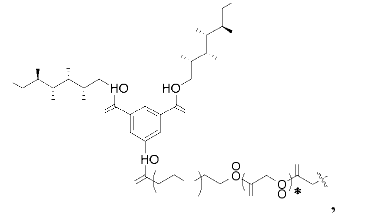 wherein r, s, t, and u are each independently an integer selected from 1-50; Rₐ is selected from a bond and C₁-C₃ alkylene, such as methylene, ethylidene, n-propylidene, or isopropylidene; R_{b} is selected from C₁-C₃ alkyl, such as methyl, ethyl, n-propyl, or isopropyl.

7. The antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to any one of claims 1-6, wherein L is selected from the following structures: 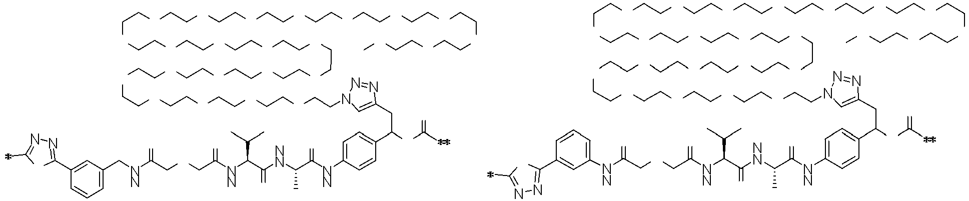 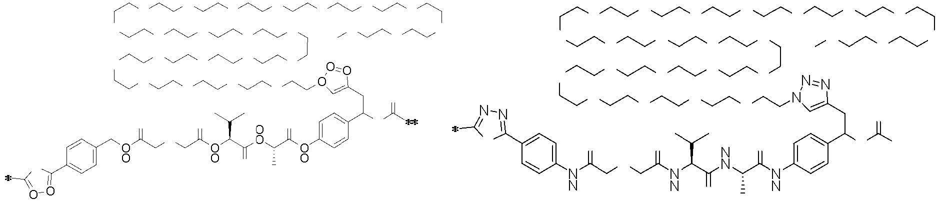 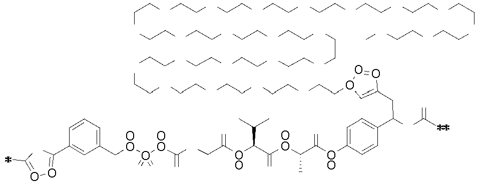 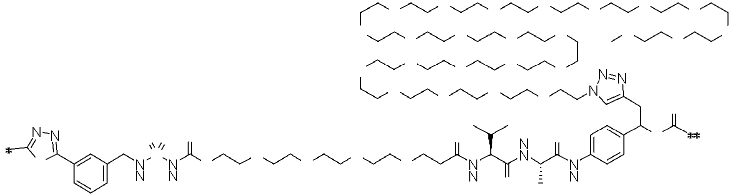 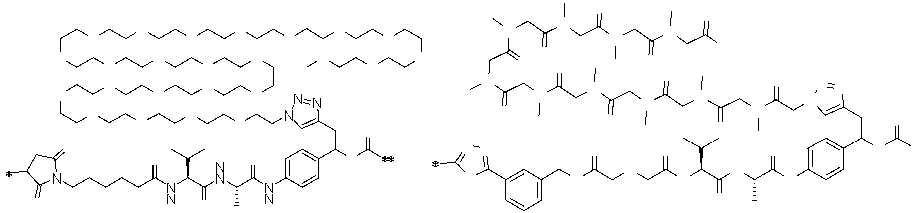 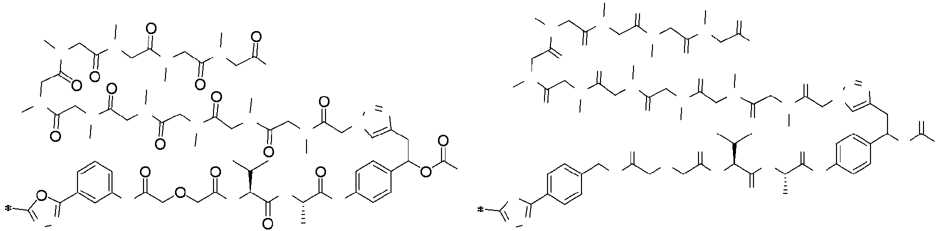 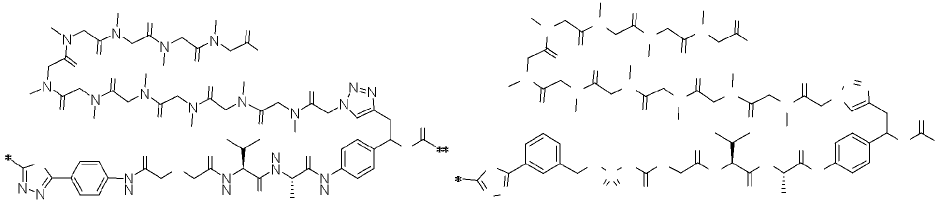 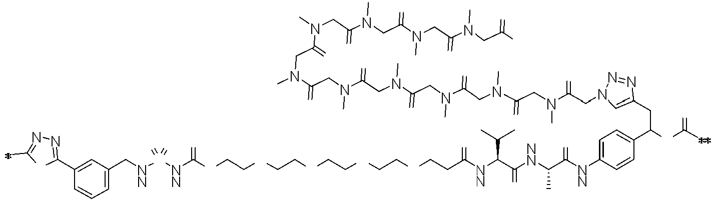 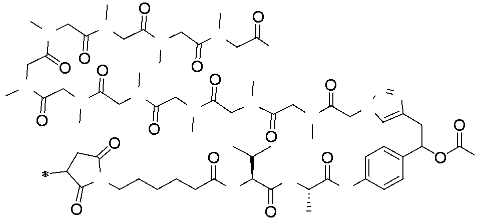 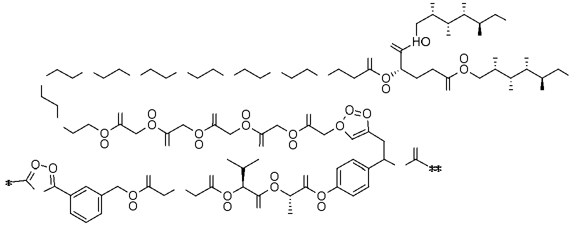 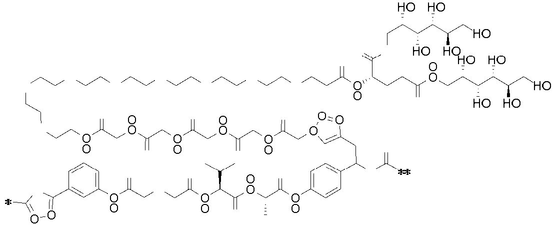 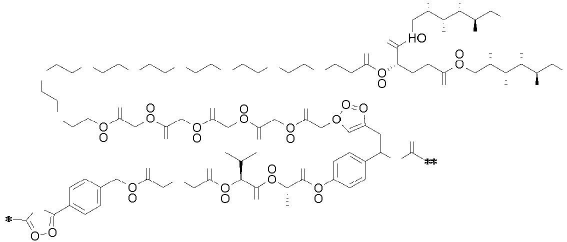 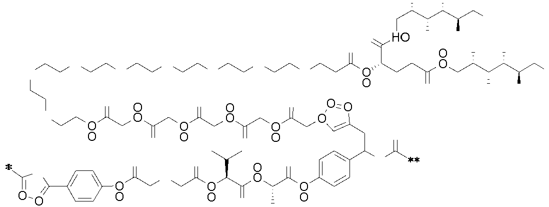 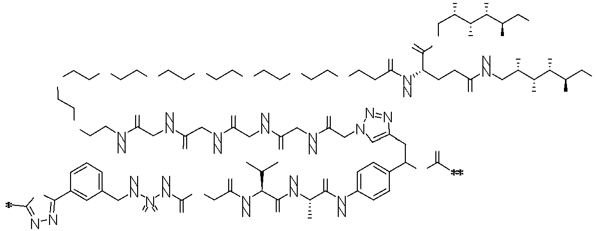 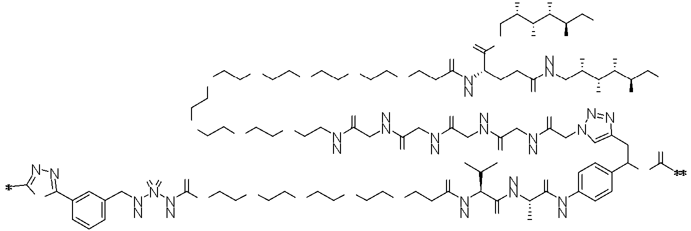 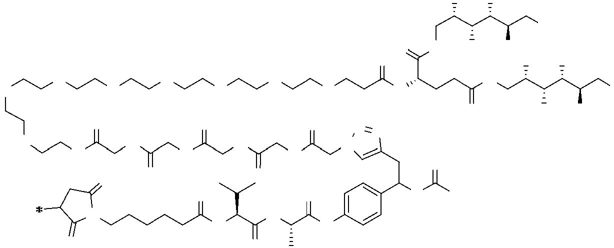 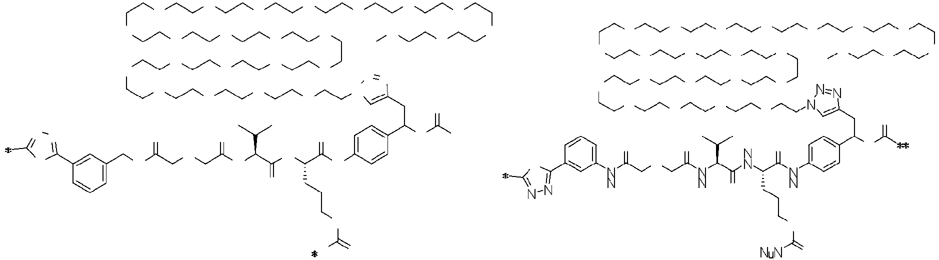 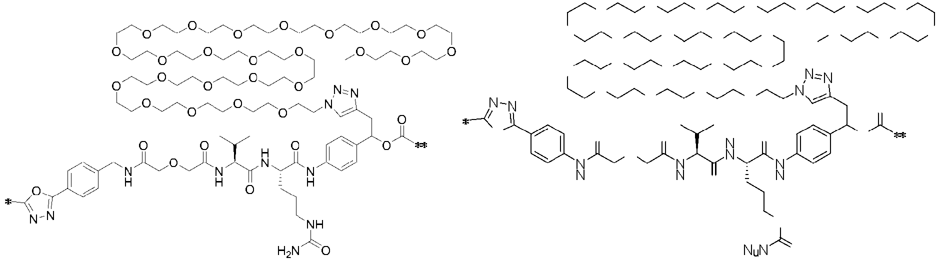 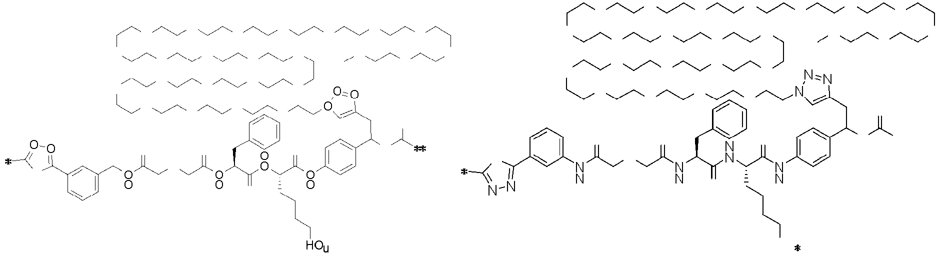 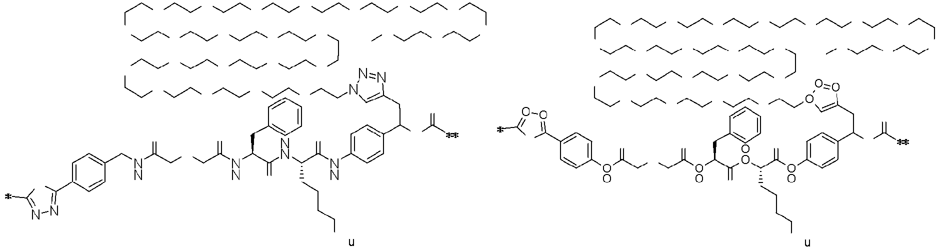 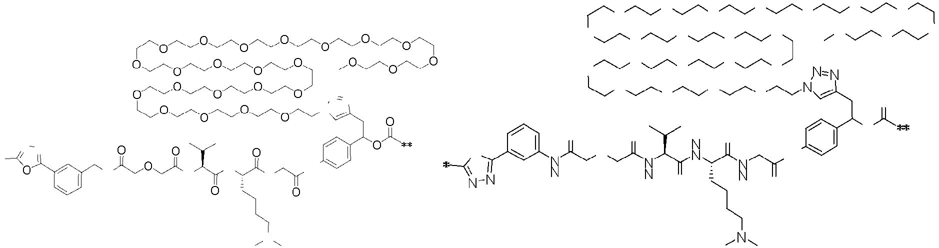 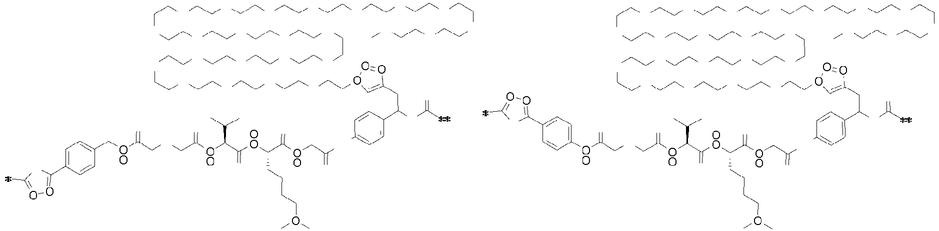 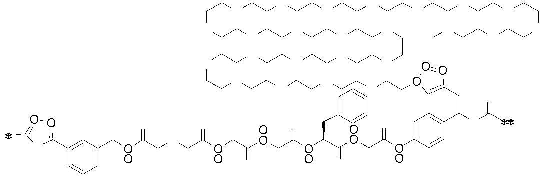 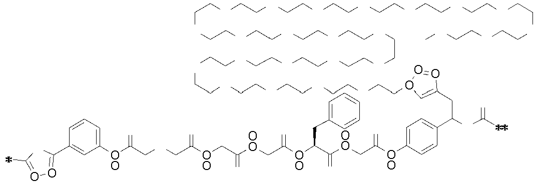 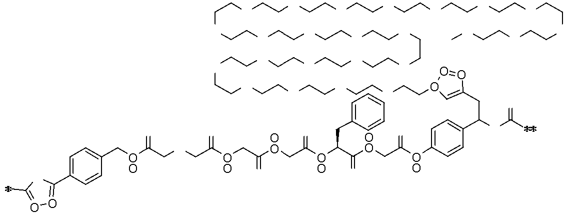 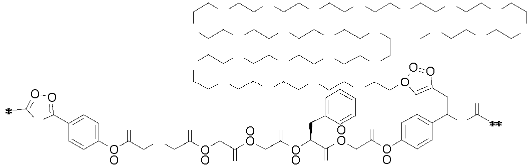 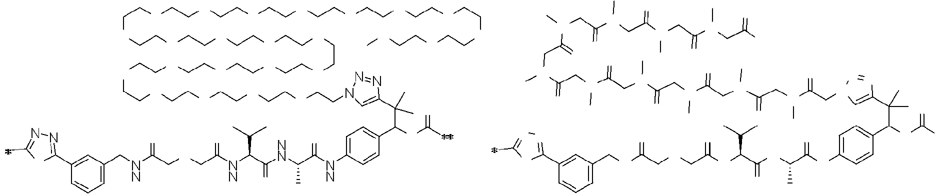 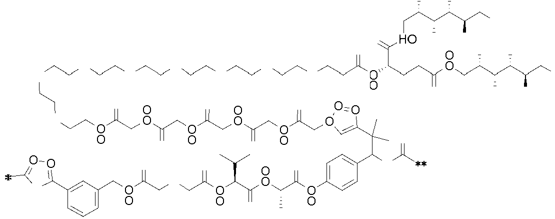 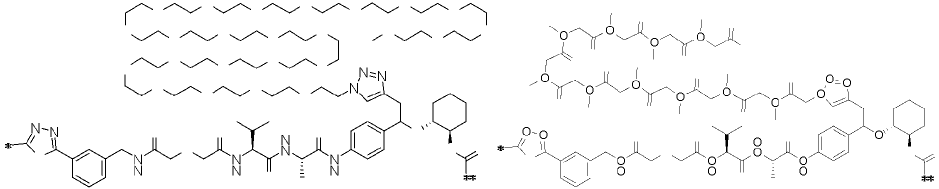 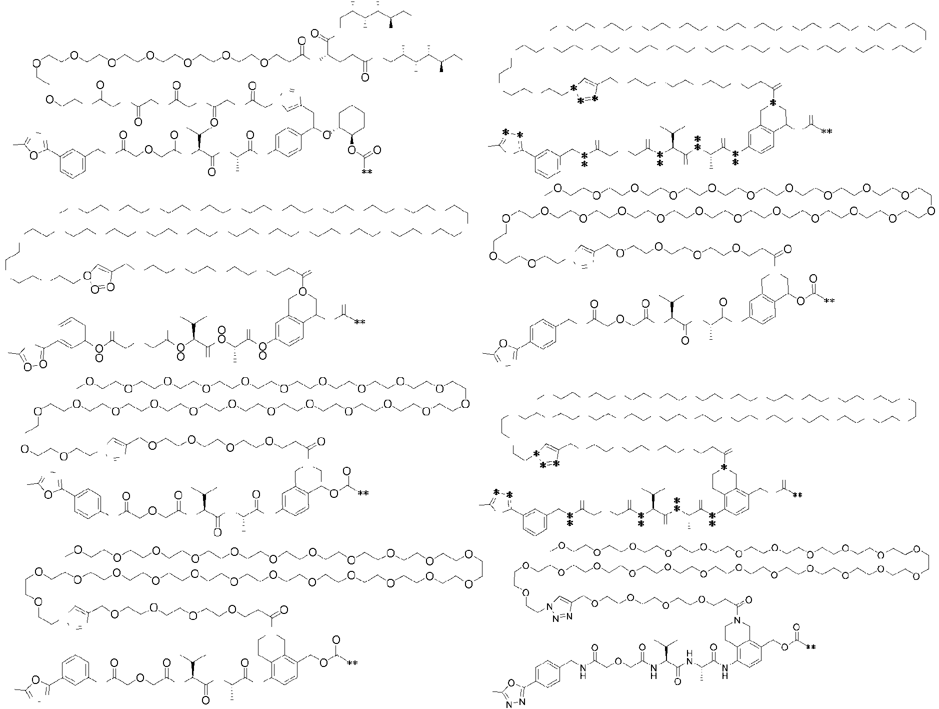 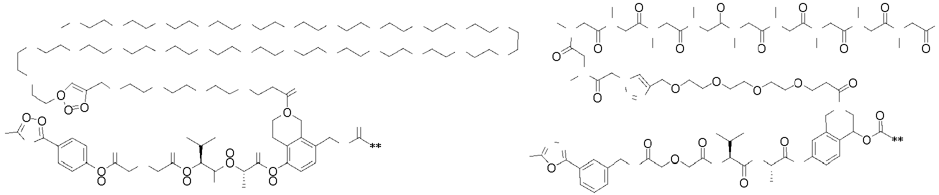 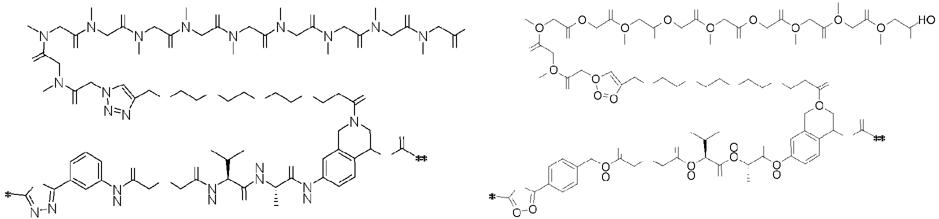 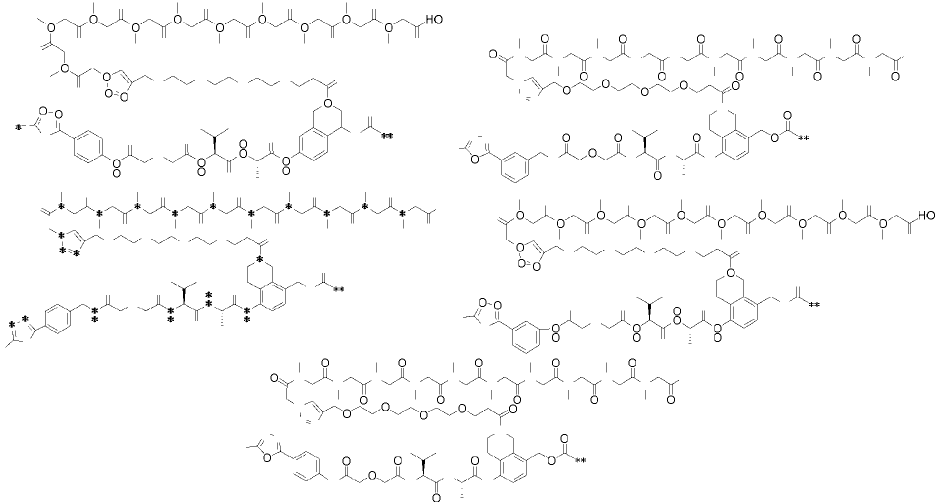 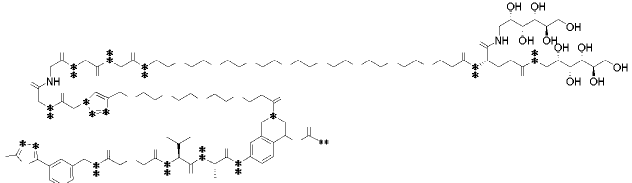 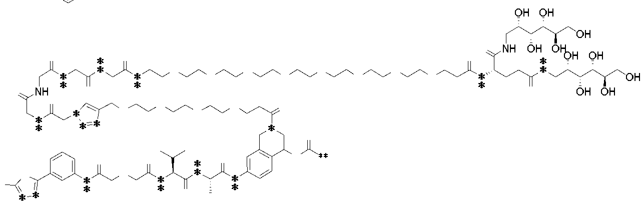 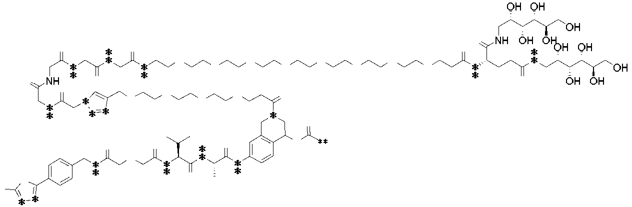 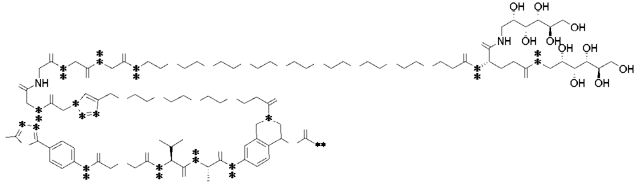 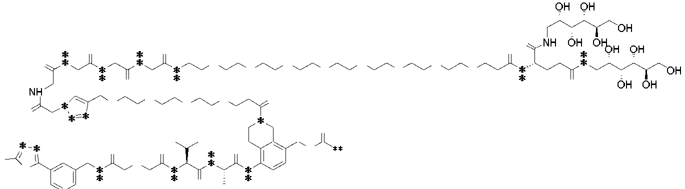 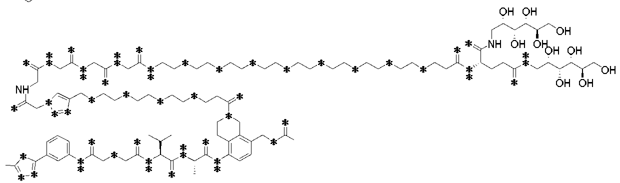 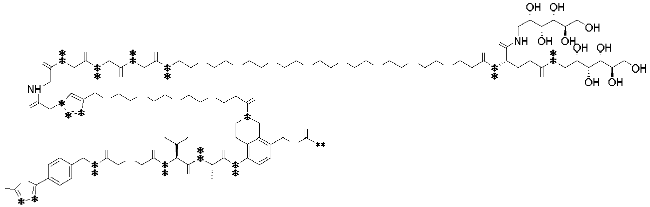 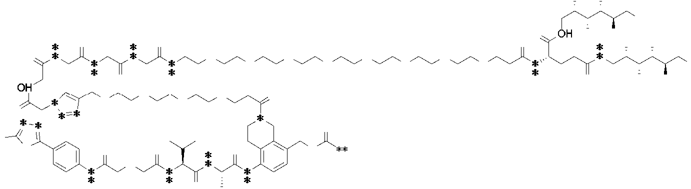 wherein * represents linkage to a sulfhydryl group of the antibody A, and ** represents linkage to the amino group of the bioactive molecule D.

8. The antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to any one of claims 1-7, wherein the molecular glue compound has a structure represented by formula (A-1); in the structure represented by formula (A-1), RA is selected from the following optionally substituted substituents: 3- to 8-membered monocyclic heterocyclyl, 6- to 14-membered spiro heterocyclyl, 5- to 14-membered fused heterocyclyl, and 5- to 14-membered bridged heterocyclyl, wherein the heteroatom in the heterocyclyl is selected from nitrogen;
preferably, RA is selected from optionally substituted 5- to 6-membered monocyclic heterocyclyl;
preferably, the optionally substituted substituent is selected from H and C₁₋₃ alkyl, such as methyl, ethyl, n-propyl, or isopropyl;
preferably, the compound having the structure represented by formula (A-1) has a structure represented by formula (A-2) or (A-3): 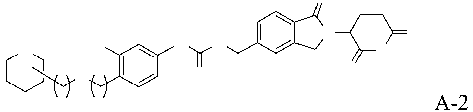 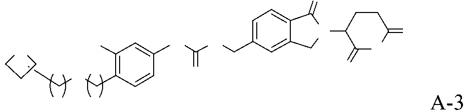 wherein m1 and n1 are each independently an integer selected from 0-6;
preferably, the compound having the structure represented by formula (A-1) has a structure represented by formula (A-4), (A-5), or (A-6): 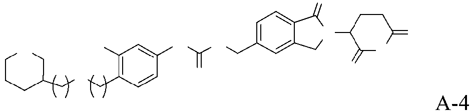 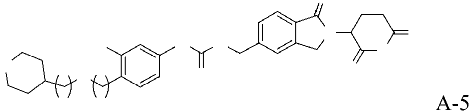 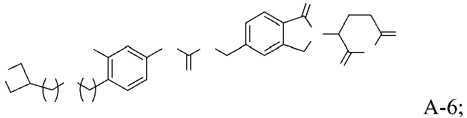
preferably, the bioactive molecule D is selected from compounds of the following formulas: 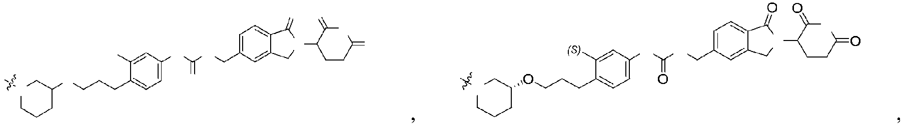 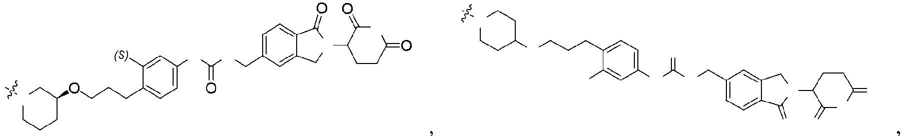
and

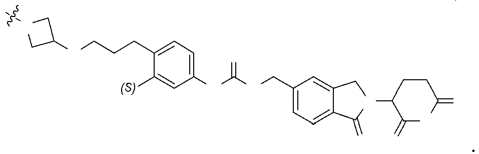

9. The antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to any one of claims 1-8, wherein A is selected from an antibody or an antigen-binding fragment thereof targeting Her2, BCMA, CD33, or CD123;
preferably, in the order of VHCDR1, VHCDR2, VHCDR3, VLCDR1, VLCDR2, and VLCDR3, a CDR combination of a heavy chain variable region VH and a light chain variable region of the antibody or the antigen-binding fragment thereof is as follows:
CDR combination I: SEQ ID NOs. 21-26,
CDR combination II: SEQ ID NOs. 27-32,
CDR combination III: SEQ ID NOs. 33-38,
CDR combination IV: SEQ ID NOs. 39-44,
CDR combination V: SEQ ID NOs. 45-50,
CDR combination VI: SEQ ID NOs. 51-56,
CDR combination VII: SEQ ID NOs. 57-62,
CDR combination VIII: SEQ ID NOs. 63-68,
CDR combination IX: SEQ ID NOs. 69-74, or
CDR combination X: SEQ ID NOs. 75-80;
also preferably, A is selected from Trastuzumab (combination of heavy and light chains: SEQ ID NOs. 1-2), 007 (pertuzumab, combination of heavy and light chains: SEQ ID NOs. 3-4), 014 (Gemtuzumab, combination of heavy and light chains: SEQ ID NOs. 5-6), 018 (CD33AB, combination of heavy and light chains: SEQ ID NOs. 7-8), 019 (combination of heavy and light chains: SEQ ID NOs. 9-10), 020 (combination of heavy and light chains: SEQ ID NOs. 11-12), 021 (combination of heavy and light chains: SEQ ID NOs. 13-14), 022 (combination of heavy and light chains: SEQ ID NOs. 15-16), 023 (heavy and light chain combination: SEQ ID NOs. 17-18), 008 (combination of heavy and light chains: SEQ ID NOs. 19-20), and biosimilars thereof.

10. The antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to any one of claims 1-9, selected from the following structures:
| No. | Structure |
|---|---|
| 1 | 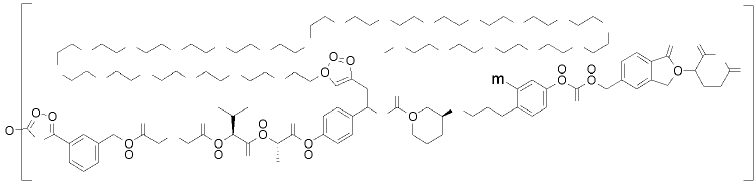 |
| 2 | 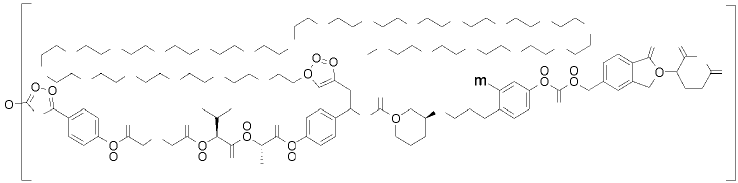 |
| 3 | 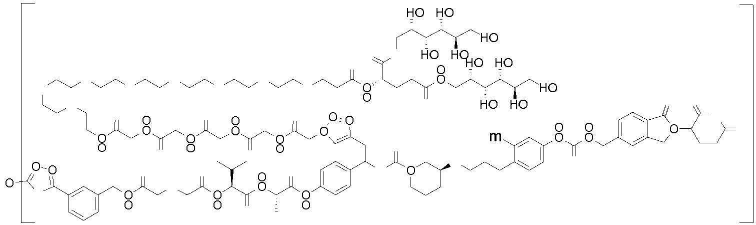 |
| 4 | 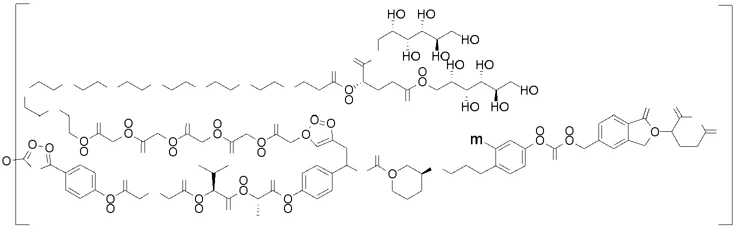 |
| 5 | 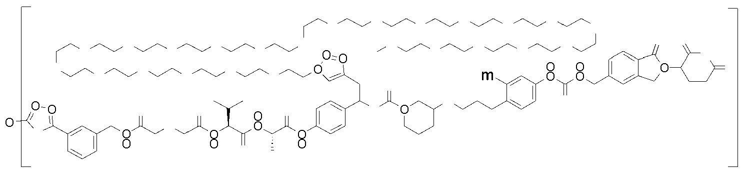 |
| 6 | 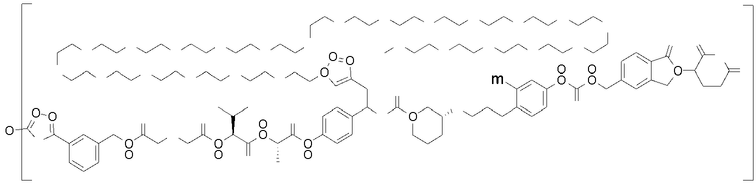 |
| 7 | 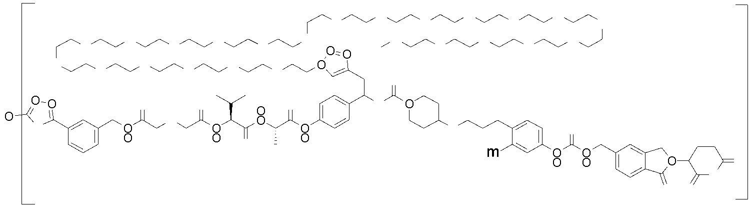 |
| 8 | 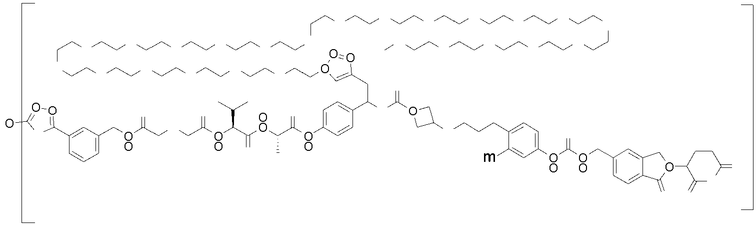 |
| 9 | 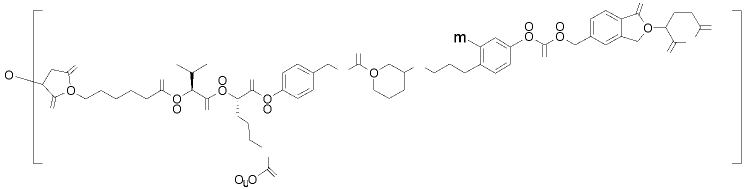 |
| 10 | 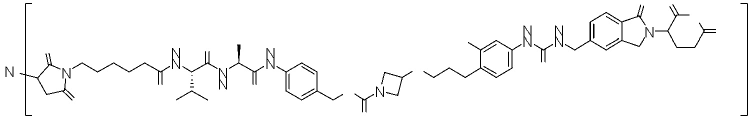 |
| 11 | 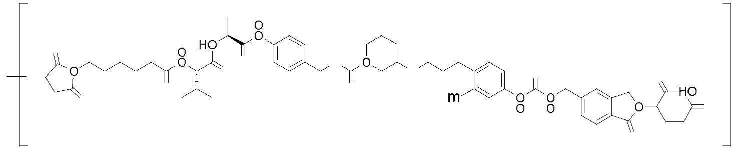 |
wherein preferably, the antibody A of structures 9-11 in the above table targets CD33 or CD123, and further preferably is 018, 019, 020, 021, 022, or 023.

11. A pharmaceutical composition, comprising the antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to any one of claims 1-10, and one or more pharmaceutically acceptable auxiliary materials.

12. Use of the antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to any one of claims 1-10, or the pharmaceutical composition according to claim 11 for the manufacturing of a medicament for the treatment of a cancer.

13. The use according to claim 12, wherein the cancer comprises liver cancer, kidney cancer, lung cancer (e.g., small cell lung cancer and non-small cell lung cancer), gastric cancer, esophageal cancer, urethral cancer, bladder cancer, colon cancer, rectal cancer, prostate cancer, breast cancer, ovarian cancer, pancreatic cancer, melanoma, hematological tumors or glioblastoma multiforme, lymphoma (e.g., Hodgkin lymphoma, non-Hodgkin lymphoma, or recurrent anaplastic large cell lymphoma), cervical cancer, uterine cancer, endometrial cancer, salivary gland cancer, glioma, neuroblastoma, sarcoma, colorectal cancer, leukemia (e.g., acute lymphoblastic leukemia, acute myeloid leukemia, acute promyelocytic leukemia, chronic myeloid leukemia, or chronic lymphocytic leukemia), bone cancer, skin cancer, thyroid cancer, etc.

14. A linker-drug compound represented by formula (II), or a tautomer, a mesomer, a racemate, an enantiomer or a diastereoisomer thereof, and a pharmaceutically acceptable salt, a hydrate, a solvate or an isotopically labeled compound thereof:
L'-D (II)
wherein L' is a linker moiety;
D is an amino-containing bioactive molecule or a pharmaceutically acceptable salt thereof, covalently linked to the linker moiety L' via an amine group in its molecular structure;
L' is represented by the following formula:
L₁'-L₂-L₃-L₄-,
wherein L₁' is selected from 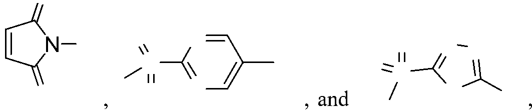 wherein ** represents linkage to L₂;
L₂, L₃, L₄, and D are as defined in claims 1-10.

15. A linker-drug compound represented by formula (II), or a tautomer, a mesomer, a racemate, an enantiomer or a diastereoisomer thereof, and a pharmaceutically acceptable salt, a hydrate, a solvate or an isotopically labeled compound thereof, having a structure shown as follows:
| No. | Structure |
|---|---|
| LD38 | 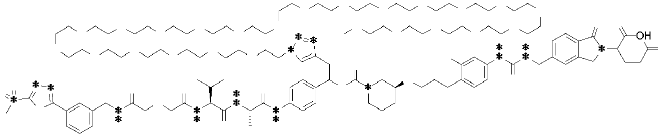 |
| LD39 | 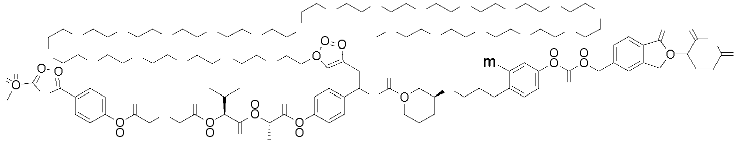 |
| LD40 | 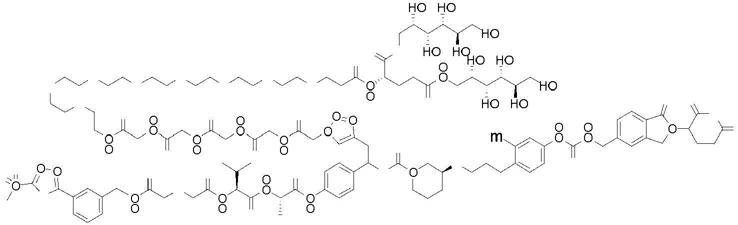 |
| LD41 | 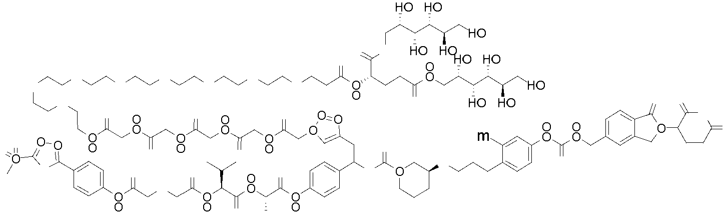 |
| LD42 | 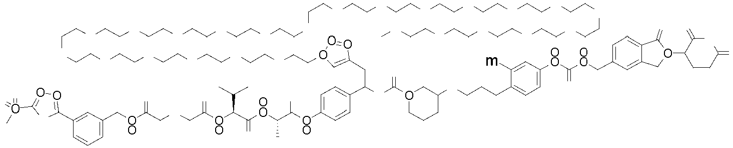 |
| LD43 | 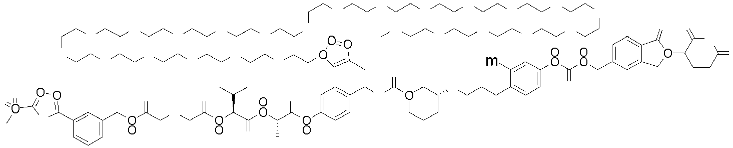 |
| LD44 | 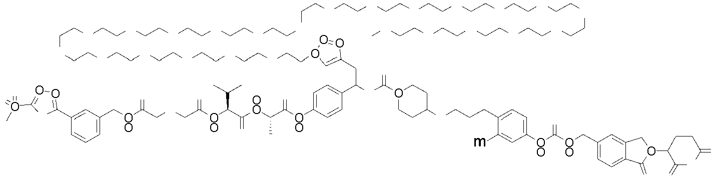 |
| LD45 | 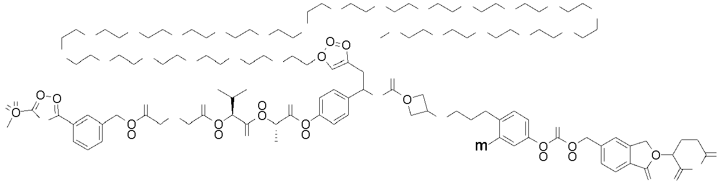 |
